(19) Europäisches Patentamt
European Patent Office
Office européen des brevets

(11) EP 3 831 849 A1

# (12) EUROPEAN PATENT APPLICATION

(43) Date of publication:
**09.06.2021 Bulletin 2021/23**

(51) Int Cl.:
***C07K 16/28*** *(2006.01)* ***C07K 16/30*** *(2006.01)*

(21) Application number: **19213002.9**

(22) Date of filing: **02.12.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(71) Applicant: **LamKap Bio beta AG**
**8808 Pfaeffikon Schwyz (CH)**

(72) Inventor: **The designation of the inventor has not yet been filed**

(74) Representative: **Schreiner, Siegfried**
**Finkenweg 1**
**82362 Weilheim (DE)**

## (54) BISPECIFIC ANTIBODIES AGAINST CEACAM5 AND CD47

(57) The present invention relates to bispecific antibodies which bind to human carcinoembryonic antigen CEACAM5 and human CD47. In addition, the present invention relates to polynucleotides encoding such bispecific antibodies and vectors and host cells comprising such polynucleotides. The invention further relates to methods for selecting and producing such antibodies and to methods of using such antibodies in the treatment of diseases. The invention also relates to the therapeutic use of the bispecific antibodies in monotherapy and in combination therapy.

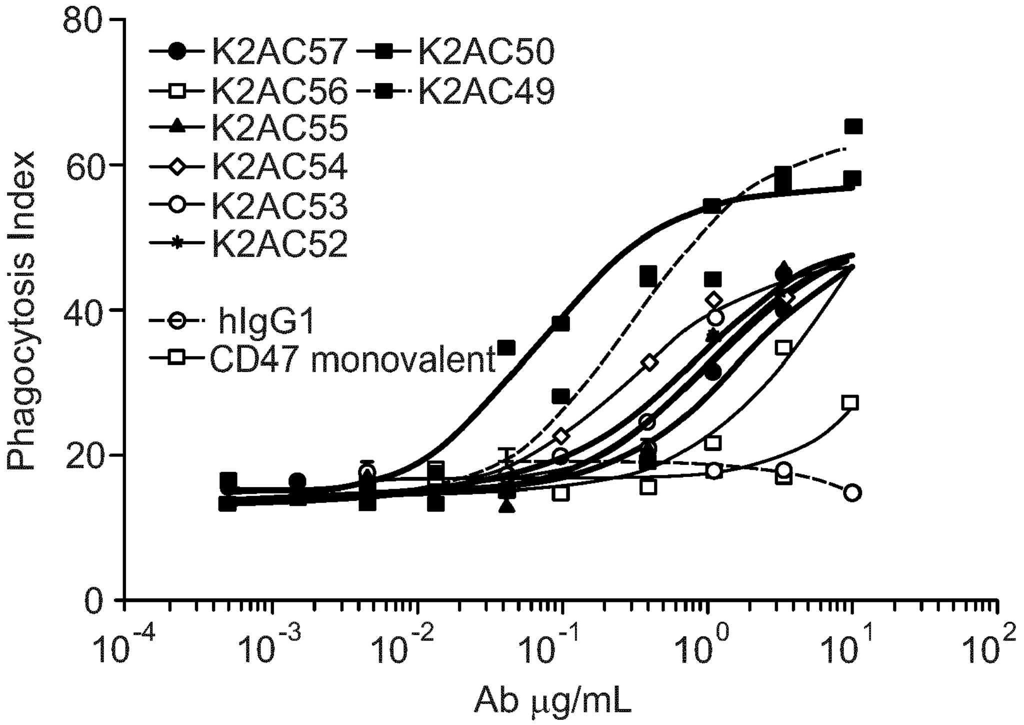


Fig. 3



Printed by Jouve, 75001 PARIS (FR)

## Description

### REFERENCE TO SEQUENCE LISTING

The content of the electronically submitted sequence listing filed with the application is incorporated herein by reference in its entirety.

### FIELD OF THE INVENTION

The present invention relates to bispecific antibodies which bind to human carcinoembryonic antigen CEACAM5 (CEA) and to human CD47 (CEAxCD47 bispecific antibodies) and in one embodiment to human CEACAM5, human CEACAM6 and to human CD47. In addition, the present invention relates to polynucleotides encoding such bispecific antibodies and vectors and host cells comprising such polynucleotides. The invention further relates to methods for selecting and producing such antibodies and to methods of using such antibodies in the treatment of diseases. The invention also relates to the therapeutic use of the CEAxCD47 bispecific antibodies in monotherapy and in combination therapy, especially with CEAxCD3 T-cell bispecific antibodies (TCB) and/or inhibitors of PD-1 or PD-L1.

### BACKGROUND OF THE INVENTION

The human CEA family contains 29 genes, of which 18 are expressed: 7 belonging to the CEA subgroup and 11 to the pregnancy-specific glycoprotein subgroup. Several CEA subgroup members are thought to possess cell adhesion properties. CEA is thought to have a role in innate immunity (Hammarström S., Semin Cancer Biol. 9(2):67-81 (1999)). Carcinoembryonic antigen (CEA, CEACAM5, Meconium antigen (MA) or CD66e; UniProtKB - P06731) is a member of the carcinoembryonic antigen-related cell adhesion molecule (CEACAM) family and a tumor-associated antigen (Gold and Freedman, J Exp. Med., 121:439-462, 1965; Berinstein N. L., J Clin Oncol., 20:2197-2207, 2002). CEACAM6 (CD66c; NCA-50/90; UniProtKB - P40199) belongs also to the carcinoembryonic antigen (CEA) family. Multiple monoclonal antibodies have been raised against CEA for research purposes, as diagnostic tools, and for therapeutic purposes (see e.g. WO2012117002 (incorporated by reference in its entirety), see also Example 8 f)). Soluble CEA - is an established tumor marker. Levels in plasma of cancer patients can go in some cases over 1000 ng/ml, whereas plasma concentrations in healthy individuals are below 10 ng/ml (e.g. Sandler B. et al Anticancer Res 1999, 19(5B), 4229-33). Hao C., Zhang G. and L. in Progress in Molecular Biology and Translational Science (2019) report that CEA plasma concentrations between 100 and 250 ng/mL can be found in a significant % of patients in Pancreatic Cancer, Colon- and Rectal Cancer, Lung Cancer and Gastric Cancer. Such high levels are especially observed when these cancers are locally advanced and/or metastatic. According to Wanebo et. al., New Eng. J. Med. (1978) 21% of recurrent/meatastatic colon cancer have sCEA above 100 ng/ml. Hohenberger et. al., Annals Surgery (1994) report in colorectal patients, stage Duke 4 and liver metastasis, that 26 % of patients have sCEA over 50 ng/mL. Jurgensmerier et al Br. J. Cancer (2013) report in rather large studies with several hundred of patients suffering from metastatic colorectal cancer sCEA above 225 ng/mL in 24% respectively 25% of these patients.

The mouse monoclonal antibody PR1A3 was raised by fusion of NS1 (P3/NS 1/I-Ag-4-1) myeloma cells with spleen cells from mice immunized with normal colorectal epithelium Richman P. I. and Bodmer W. F., Int. J. Cancer, 39:317-328, 1987 describe mouse monoclonal antibody PR1A3. Epitope mapping of PR1 A3 shows that the antibody targets the B3 domain and the GPI anchor of the CEA molecule (Durbin H. et al., Proc. Natl. Scad. Sci. USA, 91 :4313-4317, 1994). Consequently, the PR1A3 antibody binds mainly to the membrane- bound CEA, and not the soluble CEA form that can be found in the bloodstreams of cancer patients. The epitope bound by PR1 A3 is a conformational epitope, not a linear epitope (Stewart et al., Cancer Immunol Immunother, 47 (1999) 299-06). Humanized PR1 A3 (hPR1 A3) antibodies are described e.g. by Conaghhan P. J., et al., Br. J. Cancer, 98 (2008)1217-1225 and WO2012117002 (incorporated by reference in its entirety).

CEACAM5 is expressed by the cells of several tumor types including but not limited to e.g. colorectal tumors, tumors of the pancreas, lung tumors, gastric tumors etc.

These tumor cells express often also CEACAM6, expression of CEACAM6 can be well higher as expression of CEACAM5 (see e.g Blumenthal RD et.al. BMC Cancer, 2007 Jan 3;7:2).

A method for treating cancer by a combination of a human PD-1 axis antagonist and an anti-CEA/anti-CD3 bispecific antibody is mentioned in US20140242079 and WO2017118657 (each of which is incorporated by reference in its entirety) and clinical results have been published at ASCO conference 2017 (Tabernero et al, J Clin Oncol 35, 2017 (suppl;abstr 3002)). A method of treating tumors by administering immune checkpoint antagonists binding two or more different targets of an immune checkpoint pathway, and a T cell-redirecting agent binding to CEA and a T cell surface antigen is mentioned in WO2015112534. A conjugate consisting of a single domain anti-CEACAM6 antibody and urease is at present in clinical trials (NCT02309892; WO2016116907). A class I antibody binding to CEACAM5, CEACAM6 and granulocytes is mentioned in US20110064653. An anti CD3ε antibody described in the state of the art is SP34 (Yang SJ, The Journal of Immunology (1986) 137; 1097-1100). SP34 reacts with both primate and human CD3. SP34 is available from BD Biosciences. A further anti CD3 antibody described in the state of the art is UCHT-1 (see WO2000041474). A further anti CD3 antibody described in the state of the art is BC-3 (Fred Hutchinson Cancer Research Institute; used in Phase I/II trials of GvHD, Anasetti et al., Transplantation 54: 844 (1992)). SP34 differs from UCHT-1 and BC-3 in that SP-34 recognizes an epitope present on solely the ε chain of CD3 (see Salmeron et al., (1991) J. Immunol. 147: 3047) whereas UCHT-1 and BC-3 recognize an epitope contributed by both the ε and γ chains. Anti CD3 antibodies are also described in WO2007042261, WO2008119565, WO2008119566, WO2008119567, WO2010037836, WO2010037837, WO2010037838, and US8236308 (each of which is incorporated by reference in its entirety). A bispecific antibody comprising a binding part specific for CEA and a binding part specific for CD3ε is described in US20140242079A1 (incorporated by reference in its entirety).

Human CD47 (UniProtKB - Q08722 (CD47_HUMAN; IAP) is a transmembrane protein that binds the ligands thrombospondin-1 (TSP-1) and signal-regulatory protein alpha (SIRPα; CD172a; UniProtKB P78324) and can act as a "don't eat me" signal to the immune system, especially for macrophages. CD47 is involved in a range of cellular processes, including apoptosis, proliferation, adhesion, and migration. Furthermore, it plays a key role in immune and angiogenic responses. CD47 is overexpressed in different tumor cells. Antibodies against CD47 are described in the state of the art and some are in clinical trials as therapeutic agents for tumor treating (Weiskopf K. European Journal of Cancer 76 (2017) 100-109; Huang Y et al., J Thorac Dis 2017;9(2):E168-E174. Antibodies of the IgG1 subclass that bind CD47 can result in the depletion of platelets and reduction of red blood cells RBC of hemoglobin in a Fc-dependent manner (see e.g. US20140140989). For avoiding this adverse effect, in WO2017196793 there is described a mutant form of the IgG4 subclass of an anti-CD47 antibody (IgG4PE, with the S228P mutation as well as a L235E mutation to reduce FcyR binding). Such anti-CD47 antibody with severely reduced FcyR binding and effector function does not result in such platelet depletion. A single domain bispecific antibody against CD47 and CD20 was described by von Bommel PE et al., Oncoimmunol. 7 (2018) e386361 and Piccione EC et al. mAbs 7 (2015)946-956. Dheilly E. et al., Mol. Thera. 25 (2017) 523-533 (see also WO2014087248) describe a bispecific antibody against CD19 and CD47. A bispecific antibody against CD19 and CD47 comprising a common heavy chain of SEQ ID NO:5 and a variable light domain VL of SEQ ID NO:10 is described in WO2014087248 (incorporated by reference in its entirety).

Human FcRI (CD64) is restricted to monocytes/macrophages and dendritic cells (DCs) and, inducibly expressed on neutrophils and mast cells; hFc RIIA (CD32A) is expressed on all myeloid cells but not on lymphocytes; hFc RIIB (CD32B) is highly expressed only on circulating B cells and basophils (L. Cassard, F. Joensson, S. Arnaud, M. Daeron, J. Immunol.189 (2012(2995-3006), poorly expressed on 20% of the monocytes and 4% of the neutrophils, and expressed on tissue macrophages and DCs, but not on mast cells hFc RIIC (CD32C) is expressed on NK cells, monocytes, and neutrophils. hFc RIIIA (CD16A) is expressed on NK cells and monocytes/macrophages; hFcRIIIB CD16B) is expressed on neutrophils and, as recently demonstrated, on subsets of basophils. These expression patterns highlight that hFc RIIA is the only activating IgG receptor constitutively expressed by mast cells, basophils, neutrophils and eosinophils (Bruhns P., Blood 119 (2012) 5640). The biological activities of each subclass of IgG are poorly known. IgG receptors (FcγRs) are strikingly numerous in humans. They comprise high-affinity and low-affinity receptors. Both high-affinity and low-affinity FcγRs bind IgG-immune complexes with a high avidity, but only high-affinity FcγRs bind monomeric IgG. There is one high-affinity IgG receptor in humans, hFcγRI (CD64), and two families of low-affinity IgG receptors, hFcγ RIIA, IIB, and IIC (CD32), and hFcγRIIIA and IIIB (CD16). hFcγRI and hFcγRIIIA are FcγR associated activating receptors, hFcγRIIA and hFcγRIIC are single-domain activating receptors, hFcγRIIB are single-domain inhibitory receptors, and hFcγRIIIB are GPI-anchored receptors whose function is uncertain (Bruhns P. Blood 113 (2009) 3716). Several research groups have demonstrated that antibodies, lacking the 1,6- fucose on their heavy chain glycosylation, have enhanced binding affinity to the FcγRIII receptor and increased ADCC activity (Shields, R. L., et al., (2002) J Biol. Chem. 277, 26733-26740.; (2002) J Biol. Chem. 8, 8). In addition, a correlation between binding affinity to the FcγRIII receptor and ADCC activity has been established (Okazaki, A., et al., (2004) J Mol. Biol. 336, 1239-1249; Dall'Ozzo, 2004). An IgG molecule carries two N-linked oligosaccharides in its Fc region, one on each heavy chain. As any glycoprotein, an antibody is produced as a population of glycoforms which share the same polypeptide backbone but have different oligosaccharides attached to the glycosylation sites. The oligosaccharides normally found in the Fc region of serum IgG are of complex bi-antennary type (Wormald et al., Biochemistry 36: 1370-1380 (1997), with a low level of terminal sialic acid and bisecting N-acetylglucosamine (GlcNAc), and a variable degree of terminal galactosylation and core fucosylation. Some studies suggest that the minimal carbohydrate structure required for FcyR binding lies within the oligosaccharide core. Lund et al., J. Immunol. 157:4963-69 (1996). Antibodies with a reduced fucose content in glycan moieties exhibit higher antibody dependent cellular cytotoxicity (ADCC) activity compared to a normally fucosylated antibody (Niwa R et al., Cancer Res, 64, 2127-33, 2004). The mechanism behind the enhanced ADCC of a low / no-fucose antibody is its increased affinity to FcγRIIIa (CD 16). A cell line with knockout of both alleles for the gene responsible for fucose addition (α1,6-fucosyltransferase; FUT8) is described in US6946292, US7425446, US8067232 (each of which is incorporated by reference in its entirety), and under http://www.potelligent.com. Overexpression in Chinese hamster ovary (CHO) cells of β(1,4)-N-acetylglucosaminyltransferase III (GnTIII), a glycosyltransferase catalyzing the formation of bisected oligosaccharides, significantly increases the in vitro ADCC activity of antibodies produced by the engineered CHO cells. (Umaña, P. et al., Nature Biotechnol. 17:176-180 (1999), WO199954342, US20030175884(each of which is incorporated by reference in its entirety)).

Another technology which can be used to produce antibodies with reduced fucose content is described in US8642292 (incorporated herein by reference). This technology is designed to configure the stable integration of a heterologous bacterial enzyme into an antibody producer cell line like a CHO cell line or others. By this measure, the de novo synthesis of fucose from D-mannose is blocked. If in addition production cells are cultivated in fucose free medium, as a result antibodies with a stable level of afucosylation are produced.

.Mutations within the Fc domain can also alter binding properties of the Fc domain to the different Fc receptors (WO2004063351, WO2004099249; WO2005018669, WO2005063815, WO2005110474, WO2005056759, WO2005092925, WO2005018572, WO2006019447, WO2006116260, WO2006023420, WO2006047350, WO2006085967, WO2006105338, WO2007021841, WO2007008943, WO2007024249, WO2007041635, WO2007048077, WO2007044616, WO2007106707, WO2008022152, WO2008140603, WO2008036688, WO2008091798, WO2008091954, WO2008092117, WO2008098115, WO2008121160, WO2008150494, WO2010033736, WO2014113510 (each of which is incorporated by reference in its entirety)).

Considerable progress has been made in the treatment of hematological malignancies. That is in contrast to the progression made in the treatment of several types of advanced solid tumors. Progression free survival (PFS) and overall survival (OS) of those advanced tumor types, many of those rather frequent, was to some extent improved by new chemotherapy schemes with and w/o monoclonal antibodies against e.g. VEGFR or ERGFR as combination partner to chemotherapy. But in the past years for many of the advanced/metastatic solid tumors the progress of drug therapy was limited. Much hope has been put into cancer immunotherapy and there are certain, but limited, successes. Tumors develop measures to protect their cells from destruction by T-effector cells and other immune cells like macrophages. Cancer immunotherapy in the last decade(s) had certainly quite some focus and success on making T-cells fit again and to re-direct them against cancer cells. The most prominent examples are inhibitors/activators of certain immune checkpoints. E. g. checkpoint inhibitors like PD-1 axis antagonists have shown to re-activate T-effector cells to fight certain solid cancers. But not all solid tumor types are responsive and even in those responsive, it is often much less than 50% of patients having a relevant benefit from e.g. treatment with an anti-PD-1 or PD-L1 antibody.

Adoptive T-cell therapy with CAR T-cells and also therapy with T-cell bispecific antibodies delivered promising clinical results in hematological malignancies. But clinical studies with adoptive T-cell therapies, e.g. CAR T-cells, in various solid tumors mostly showed no or only minor response rates (e.g. Xu et. al. Expert Review of Anticancer Therapy 2017, 17, 1099-1106). US20140242079 and WO2017055389 (each of which is incorporated by reference in its entirety) describe CEAxCD3 T-cell bispecific antibodies. One antibody from US20140242079 and one from WO2017055389 are both in clinical development (see clinicaltrials.gov; RO6958688 in NCT3866239 and RO7172508 in NCT03539484). These T-cell bispecific antibodies bind to different epitopes of CEAxCD3 and have different tumor cell killing potency. Regarding tumor cell killing in an in vitro assay with human T-cells, most potent CEAxCD3 T-cell bispecific antibodies described in WO201705389 are by a factor of 10 to 100 or more potent than RO6958688/cibisatamab (CEA-TCB).

Until recently results of clinical trials with T-cell bispecific antibodies TAA x CD3 (TAA = Tumor Associated Antigen) in patients with advanced solid tumors were disappointing. But preliminary phase 1 results have been published at ASCO 2017 for the CEAxCD3 T-cell bispecific antibody CEA-TCB (RO6958688/cibisatamab, see for example Bacac et al Clin. Cancer Res., 22(13), 3286-97 (2016); and US20140242079) showing in advanced colorectal cancer patients in monotherapy partial responses and stable disease (J.Tabernero et.al., J. Clin. Oncol. 35, 2017 (suppl. Abstr. 3002)). At clinically active doses plasma concentrations of e.g. 300 nM have been reached for cibisatamab. More partial responses and stable disease occurred when CEA-TCB was combined with a PD-L1 inhibiting antibody. These data show that efficacy can be achieved with CEA-TCB in advanced solid tumors. But in monotherapy and also in the combination with a PD-L1 inhibitor, most of the patients were still progressing and those reacting showed at best partial responses and stable disease, but no complete responses have been achieved. One approach to get better results could be to add to T-cell bispecific antibodies not only an inhibitor of PD-1 checkpoint axis, but to add further checkpoint inhibitors or agonists. But so far, to the best of our knowledge, there are no promising clinical data for such a combination approach available. Limited availability of T-cells within advanced solid tumors is certainly an important mechanism limiting the efficacy achievable with T-cell bispecific antibodies plus PD-1 axis inhibitors and/or other checkpoint inhibitors or agonists for T-cells. Instead of adding to the combination of a T-cell bispecific antibody and a PD-1 axis inhibitor another therapeutic agent aiming to re-direct T-cells against tumor cells of advanced solid tumors, it may be more successful to add a therapeutic agent re-directing to the tumor cells other immune cells, especially macrophages or macrophages and natural killer NK-cells. This invention deals with bispecific antibodies re-directing macrophages and also NK-cells against CEACAM5 or CEACAM5 and CEACAM6 expressing solid tumors as a monotherapy or in combination with e.g. T-cell bispecific antibodies and/or PD-1/PD-L1 inhibiting antibodies.

The disappointing results with CAR T-cells in solid tumors may have a simple explanation - the number of CAR T-cells penetrating the solid tumor and distributed in it are just not sufficient. This is certainly different in the majority of haematological malignancies; CAR T-cells can well access the tumor cells, explaining the difference of high efficacy in these malignancies compared to disappointing efficacy in solid tumors. In addition, CAR T-cells may be heavily suppressed by the tumor microenvironment (TME) which is mostly strongly immune suppressive.

Monoclonal antibodies and also bispecific antibodies used in therapy can cause a variety of adverse effects. An important toxicity issue is the cytokine-release syndrome (CRS), which was for example found in therapy with alemtuzumab, muromonab-CD3, rituximab, and CD19 x CD3 bispecific antibody blinatumomab. It was also found that treatment with anti-CD47 antibodies induce increased amounts of pro-inflammatory cytokines after anti-CD47 antibody mediated phagocytosis (see e.g. US20160144009). Known adverse events of anti-CD47 monoclonal antibodies with wt IgG1 Fc are increased red blood cell RBC phagocytosis/lysis and platelet activation.

The present invention provides bispecific antibodies with a first binding part either binding to human CEACAM5 and not to CEACAM6 or binding to CEACAM 5 and 6 in a balanced manner and a second binding part binding to human CD47. In one embodiment the bispecific antibodies according to the invention do not bind to CEACAM1 to avoid phagocytosis/killing of immune cells because CEACAM1 is widely expressed on immune cells. In one embodiment the bispecific antibodies are designated to the treatment of solid tumors. In one embodiment the bispecific antibodies combine high efficacy with low toxicity, low immunogenicity and favourable pharmacokinetic properties. In one embodiment the bispecific antibodies according to this invention induce their anti-tumor cells effects mainly via optimized phagocytosis/ADCP (antibody dependent cellular phagocytosis) and ADCC (antibody dependent cellular cytotoxicity) due to involvement of immune cells, especially macrophages and NK-cells. In one embodiment the present invention also provides bispecific antibodies specifically binding to human CEACAM5 or human CEACAM5 and 6 and human CD47 designated for the combination treatment with CEAxCD3 T-cell bispecific antibodies like RO6958688, RO7172508 and other CEAxCD3 T-cell bispecific antibodies e.g. as described below and showing strong phagocytosis of tumor cells like MKN-45 in the presence of human macrophages. The present invention provides also monoclonal antibodies specifically binding to human CEACAM5 or human CEACAM5 and CEACAM6 and bispecific antibodies comprising such monoclonal antibodies and their uses, especially for use in the treatment of solid tumors.

Figure 1 provides an overview on the general structure of the bispecific antibodies of this invention.

## SUMMARY OF THE INVENTION

In one embodiment, the invention relates to a monoclonal antibody (further named also as "AC CEA") specifically binding to human CEACAM5 (further named also as "CEA) and cynomolgus CEACAM5, characterized in comprising

a) as heavy chain variable region a heavy chain variable region comprising a CDRH1 of SEQ ID NO:1, a CDRH2 of SEQ ID NO:2 and a CDRH3 of SEQ ID NO:3, and

b) as light chain variable region a light chain variable region comprising a CDRL set selected from the group consisting of

b1) a CDRL1 of SEQ ID NO:17, CDRL2 of SEQ ID NO:18, and CDRL3 of SEQ ID NO:19,
b2) a CDRL1 of SEQ ID NO:20, CDRL2 of SEQ ID NO:21, and CDRL3 of SEQ ID NO:22,
b3) a CDRL1 of SEQ ID NO:23, CDRL2 of SEQ ID NO:24, and CDRL3 of SEQ ID NO:25,
b4) a CDRL1 of SEQ ID NO:26, CDRL2 of SEQ ID NO:27, and CDRL3 of SEQ ID NO:28,
b5) a CDRL1 of SEQ ID NO:29, CDRL2 of SEQ ID NO:30, and CDRL3 of SEQ ID NO:31,
b6) a CDRL1 of SEQ ID NO:32, CDRL2 of SEQ ID NO:33, and CDRL3 of SEQ ID NO:34,
b7) a CDRL1 of SEQ ID NO:35, CDRL2 of SEQ ID NO:36, and CDRL3 of SEQ ID NO:37,
b8) a CDRL1 of SEQ ID NO:38, CDRL2 of SEQ ID NO:39, and CDRL3 of SEQ ID NO:40,
b9) a CDRL1 of SEQ ID NO:41, CDRL2 of SEQ ID NO:42, and CDRL3 of SEQ ID NO:43,
b10) a CDRL1 of SEQ ID NO:44, CDRL2 of SEQ ID NO:45, and CDRL3 of SEQ ID NO:46,
b11) a CDRL1 of SEQ ID NO:47, CDRL2 of SEQ ID NO:48, and CDRL3 of SEQ ID NO:49,
b12) a CDRL1 of SEQ ID NO:50, CDRL2 of SEQ ID NO:51, and CDRL3 of SEQ ID NO:52,
b13) a CDRL1 of SEQ ID NO:53, CDRL2 of SEQ ID NO:54, and CDRL3 of SEQ ID NO:55,
b14) a CDRL1 of SEQ ID NO:56, CDRL2 of SEQ ID NO:57, and CDRL3 of SEQ ID NO:58,
b15) a CDRL1 of SEQ ID NO:59, CDRL2 of SEQ ID NO:60, and CDRL3 of SEQ ID NO:61,
b16) a CDRL1 of SEQ ID NO:62, CDRL2 of SEQ ID NO:63, and CDRL3 of SEQ ID NO:64,
b17) a CDRL1 of SEQ ID NO:71, CDRL2 of SEQ ID NO:72, and CDRL3 of SEQ ID NO:73,
b18) a CDRL1 of SEQ ID NO:142, CDRL2 of SEQ ID NO:143, and CDRL3 of SEQ ID NO:144,
b19) a CDRL1 of SEQ ID NO:145, CDRL2 of SEQ ID NO:146, and CDRL3 of SEQ ID NO:147,
b20) a CDRL1 of SEQ ID NO:148, CDRL2 of SEQ ID NO:149, and CDRL3 of SEQ ID NO:150,
b21) a CDRL1 of SEQ ID NO:151, CDRL2 of SEQ ID NO:152, and CDRL3 of SEQ ID NO:153,

b22) a CDRL1 of SEQ ID NO:154, CDRL2 of SEQ ID NO:155, and CDRL3 of SEQ ID NO:156, and
b23) a CDRL1 of SEQ ID NO:157, CDRL2 of SEQ ID NO:158, and CDRL3 of SEQ ID NO:159.

In one embodiment, the invention relates to an AC CEA according to the invention, characterized in comprising

a) a heavy chain variable region VH having at least 90%, 91%, 92%, 93%, 94%, 95%, 96%, 97%, 98%, 99%, or 100% amino acid identity to SEQ ID NO:4, comprising a CDRH1 of SEQ ID NO:1, a CDRH2 of SEQ ID NO:2, and a CDRH3 of SEQ ID NO:3, and
b) as light chain a light chain selected from the group consisting of

b1) a light chain having at least 90%, 91%, 92%, 93%, 94%, 95%, 96%, 97%, 98%, 99%, or 100% amino acid identity to SEQ ID NO:74, and comprising
a CDRL1 of SEQ ID NO:17, CDRL2 of SEQ ID NO:18, and CDRL3 of SEQ ID NO:19,
b2) a light chain having at least 90%, 91%, 92%, 93%, 94%, 95%, 96%, 97%, 98%, 99%, or 100% amino acid identity to SEQ ID NO:75, and comprising a CDRL1 of SEQ ID NO:20, CDRL2 of SEQ ID NO:21, and CDRL3 of SEQ ID NO:22,
b3) a light chain having at least 90%, 91%, 92%, 93%, 94%, 95%, 96%, 97%, 98%, 99%, or 100% amino acid identity to SEQ ID NO:76, and comprising a CDRL1 of SEQ ID NO:23, CDRL2 of SEQ ID NO:24, and CDRL3 of SEQ ID NO:25,
b4) a light chain having at least 90%, 91%, 92%, 93%, 94%, 95%, 96%, 97%, 98%, 99%, or 100% amino acid identity to SEQ ID NO:77, and comprising
a CDRL1 of SEQ ID NO:17, CDRL2 of SEQ ID NO:18, and CDRL3 of SEQ ID NO:19,
b5) a light chain having at least 90%, 91%, 92%, 93%, 94%, 95%, 96%, 97%, 98%, 99%, or 100% amino acid identity to SEQ ID NO:78, and comprising a CDRL1 of SEQ ID NO:20, CDRL2 of SEQ ID NO:21, and CDRL3 of SEQ ID NO:22,
b6) a light chain having at least 90%, 91%, 92%, 93%, 94%, 95%, 96%, 97%, 98%, 99%, or 100% amino acid identity to SEQ ID NO:79, and comprising a CDRL1 of SEQ ID NO:23, CDRL2 of SEQ ID NO:24, and CDRL3 of SEQ ID NO:25,
b7) a light chain having at least 90%, 91%, 92%, 93%, 94%, 95%, 96%, 97%, 98%, 99%, or 100% amino acid identity to SEQ ID NO:80, and comprising a CDRL1 of SEQ ID NO:26, CDRL2 of SEQ ID NO:27, and CDRL3 of SEQ ID NO:28,
b8) a light chain having at least 90%, 91%, 92%, 93%, 94%, 95%, 96%, 97%, 98%, 99%, or 100% amino acid identity to SEQ ID NO:81, and comprising a CDRL1 of SEQ ID NO:29, CDRL2 of SEQ ID NO:30, and CDRL3 of SEQ ID NO:31,
b9) a light chain having at least 90%, 91%, 92%, 93%, 94%, 95%, 96%, 97%, 98%, 99%, or 100% amino acid identity to SEQ ID NO:82, and comprising a CDRL1 of SEQ ID NO:32, CDRL2 of SEQ ID NO:33, and CDRL3 of SEQ ID NO:34,
b10) a light chain having at least 90%, 91%, 92%, 93%, 94%, 95%, 96%, 97%, 98%, 99%, or 100% amino acid identity to SEQ ID NO:83, and comprising a CDRL1 of SEQ ID NO:35, CDRL2 of SEQ ID NO:36, and CDRL3 of SEQ ID NO:37,
b11) a light chain having at least 90%, 91%, 92%, 93%, 94%, 95%, 96%, 97%, 98%, 99%, or 100% amino acid identity to SEQ ID NO:84, and comprising a CDRL1 of SEQ ID NO:38, CDRL2 of SEQ ID NO:39, and CDRL3 of SEQ ID NO:40,
b12) a light chain having at least 90%, 91%, 92%, 93%, 94%, 95%, 96%, 97%, 98%, 99%, or 100% amino acid identity to SEQ ID NO:85, and comprising a CDRL1 of SEQ ID NO:41, CDRL2 of SEQ ID NO:42, and CDRL3 of SEQ ID NO:43,
b13) a light chain having at least 90%, 91%, 92%, 93%, 94%, 95%, 96%, 97%, 98%, 99%, or 100% amino acid identity to SEQ ID NO:86, and comprising a CDRL1 of SEQ ID NO:44, CDRL2 of SEQ ID NO:45, and CDRL3 of SEQ ID NO:46,
b14) a light chain having at least 90%, 91%, 92%, 93%, 94%, 95%, 96%, 97%, 98%, 99%, or 100% amino acid identity to SEQ ID NO:87, and comprising a CDRL1 of SEQ ID NO:47, CDRL2 of SEQ ID NO:48, and CDRL3 of SEQ ID NO:49,
b15) a light chain having at least 90%, 91%, 92%, 93%, 94%, 95%, 96%, 97%, 98%, 99%, or 100% amino acid identity to SEQ ID NO:88, and comprising a CDRL1 of SEQ ID NO:50, CDRL2 of SEQ ID NO:51, and CDRL3 of SEQ ID NO:52,
b16) a light chain having at least 90%, 91%, 92%, 93%, 94%, 95%, 96%, 97%, 98%, 99%, or 100% amino acid identity to SEQ ID NO:89, and comprising a CDRL1 of SEQ ID NO:53, CDRL2 of SEQ ID NO:54, and CDRL3 of SEQ ID NO:55,

b17) a light chain having at least 90%, 91%, 92%, 93%, 94%, 95%, 96%, 97%, 98%, 99%, or 100% amino acid identity to SEQ ID NO:90, and comprising a CDRL1 of SEQ ID NO:56, CDRL2 of SEQ ID NO:57, and CDRL3 of SEQ ID NO:58,
b18) a light chain having at least 90%, 91%, 92%, 93%, 94%, 95%, 96%, 97%, 98%, 99%, or 100% amino acid identity to SEQ ID NO:91, and comprising a CDRL1 of SEQ ID NO:59, CDRL2 of SEQ ID NO:60, and CDRL3 of SEQ ID NO:61,
b19) a light chain having at least 90%, 91%, 92%, 93%, 94%, 95%, 96%, 97%, 98%, 99%, or 100% amino acid identity to SEQ ID NO:92, and comprising a CDRL1 of SEQ ID NO:62, CDRL2 of SEQ ID NO:63, and CDRL3 of SEQ ID NO:64,
b20) a light chain having at least 90%, 91%, 92%, 93%, 94%, 95%, 96%, 97%, 98%, 99%, or 100% amino acid identity to SEQ ID NO:167, and comprising a CDRL1 of SEQ ID NO:71, CDRL2 of SEQ ID NO:72, and CDRL3 of SEQ ID NO:73,
b21) a light chain having at least 90%, 91%, 92%, 93%, 94%, 95%, 96%, 97%, 98%, 99%, or 100% amino acid identity to SEQ ID NO:168, and comprising a CDRL1 of SEQ ID NO:142, CDRL2 of SEQ ID NO:143, and CDRL3 of SEQ ID NO:144
b22) a light chain having at least 90%, 91%, 92%, 93%, 94%, 95%, 96%, 97%, 98%, 99%, or 100% amino acid identity to SEQ ID NO:169, and comprising a CDRL1 of SEQ ID NO:145, CDRL2 of SEQ ID NO:146, and CDRL3 of SEQ ID NO:147,
b23) a light chain having at least 90%, 91%, 92%, 93%, 94%, 95%, 96%, 97%, 98%, 99%, or 100% amino acid identity to SEQ ID NO:170, and comprising a CDRL1 of SEQ ID NO:148, CDRL2 of SEQ ID NO:149, and CDRL3 of SEQ ID NO:150,
b24) a light chain having at least 90%, 91%, 92%, 93%, 94%, 95%, 96%, 97%, 98%, 99%, or 100% amino acid identity to SEQ ID NO:171, and comprising a CDRL1 of SEQ ID NO:151, CDRL2 of SEQ ID NO:152, and CDRL3 of SEQ ID NO:153,
b25) a light chain having at least 90%, 91%, 92%, 93%, 94%, 95%, 96%, 97%, 98%, 99%, or 100% amino acid identity to SEQ ID NO:172, and comprising a CDRL1 of SEQ ID NO:154, CDRL2 of SEQ ID NO:155, and CDRL3 of SEQ ID NO:156, and
b26) a light chain having at least 90%, 91%, 92%, 93%, 94%, 95%, 96%, 97%, 98%, 99%, or 100% amino acid identity to SEQ ID NO:173, and comprising a CDRL1 of SEQ ID NO:157, CDRL2 of SEQ ID NO:158, and CDRL3 of SEQ ID NO:159.

In one embodiment, the invention relates to AC CEA according to the invention, characterized in comprising as heavy chain variable region a heavy chain variable region having at least 90%, 91%, 92%, 93%, 94%, 95%, 96%, 97%, 98%, 99%, or 100% amino acid sequence identity to the heavy chain region of SEQ ID NO:5 and as light chain a light chain having at least 90%, 91%, 92%, 93%, 94%, 95%, 96%, 97%, 98%, 99%, or 100% amino acid sequence identity to a light chain selected from the group of

a) the light chain of SEQ ID NO:74,
b) the light chain of SEQ ID NO:75,
c) the light chain of SEQ ID NO:76,
d) the light chain of SEQ ID NO:77,
e) the light chain of SEQ ID NO:78,
f) the light chain of SEQ ID NO:79,
g) the light chain of SEQ ID NO:80,
h) the light chain of SEQ ID NO:81,
i) the light chain of SEQ ID NO:82,
k) the light chain of SEQ ID NO:83,
l) the light chain of SEQ ID NO:84,
m) the light chain of SEQ ID NO:85,
n) the light chain of SEQ ID NO:86,
o) the light chain of SEQ ID NO:87,
p) the light chain of SEQ ID NO:88,
q) the light chain of SEQ ID NO:89,
r) the light chain of SEQ ID NO:90,
s) the light chain of SEQ ID NO:91,
t) the light chain of SEQ ID NO:92,
u) the light chain of SEQ ID NO:167,
v) the light chain of SEQ ID NO:168,

w) the light chain of SEQ ID NO:169,
x) the light chain of SEQ ID NO:170,
y) the light chain of SEQ ID NO:171,
z) the light chain of SEQ ID NO:172,
aa) the light chain of SEQ ID NO:173,
ab) the light chain of SEQ ID NO:174,
ac) the light chain of SEQ ID NO:175,
ad) the light chain of SEQ ID NO:176,
ae) the light chain of SEQ ID NO:177,
af) the light chain of SEQ ID NO:178,
ag) the light chain of SEQ ID NO:179, and
ah) the light chain of SEQ ID NO:180.

In one embodiment, the invention relates to AC CEA according to the invention, characterized in comprising as heavy chain region a heavy chain region of SEQ ID NO:5 and as light chain a light chain selected from the group of

a) the light chain of SEQ ID NO:74,
b) the light chain of SEQ ID NO:75,
c) the light chain of SEQ ID NO:76,
d) the light chain of SEQ ID NO:77,
e) the light chain of SEQ ID NO:78,
f) the light chain of SEQ ID NO:79
g) the light chain of SEQ ID NO:80,
h) the light chain of SEQ ID NO:81,
i) the light chain of SEQ ID NO:82,
k) the light chain of SEQ ID NO:83,
l) the light chain of SEQ ID NO:84,
m) the light chain of SEQ ID NO:85,
n) the light chain of SEQ ID NO:86,
o) the light chain of SEQ ID NO:87,
p) the light chain of SEQ ID NO:88,
q) the light chain of SEQ ID NO:89,
r) the light chain of SEQ ID NO:90,
s) the light chain of SEQ ID NO:91,
t) the light chain of SEQ ID NO:92,
u) the light chain of SEQ ID NO:167,
v) the light chain of SEQ ID NO:168,
w) the light chain of SEQ ID NO:169,
x) the light chain of SEQ ID NO:170,
y) the light chain of SEQ ID NO:171,
z) the light chain of SEQ ID NO:172,
aa) the light chain of SEQ ID NO:173,
ab) the light chain of SEQ ID NO:174,
ac) the light chain of SEQ ID NO:175,
ad) the light chain of SEQ ID NO:176,
ae) the light chain of SEQ ID NO:177,
af) the light chain of SEQ ID NO:178,
ag) the light chain of SEQ ID NO:179, and
ah) the light chain of SEQ ID NO:180.

In one embodiment the AC CEA is a Fab or a $F(ab)_2$ fragment. In one embodiment such Fab fragment is the first binding part of a bispecific antibody. In one embodiment the AC CEA is specifically binding to human CEACAM5, cynomolgus CEACAM5 and human CEACAM6.

In one embodiment, the invention relates to a AC CEA antibody according to the invention, characterized in that said antibody competes for binding to CEACAM5 with the anti-CEACAM5 antibody SM3E, said antibody SM3E comprises as VK and VH domains VK and VH of sequences SEQ ID NO:110 and 111.

In one embodiment, the invention relates to a AC CEA antibody according to the invention, characterized in that said antibody is a bispecific antibody. In one embodiment, the invention relates to an AC CEA antibody according to the invention, characterized in that said antibody is a bispecific antibody comprising as first binding part said AC CEA as monovalent light and heavy chain (VHCH1) and binding in the second binding part specifically to human CD47.

In one embodiment, the invention relates to a bispecific antibody (further named also as "", "CEAxCD47 bispecific antibody", or "bispecific antibody according to the invention") comprising a first binding part specifically binding to human CEACAM5 (further named also as "CEA") and a second binding part specifically binding to human CD47 (further named also as "CD47") characterized in that:

a) the first binding part comprises as heavy chain variable region a heavy chain variable region comprising a CDRH1 of SEQ ID NO:1, a CDRH2 of SEQ ID NO:2 and a CDRH3 of SEQ ID NO:3,
b) the first binding part comprises as light chain variable region a light chain variable region comprising a CDRL set selected from the group consisting of

b1) a CDRL1 of SEQ ID NO:17, CDRL2 of SEQ ID NO:18, and CDRL3 of SEQ ID NO:19,
b2) a CDRL1 of SEQ ID NO:20, CDRL2 of SEQ ID NO:21, and CDRL3 of SEQ ID NO:22,
b3) a CDRL1 of SEQ ID NO:23, CDRL2 of SEQ ID NO:24, and CDRL3 of SEQ ID NO:25,
b4) a CDRL1 of SEQ ID NO:26, CDRL2 of SEQ ID NO:27, and CDRL3 of SEQ ID NO:28,
b5) a CDRL1 of SEQ ID NO:29, CDRL2 of SEQ ID NO:30, and CDRL3 of SEQ ID NO:31,
b6) a CDRL1 of SEQ ID NO:32, CDRL2 of SEQ ID NO:33, and CDRL3 of SEQ ID NO:34,
b7) a CDRL1 of SEQ ID NO:35, CDRL2 of SEQ ID NO:36, and CDRL3 of SEQ ID NO:37,
b8) a CDRL1 of SEQ ID NO:38, CDRL2 of SEQ ID NO:39, and CDRL3 of SEQ ID NO:40,
b9) a CDRL1 of SEQ ID NO:41, CDRL2 of SEQ ID NO:42, and CDRL3 of SEQ ID NO:43,
b10) a CDRL1 of SEQ ID NO:44, CDRL2 of SEQ ID NO:45, and CDRL3 of SEQ ID NO:46,
b11) a CDRL1 of SEQ ID NO:47, CDRL2 of SEQ ID NO:48, and CDRL3 of SEQ ID NO:49,
b12) a CDRL1 of SEQ ID NO:50, CDRL2 of SEQ ID NO:51, and CDRL3 of SEQ ID NO:52,
b13) a CDRL1 of SEQ ID NO:53, CDRL2 of SEQ ID NO:54, and CDRL3 of SEQ ID NO:55,
b14) a CDRL1 of SEQ ID NO:56, CDRL2 of SEQ ID NO:57, and CDRL3 of SEQ ID NO:58,
b15) a CDRL1 of SEQ ID NO:59, CDRL2 of SEQ ID NO:60, and CDRL3 of SEQ ID NO:61,
b16) a CDRL1 of SEQ ID NO:62, CDRL2 of SEQ ID NO:63, and CDRL3 of SEQ ID NO:64,
b17) a CDRL1 of SEQ ID NO:71, CDRL2 of SEQ ID NO:72, and CDRL3 of SEQ ID NO:73,
b18) a CDRL1 of SEQ ID NO:142, CDRL2 of SEQ ID NO:143, and CDRL3 of SEQ ID NO:144,
b19) a CDRL1 of SEQ ID NO:145, CDRL2 of SEQ ID NO:146, and CDRL3 of SEQ ID NO:147,
b20) a CDRL1 of SEQ ID NO:148, CDRL2 of SEQ ID NO:149, and CDRL3 of SEQ ID NO:150,
b21) a CDRL1 of SEQ ID NO:151, CDRL2 of SEQ ID NO:152, and CDRL3 of SEQ ID NO:153,
b22) a CDRL1 of SEQ ID NO:154, CDRL2 of SEQ ID NO:155, and CDRL3 of SEQ ID NO:156, and
b23) a CDRL1 of SEQ ID NO:157, CDRL2 of SEQ ID NO:158, and CDRL3 of SEQ ID NO:159, and

c) the second binding part comprises as heavy chain variable region a heavy chain variable region comprising a CDRH1 of SEQ ID NO:1, a CDRH2 of SEQ ID NO:2 and a CDRH3 of SEQ ID NO:3, and as light chain variable region a light chain variable region comprising a CDRL1 of SEQ ID NO:7, a CDRL2 of SEQ ID NO:8, and a CDRL3 of SEQ ID NO:9.

In one embodiment, the invention relates to a bispecific antibody according to the invention, characterized in comprising

a) in the first binding part a heavy chain region having at least 90%, 91%, 92%, 93%, 94%, 95%, 96%, 97%, 98%, 99%, or 100% amino acid identity to SEQ ID NO:5, comprising a CDRH1 of SEQ ID NO:1, a CDRH2 of SEQ ID NO:2, and a CDRH3 of SEQ ID NO:3, and
b) as light chain a light chain selected from the group consisting of

b1) a light chain having at least 90%, 91%, 92%, 93%, 94%, 95%, 96%, 97%, 98%, 99%, or 100% amino acid identity to SEQ ID NO:77, and comprising
a CDRL1 of SEQ ID NO:17, CDRL2 of SEQ ID NO:18, and CDRL3 of SEQ ID NO:19,
b2) a light chain having at least 90%, 91%, 92%, 93%, 94%, 95%, 96%, 97%, 98%, 99%, or 100% amino acid identity to SEQ ID NO:78, and comprising a CDRL1 of SEQ ID NO:20, CDRL2 of SEQ ID NO:21, and CDRL3 of SEQ ID NO:22,
b3) a light chain having at least 90%, 91%, 92%, 93%, 94%, 95%, 96%, 97%, 98%, 99%, or 100% amino acid identity to SEQ ID NO:79, and comprising a CDRL1 of SEQ ID NO:23, CDRL2 of SEQ ID NO:24, and CDRL3 of SEQ ID NO:25,

b4) a light chain having at least 90%, 91%, 92%, 93%, 94%, 95%, 96%, 97%, 98%, 99%, or 100% amino acid identity to SEQ ID NO:80, and comprising a CDRL1 of SEQ ID NO:26, CDRL2 of SEQ ID NO:27, and CDRL3 of SEQ ID NO:28,
b5) a light chain having at least 90%, 91%, 92%, 93%, 94%, 95%, 96%, 97%, 98%, 99%, or 100% amino acid identity to SEQ ID NO:81, and comprising a CDRL1 of SEQ ID NO:29, CDRL2 of SEQ ID NO:30, and CDRL3 of SEQ ID NO:31,
b6) a light chain having at least 90%, 91%, 92%, 93%, 94%, 95%, 96%, 97%, 98%, 99%, or 100% amino acid identity to SEQ ID NO:82, and comprising a CDRL1 of SEQ ID NO:32, CDRL2 of SEQ ID NO:33, and CDRL3 of SEQ ID NO:34,
b7) a light chain having at least 90%, 91%, 92%, 93%, 94%, 95%, 96%, 97%, 98%, 99%, or 100% amino acid identity to SEQ ID NO:83, and comprising a CDRL1 of SEQ ID NO:35, CDRL2 of SEQ ID NO:36, and CDRL3 of SEQ ID NO:37,
b8) a light chain having at least 90%, 91%, 92%, 93%, 94%, 95%, 96%, 97%, 98%, 99%, or 100% amino acid identity to SEQ ID NO:84, and comprising a CDRL1 of SEQ ID NO:38, CDRL2 of SEQ ID NO:39, and CDRL3 of SEQ ID NO:40,
b9) a light chain having at least 90%, 91%, 92%, 93%, 94%, 95%, 96%, 97%, 98%, 99%, or 100% amino acid identity to SEQ ID NO:85, and comprising a CDRL1 of SEQ ID NO:41, CDRL2 of SEQ ID NO:42, and CDRL3 of SEQ ID NO:43,
b10) a light chain having at least 90%, 91%, 92%, 93%, 94%, 95%, 96%, 97%, 98%, 99%, or 100% amino acid identity to SEQ ID NO:86, and comprising a CDRL1 of SEQ ID NO:44, CDRL2 of SEQ ID NO:45, and CDRL3 of SEQ ID NO:46,
b11) a light chain having at least 90%, 91%, 92%, 93%, 94%, 95%, 96%, 97%, 98%, 99%, or 100% amino acid identity to SEQ ID NO:87, and comprising a CDRL1 of SEQ ID NO:47, CDRL2 of SEQ ID NO:48, and CDRL3 of SEQ ID NO:49,
b12) a light chain having at least 90%, 91%, 92%, 93%, 94%, 95%, 96%, 97%, 98%, 99%, or 100% amino acid identity to SEQ ID NO:88, and comprising a CDRL1 of SEQ ID NO:50, CDRL2 of SEQ ID NO:51, and CDRL3 of SEQ ID NO:52,
b13) a light chain having at least 90%, 91%, 92%, 93%, 94%, 95%, 96%, 97%, 98%, 99%, or 100% amino acid identity to SEQ ID NO:89, and comprising a CDRL1 of SEQ ID NO:53, CDRL2 of SEQ ID NO:54, and CDRL3 of SEQ ID NO:55,
b14) a light chain having at least 90%, 91%, 92%, 93%, 94%, 95%, 96%, 97%, 98%, 99%, or 100% amino acid identity to SEQ ID NO:90, and comprising a CDRL1 of SEQ ID NO:56, CDRL2 of SEQ ID NO:57, and CDRL3 of SEQ ID NO:58,
b15) a light chain having at least 90%, 91%, 92%, 93%, 94%, 95%, 96%, 97%, 98%, 99%, or 100% amino acid identity to SEQ ID NO:91, and comprising a CDRL1 of SEQ ID NO:59, CDRL2 of SEQ ID NO:60, and CDRL3 of SEQ ID NO:61,
b16) a light chain having at least 90%, 91%, 92%, 93%, 94%, 95%, 96%, 97%, 98%, 99%, or 100% amino acid identity to SEQ ID NO:92, and comprising a CDRL1 of SEQ ID NO:62, CDRL2 of SEQ ID NO:63, and CDRL3 of SEQ ID NO:64,
b17) a light chain having at least 90%, 91%, 92%, 93%, 94%, 95%, 96%, 97%, 98%, 99%, or 100% amino acid identity to SEQ ID NO:174, and comprising a CDRL1 of SEQ ID NO:71, CDRL2 of SEQ ID NO:72, and CDRL3 of SEQ ID NO:73,
b18) a light chain having at least 90%, 91%, 92%, 93%, 94%, 95%, 96%, 97%, 98%, 99%, or 100% amino acid identity to SEQ ID NO:175, and comprising a CDRL1 of SEQ ID NO:142, CDRL2 of SEQ ID NO:143, and CDRL3 of SEQ ID NO:144
b19) a light chain having at least 90%, 91%, 92%, 93%, 94%, 95%, 96%, 97%, 98%, 99%, or 100% amino acid identity to SEQ ID NO:176, and comprising a CDRL1 of SEQ ID NO:145, CDRL2 of SEQ ID NO:146, and CDRL3 of SEQ ID NO:147,
b20) a light chain having at least 90%, 91%, 92%, 93%, 94%, 95%, 96%, 97%, 98%, 99%, or 100% amino acid identity to SEQ ID NO:177, and comprising a CDRL1 of SEQ ID NO:148, CDRL2 of SEQ ID NO:149, and CDRL3 of SEQ ID NO:150,
b21) a light chain having at least 90%, 91%, 92%, 93%, 94%, 95%, 96%, 97%, 98%, 99%, or 100% amino acid identity to SEQ ID NO:178, and comprising a CDRL1 of SEQ ID NO:151, CDRL2 of SEQ ID NO:152, and CDRL3 of SEQ ID NO:153,
b22) a light chain having at least 90%, 91%, 92%, 93%, 94%, 95%, 96%, 97%, 98%, 99%, or 100% amino acid identity to SEQ ID NO:179, and comprising a CDRL1 of SEQ ID NO:154, CDRL2 of SEQ ID NO:155, and CDRL3 of SEQ ID NO:156, and
b23) a light chain having at least 90%, 91%, 92%, 93%, 94%, 95%, 96%, 97%, 98%, 99%, or 100% amino acid identity to SEQ ID NO:180, and comprising a CDRL1 of SEQ ID NO:157, CDRL2 of SEQ ID NO:158, and CDRL3 of SEQ ID NO:159, and

c) in the second binding part a heavy chain region having at least 90%, 91%, 92%, 93%, 94%, 95%, 96%, 97%, 98%, 99%, or 100% amino acid identity to SEQ ID NO:5, comprising a CDRH1 of SEQ ID NO:1, a CDRH2 of SEQ ID NO:2, and a CDRH3 of SEQ ID NO:3, and a light chain having at least 90%, 91%, 92%, 93%, 94%, 95%, 96%, 97%, 98%, 99%, or 100% amino acid identity to SEQ ID NO:11, comprising a CDRL1 of SEQ ID NO:7, a CDRL2 of SEQ ID NO:8, and a CDRL3 of SEQ ID NO:9.

In one embodiment, the invention relates to a bispecific antibody according to the invention, characterized in comprising

a) in the first binding part a heavy chain region having at least 90%, 91%, 92%, 93%, 94%, 95%, 96%, 97%, 98%, 99%, or 100% amino acid identity to SEQ ID NO:5, comprising a CDRH1 of SEQ ID NO:1, a CDRH2 of SEQ ID NO:2, and a CDRH3 of SEQ ID NO:3, and
b) as light chain a light chain selected from the group consisting of

b1) a light chain having at least 90%, 91%, 92%, 93%, 94%, 95%, 96%, 97%, 98%, 99%, or 100% amino acid identity to SEQ ID NO:74, and comprising
a CDRL1 of SEQ ID NO:17, CDRL2 of SEQ ID NO:18, and CDRL3 of SEQ ID NO:19,
b2) a light chain having at least 90%, 91%, 92%, 93%, 94%, 95%, 96%, 97%, 98%, 99%, or 100% amino acid identity to SEQ ID NO:75, and comprising a CDRL1 of SEQ ID NO:20, CDRL2 of SEQ ID NO:21, and CDRL3 of SEQ ID NO:22,
b3) a light chain having at least 90%, 91%, 92%, 93%, 94%, 95%, 96%, 97%, 98%, 99%, or 100% amino acid identity to SEQ ID NO:76, and comprising a CDRL1 of SEQ ID NO:23, CDRL2 of SEQ ID NO:24, and CDRL3 of SEQ ID NO:25,
b20) a light chain having at least 90%, 91%, 92%, 93%, 94%, 95%, 96%, 97%, 98%, 99%, or 100% amino acid identity to SEQ ID NO:167, and comprising a CDRL1 of SEQ ID NO:71, CDRL2 of SEQ ID NO:72, and CDRL3 of SEQ ID NO:73,
b21) a light chain having at least 90%, 91%, 92%, 93%, 94%, 95%, 96%, 97%, 98%, 99%, or 100% amino acid identity to SEQ ID NO:168, and comprising a CDRL1 of SEQ ID NO:142, CDRL2 of SEQ ID NO:143, and CDRL3 of SEQ ID NO:144
b22) a light chain having at least 90%, 91%, 92%, 93%, 94%, 95%, 96%, 97%, 98%, 99%, or 100% amino acid identity to SEQ ID NO:169, and comprising a CDRL1 of SEQ ID NO:145, CDRL2 of SEQ ID NO:146, and CDRL3 of SEQ ID NO:147,
b23) a light chain having at least 90%, 91%, 92%, 93%, 94%, 95%, 96%, 97%, 98%, 99%, or 100% amino acid identity to SEQ ID NO:170, and comprising a CDRL1 of SEQ ID NO:148, CDRL2 of SEQ ID NO:149, and CDRL3 of SEQ ID NO:150,
b24) a light chain having at least 90%, 91%, 92%, 93%, 94%, 95%, 96%, 97%, 98%, 99%, or 100% amino acid identity to SEQ ID NO:171, and comprising a CDRL1 of SEQ ID NO:151, CDRL2 of SEQ ID NO:152, and CDRL3 of SEQ ID NO:153,
b25) a light chain having at least 90%, 91%, 92%, 93%, 94%, 95%, 96%, 97%, 98%, 99%, or 100% amino acid identity to SEQ ID NO:172, and comprising a CDRL1 of SEQ ID NO:154, CDRL2 of SEQ ID NO:155, and CDRL3 of SEQ ID NO:156, and
b26) a light chain having at least 90%, 91%, 92%, 93%, 94%, 95%, 96%, 97%, 98%, 99%, or 100% amino acid identity to SEQ ID NO:173, and comprising a CDRL1 of SEQ ID NO:157, CDRL2 of SEQ ID NO:158, and CDRL3 of SEQ ID NO:159, and

c) in the second binding part a heavy chain region having at least 90%, 91%, 92%, 93%, 94%, 95%, 96%, 97%, 98%, 99%, or 100% amino acid identity to SEQ ID NO:5, comprising a CDRH1 of SEQ ID NO:1, a CDRH2 of SEQ ID NO:2, and a CDRH3 of SEQ ID NO:3, and as light chain a light chain having at least 90%, 91%, 92%, 93%, 94%, 95%, 96%, 97%, 98%, 99%, or 100% amino acid identity to SEQ ID NO:181, comprising a CDRL1 of SEQ ID NO:7, a CDRL2 of SEQ ID NO:8, and a CDRL3 of SEQ ID NO:9.

In one embodiment, the invention relates to a bispecific antibody according to the invention, characterized in comprising in the first binding part a heavy chain region sequence having at least 90%, 91%, 92%, 93%, 94%, 95%, 96%, 97%, 98%, 99%, or 100% amino acid sequence identity to the heavy chain region of SEQ ID NO:5 and as light chain a light chain having at least 90%, 91%, 92%, 93%, 94%, 95%, 96%, 97%, 98%, 99%, or 100% amino acid sequence identity to a light chain selected from the group of

a) the light chain of SEQ ID NO:77,
b) the light chain of SEQ ID NO:78,
c) the light chain of SEQ ID NO:79,
d) the light chain of SEQ ID NO:80,
e) the light chain of SEQ ID NO:81,
f) the light chain of SEQ ID NO:82,
g) the light chain of SEQ ID NO:83,
h) the light chain of SEQ ID NO:84,
i) the light chain of SEQ ID NO:85,
k) the light chain of SEQ ID NO:86,
l) the light chain of SEQ ID NO:87,
m) the light chain of SEQ ID NO:88,
n) the light chain of SEQ ID NO:89,
o) the light chain of SEQ ID NO:90,
p) the light chain of SEQ ID NO:91,
r) the light chain of SEQ ID NO:92,
s) the light chain of SEQ ID NO:174,
t) the light chain of SEQ ID NO:175,
u) the light chain of SEQ ID NO:176,
v) the light chain of SEQ ID NO:177,
w) the light chain of SEQ ID NO:178,
x) the light chain of SEQ ID NO:179, and
y) the light chain of SEQ ID NO:180, and
b) comprising in the second binding part a heavy chain region sequence having at least 90%, 91%, 92%, 93%, 94%, 95%, 96%, 97%, 98%, 99%, or 100% amino acid sequence identity to the heavy chain region of SEQ ID NO:5 and as light chain a light chain having at least 90%, 91%, 92%, 93%, 94%, 95%, 96%, 97%, 98%, 99%, or 100% amino acid sequence identity to a light chain of SEQ ID NO:11.

In one embodiment, the invention relates to a bispecific antibody according to the invention, characterized in comprising in the first binding part a heavy chain region sequence having at least 90%, 91%, 92%, 93%, 94%, 95%, 96%, 97%, 98%, 99%, or 100% amino acid sequence identity to the heavy chain region of SEQ ID NO:5 and as light chain a light chain having at least 90%, 91%, 92%, 93%, 94%, 95%, 96%, 97%, 98%, 99%, or 100% amino acid sequence identity to a light chain selected from the group of

a) the light chain of SEQ ID NO:74,
b) the light chain of SEQ ID NO:75,
c) the light chain of SEQ ID NO:76,
d) the light chain of SEQ ID NO:167,
e) the light chain of SEQ ID NO:168,
f) the light chain of SEQ ID NO:169,
g) the light chain of SEQ ID NO:170,
h) the light chain of SEQ ID NO:171,
i) the light chain of SEQ ID NO:172,
k) the light chain of SEQ ID NO:173, and
b) comprising in the second binding part a heavy chain region sequence having at least 90%, 91%, 92%, 93%, 94%, 95%, 96%, 97%, 98%, 99%, or 100% amino acid sequence identity to the heavy chain region of SEQ ID NO:5 and as light chain a light chain having at least 90%, 91%, 92%, 93%, 94%, 95%, 96%, 97%, 98%, 99%, or 100% amino acid sequence identity to a light chain of SEQ ID NO:181.

In one embodiment, the invention relates to a bispecific antibody according to the invention, characterized in comprising in the first binding part a heavy chain region sequence of SEQ ID NO:5 and as light chain a light chain selected from the group of

a) the light chain of SEQ ID NO:77,
b) the light chain of SEQ ID NO:78,
c) the light chain of SEQ ID NO:79,

d) the light chain of SEQ ID NO:80,
e) the light chain of SEQ ID NO:81,
f) the light chain of SEQ ID NO:82,
g) the light chain of SEQ ID NO:83,
h) the light chain of SEQ ID NO:84,
i) the light chain of SEQ ID NO:85,
k) the light chain of SEQ ID NO:86,
l) the light chain of SEQ ID NO:87,
m) the light chain of SEQ ID NO:88,
n) the light chain of SEQ ID NO:89,
o) the light chain of SEQ ID NO:90,
p) the light chain of SEQ ID NO:91,
r) the light chain of SEQ ID NO:92,
s) the light chain of SEQ ID NO:174,
t) the light chain of SEQ ID NO:175,
u) the light chain of SEQ ID NO:176,
v) the light chain of SEQ ID NO:177,
w) the light chain of SEQ ID NO:178,
x) the light chain of SEQ ID NO:179, and
y) the light chain of SEQ ID NO:180, and
b) comprising in the second binding part a heavy chain region sequence of SEQ ID NO:5 and as light chain a light chain of SEQ ID NO:11.

In one embodiment, the invention relates to a bispecific antibody according to the invention, characterized in comprising in the first binding part a heavy chain region sequence of SEQ ID NO:5 and as light chain a light chain selected from the group of

a) the light chain of SEQ ID NO:74,
b) the light chain of SEQ ID NO:75,
c) the light chain of SEQ ID NO:76,
d) the light chain of SEQ ID NO:167,
e) the light chain of SEQ ID NO:168,
f) the light chain of SEQ ID NO:169,
g) the light chain of SEQ ID NO:170,
h) the light chain of SEQ ID NO:171,
i) the light chain of SEQ ID NO:172,
k) the light chain of SEQ ID NO:173,
and
b) comprising in the second binding part a heavy chain region sequence of SEQ ID NO:5 and as light chain a light chain of SEQ ID NO:181.

In one embodiment, the invention relates to a bispecific antibody according to the invention, characterized in comprising in the first binding part as variable heavy chain region a variable heavy chain region of SEQ ID NO:4.
In one embodiment, the invention relates to a bispecific antibody according to the invention, characterized in comprising in the second binding part as variable heavy chain region a variable heavy chain region of SEQ ID NO:4.
In one embodiment, the invention relates to a bispecific antibody according to the invention, characterized in comprising in the second binding part as variable light chain region a variable light chain region of SEQ ID NO:10.
In one embodiment, the invention relates to a bispecific antibody according to the invention, characterized in specifically binding to human CEACAM5 and cynomolgus CEACAM5.
In one embodiment, the invention relates to a bispecific antibody according to the invention, characterized in specifically binding to human CEACAM5, cynomolgus CEACAM5 and human CEACAM6.
In one embodiment, the invention relates to a bispecific antibody according to the invention, characterized in that said bispecific antibody competes for binding to CEACAM5 with the anti-CEACAM5 antibody SM3E, which comprises as VK and VH domains VK and VH of sequences SEQ ID NO:110 and 111.
In one embodiment the bispecific antibody is K2AC53 and K2AC54 and derivatives comprising the CDR regions and/or the light and heavy chains of said antibodies as described above.
In one embodiment, the invention relates to a bispecific antibody specifically binding to human CEACAM5 and human CD47 characterized in that the Fc region has been glycoengineered to have a reduced number of fucose residues as compared to the same but non-glycoengineered bispecific antibody.

In one embodiment, the present invention provides a bispecific antibody, characterized in specifically binding to human CEACAM5 and not to human CEACAM6 in the first binding part and specifically binding to human CD47 in the second binding part. The term "not binding to human CEACAM6" means that in an ELISA based binding assay (example 8f) to the recombinant human CEACAM6 protein in one embodiment a concentration dependent binding curve cannot be established due to very weak or no binding or in another embodiment EC50 for binding is 100 or more times higher than EC50 for binding to recombinant human CEACAM5.

In one embodiment, the present invention provides a bispecific antibody, characterized in specifically binding to human CEACAM5 and human CEACAM6 in the first binding part and to human CD47 in the second binding part. In one embodiment the invention relates to a bispecific antibody CEAxCD47 specifically binding in a balanced manner to human CEACAM5 and human CEACAM6. In one embodiment the bispecific antibody is characterized in binding to human recombinant CEACAM5 and CEACAM6, characterized in that the EC50 values of binding to human CEACAM5 and human CEACAM6 differing by less than a factor of 10. In one embodiment the bispecific antibody is characterized in binding to human CEACAM5 and CEACAM6, characterized in that the EC50 values of binding to human CEACAM5 and human CEACAM6 differing by less than a factor of 5 (balanced CEACAM5 and CEACAM6 binding, binding in balanced manner, see figures 4 and 5 and table 6). Binding is measured in a streptavidin/biotin-based ELISA (see example 8f). Such bispecific antibodies are e.g. K2AC49 and K2AC50 and derivatives comprising the CDR regions and/or the light and heavy chains of said antibodies as described above.

In one embodiment the bispecific antibody according to the invention is characterized in specifically binding to human CEACAM5 and human CEACAM6 in the first binding part and human CD47 in the second binding part, characterized in

a) that the first binding part comprises as heavy chain variable region a heavy chain variable region comprising as CDRs a CDRH1 of SEQ ID NO:1, CDRH2 of SEQ ID NO:2 and CDRH3 of SEQ ID NO:3 and as light chain variable region a light chain variable region comprising as CDRs a CDRL1 of SEQ ID NO: 44, a CDRL2 of SEQ ID NO: 45, and a CDRL3 of SEQ ID NO: 46 and

b) that the second binding part comprises as heavy chain variable region a heavy chain variable region comprising as CDRs a CDRH1 of SEQ ID NO:1, CDRH2 of SEQ ID NO:2 and CDRH3 of SEQ ID NO:3 and as light chain variable region a light chain variable region comprising as CDRs a CDRL1 of SEQ ID NO:7, CDRL2 of SEQ ID NO:8, and CDRL3 of SEQ ID NO:9.

In one embodiment the bispecific antibody according to the invention is characterized in specifically binding to human CEACAM5 and cynomolgus CEACAM5 in the first binding part and human CD47 in the second binding part, characterized in

b) a) that the first binding part comprises as heavy chain variable region a heavy chain variable region comprising as CDRs a CDRH1 of SEQ ID NO:1, CDRH2 of SEQ ID NO:2 and CDRH3 of SEQ ID NO:3 and as light chain variable region a light chain variable region comprising as CDRs a CDRL1 of SEQ ID NO: 53, a CDRL2 of SEQ ID NO: 54, and a CDRL3 of SEQ ID NO: 55, and

b) that the second binding part comprises as heavy chain variable region a heavy chain variable region comprising as CDRs a CDRH1 of SEQ ID NO:1, CDRH2 of SEQ ID NO:2 and CDRH3 of SEQ ID NO:3 and as light chain variable region a light chain variable region comprising as CDRs a CDRL1 of SEQ ID NO:7, CDRL2 of SEQ ID NO:8, and CDRL3 of SEQ ID NO:9.

In one embodiment the present invention provides a bispecific antibody, specifically binding to human CEACAM5 and human CEACAM6 in the first binding part and human CD47 in the second binding part, characterized in

a) that the first binding part comprises a heavy chain of SEQ ID NO:5 or of SEQ ID NO:6 and as light chain a light chain of SEQ ID NO:86 and

b) that the second binding part comprises a heavy chain of SEQ ID NO:5 or of SEQ ID NO:6 and as light chain a light chain of SEQ ID NO:11.

In one embodiment the present invention provides a bispecific antibody, specifically binding to human CEACAM5 and cynomolgus CEACAM5 in the first binding part and human CD47 in the second binding part, characterized in

a) that the first binding part comprises a heavy chain of SEQ ID NO:5 or of SEQ ID NO:6 and as light chain a light chain of SEQ ID NO:89 and

b) that the second binding part comprises a heavy chain of SEQ ID NO:5 or of SEQ ID NO:6 and as light chain a light chain of SEQ ID NO:11.

In one embodiment of the invention the antibody is characterized in a balanced human/cynomolgus CEACAM5 binding (EC50 for binding to recombinant human CEACAM5 and cynomolgus CEACAM5 do not differ by more than a factor of 10, in one embodiment a factor of 5). In one embodiment of the invention these antibodies are characterized in human CEACAM6 binding with an EC50 for binding to the recombinant human CEACAM6 (ELISA based assay, see Example 8f) of 1 nM or lower.

In one embodiment the present invention provides a bispecific antibody, specifically binding to human CEACAM5 and cynomolgus CEACAM5 in the first binding part and human CD47 in the second binding part, characterized in

a) that the first binding part comprises a heavy chain variable region comprising as CDRs a CDRH1 of SEQ ID NO:1, CDRH2 of SEQ ID NO:2 and CDRH3 of SEQ ID NO:3 and as light chain variable region a light chain variable region comprising as CDRs a CDRL1 of SEQ ID NO: 53, a CDRL2 of SEQ ID NO: 54, and a CDRL3 of SEQ ID NO: 55, and

b) that the second binding part comprises a heavy chain variable region comprising as CDRs a CDRH1 of SEQ ID NO:1, CDRH2 of SEQ ID NO:2 and CDRH3 of SEQ ID NO:3 and as light chain variable region a light chain variable region comprising as CDRs a CDRL1 of SEQ ID NO:7, CDRL2 of SEQ ID NO:8, and CDRL3 of SEQ ID NO:9. In one embodiment of the invention this antibody is characterized in a balanced human/cynomolgus CEACAM5 binding (antibody K2AC54). Balanced binding means that the binding curves to recombinant human and cynomolgus CEACAM5 do not differ by more than a factor of 10 regarding the EC50 (see also fig.6; the range of factor 10 means EC50 of CEACAM5:CEACAM6 is 1:10 to 10:1; analogous for factor 5). In one embodiment the binding curves to recombinant human and cynomolgus CEACAM5 do not differ by more than a factor of 5 regarding the EC50 (see also fig.6). This balanced binding to human and cynomolgus CEACAM5 together with the cross reactivity of the CD47 arm used in all the bsAb of this invention to cynomolgus CD47 will enable to study the toxicological profile of the bsAb according to the invention in cynomolgus monkeys.

In one embodiment, the invention relates to a bispecific antibody specifically binding to human CEACAM5 and human CD47, the bispecific antibody comprising a first binding part, specifically binding to human CEACAM5 and a second binding part, specifically binding to human CD47, characterized in that said bispecific antibody competes with the anti-CEA antibody SM3E, comprising as VK and VH domains VK and VH of sequences SEQ ID NO:110 and 111, for binding to CEACAM5.

In one embodiment, the invention relates to a bispecific antibody specifically binding to human CEACAM5 and human CD47, the bispecific antibody comprising a first binding part, specifically binding to human CEACAM5 and a second binding part, specifically binding to human CD47, characterized in that said bispecific antibody does not compete with anti-CEA antibodies SAR, comprising as VK and VH domains VK and VH of sequences SEQ ID NO:112 and 113, and CH1A1A, comprising as VK and VH domains VK and VH of sequences SEQ ID NO:114 and 115 for binding to CEACAM5.

In one embodiment, the invention relates to a bispecific antibody specifically binding to human CEACAM5 and human CD47, the bispecific antibody comprising a first binding part, specifically binding to human CEACAM5 and a second binding part, specifically binding to human CD47, characterized in that the EC50 value of phagocytosis index curve of said bispecific antibody is in the range of 0.01 to 10 times of the EC50 value of reference antibody K2AC54 under the same experimental conditions. In further embodiments the range is 0.01 to 10, 0.1 to 10, 0.2 to 10, 0.3 to 10, or 0.5 to 10. EC50 values of phagocytosis are measured as EC50 values of the phagocytosis index curve (imaging-based phagocytosis assay, see Example 9 and figure 3). In one embodiment the EC50 value is in such range in the presence of 1mg/ml human IgG or without human IgG.

In one embodiment, the invention relates to a bispecific antibody specifically binding to human CEACAM5 and human CD47, the bispecific antibody comprising a first binding part, specifically binding to human CEACAM5 and a second binding part, specifically binding to human CD47, characterized in that in presence of 1mg/ml human IgG the maximal phagocytosis index (see example 9e; CellInsight™ based assay) of said bispecific antibody is not decreased for 30% or more in comparison to the maximal phagocytosis index measured under the same experimental conditions but without addition of human IgG.

In one embodiment the bispecific antibody is characterized in being monovalent for the first binding part and monovalent for the second binding part.

In one embodiment, the constant and variable framework region sequences are human.

In one embodiment, the bispecific antibody is characterized in that each of the first and second binding part comprises an immunoglobulin heavy chain and an immunoglobulin light chain. In one embodiment the bispecific antibody is characterized in being of human IgG1 type. In one embodiment the bispecific antibody is a full-length antibody.

In one embodiment the bispecific antibody according to the invention is characterized in comprising a first binding part specific for CEA, comprising a lambda light chain variable domain and a lambda light chain constant domain and a second binding part specific for CD47, comprising a kappa light chain variable domain and a kappa light chain constant domain (κλ bispecific antibody, κλ Body, type 1; see table 1). In one such embodiment the second binding part comprises as light chain LC (CD47 VKCK) the light chain of SEQ ID NO:11 and in the first binding part the constant light chain domain is the lambda light chain constant domain of SEQ ID NO:15. The kappa light chain of SEQ ID NO:11 comprises as variable light chain domain the variable light chain domain of SEQ ID NO:10 (Mab CD47 VK) and as constant light chain domain the constant light chain domain of SEQ ID NO:13 (Mab CD47 CK).

In one embodiment the bispecific antibody according to the invention is characterized in comprising a first binding part specifically binding to CEA, comprising a kappa light chain variable domain and a lambda light chain constant domain (hybrid light chain) and a second binding part specifically binding to CD47, comprising a kappa light chain variable domain and a kappa light chain constant domain (hybrid versions of bispecific antibodies K2AC41, K2AC42,K2AC43, K2AC60, K2AC61, K2AC62, K2AC63, K2AC64, K2AC65, and K2AC66; κλ bispecific antibody, κλ Body, type 2, see table 1 and fig.1B). In one such embodiment the second binding part comprises as kappa light chain (CD47 VKCK) the kappa light chain of SEQ ID NO:11, and in the first binding part the lambda constant light chain domain of SEQ ID NO:15 (AC CEA CL). The kappa light chain of SEQ ID NO:11 comprises as variable light chain domain the variable light chain of SEQ ID NO:10 (CD47 VK) and as constant light chain the constant light chain of SEQ ID NO:13 (CD47 CK).

In one embodiment the bispecific antibody according to the invention is characterized in comprising a first binding part specifically binding to CEA, comprising a kappa light chain variable domain and a kappa light chain constant domain and a second binding part specifically binding to CD47, comprising a kappa light chain variable domain and a lambda light chain constant domain (hybrid bispecific antibodies; κλ bispecific antibody, κλ Body, type 3, fig.1C). In one such embodiment the second binding part comprises as light chain the light chain of SEQ ID NO:181 (MabCD47 VKCL, hybrid light chain) and in the first binding part the kappa CL of SEQ ID NO:16. The light chain of SEQ ID NO:181 (MabCD47 VKCL) comprises as variable light chain domain the kappa variable light chain domain of SEQ ID NO:10 (CD47 VK) and as constant light chain domain the lambda constant light chain domain of SEQ ID NO:14 (CD47 CL).

In one embodiment the bispecific antibody according to the invention is of fully human bispecific IgG (especially IgG1) format and in addition a κλ bispecific antibody of type 1, type 2 or type 3.

In one embodiment the bispecific antibody according to the invention is characterized in being a κλ bispecific antibody of type 1, type 2, or type 3 and comprising a common heavy chain (cHC). In one embodiment the common heavy chain comprises as variable heavy chain a variable heavy chain of SEQ ID NO:4. In one embodiment the bispecific antibody according to the invention is characterized in comprising a common heavy chain of SEQ ID NO:5

In one embodiment the bispecific antibody is characterized in binding to human CD47 with a binding affinity ($K_D$) of 100 nM to 600nM, in one embodiment with a binding affinity of 100 nM to 500nM.

In one embodiment the bispecific antibody is characterized in binding to MKN-45 cells with an EC50 value of 1 to 250 nM, in one embodiment with an EC50 value of 1 to 200 nM. In one embodiment with an EC50 value of 1 to 150 nM. In one embodiment the bispecific antibody is characterized in binding to MKN-45 cells with a value of 1 to 30 nM. In one embodiment the bispecific antibody is characterized in binding to MKN-45 cells with an EC50 value of 10 to 30 nM. In one embodiment the bispecific antibody is characterized in binding to MKN-45 cells with an EC50 value of 10 to 100 nM.

In one embodiment the bispecific antibody is characterized in that it does not cross-react with human CEACAM1.

In one embodiment the bispecific antibody according to the invention is characterized in that a bispecific antibody specifically binding to human CEACAM5 and CD3ε (further named also as CEA-TCB), comprising as heavy chains the heavy chains of SEQ ID NO:107 and 108 and as light chains the light chains of SEQ ID NO: 106 and 109 in a concentration of 300 nM does not shift the EC50 of the binding curve of the bispecific antibody of the invention to MKN-45 cells by more than a factor of 3, in one embodiment towards higher concentrations. In such case the bispecific antibody according to the invention and CEA-TCB are defined as "not competitive" and considered able to bind simultaneously to CEA without significantly interfering in binding to said CEA. CEA-TCB is in clinical trials (cibisatamab or RO 6958688; ClinicalTrials.gov NCT03866239).

In one embodiment the bispecific antibody according to the invention is characterized that a bispecific antibody specifically binding to human CEACAM5 and CD3ε (further named also as CEA-TCB 1), comprising as heavy and light chains the chains of amino acid sequences SEQ ID NO: 102 to 105 in a concentration of 30 nM does not shift the EC50 of the binding curve of the bispecific antibody of the invention to MKN-45 cells by more than a factor of 3, in one embodiment towards higher concentrations. In such case the bispecific antibody according to the invention and CEA-TCB1 are defined as "not competitive" and considered able to bind simultaneously to CEA without significantly interfering in binding to said CEA. In such case the bispecific antibody according to the invention and CEA-TCB and/or CEA-TCB 1 are defined as "not competitive" and considered able to bind simultaneously to CEA without significantly interfering in their binding to said CEA.

In one embodiment the bispecific antibody according to the invention is characterized in that a bispecific antibody specifically binding to human CEACAM5 and CD3ε (further named also as CEA-TCB 1), comprising as heavy and light chains the chains of amino acid sequences SEQ ID NO: 102 to 105, in a concentration of 30 nM does not shift the EC50 of the phagocytosis index curve of the bispecific antibody of the invention to MKN-45 cells by more than a factor of 3, in one embodiment towards higher concentrations. In such case the bispecific antibody according to the invention and CEA-TCB 1 are defined as "not competitive" and considered able to bind simultaneously to CEA without significantly interfering in their binding to said CEA, and can therefore develop its effect on phagocytosis (CEAxCD47) undisturbed and also its effect on T-cell activation (CEAxTCB1) undisturbed, even if therapeutic levels of both drugs are simultaneously present in the tumor tissue.

In one embodiment the bispecific antibody according to the invention is characterized that a bispecific antibody specifically binding to human CEACAM5 and CD3ε (further named also as CEA-TCB), comprising as heavy and light chains the chains of amino acid sequences SEQ ID NO: 106 to 109 in a concentration of 300 nM does not shift the EC50 of the phagocytosis index curve of the bispecific antibody of the invention to MKN-45 cells by more than a factor of 3, in one embodiment towards higher concentrations.. In such case the bispecific antibody according to the invention and CEA-TCB are defined as "not competitive" and considered able to bind simultaneously to CEA without significantly interfering in their binding to said CEA and can therefore develop its effect on phagocytosis (CEAxCD47) undisturbed and also its effect on T-cell activation (CEA-TCB) undisturbed, even if therapeutic levels of both drugs are simultaneously present in the tumor tissue. This facilitates combination treatment of CEA-TCB/TCB1 with CEAxCD47 of this invention.

The sequences of SEQ ID NO: 106 to 109 and SEQ ID NO:116 are according to US20140242079 (CEA-TCB) and the sequences of SEQ ID NO:102 to 105 and SEQ ID NO:117 are according to WO2017055389 (CEA-TCB1).

In one embodiment the CEAxCD47 bispecific antibodies of the invention combined with CEAxCD3 bispecific antibodies like CEA-TCB and CEA-TCB 1 show at least additive or even synergistic % killing of tumor cells in an assay containing e.g. MKN-45 tumor cells and human macrophages and T-cells derived from the same volunteer human donor.

In one embodiment, the bispecific antibody is characterized in comprising a common heavy chain (cHC) as heavy chain of the first binding part and as heavy chain of the second binding part. In one embodiment, the bispecific antibody is characterized in that said common heavy chain of each binding part comprises as CDRs CDRH1 of SEQ ID NO:1, CDRH2 of SEQ ID NO:2 and CDRH3 of SEQ ID NO:3. In one embodiment, the bispecific antibody is characterized in that said common heavy chain of each binding part comprises as common variable heavy domain SEQ ID NO:4.

In one embodiment, the bispecific antibody according to the invention is characterized in inhibiting the interaction between CD47 on MKN-45 cells with an IC50 of 0.1 to 10 nM. SIRPα (SIRPα, CD172a; UniProtKB P78324) is used in a concentration of 200 ng/ml (His tagged soluble SIRPα). Details of the assay are described in example 8 (SIRPα Blocking Activity of CD47 Antibodies), and results are shown in Table 4.

In one embodiment the bispecific antibody of the invention is characterized in a concentration dependent phagocytosis (ADCP) of CEACAM5 and/or CEACAM6 expressing tumor cell lines like MKN-45 cells by human macrophages at an EC50 of the bispecific antibody below 40nM, in one embodiment below 10 nM (1 μg/mL are approx. 6.6 nM). ADCP is measured according to the invention as phagocytosis index (EC50 or maximum) by imaging, usually with an E:T ratio of 1:3 (human macrophages;target cells (tumor cells); see e.g. Fig.3 and table 5 for EC50 values and for max. index of phagocytosis). Results in figure 3 have been obtained with E:T of 1:3. Details of the assay are described in example 9e).1. (Imaging assay based on CellInsight CX5). If not otherwise stated, phagocytosis index values are measured by such imaging method.

ADCP can be also measured by Flow Cytometry with an E:T ratio of e.g. 3:1 (human macrophages;target cells (tumor cells). Details of the assay are described in example 9e).2 (Flow cytometry based ADCP assay).

In one embodiment, the bispecific antibody is characterized in specifically binding to CEACAM5 but is not competing for binding to CEACAM5 on tumor cells like MKN-45 with CEA-TCB and/or CEA-TCB1.

In one embodiment, the bispecific antibody according to the invention is characterized in that the EC50 value for the binding to MKN-45 cells (EC50 between 1 and 250 nM) is increased by less than a factor of three by addition of CEA-TCB at a concentration of 300 nM respectively by addition of CEA-TCB1 at a concentration of 30 nM (no competition).

In one embodiment, the CEAxCD47 antibodies of the invention show a 100 or more times higher EC50 for RBC phagocytosis compared to the EC50 measured in the same assay with B6H12.2 (ATCC® HB9771™; for assay see Example 15).

In one embodiment, the CEAxCD47 antibodies of the invention (carrying wt IgG1 Fc w/o or with afucosylation) do not show significant platelet activation in concentrations up to 200 μg/mL (see Example 15 for the assay used).

In another embodiment, the present invention relates to a bispecific antibody according to the invention that has been glycoengineered to have an Fc region with modified oligosaccharides.

In one embodiment the bispecific antibody according to the invention comprises a reduced amount of fucose in the oligosaccharide chain(s). It was surprisingly found, that such a glycoengineered bispecific antibody according to the invention is characterized in an at least 3 times lower EC50 value for the phagocytosis index curve measured by the imaging based assay) as the same not glycoengineered (parent) bispecific antibody if measured under the same experimental conditions. In one embodiment EC50 for the phagocytosis index is 5 to 10 times lower, or 10 to 30 times lower). In one embodiment, the Fc region has been modified to have a reduced number of fucose residues as compared to the same but non-glycoengineered bispecific antibody. In another embodiment, the Fc region has an increased proportion of bisected oligosaccharides as compared to the non-glycoengineered bispecific antibody. In yet another embodiment, the bisected oligosaccharides are predominantly bisected complex. In another embodiment, the glycoengineered antigen binding molecules of the invention have an increased proportion of bisected, nonfucosylated oligosaccharides in the Fc region of said bispecific antibody as compared to the non-glycoengineered bispecific antibody. Alternatively, the bispecific antibodies of the invention may have an increased ratio of GlcNAc residues to fucose residues in the Fc region compared to the non-glycoengineered bispecific antibody. In one embodiment, the bisected, nonfucosylated oligosaccharides are predominantly in hybrid form. Alternatively, the bisected, nonfucosylated oligosaccharides are predominantly complex type.

In one embodiment the bispecific antibody according to the invention is characterized in that 50% to 100% of the N-linked oligosaccharides in the Fc region are nonfucosylated.

In one embodiment the bispecific antibody according to the invention is characterized in that the fucose amount in the oligosaccharide chain(s) of the bispecific antibody according to the invention is reduced by 80% to 100% compared to the fucose content of the respective antibody, if no afucosylation method is applied.

In one embodiment the bispecific antibody is characterized in that 50% to 100% of the N-linked oligosaccharides in the Fc region are bisected.

In one embodiment the bispecific antibody is characterized that 80% to 100% of the N-linked oligosaccharides in the Fc region are bisected and nonfucosylated.

In one embodiment the bispecific antibody is characterized in that concentration/ADCC curve (decrease of EC50 or increase of maximum of ADCC) induced by said glycoengineered antibody is increased by at least a factor of 1.2 compared to the ADCC induced by the same but non-glycoengineered bispecific antibody. In one embodiment ADCC is increased by a factor of 1.2 to 2.0.

In one embodiment the bispecific antibody is characterized in an at least 3 times lower EC50 value for the phagocytosis index curve measured by the imaging based assay as compared to the same but not glycoengineered (parent) bispecific antibody if measured under the same experimental conditions. In one embodiment EC50 for the phagocytosis index is 5 to 10 times lower, or 10 to 30 times lower

In one embodiment the bispecific antibody is characterized in that the maximal phagocytosis index induced by said glycoengineered antibody and measured by flow cytometry is increased by at least a factor of 1.2 compared to the maximal phagocytosis index induced by the same but non-glycoengineered bispecific antibody. In one embodiment maximal phagocytosis index is increased by a factor of 1.2 to 2.0.

In one embodiment the bispecific antibody is characterized in that the maximal phagocytosis index induced by said glycoengineered antibody and measured by imaging is increased by at least a factor of 1.2 compared to maximal phagocytosis index induced by the same but non-glycoengineered bispecific antibody. In one embodiment maximal phagocytosis index is increased by a factor of 1.2 to 2.0.

In one embodiment the bispecific antibody according to the invention is characterized in comprising one, two or three amino acid substitutions in the Fc region ("Fc amino acid substitution") selected from the group consisting of mono-substitutions S239D, I332E, G236A, of bi-substitutions I332E and G236A, S239D and I332E, S239D and G236A, and of triple-substitution S329D and I332E and G236A.

In one embodiment the bispecific antibody according to the invention is characterized in comprising one, two or three amino acid substitutions in the Fc region selected from the group consisting of mono-substitutions S239D, I332E, G236A, of bi-substitutions I332E and G236A, S239D and I332E, S239D and G236A, and triple-substitution S329D and I332E and G236A and a Fc region which has been glycoengineered to have a reduced number of fucose residues as compared to the same but non-glycoengineered bispecific antibody.

In one embodiment the bispecific antibody comprising said substitutions in the Fc region is characterized in that concentration/ADCC curve (decrease of EC50 or increase of maximum of ADCC) induced by said amino acid substituted antibody is increased by at least a factor of 1.2 compared to the ADCC induced by said antibody comprising none of said amino acid substitutions in the Fc region. In one embodiment ADCC is increased by a factor of 1.2 to 2.0.

In one embodiment the bispecific antibody comprising said substitutions in the Fc region is characterized in an at least 3 times lower EC50 value for the phagocytosis index curve measured by the imaging based assay as compared to the same (parent) bispecific antibody comprising none of said amino acid substitutions in Fc region, if measured under the same experimental conditions. In one embodiment EC50 for the phagocytosis index is 5 to 10 times lower, or 10 to 30 times lower. In one embodiment the bispecific antibody comprising said substitutions in the Fc region is characterized in that flow cytometry determined maximal phagocytosis (ADCP) induced by said amino acid substituted antibody is increased by at least a factor of 1.2 compared to the ADCP induced by said antibody comprising none of said amino acid substitutions in the Fc region. In one embodiment ADCP is increased by a factor of 1.2 to 2.0. In one embodiment the bispecific antibody comprising said substitutions in the Fc region is characterized in that by imaging determined maximal phagocytosis index induced by said amino acid substituted antibody is increased by at least a factor of 1.2 compared to the ADCP induced by said antibody comprising none of said amino acid substitutions in the Fc region. In one embodiment ADCP is increased by a factor of 1.2 to 2.0.

In one embodiment, the bispecific antibody according to the invention is characterized in that 50% to 100%, 60% to 100%, 70% to 100% or 80% to 100% of the N-linked oligosaccharides in the Fc region are non-fucosylated. In one embodiment, the bispecific antibody according to the invention is characterized in 50% to 100%, 60% to 100%, 70% to 100% or 80% to 100% of the N-linked oligosaccharides in the Fc region are bisected. In one embodiment, the bispecific antibody according to the invention is characterized in that 50% to 100%, 60% to 100%, 70% to 100% or 80% to 100% of the N-linked oligosaccharides in the Fc region are bisected, nonfucosylated.

In one embodiment, the glycoengineered bispecific antibody comprises increased effector functions compared to the non-glycoengineered bispecific antibody comprising as common heavy chain SEQ ID NO:6 (common heavy chain of parent bispecific antibody, produced in a CHO K1 cell line CHO-K1 (ATCC® CCL-61™ at standard conditions as defined below).

In one embodiment, the bispecific antibody according to the invention is characterized in that said glycoengineered bispecific antibody comprises one or more increased effector functions such as those from the group consisting of increased binding affinity to FcγRs, increased binding of macrophages (increased antibody dependent cellular phagocytosis; ADCP), increased binding of NK cells (increased antibody-mediated cellular cytotoxicity; ADCC), and increased binding to monocytes.

The concentration/phagocytosis index curve measured for the anti-CD47 monoclonal antibody hu5F9-G4 (tested in clinical trials since 2014, see e.g. clinicaltrial.gov) is strongly reduced by the addition of human IgG added in physiological concentrations of 1 mg/mL to the assay (increase of EC50 and decrease of the maximum of the phagocytosis curve measured in imaging based assay).

In one embodiment the CEAxCD47 antibodies of the invention show only a small shift of a factor of 3 or below of EC50 and 30% decrease or less of the maximum of the concentration/phagocytosis index curve if 1mg/mL human IgG is added compared to same assay but without addition of human IgG.

In one embodiment the CEAxCD47 antibodies of the invention are characterized in that addition of 1 mg /mL of human IgG to the imaging based phagocytosis assay causes a less than a factor of 0.8 reduction of the maximum of the concentration/phagocytosis index curve and/or a less than a factor of 3 shift of the EC50 towards higher concentrations.

A further embodiment of the invention is an isolated polynucleotide characterized in encoding a bispecific antibody according to the invention.

A further embodiment of the invention is an expression vector comprising the polynucleotide according to the invention. Appropriate polynucleotides are described in SEQ ID NO:12, 119 -141, and 160 - 166.

A further embodiment of the invention is a host cell comprising the expression vector according to the invention.

A further embodiment of the invention is a method for the production of a bispecific antibody according to the invention, characterized in comprising:

a) culturing a host cell comprising an expression vector encoding said bispecific antibody under conditions which permit the production of said antibody of the invention, and
b) isolating said antibody wherein said antibody is capable of specifically binding to CEACAM5 and CD47.

In one embodiment, the invention is characterized in comprising a method for producing a glycoengineered bispecific antibody according to the invention, lacking fucose or with a reduced amount of fucose on its oligosaccharide chains in a host cell, said method comprising:

a) providing a host cell comprising a first polynucleotide encoding GDP-6-deoxy-D-lyxo-4-hexylose reductase (RMD) and a second polynucleotide encoding the bispecific antibody according to the invention,

ii) expressing GDP-6-deoxy-D-lyxo-4-hexylose reductase encoded by the first polynucleotide and the bispecific antibody according to the invention encoded by the second polynucleotide in said cell, whereby said cell is cultivated in one embodiment in a fucose free medium, and
iii) isolating said bispecific antibody from said cell under conditions which permit the production of said bispecific antibody of the invention, and which permit that the oligosaccharides of the Fc region of said bispecific antibody are lacking fucose in an amount of 80% to 100%; and

b) isolating said glycoengineered bispecific antibody wherein said glycoengineered bispecific antibody is capable of specifically binding to CEACAM5 and to CD47 or CEACAM5 and CEACAM5 and CEACAM6 and to CD47.

The second polypeptide encoding the antibody of the invention can be one polypeptide encoding all respective two different light chains and the common heavy chain or separate polypeptides, encoding separately the respective light and heavy chains. Also the expression vector can be one, two or three vectors expressing the respective two different light chains and the common heavy chain.

In one embodiment, the invention is characterized in comprising a method for producing a glycoengineered bispecific antibody according to the invention in a host cell, said method comprising:

a) culturing a host cell glycoengineered to express at least one nucleic acid encoding a polypeptide having β(1,4)-N-acetylglucosaminyltransferase III activity under conditions which permit the production of said bispecific antibody of the invention, and which permit the modification of the oligosaccharides present on the Fc region of said bispecific antibody; and
b) isolating said glycoengineered bispecific antibody wherein said glycoengineered bispecific antibody is capable of specifically binding to CEACAM5 and CD47.

In one embodiment, the invention is characterized in comprising a method for producing a glycoengineered bispecific antibody in a host cell, said method comprising:

a) culturing a host cell glycoengineered by targeted disruption of the FUT8 gene under conditions which permit the production of said bispecific antibody of the invention, and which permit the modification of the oligosaccharides present on the Fc region of said bispecific antibody, and
b) isolating said glycoengineered bispecific antibody wherein said glycoengineered bispecific antibody is capable of specifically binding to CEACAM5 and CD47.

In one embodiment, the invention is characterized in comprising a method for producing a Fc substituted bispecific antibody according to the invention in a host cell, said method comprising:

a) culturing a host cell comprising an expression vector encoding a Fc substituted, bispecific antibody of the invention under conditions which permit the production of said bispecific antibody, and
b) isolating said Fc substituted bispecific antibody wherein said bispecific antibody is capable of specifically binding to CEACAM5 and CD47.

A further embodiment of the invention is a method of inducing cell lysis of a tumor cell comprising contacting the tumor cell with a bispecific antibody according to the invention. The tumor cell is a human tumor cell, preferably in a patient.

A further embodiment of the invention is a method according to the invention, characterized in that the tumor cell is a colorectal cancer cell, NSCLC (non-small cell lung cancer) cell, gastric cancer cell, pancreatic cancer cell, breast cancer cell, or another tumor cell expressing CEACAM5 or CEACAM5 and CEACAM6.

A further embodiment of the invention is a method of treating a subject having a cancer that expresses CEACAM5 or CEACAM5 and CEACAM6, the method comprising administering to the subject a therapeutically effective amount of a bispecific antibody according to the invention.

A further embodiment of the invention is a method of increasing survival time in a subject having a cancer that expresses CEACAM5 or CEACAM5 and CEACAM6, said method comprising administering to said subject a therapeutically effective amount of a bispecific antibody according to the invention.

A further embodiment of the invention is a method according to the invention, characterized in that the cancer is colorectal cancer, non-small cell lung cancer (NSCLC), gastric cancer, pancreatic cancer or breast.

A further embodiment of the invention is a method according to the invention, characterized in that a bispecific antibody according to the invention is administered in combination with chemotherapy or radiation therapy to a human subject.

A further embodiment of the invention is a method of treating a subject having a cancer that expresses CEACAM5 or CEACAM5 and CEACAM6, the method comprising administering to the subject a therapeutically effective amount of a bispecific antibody according to the invention, characterized in that the EC50 value of phagocytosis of said bispecific antibody is in the range of 0.01 to 10 times of the E50 value of reference antibody K2AC54 under the same experimental conditions and in the presence and/or without of 1mg/ml human IgG. In further embodiments the range is 0.01 to 10, 0.1 to 10, 0.2 to 10, 0.3 to 10, or 0.5 to 10. In one embodiment the bispecific antibody is characterized in binding to human CD47 with a binding affinity of 100 nM to 600nM, in one embodiment with a binding affinity of 100 nM to 500nM. Bispecific antibody K2AC54 is described by sequences SEQ ID NO:1 to 13, 15, 53, 54, 55 and 89.

A further embodiment of the invention is the use of a bispecific antibody according to the invention in a method of treating a subject having a cancer that expresses CEACAM5 or CEACAM5 and CEACAM6, the method comprising administering to the subject a therapeutically effective amount of a bispecific antibody according to the invention, characterized in that the EC50 value of phagocytosis of said bispecific antibody is in the range of 0.01 to 10 times of the E50 value of reference antibody K2AC54 under the same experimental conditions and in the presence or without of 1mg/ml human IgG. In further embodiments the range is 0.01 to 10, 0.1 to 10, 0.2 to 10, 0.3 to 10, 0.5 to 10 or 0. In one embodiment the bispecific antibody is characterized in binding to human CD47 with a binding affinity of 100 nM to 600nM, in one embodiment with a binding affinity of 100 nM to 500nM.

ADCC and ADCP/phagocytosis index values of antibodies according to the invention are not or only to a low extend affected by human IgG in a concentration of 1 mg/ml (1 mg/ml or even higher human IgG is the magnitude of the concentration of IgG found in the blood respectively plasma of most patients), whereas for an anti-CD47 antibody of the state of the art (hu5F9-G4), ADCC and ADCP values are strongly reduced in the presence of 1 mg/mL human IgG.

A further embodiment of the invention is the use of the bispecific antibody according to the invention in the manufacture of a medicament for treating a subject having a cancer that expresses CEACAM5 or CEACAM5 and CEACAM6.

A further embodiment of the invention is the use of the bispecific antibody according to the invention in the manufacture of a medicament according to the invention, characterized in that the cancer is selected from the group consisting of colorectal cancer, non-small cell lung cancer (NSCLC), gastric cancer, pancreatic cancer and breast cancer.

A further embodiment of the invention is a bispecific antibody according to the invention, for use in simultaneous, separate, or sequential combination with a second bispecific antibody comprising a third binding part specifically binding to human CEACAM5, and a fourth binding part specifically binding to human CD3ε in the treatment of a subject having a cancer that expresses CEACAM5 or CEACAM5 and CEACAM6. A further embodiment of the invention is a bispecific antibody according to the invention, for use in simultaneous, separate, or sequential combination with a second bispecific antibody comprising a third binding part specifically binding to human CEACAM5 and a fourth binding part specifically binding to an epitope of human CD3ε, said epitope comprising the amino acid sequence of SEQ ID NO:118 in the treatment of a subject having a cancer that expresses CEACAM5 or CEACAM5 and CEACAM6.

A further embodiment of the invention is a bispecific antibody according to the invention, for use in simultaneous, separate, or sequential combination with CEA-TCB or CEA/TCB1 in the treatment of a subject having a cancer that expresses CEACAM5 or CEACAM5 and CEACAM6.

A further embodiment of the invention is a bispecific antibody according to the invention, characterized in not competing with said second bispecific antibody for use in simultaneous, separate, or sequential combination with said second bispecific antibody in the treatment of a subject having a cancer that expresses CEACAM5 or CEACAM5 and CEACAM6.

A further embodiment of the invention is a bispecific antibody according to the invention, characterized in not competing with CEA-TCB or CEA-TCB 1 for use in simultaneous, separate, or sequential combination with said CEA-TCB or CEA-TCB 1 in the treatment of a subject having a cancer that expresses CEACAM5 or CEACAM5 and CEACAM6.

A further embodiment of the invention is a bispecific antibody according to the invention, characterized in competing with CEA-TCB or CEA-TCB1 for use in simultaneous, separate, or sequential combination with said CEA-TCB or CEA-TCB 1 in the treatment of a subject having a cancer that expresses CEACAM5 or CEACAM5 and CEACAM6.

A further embodiment of the invention is a bispecific antibody according to the invention, for use in simultaneous, separate, or sequential combination with a second bispecific antibody comprising a third binding part specifically binding to human CEACAM5, comprising as heavy chain region a heavy chain variable region of SEQ ID NO:98 and as light chain variable region a light chain variable region of SEQ ID NO:99 and a fourth binding part specifically binding to human CD3ε, comprising as heavy chain variable region a heavy chain variable region of SEQ ID NO:100 and as light chain variable region a light chain variable region of SEQ ID NO:101.

A further embodiment of the invention is a bispecific antibody according to the invention, for use according to the invention, characterized in that the bispecific antibody according to the invention and the second bispecific antibody are administered to said subject alternately in 6 to 15 day intervals. A further embodiment of the invention is a bispecific antibody according to the invention, for use according to the invention, characterized in that the bispecific antibody according to the invention and the second bispecific antibody are administered to said subject simultaneously in 6 to 15 day intervals.

A further embodiment of the invention is a first bispecific antibody according to the invention, comprising a first binding part, specifically binding to human CEACAM5 and a second binding part, specifically binding to human CD47, for use in simultaneous, separate, or sequential combination in the treatment of a subject having a cancer that expresses CEACAM5 or CEACAM5 and CEACAM6, with a second bispecific antibody, comprising a third binding part specifically binding to human CEACAM5, comprising as as heavy chain variable region heavy chain variable region of SEQ ID NO:116 and as light chain variable region a light chain variable region of SEQ ID NO:117 and a fourth binding part specifically binding to an epitope of human CD3ε, comprising the amino acid sequence of SEQ ID NO:118, whereby said second bispecific antibody in a concentration of 300 nM does not shift the EC50 value of the phagocytosis index curve to MKN-45 cells of the bispecific antibody according to the invention by more than a factor of 3, in one embodiment towards higher concentrations.

A further embodiment of the invention is a first bispecific antibody according to the invention, comprising a first binding part, specifically binding to human CEACAM5 and a second binding part, specifically binding to human CD47, for use in simultaneous, separate, or sequential combination in the treatment of a subject having a cancer that expresses CEACAM5 or CEACAM5 and CEACAM6, with a second bispecific antibody comprising a third binding part specifically binding to human CEACAM5, comprising as heavy chain variable region a heavy chain variable region of SEQ ID NO:108 and as light chain variable region a light chain variable region of SEQ ID NO:109 and a fourth binding part specifically binding to human CD3ε, comprising as heavy chain variable region a heavy chain variable region of SEQ ID NO:100 and as light chain variable region a light chain variable region of SEQ ID NO:101, whereby said second bispecific antibody in a concentration of 30 nM does not shift the EC50 of the binding curve to MKN-45 cells of the bispecific antibody according to the invention by more than a factor of 3, in one embodiment towards higher concentrations.

A further embodiment of the invention is a first bispecific antibody according to the invention, for use in simultaneous, separate, or sequential combination in the treatment of a subject having a cancer that expresses CEACAM5 or CEACAM5 and CEACAM6, with CEA-TCB or CEA-TCB1, whereby said CEA-TCB in a concentration of 300 nM or CEA-TCB1in a concentration of 30 nM do not shift the EC50 of the binding curve to MKN-45 cells of the bispecific antibody according to the invention by more than a factor of 3, in one embodiment towards higher concentrations.

A further embodiment of the invention is a first bispecific antibody according to the invention, comprising a first binding part, specifically binding to human CEACAM5 and a second binding part, specifically binding to human CD47 according to the invention, for use according to the invention, characterized in that said cancer is colorectal cancer, non-small cell lung cancer (NSCLC), gastric cancer, pancreatic cancer and breast cancer.

A further embodiment of the invention is a composition comprising a bispecific antibody according to the invention, characterized in not competing with said second bispecific antibody as defined above for use in the treatment of a subject having a cancer that expresses CEACAM5 or CEACAM5 and CEACAM6.

A further embodiment of the invention is a composition comprising a bispecific antibody according to the invention, characterized in not competing with a second bispecific antibody comprising a third binding part specifically binding to human CEACAM5, comprising as heavy chain variable region a heavy chain variable region of SEQ ID NO:116 and as light chain variable region a light chain variable region of SEQ ID NO:117 and a fourth binding part specifically binding to an epitope of human CD3ε, comprising the amino acid sequence of SEQ ID NO:118, for use in the treatment of a subject having a cancer that expresses CEACAM5 or CEACAM5 and CEACAM6.

A further embodiment of the invention is a composition comprising a bispecific antibody according to the invention, characterized in not competing with a second bispecific antibody comprising a third binding part specifically binding to human CEACAM5, comprising as heavy chain variable region a heavy chain variable region of SEQ ID NO:108 and as light chain variable region a light chain variable region of SEQ ID NO:109 and a fourth binding part specifically binding to human CD3ε, comprising as heavy chain variable region a heavy chain variable region of SEQ ID NO:100 and as light chain variable region a light chain variable region of SEQ ID NO:101, for use in the treatment of a subject having a cancer that expresses CEACAM5 or CEACAM5 and CEACAM6. A further embodiment of the invention is a composition comprising a bispecific antibody according to the invention, characterized in not competing with CEA-TCB and/or CEA-TCB 1.

A further embodiment of the invention is a method for the treatment of a human patient diagnosed with a tumor (cancer), especially a solid tumor, especially a solid cancer that expresses CEACAM5 or CEACAM5 and CEACAM6, especially colorectal cancer, non-small cell lung cancer (NSCLC), gastric cancer, pancreatic cancer and breast cancer, comprising administering an effective amount of an bispecific antibody according to the invention and a second bispecific antibody as described above, against CEA and CD3 (in one embodiment CEA-TCB or CEA-TCB 1), to the human patient, the method comprising subsequently:

administering to the patient a dose of 0.1 to 10 mg/kg, in a further embodiment of 0.5 to 10 mg/kg, in a further embodiment of 1 to 2 mg/kg of said second anti CEAxCD3 antibody, e.g. weekly over 4 to 12 weeks or q2w, over 4 to 12 weeks and administering after these 4 to 12 weeks and after waiting for additional 2 or 3 or 4 elimination half-lives of said anti CEAxCD3 antibody to the patient a dose of 0.1 to 20 mg/kg of an antibody according to the invention,
administering to the patient said antibody according to the invention q1, q2w, q3w or optionally q4w for e.g. 12 more weeks, waiting 2 or 3 or 4 elimination half-lives of said antibody according to the invention and then optionally repeating said cycle of CEA x CD3 bispecific antibody administration followed by CEA x CD47 bispecific antibody administration and optionally repeat again that cycle etc.

As said CEA x CD3 bispecific antibody and the CEA x CD47 bispecific antibody according to this invention are not competitive, the two bispecific antibodies can also be administered in a manner ("simultaneous manner") that the patient experiences therapeutically effective plasma and tissue concentrations of both bispecific antibodies in parallel, e.g. by administration to the patient at about the same time a dose of 0.1 to 10 mg/kg, in a further embodiment of 0.5 to 10 mg/kg, in a further embodiment of 1 to 2 mg/kg of the CEA x CD3 bispecific antibody and 1 to 20 mg/kg of the CEA x CD47 bispecific antibody of this invention, followed by one or more of these combined administrations at a frequency of q1w or q2w or q3w or optionally q4w.

The term "q1w" means administration once a week; q2w means administration every two weeks etc. For safety reasons it may be needed in one embodiment to start the therapy with the said second antibody CEAxCD3 w/o adding a bsAb of the invention and to start simultaneous administration of the two bsAb only after Cytokine Release Syndrome CRS typical for a CEAxCD3 is over (usually after 2 or 3 doses of a TAAxCD3 antibody).

A further embodiment of the invention is a pharmaceutical composition comprising an antibody according to the invention and a pharmaceutically acceptable excipient or carrier.

A further preferred embodiment of the invention is a pharmaceutical composition comprising an antibody according to the invention for use as a medicament.

A further preferred embodiment of the invention is a pharmaceutical composition comprising an antibody according to the invention for use as a medicament in the treatment of solid tumor disorders. A further preferred embodiment of the invention is a pharmaceutical composition comprising an antibody according to the invention for use as a medicament in the treatment of colorectal cancer, NSCLC (non-small cell lung cancer), gastric cancer, pancreatic cancer or breast cancer.

A further embodiment of the invention is a composition comprising a bispecific antibody according to the invention, for use in simultaneous, separate, or sequential combination in the treatment of a subject having a cancer that expresses CEACAM5 or CEACAM5 and CEACAM6, with a second bispecific antibody, comprising a third binding part specifically binding to human CEACAM5, comprising as heavy chain variable region a heavy chain variable region of SEQ ID NO:116 and as light chain variable region a light chain variable region of SEQ ID NO:117 and a fourth binding part specifically binding to an epitope of human CD3ε, comprising the amino acid sequence of SEQ ID NO:118, whereby said second bispecific antibody in a concentration of 300 nM does not shift the EC50 of the binding curve to MKN-45 cells of the bispecific antibody according to the invention by more than a factor of 3, in one embodiment towards higher concentrations.

A further embodiment of the invention is a composition comprising a bispecific antibody according to the invention, for use in simultaneous, separate, or sequential combination in the treatment of a subject having a cancer that expresses CEACAM5 or CEACAM5 and CEACAM6, with a second bispecific antibody comprising a third binding part specifically binding to human CEACAM5, comprising as heavy chain variable region a heavy chain variable region of SEQ ID NO:108 and as light chain variable region a light chain variable region of SEQ ID NO:109 and a fourth binding part specifically binding to human CD3ε, comprising as heavy chain variable region a heavy chain variable region of SEQ ID NO:100 and as light chain variable region a light chain variable region of SEQ ID NO:101, whereby said second bispecific antibody in a concentration of 30 nM does not shift the EC50 of the binding curve to MKN-45 cells of the bispecific antibody according to the invention by more than a factor of 3, towards higher concentrations.

A further embodiment of the invention is a composition according to the invention, characterized in that the cancer is colorectal cancer, non-small cell lung cancer (NSCLC), gastric cancer, pancreatic cancer, or breast cancer.

A further embodiment of the invention is the use of an antibody according to the invention for the manufacture of a pharmaceutical composition.

A further embodiment of the invention is the use of an antibody according to the invention and a pharmaceutically acceptable excipient or carrier for the manufacture of a pharmaceutical composition.

A further embodiment of the invention is the use of an antibody according to the invention for the manufacture of a medicament in the treatment of solid tumor disorders.

A further embodiment of the invention is the use of an antibody according to the invention in the treatment of colorectal cancer, NSCLC (non-small cell lung cancer), gastric cancer, pancreatic cancer or breast cancer.

Another aspect of the invention provides a method of inducing cell lysis of a tumor cell comprising contacting the tumor cell with the bispecific antibody of any of above described embodiments. In some embodiments, the tumor cell is a colorectal cancer cell, NSCLC (non-small cell lung cancer), gastric cancer cell, pancreatic cancer cell or breast cancer cell.

In one embodiment, the cell lysis is induced by antibody dependent cellular phagocytosis and/or antibody dependent cellular cytotoxicity of the bispecific antibody.

Another aspect of the invention provides a method of treating a subject having a cancer that abnormally expresses CEACAM5 or CEACAM5 and CEACAM6, the method comprising administering to the subject a therapeutically effective amount of the bispecific antibody of any of above described embodiments.

Another aspect of the invention provides a method of treating a subject having a cancer that abnormally expresses CEACAM5 or CEACAM5 and CEACAM6, the method comprising administering to the subject a therapeutically effective amount of the bispecific antibody of any of above described embodiments in combination with a bispecific antibody binding to human CEA and human CD3. As the CEAxCD3 bispecific antibodies and the CEAxCD47 bispecific antibodies according to the invention are not or only minimally competing they can be not only given sequentially but also in parallel (simultaneously) which may well be an advantage because tumor cell killing via engagement of T-cells by the CEAxCD3 bispecific antibody and at the same time via engagement of macrophages by the CEAxCD47 bispecific antibody is additive or may be even synergistic, which means efficacy is increased if both drugs are given in parallel.

Another aspect of the invention provides a method of increasing progression free survival and/or overall survival time in a subject having a cancer that abnormally expresses CEACAM5 or CEACAM5 and CEACAM6, said method comprising administering to said subject a therapeutically effective amount of the bispecific antibody of any of above described embodiments. In one embodiment, the cancer is colorectal cancer, non-small cell lung cancer (NSCLC), gastric cancer, pancreatic cancer or breast cancer or another cancer expressing CEACAM5 or CEACAM5 and CEACAM6.

In certain embodiments of these methods, the bispecific antibody is administered in combination with chemotherapy or radiation therapy. In one embodiment, the subject is a patient suffering from colorectal cancer or lung cancer or gastric cancer or pancreatic cancer or breast cancer or another cancer expressing CEACAM5 or CEACAM5 and CEACAM6.

Another aspect of the invention provides a method of treating a subject having a cancer that abnormally expresses CEACAM5 or CEACAM5 and CEACAM6, the method comprising administering to the subject a therapeutically effective amount of the bispecific antibody of any of above described embodiments in combination with a bispecific antibody against human CEA and human CD3epsilon.

Another aspect of the invention provides a method of increasing progression free survival time and/or overall survival time in a subject having a cancer that abnormally expresses CEACAM5 or CEACAM5 and CEACAM6, said method comprising administering to said subject a therapeutically effective amount of the bispecific antibody of any of above described embodiments. In one embodiment, the cancer is colorectal cancer, non-small cell lung cancer (NSCLC), gastric cancer, pancreatic cancer or breast cancer.

In certain embodiments of these methods, the bispecific antibody is administered in combination with chemotherapy or radiation therapy. In one embodiment, the subject is a cancer patient with colorectal cancer or lung cancer or gastric cancer or pancreatic cancer or breast cancer or another CEACAM5 or CEACAM5 and CEACAM6 expressing cancer.

Another embodiment of the invention provides the use of a bispecific antibody according to the invention for any of the above described methods of treatment. In one embodiment, the cancer is selected from the group consisting of colorectal cancer, non-small cell lung cancer (NSCLC), gastric cancer, pancreatic cancer and breast cancer.

## BRIEF DESCRIPTION OF THE FIGURES

**Fig. 1** shows the general molecular structure of the bispecific antibodies of this invention binding to either CEACAM5 or CEACAM5 and 6 in the first binding part and to CD47 in the second binding part. Figure 1A: non hybrid format with lambda light chain (lambda variable light chain VL(1) and lambda constant light chain CL(1) in the CEACAM binding part) and kappa light chain (kappa variable light chain VK(2) and kappa constant light chain CK(2) in the CD47 binding part). Figure 1B: hybrid format with a hybrid light chain (kappa variable light chain VK(1) and lambda constant light chain CL(1) in the CEACAM binding part) and kappa light chain (kappa variable light chain VK(2) and kappa constant light chain CK(2) in the CD47 binding part). Figure 1C: alternative hybrid format with a kappa light chain (kappa variable light chain VK(1) and kappa constant light chain CK(1) in the CEACAM binding part) and hybrid light chain (kappa variable light chain VK(2) and lambda constant light chain CL(2) in the CD47 binding part) ; the Fc part can be wildtype IgG1 or for increased phagocytosis and ADCC be afucosylated or carry aa mutation(s) or both.

**Fig. 2** shows concentration dependent binding of CD47xCEACAM5 bispecific antibodies of the invention as compared to the corresponding CD47 monovalent anti-CD47 antibody on target cells (MKN-45) expressing CD47 and CEA. For comparison/as a negative control also an irrelevant hIgG1 was tested.

**Fig. 3** shows concentration dependent phagocytosis of MKN-45 wt cells induced by 6 different CD47xCEACAM5 bispecific antibodies of the invention and 2 different CD47xCEACAM5/CEACAM6 bispecific antibodies of the invention (K2AC49 and 50) as compared to the corresponding anti-CD47 monovalent antibody and an irrelevant hIgG1 control.

**Fig. 4** shows concentration dependent binding of CEACAM5/CEACAM6 mAb AC49 of the invention to human CEACAM5 and human CEACAM6 measured by an ELISA based assay as compared to the binding of an irrelevant protein (control).

**Fig. 5** shows concentration dependent binding of CEACAM5/CEACAM6 mAb AC50 of the invention to human CEACAM5 and human CEACAM6 measured by an ELISA based assay as compared to the binding to an irrelevant protein (control).

**Fig. 6** shows concentration dependent binding of the CEACAM5 mAb AC54 of the invention to human CEACAM5 measured by an ELISA based assay as compared to the binding to cynomolgus CEACAM5 and an irrelevant protein (control).

**Fig. 7** shows concentration dependent phagocytosis of MKN-45 wt cells induced by CD47xCEACAM5 bispecific antibodies K2AC21, 54, 58 and 59 as compared to the corresponding anti-CD47 monovalent antibody and an irrelevant hIgG1 control. K2AC21 (resp. AC21) is the parenteral bsAb from which K2AC58 and K2AC59 (resp. the AC CEA anti-CEACAM antibodies) were derived during a lead optimizing (LO) procedure.

## DETAILED DESCRIPTION OF THE INVENTION

Terms are used herein as generally used in the art, unless otherwise defined as follows.

As used herein, the term "antigen binding part, binding part" refers in its broadest sense to a part of an antibody that specifically binds an antigenic determinant such as CEA, CD47 and CD3.

More specifically, as used herein, a binding part that binds membrane-bound human carcinoembryonic antigen (CEA, same as CEACAM5) or to CD47 specifically binds to CEA or CD47, more particularly to cell surface or membrane-bound CEA or CD47. Therefore, each binding part binds either to CEA or CD47. By "specifically binding, specific for, binding to" is meant that the binding is selective for the antigen and can be discriminated from unwanted or nonspecific interactions. In some embodiments, the extent of binding of an anti-target antibody to an unrelated, non-target protein is about 10-fold preferably >1 00-fold less than the binding of the antibody to said target as measured, e.g., by surface plasmon resonance (SPR) e.g. Biacore®, enzyme-linked immunosorbent (ELISA) or flow cytometry (FACS). Targets are the proteins discussed herein - e.g. CEA, CD47, and CD3ε. In one embodiment the CEA binding part binds in addition to CEACAM6. "Specifically binding to CEA, CD47, binding to CEA, CD47, specific for CEA, CD47" refers in one embodiment to an antibody, e.g., bispecific antibody, that is capable of binding to the targets CEA and. CD47 with sufficient affinity such that the antibody is useful as a therapeutic agent in targeting tumor cells expressing CEACAM5 or CEACAM5 and CEACAM6 and CD47. The term "binding to MKN-45 cells with an EC50 value of" refers to an EC50 value measured by FACS/flow cytometry (see example 10a).

In one embodiment the bispecific antibody according to the invention binds to cynomolgus CEACAM5 as well as human CEACAM5. In one embodiment the bispecific antibody is characterized in binding to human CEACAM5 and cynomolgus CEACAM5 with an EC50 ratio of 1:10 to 10:1, in one embodiment 1:5 to 5:1, or in one embodiment 1:3 to 3:1.

As used herein, the term "antibody" refers to an antibody comprising two heavy chains and two light chains. In one embodiment the antibody is a full-length antibody. As used herein, the term "antibody heavy chain" refers to an antibody heavy chain, consisting of a variable region and a constant region as defined for a full-length antibody. As used herein, the term "antibody light chain" refers to an antibody light chain, consisting of a variable region and a constant region as defined for a full-length antibody.

The term "full-length antibody" denotes an antibody consisting of two "full-length antibody heavy chains" and two "full-length antibody light chains". A "full-length antibody heavy chain" is a polypeptide consisting in N-terminal to C- terminal direction of an antibody heavy chain variable domain (VH), an antibody constant heavy chain domain 1 (CH1), an antibody hinge region (HR), an antibody heavy chain constant domain 2 (CH2), and an antibody heavy chain constant domain 3 (CH3), abbreviated as VH-CH-HR-CH2-CH3. A "full-length antibody light chain" is a polypeptide consisting in N-terminal to C- terminal direction of an antibody light chain variable domain (VL), and an antibody light chain constant domain (CL), abbreviated as VL-CL. The antibody light chain constant domain (CL) can be κ (kappa) or λ (lambda). The two full-length antibody domains are linked together via inter-polypeptide disulphide bonds between the CL domain and the CH1 domain and between the hinge regions of the full-length antibody heavy chains. Examples of typical full-length antibodies are natural antibodies like IgG (e.g. IgG 1 and IgG2), IgM, IgA, IgD, and IgE. The full-length antibody according to the invention is in one embodiment of human IgG1 type, in one further embodiment comprising one or more amino acid substitutions in the Fc part as defined below and/or being glycoengineered at polysaccharide chain attached to Asn297. The full-length antibody according to the invention comprise two binding parts each formed by a pair of VH and VL, one binding to CEA and the other binding to CD47.

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). HVRs are non-contiguous antigen combining sites (also known as antigen binding regions). Generally, antibodies comprise six HVRs: three in the VH (HVRH1, HVRHH2, HVRH3), and three in the VL (HVRL1, HVRL2, HVRL3). Exemplary HVRs herein include:

(a) CDRs occurring at amino acid residues 24-34 (HVRL1), 50-56 (HVRL2), 89-97 (HVRL3), 31-35b (HVRH1), 50-65 (HVRH2), and 95-102 (HVRH3) (Kabat et al., Sequences of Proteins of Immunological Interest, 5th Ed. Public Health Service, National Institutes of Health, Bethesda, MD (1991); which is incorporated herein by reference);

(b) hypervariable loops occurring at amino acid residues 26-32 (HVRL1), 50-52 (HVRL2), 91-96 (HVRL3), 26-32 (HVRH1), 53-55 (HVRH2), and 96-101 (HVRH3) (Chothia and Lesk, J. Mol. Biol. 196:901-917 (1987); incorporated herein by reference);

(c) antigen contacts occurring at amino acid residues 27-36 (HVRL1), 46-55 (HVRL2), 89-96 (HVRL3), 30-35b (HVRH1), 47-58 (HVRH2), and 93-101 (HVRH3) (MacCallum et al. J. Mol. Biol. 262: 732-745 (1996); incorporated herein by reference); and (d) combinations of (a), (b), and/or (c), including HVR amino acid residues 46- 56 (HVRL2), 47-56 (HVRL2), 48-56 (HVRL2), 49-56 (HVRL2), 26-35 (HVRH1), 26-35b (HVRH1), 49- 65 (HVRH2), 93-102 (HVRH3), and 94-102 (HVRH3).

As used herein and mentioned above, "Complementarity determining region(s)" ("CDR") describe the non-contiguous antigen combining sites (also known as antigen binding regions) found within the variable region of both heavy and light chain polypeptides. CDRs are also referred to as "hypervariable regions (HVRs)" and that term is used interchangeably herein with the term "CDR" in reference to the portions of the variable region that form the antigen binding regions. This particular region has been described by (Kabat et al., supra,). The appropriate amino acid residues which encompass the CDRs as defined by Kabat are set forth below in the sequence list table. The exact residue numbers which encompass a particular CDR will vary depending on the sequence and size of the CDR. Those skilled in the art can routinely determine which residues comprise a particular CDR given the variable region amino acid sequence of the antibody. As used herein the term "comprising a CDRL1 of SEQ ID NO:x" refers to that the CDRL1 region of the referred variable light chain is of SEQ ID NO:x (comprising as CDRL1 a CDRL1 of SEQ ID NO:x). This is true also for the other CDRs.

Unless otherwise indicated, HVR residues are numbered herein according to Kabat et al., supra and named as "CDRs".

The term "percent (%) amino acid sequence identity" as used herein is defined as the percentage of amino acid residues in an antibody sequence that are identical with the amino acid residues in the reference antibody sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, Clustal W, Megalign (DNASTAR) software or the FASTA program package. Those skilled in the art can determine appropriate parameters for aligning sequences, including any algorithms needed to achieve maximal alignment over the full length of the sequences being compared. For purposes herein, however, % amino acid sequence identity values are generated using the ggsearch program of the FASTA package version 36.3.8c or later with a BLOSUM50 comparison matrix. The FASTA program package was authored by W. R. Pearson and D. J. Lipman (1988), "Improved Tools for Biological Sequence Analysis", PNAS 85:2444-2448; W. R. Pearson (1996) "Effective protein sequence comparison" Meth. Enzymol. 266:227-258; and Pearson et. al. (1997) Genomics 46:24-36 and is publicly available from http://fasta.bioch.virginia.edu/fasta_www2/fasta_down.shtml. Alternatively, a public server accessible at http://fasta.bioch.virginia.edu/fasta_www2/index.cgi can be used to compare the sequences, using the ggsearch (global protein:protein) program and default options (BLOSUM50; open: -10; ext: -2; Ktup = 2) to ensure a global, rather than local, alignment is performed. Percent amino acid identity is given in the output alignment header.

As used herein, the term "90%, 91%, 92%, 93%, 94%, 95%, 96%, 97%, 98%, 99%, or 100% identity" as used in an embodiment of the invention means that in this embodiment all such sequences are either of 90% identity, 91% identity, 92% identity, 93% identity, 94% identity, 95% identity, 96% identity, 97% identity, 98% identity, 99% identity, or 100% identity (fully identical).

As used herein, the term "Fc region; Fc domain" refers to a C-terminal region of an IgG heavy chain; in case of an IgG1 antibody, the C-terminal region comprises -CH2-CH3 (see above). Although the boundaries of the Fc region of an IgG heavy chain might vary slightly, the human IgG heavy chain Fc region is usually defined to stretch from the amino acid residue at position Cys226 to the carboxyl-terminus.

Constant regions are well known in the state of the art and e.g. described by Kabat, E.A., (see e.g. Johnson, G., and Wu, T.T., Nucleic Acids Res.28 (2000) 214-218; Kabat, E.A., et al, Proc. Natl. Acad. Sci. USA 72 (1975) 2785-2788).

The term "epitope" includes any polypeptide determinant capable of specific binding to an antibody. In certain embodiments, "epitope" includes chemically active surface groupings of molecules such as amino acids, sugar side chains, phosphoryl, or sulfonyl, and, in certain embodiments, may have specific three dimensional structural characteristics, and or specific charge characteristics. An epitope is a region of a target that is bound by an antibody. In one embodiment the bispecific antibody of the invention binds to the N-terminal domain of CEACAM5 (Ig-like V-type domain of amino acids 35 - 144, UniProtKB - P06731). Binding location of the CEAxCD47 bispecific antibodies to CEACAM5 is achieved via epitope binning. In epitope binning, antibodies are tested in a pairwise combinatorial manner, and antibodies that compete for the same binding region are grouped together into bins. Competition testing is performed herein with anti-CEA antibodies according to the state of the art and as described herein. In one embodiment the bispecific antibody of the invention competes for binding to CEACAM5 with reference antibody SM3E (bin 1). In one embodiment the bispecific antibody of the invention does not compete for binding to CEACAM5 with reference antibodies SAR (bin 2). Competition is measured by an assay wherein biotinylated human CEACAM5 in a concentration of 0.5 μg/ml is immobilized and incubated with 10 μg/ml of the reference. CEACAM5 antibodies comprising the CEACAM5 binding part of the CEAxCD47 bispecific antibody of the present invention are added at 0.2 μg/ml for 1 hour at room temperature. The plate is washed and the bound CEACAM5 mAbs are detected.

As used herein, the term "a common heavy chain (cHC)" refers to a polypeptide consisting in N-terminal to C-terminal direction of an antibody heavy chain variable domain (VH), an antibody constant heavy chain domain 1 (CH1), an antibody hinge region (HR), an antibody heavy chain constant domain 2 (CH2), and an antibody heavy chain constant domain 3 (CH3), abbreviated as VH-CH-HR-CH2-CH3. Common heavy chains suitable for the bispecific antibodies according to the invention are heavy chains of an anti-CD47 antibody as described in WO2012023053, WO2013088259, WO2014087248, and WO2016156537 (each of which is incorporated by reference in its entirety). In one embodiment common heavy chain of the bispecific antibody according to the invention comprises as heavy chain CDRs a CDRH1 of SEQ ID NO:1, a CDRH2 of SEQ ID NO:2 and a CDRH3 of SEQ ID NO:3. In one embodiment the cHC of the bispecific antibody according to the invention comprises as heavy chain variable region VH a VH region of SEQ ID NO:4. In one embodiment the common heavy chain cHC of the bispecific antibody according to the invention is of SEQ ID NO:5 (VH-CH1). In one embodiment the common heavy chain cHC of the bispecific antibody according to the invention is of SEQ ID NO:6. SEQ ID NO:6, is a heavy chain comprising in addition an IgG1 Fc part. In one embodiment the antibody according to the invention is a κλ bispecific antibody comprising a cHC (κλ Body).

"The κλ Body format allows the affinity purification of bispecific antibodies which are undistinguishable from a standard IgG molecule and with characteristics that are undistinguishable from a standard monoclonal antibody (see e.g. WO2013088259, WO2012023053), promising no or low immunogenicity potential in patients.

Bispecific antibodies of the invention, comprising a common heavy chain, can be made for example according to WO2012023053 (incorporated by reference in its entirety). The methods described in WO2012023053 generate bispecific antibodies that are identical in structure to a human immunoglobulin. This type of molecule is composed of two copies of a unique heavy chain polypeptide, a first light chain variable region fused to a constant Kappa domain and second light chain variable region fused to a constant Lambda domain. One binding site displays specificity to CEA and the other site displays specificity to CD47, wherein to each the heavy and the respective light chain contribute. The light chain variable regions can be of the Lambda or Kappa family and are preferably fused to a Lambda and Kappa constant domains, respectively. This is preferred in order to avoid the generation of non-natural polypeptide junctions. However, it is also possible to obtain bispecific antibodies of the invention by fusing a Kappa light chain variable domain to a constant Lambda domain for a first specificity or fusing a Lambda light chain variable domain to a constant Kappa domain for the second specificity. The other light chain is then always fully kappa (VL and CL) or fully lambda. The bispecific antibodies described in WO 2012023053 are "κλ Bodies". This κλ-Body format allows the affinity purification of a bispecific antibody that is undistinguishable from a standard IgG molecule with characteristics that are undistinguishable from a standard monoclonal antibody and, therefore, favourable as compared to previous formats including e.g. amino acid bridges or other unnatural elements. The formats of the bispecific antibodies of the invention are shown in table 1A and figures 1A, B, C).

**Table 1A (see also fig.IA, B, C; K2AC54, K2AC41, AC41K2 H-CL1 can be taken as exemplary formats)**

| CEACAM CD47 bsAb | CEACAM variable light chain | CEACAM constant light chain | CD47 variable light chain | CD47 constant light chain | Non-hybrid format | Hybrid format in binding part |
|---|---|---|---|---|---|---|
| K2AC41 | kappa | lambda | kappa | kappa | | CEACAM5 |
| AC41K2 H-CL1 | kappa | kappa | kappa | lambda | | CD47 |
| K2AC42 | kappa | lambda | kappa | kappa | | CEACAM5 |
| AC42K2 H-CL1 | kappa | kappa | kappa | lambda | | CD47 |
| K2AC43 | kappa | lambda | kappa | kappa | | CEACAM5 |

(continued)

| CEACAM CD47 bsAb | CEACAM variable light chain | CEACAM constant light chain | CD47 variable light chain | CD47 constant light chain | Non-hybrid format | Hybrid format in binding part |
|---|---|---|---|---|---|---|
| AC43K2 H-CL1 | kappa | kappa | kappa | lambda | | CD47 |
| K2AC44 | lambda | lambda | kappa | kappa | X | |
| K2AC45 | lambda | lambda | kappa | kappa | X | |
| K2AC46 | lambda | lambda | kappa | kappa | X | |
| K2AC47 | lambda | lambda | kappa | kappa | X | |
| K2AC48 | lambda | lambda | kappa | kappa | X | |
| K2AC49 | lambda | lambda | kappa | kappa | X | |
| K2AC50 | lambda | lambda | kappa | kappa | X | |
| K2AC52 | lambda | lambda | kappa | kappa | X | |
| K2AC53 | lambda | lambda | kappa | kappa | X | |
| K2AC54 | lambda | lambda | kappa | kappa | X | |
| K2AC55 | lambda | lambda | kappa | kappa | X | |
| K2AC56 | lambda | lambda | kappa | kappa | X | |
| K2AC57 | lambda | lambda | kappa | kappa | X | |
| K2AC60 | kappa | lambda | kappa | kappa | | CEACAM5 |
| AC60K2 H-CL1 | kappa | kappa | kappa | lambda | | CD47 |
| K2AC61 | kappa | lambda | kappa | kappa | | CEACAM5 |
| AC61K2 H-CL1 | kappa | kappa | kappa | lambda | | CD47 |
| K2AC62 | kappa | lambda | kappa | kappa | | CEACAM5 |
| AC62K2 H-CL1 | kappa | kappa | kappa | lambda | | CD47 |
| K2AC63 | kappa | lambda | kappa | kappa | | CEACAM5 |
| AC63K2 H-CL1 | kappa | kappa | kappa | lambda | | CD47 |
| K2AC64 | kappa | lambda | kappa | kappa | | CEACAM5 |
| AC64K2 H-CL1 | kappa | kappa | kappa | lambda | | CD47 |
| K2AC65 | kappa | lambda | kappa | kappa | | CEACAM5 |
| AC65K2 H-CL1 | kappa | kappa | kappa | lambda | | CD47 |
| K2AC66 | kappa | lambda | kappa | kappa | | CEACAM5 |
| AC66K2 H-CL1 | kappa | kappa | kappa | lambda | | CD47 |

The bispecific antibodies according to the invention bind all to bin 1, i.e. distal of the cell membrane. Bispecific antibodies K2AC21 and the derivatives K2AC58 and K2AC59 found by a LO procedure (see Example 5) applied to AC21, are competing with SAR and therefore binding to bin 2, i.e. proximal to the cell membrane. The formats of these bispecific antibodies are shown in table 1B for comparison.

**Table 1B**

| **CEACAM CD47 bsAb** | **CEACAM variable light chain** | **CEACAM constant light chain** | **CD47 variable light chain** | **CD47 constant light chain** |
|---|---|---|---|---|
| K2AC58 | lambda | lambda | kappa | kappa |
| K2AC59 | lambda | lambda | kappa | kappa |
| K2AC21 | lambda | lambda | kappa | kappa |

K2AC21 comprises in the first binding part as light chain a light chain having of SEQ ID NO:185, and comprises as a CDRL1 of SEQ ID NO:182, CDRL2 of SEQ ID NO:183, CDRL3 of SEQ ID NO:184, in the second binding part as light chain a light chain of SEQ ID NO:11 and as heavy chain a common heavy chain of SEQ ID NO:4.

K2AC58 comprises in the first binding part as light chain a light chain having of SEQ ID NO:93, and comprises as a CDRL1 of SEQ ID NO:65, CDRL2 of SEQ ID NO:66, CDRL3 of SEQ ID NO:67, in the second binding part as light chain a light chain of SEQ ID NO:11 and as heavy chain a common heavy chain of SEQ ID NO:4.

K2AC59 comprises in the first binding part as light chain a light chain having of SEQ ID NO:94, and comprises as a CDRL1 of SEQ ID NO:68, CDRL2 of SEQ ID NO:69, CDRL3 of SEQ ID NO:70, in the second binding part as light chain a light chain of SEQ ID NO:11 and as heavy chain a common heavy chain of SEQ ID NO:4.

Phagocytosis achieved with K2AC21, K2AC58 and K2AC59 is surprisingly strongly inferior to phagocytosis achieved with e.g. reference biAb K2AC54 or other antibodies of the invention binding to bin 1 (see e.g. figure 7 and 3). Binding closer to the membrane seems to be a disadvantage compared to binding distal to the membrane.

In one embodiment, the term "K2ACxx" refers to a bispecific CEACAM5xCD47 antibody according to the invention as defined by the light and heavy chain CDRs. In one embodiment, the term "K2ACxx" refers to a bispecific CEACAM5xCD47 antibody according to the invention as defined by the light and heavy chain CDRs and light and chains which are 90%, 91%, 92%, 93%, 94%, 95%, 96%, 97%, 98%, 99%, or 100% identical to the respective light chains. In one embodiment, the term "K2ACxx" refers to a bispecific CEACAM5xCD47 antibody according to the invention as defined by the light and heavy chain CDRs and the respective light chains. In one embodiment, the term "K2ACxx" refers to a bispecific CEACAM5xCD47 antibody according to the invention as defined by the light and heavy chain CDRs, and the respective light and heavy chains. The respective combinations of CDRs, light and heavy chains can be seen from tables 1 and 2.

As used herein, the term "CEA, CEACAM5" refers to human carcinoembryonic antigen (CEA, CEACAM-5 or CD66e; UniProtKB - P06731) which is a cell surface glycoprotein and a tumor-associated antigen (Gold and Freedman, J Exp. Med., 121:439-462, 1965; Berinstein NL, J Clin Oncol., 20:2197-2207, 2002). As used herein, the term "CEACAM6" refers to human CEACAM6 (CD66c; UniProtKB - P40199), which is also a member of the carcinoembryonic antigen-related cell adhesion molecule (CEACAM) family. The term "cynomolgus CEACAM5" relates to macaca fascicularis CEACAM5 (NCBI Reference Sequence: XP_005589491.1). As used herein, the term "CEACAM1" refers to human CEACAM1 (UniProtKB - P13688 (CEAM1_HUMAN) which is also a member of the carcinoembryonic antigen-related cell adhesion molecule (CEACAM) family. Further information and information on other members of the CEA family can be found under http://www.uniprot.org.

In one embodiment the bispecific antibody according to the invention is not competitive with CEA-TCB, or CEA-TCB1. MAB CEA and said). A bispecific anti-CEA x anti-CD3ε antibody (CEA-TCB) is described in Bacac et al Clin. Cancer Res., 22(13), 3286-97 (2016) and the variable chainsof CEA-TCB are described in US20140242079 (SEQ ID NO:21 and 27 of US20140242079 (incorporated by reference in its entirety). A further bispecific CEAxCD3 Mab (CEA-TCB1) is described in WO2017055389 as molecule B "2+1 IgG CrossFab, inverted" with charge modifications (VH/VL exchange in CD3 binder, charge modification in CEA binder, humanized CEA binder) (see SEQ ID NOs 34, 36-38 of WO2017055389 (incorporated by reference in its entirety)). As used herein in one embodiment "bispecific CEA x CD3 antibody" refers to antibody CEA-TCB or antibody CEA-TCB 1.

"As used herein, the terms "specifically binding to CD47, binding to CD47, CD47 binding part" refer in the context of the bispecific antibodies according to the invention to specificity for human CD47. Human CD47 is a multi-pass membrane protein and comprises three extracellular domains (amino acids 19-141, 198-207, and 257-268; see UniProtKB - Q08722). As used herein the term "binding affinity to CD47" is measured quantitatively by SPR.

In one embodiment binding of the bispecific antibody according to the invention to CD47 occurs via one or more of said extracellular domains. In one embodiment, the bispecific antibodies according to the invention inhibit the interaction between human CD47 and human SIRPα.

In one embodiment the second binding part of the antibody according to the invention (specifically binding to human CD47) is characterized by a light chain comprising as light chain CDRs a CDRL1 of SEQ ID NO:7, a CDRL2 of SEQ ID NO:8, and a CDRL3 of SEQ ID NO:9 , and a heavy chain comprising as heavy chain CDRs a CDRL1 of SEQ ID NO:1, a CDRL2 of SEQ ID NO:2, and a CDRL3 of SEQ ID NO:3. In one embodiment the second binding part of the antibody according to the invention (specifically binding to human CD47) is characterized by a kappa light chain variable region of SEQ ID NO:10. In one embodiment the second binding part of the antibody according to the invention (specifically binding to human CD47) is characterized by a kappa light chain of SEQ ID NO:11. In one embodiment the second binding part of the antibody according to the invention (specifically binding to human CD47) is characterized by a kappa/lambda light chain of SEQ ID NO:181. In one embodiment the second binding part of the antibody according to the invention (specifically binding to human CD47) is characterized by a heavy chain variable region of SEQ ID NO:4. In one embodiment the second binding part of the antibody according to the invention (specifically binding to human CD47) is characterized by a heavy chain of SEQ ID NO:5. In one embodiment the second binding part of the antibody according to the invention (specifically binding to human CD47) is characterized by a heavy chain of SEQ ID NO:6.

As used herein, the terms "specifically binding to CEA, binding to CEA, CEA binding part" refer in the context of the bispecific antibodies according to the invention to specificity for CEACAM5 on the surface of a cell. Binding to CEA (CEACAM5) on cells is preferably measured with gastric adenocarcinoma MKN-45 cells comprising approximately 150.000 CEA copies per cell. The concentration of the antibody according to the invention is varied in an appropriate range in regard to a resulting EC50 value for binding to MKN-45 cells as defined above.

As used herein, the term "membrane-bound human CEA" refers to human carcinoembryonic antigen (CEA) that is bound to a membrane-portion of a cell or to the surface of a cell, in particular, the surface of a tumor cell. The term "membrane-bound human CEA" may, in certain circumstances, refer to CEA which is not bound to the membrane of a cell, but which has been constructed so as to preserve the membrane bound CEA epitope to which the antibody according to the invention binds. As used herein, the terms "cross-reactivity against CEACAM6, specifically binding to CEACAM6, binding to CEACAM6, CEACAM6 binding part" refer in the context of the bispecific antibodies according to the invention that the bispecific antibody according to the invention recognizes specifically CEACAM5 and CEACAM6 on the surface (membrane) of a cell. In one embodiment the bispecific antibodies according to the invention are specifically binding to membrane-bound CEACAM6, when compared to binding to membrane-bound CEA. The ratio of the occupancy of CEACAM5 to CEACAM6 receptors on a cell surface by a given bispecific antibody of the invention is dependent on the binding affinities to CEACAM5 respectively CEACAM6 and can be easily calculated if these binding affinities have been measured, e.g. by SPR.

In certain embodiments, an antibody that specifically binds to CEACAM5 does not bind to carcinoembryonic antigen-related cell adhesion proteins such as, CEACAM1 (UniProtKB - P13688), CEACAM3 (UniProtKB - P40198), CEACAM4 (UniProtKB - 075871), CEACAM7 (UniProtKB - Q14002) and CEACAM8 (CD67 antigen, NCA 95, CD66b, UniProtKB - P31997). In one embodiment, the AC CEA antibody according to the invention specifically binds to human CEACAM5 and specifically to cynomolgus CEACAM5. In one embodiment, the AC CEA antibody according to the invention specifically binds to human CEACAM5 and cynomolgus CEACAM5 and binds also specifically to human CEACAM6 at similar EC50 as to human CEACAM5 (balanced binding). In one embodiment, the bispecific antibody according to the invention specifically binds to human CEACAM5 and specifically to cynomolgus CEACAM5. In one embodiment, the bispecific antibody according to the invention specifically binds to human CEACAM5 and cynomolgus CEACAM5 binds also specifically to human CEACAM6 at similar EC50as to human CEACAM5 (balanced binding).

As used herein, the terms "no substantial cross-reactivity against CEACAM1 and/ or CEACAM3, CEACAM4, CEACAM6, CEACAM7 and CEACAM8, non-binding to said CEACAM" refer in the context of the bispecific antibodies according to the invention that such antibodies do not show any relevant binding to said membrane-bound CEACAM at therapeutic plasma concentrations (1 to 1000 nM), when compared to membrane-bound CEACAM5. Non-binding to CEACAM1 and/ or CEACAM3, CEACAM4, CEACAM6 and/or CEACAM8 or to other CEACAM family members can be determined by flow cytometry based measurement of the binding curve to recombinant CHO or PEAK cells expressing said CEACAM or measured by an ELISA based assay measuring the binding to the recombinant CEACAM proteins. The term "not binding to" means in one embodiment that in an ELISA based binding assay (example 8f) to the respective protein a concentration dependent binding curve cannot be established due to very weak or no binding, or the respective binding curve is close to a control. In another embodiment EC50 for binding for such non-binders is 100 or more times higher than EC50 for binding to human CEACAM5. OD values for such non-binders will be usually about equal to that of the limit of detection and/or close to control (human IgG). As used herein, the terms "does not bind, no binding to" for a compound mentioned herein (e.g. human IgG or CEACAM family unrelated tumor-associated proteins, e.g. HER3), refer also to such non-relevant binding or non crossreactivity; e.g. in an ELISA based assay CEACAM family members are e.g. discussed in Kuespert K Current opinion in Cell Biol 18 (2006) 565-571.

As used herein, the term "bispecific antibody binding to human CEA and human CD3, CEAxCD3 Mab" means a bispecific antibody binding to human CEACAM5 and CD3ε. Such antibodies are for example "CEA-TCB" and "CEA-TCB1". As used herein "CEA-TCB "refers to a bispecific antibody binding to CEA and CD3 as described in US20140242079 (incorporated by reference in its entirety) as SEQ ID NO:1, 2, 21, and 22. The amino acid sequences of CEA-TCB are also described as SEQ ID NO:106 to 109 of the present invention. As used herein "CEA-TCB1" refers to molecule B in the "2+1 IgG CrossFab, inverted" format with charge modifications (VH/VL exchange in CD3 binder, charge modification in CEA binder, humanized CEA binder); SEQ ID NOs 34, 36-38 of WO2017055389 (incorporated by reference in its entirety)). The amino acid sequences of CEA-TCB1 are described as SEQ ID NO:102 to 105 of the present invention. Further CEAxCD3 Mabs are described in WO2007071426, WO2013012414, WO2015112534, WO2017118675, US20140242079 and WO2017055389 (each of which is incorporated by reference in its entirety). A further CEAxCD3 Mab is RO6958688 (see e.g. Bacac et al Clin. Cancer Res., 22(13), 3286-97 (2016). In one embodiment said CEAxCD3 Mab according to the invention is not competitive and/or does not bind to the same epitope of human CEACAM5 as CEA-TCB or CEA-TCB1.

As used herein "CD3 Mab, antibody against CD3" refers to human CD3ε (UniProtKB - P07766 (CD3EHUMAN). The term "antibody against CD3ε, anti CD3ε antibody" relates to an antibody specifically binding to CD3ε. In one embodiment, the antibody against CD3ε is specifically binding to the same epitope as anti-CD3 antibody SP34 (BD Biosciences Catalog No.565983). In one embodiment, the antibody against CD3ε is specifically binding to an epitope of human CD3ε, comprising the amino acid sequence of SEQ ID NO:118. In one embodiment, the antibody against CD3ε is specifically binding to human CD3ε and comprises a heavy chain variable region of SEQ ID NO:100 and a light chain variable region of SEQ ID NO:101.

In one embodiment the bispecific antibody of the invention does not compete with CEA-TCB and/or CEA-TCB1 for binding on CEA as presented on MKN-45 cells. Therefore CEA-TCB in a concentration of 300 nM (CEA-TCB) or 30 nM (CEA-TCB1) do not shift the EC50 of the phagocytosis index curve of said the bispecific antibody of the invention for MKN-45 cells by more than a factor of 3, in one embodiment towards higher concentrations.

300 nM are a concentration measured in patient plasma at therapeutically effective doses of CEA-TCB ((J.Tabernero et.al., J. Clin. Oncol. 35, 2017 (suppl. Abstr. 3002)). CEA-TCB1 is in preclinical investigations approx. 10 to 100 times more potent than CEA-TCB (binding affinity, tumor cell lysis, WO2017055389), therefore the shift of the EC50 is tested at 30 nM.

Competition in binding can be determined by flow cytometry based measurement of the binding curve to MKN-45 cells and determination of the EC50 of this binding curve. Non-competition means that EC50 is shifted by less than a factor of 3, in one embodiment to towards higher concentrations, if 300 nM of CEA-TCB are added to the assay. 300 nM are a concentration in the range of therapeutically active doses/plasma-concentrations of CEA x CD3 bispecific antibody (CEA-TCB) (J.Tabernero et.al., J. Clin. Oncol. 35, 2017 (suppl. Abstr. 3002)). Non-competition by CEA-TCB1 means that EC50 is shifted by less than a factor of 3 if 30 nM of CEA-TCB 1 are added to the assay.

Competition in binding can be determined by flow cytometry based measurement of the binding curve to MKN-45 cells and determination of the EC50 of this binding curve. Non-competition means that EC50 is changed by less than a factor of 3 if 300 nM of CEA-TCB are added to the assay. 300 nM are a concentration in the range of therapeutically active doses/plasma-concentrations of CEA x CD3 bispecific antibody (CEA-TCB) (J.Tabernero et.al., J. Clin. Oncol. 35, 2017 (suppl. Abstr. 3002)).

Non-competition by CEA-TCB1 means that EC50 is changed by less than a factor of 3 if 30 nM of CEA-TCB1 are added to the assay.

As used herein, the term "not competitive" means that a second antibody (bispecific antibody against CEAxCD3ε, like CEA-TCB or CEA-TCB1) in a concentration of 300 nM (CEA-TCB) or 30 nM (CEA-TCB1) does not shift the EC50 of the binding curve of the bispecific antibody of the invention to MKN-45 cells by more than a factor of 3, in one embodiment towards higher concentrations.

As used herein the term "complementarity determining region" ("CDR") describes the non-contiguous antigen combining sites (also known as antigen binding regions) found within the variable region of both heavy and light chain polypeptides. CDRs are also referred to as "hypervariable regions" and that term is used interchangeably herein with the term "CDR" in reference to the portions of the variable region that form the antigen binding regions. This particular region has been described by Kabat et al., U.S. Dept. of Health and Human Services, "Sequences of Proteins of Immunological Interest" (1983) and by Chothia et al., J. Mol. Biol. 196:901-917 (1987). Kabat et al. also defined a numbering system for variable domain sequences that is applicable to any antibody. One of ordinary skill in the art can unambiguously assign this system of "Kabat numbering" to any variable domain sequence, without reliance on any experimental data beyond the sequence itself. As used herein, "Kabat numbering" refers to the numbering system set forth by Kabat et al., U.S. Dept. of Health and Human Services, "Sequence of Proteins of Immunological Interest" (1983). Unless otherwise specified, references to the numbering of specific amino acid residue positions in bispecific antibody according to the invention are according to the Kabat numbering system.

As used herein the term "ADCP" refers to antibody-dependent cell-mediated phagocytosis.

As used herein "phagocytosis, EC50 value of phagocytosis, maximum of phagocytosis, phagocytosis index" according to the invention refer to phagocytosis measured with MKN-45 cells by "imaging". An appropriate imaging method, with incubation at an effector (macrophages):target (tumor) cell ratio of e.g. 1:1 or 1:3 and with the "phagocytosis index" as readout (Imaging determined ADCP") is described in Example 9. As used herein "phagocytosis of said bispecific antibody" means phagocytosis caused/induced by said antibody.

Antibody K2AC49 comprises a first binding part, specifically binding to human CEACAM5 and human CEACAM6 and a second binding part, specifically binding to human CD47, whereby the first binding part comprises as heavy chain a heavy chain of SEQ ID NO:5 and as light chain a light chain of SEQ ID NO:85, and that the second binding part comprises as heavy chain a heavy chain of SEQ ID NO:5 and as light chain a light chain of SEQ ID NO:11. In one embodiment antibody K2AC49 comprises as common heavy chain a heavy chain of SEQ ID NO:6. Antibody K2AC50 comprises a first binding part, specifically binding to human CEACAM5 and human CEACAM6 and a second binding part, specifically binding to human CD47, whereby the first binding part comprises as heavy chain a heavy chain of SEQ ID NO:5 and as light chain a light chain of SEQ ID NO:86, and that the second binding part comprises as heavy chain a heavy chain of SEQ ID NO:5 and as light chain a light chain of SEQ ID NO:11. In one embodiment antibody K2AC50 comprises as common heavy chain a heavy chain of SEQ ID NO:6. Antibody K2AC54 comprises a first binding part, specifically binding to human CEACAM5 and a second binding part, specifically binding to human CD47, whereby the first binding part comprises as heavy chain a heavy chain of SEQ ID NO:5 and as light chain a light chain of SEQ ID NO:89, and that the second binding part comprises a heavy chain of SEQ ID NO:5 and as light chain a light chain of SEQ ID NO:11. In one embodiment antibody K2AC54 comprises as common heavy chain a heavy chain of SEQ ID NO:6. Antibody K2AC53 comprises a first binding part, specifically binding to human CEACAM5 and a second binding part, specifically binding to human CD47, whereby the first binding part comprises as heavy chain a heavy chain of SEQ ID NO:5 and as light chain a light chain of SEQ ID NO:88, and that the second binding part comprises as heavy chain a heavy chain of SEQ ID NO:5 and as light chain a light chain of SEQ ID NO:11. Bispecific antibodies K2AC21, K2AC41 to K2AC66 comprise in the second binding part the same heavy chain (SEQ ID NO:5 or 6) and light chain (SEQ ID NO:11), but differ in the first binding part light chain (see table 2, sequence list, Seq ID NO: 185, SEQ ID NO:74 to 95, SEQ ID NO: 167 to 173). For further information on phagocytosis in the field, phagocytosis can also be measured by a flow cytometry based method as % phagocytosis and at a ratio of e.g. 3 human macrophages to 1 target/tumor-cell ("flow cytometry determined ADCP").

The terms "human IgG, hIgG" refers to a commercially available clinical-grade homogeneous preparation of human immunoglobulin IgG (e.g. from company Bio-rad.com) that does not bind specifically to CD47 and CEACAM5.

Antibodies produced in CHO cells typically have complex biantennary structures with very low or no bisecting-N-acetylglucosamine (bisecting GlcNAc) and high levels of core fucosylation. Overexpression of N-acetylglucosaminyl-transferase III has been used to increase the fraction of bisecting GlcNAc that resides on antibodies to improve antibody-dependent cellular cytotoxicity (ADCC). RNAi and gene deletion technologies have also been used to decrease or eliminate the fucose on antibodies to dramatically increase ADCC activity (Davis J. et al.; Biotechnol. Bioeng. 2001;74:288-294; Saba JA, et al.; Anal. Biochem. 2002;305:16-31; Kanda Y, et al.; J. Biotechnol. 2007;130:300-310; Mori K, et al.; Biotechnol. Bioeng. 2004;88:901-908).

In one embodiment, the bispecific antibody according to the invention is glycoengineered. In one embodiment the glycoengineered bispecific antibody according to the invention has increased ADCC and/or ADCP activity (decreased EC50 and/or higher maximum of phagocytosis index) compared to the bispecific antibody comprising an Fc part included in SEQ ID NO:6 (parent antibody), comprising glycosylation according to a production in a CHO K1 cell line (ATCC® CCL-61™) at standard conditions (1000ml vessel, temperature 37°C, pH 7.0, impeller speed 80 rpm, minimum dissolved oxygen 30%; cultivation time 14 days).

In a more particular embodiment, the increase in ADCC (decrease of EC50 and/or increase of maximum) is by a factor of 1.2 to 2.0 or even at least 2.0 as compared to said parent.

In a more particular embodiment, the increase in ADCP (decrease of EC50 of the phagocytosis index curve) is by a factor of at least 3 or even 5 or more as compared to said parent.

As used herein, the term "polypeptide having GnTIII activity, GnTIII" refers to polypeptides that are able to catalyze the addition of a N-acetylglucosamine (GlcNAc) residue in β-1-4 linkage to the β-linked mannoside of the trimannosyl core of N-linked oligosaccharides, eg. β-1,4-mannosyl-glycoprotein4-β-N-acetylglucosaminyl-transferase (EC 2.4.1.144).

As used herein the term "FUT8" refers to α1,6-fucosyltransferase (EC:2.4.1.68).

As used herein, the term "effector function, Fc-mediated cellular cytotoxicity" refers to those biological activities attributable to the Fc region (a native sequence Fc region or amino acid sequence variant Fc region) of an antibody. Examples of antibody effector functions include, but are not limited to, Fc receptor binding affinity, antibody-dependent cellular cytotoxicity (ADCC), antibody-dependent cellular phagocytosis (ADCP), cytokine secretion, immune-complex-mediated antigen uptake by antigen-presenting cells, down-regulation of cell surface receptors, etc. Such immune mechanism is leading to the lysis of "targeted cells" by "human immune effector cells."

As used herein, the term "GDP-6-deoxy-D-lyxo-4-hexulose reductase (RMD)" refers to an enzyme (UniProtKB - F0J3S3) which uses GDP-6-deoxy-D-lyxo4-hexulose as a substrate. GDP-6-deoxy-D-lyxo-4-hexulose reductase (RMD) reduces the substrate GDP-6-deoxy-D-lyxo-4-hexulose to GDP-D-rhamnose. GDP-D-Rhamnose is a nucleotide sugar donor for D-rhamnosylation in bacteria and does not occur in vertebrates. Vertebrate cells also lack specific rhamnosyltransferases so that GDP-D-Rhamnose cannot be incorporated into nascent glycostructures of glycoproteins or glycolipids within vertebrate cells (see US8642292, incorporated herein by reference).

As used herein, the term "glycoengineered antibody" refers to a bispecific antibody according to the invention which comprises a reduced amount of fucosylated and/or bisecting oligosaccharides attached to the Fc region of said antibody, usually at amino acid Asn297, compared to a parent antibody.

As used herein, the term "inhibiting fucose synthesis" refers to a method blocking the de novo synthesis of fucose in the host cell by use of RMD and - in one embodiment - culturing in addition the host cell in fucose free medium as described in US8642292.

As used herein, the term "parent antibody, parent bispecific antibody" in the context of glycoengineering refers to a bispecific antibody according to the invention which comprises the same amino acid composition as the glycoengineered antibody but is non-glycoengineered.

For such comparison the parent antibody and the glycoengineered antibody are produced in the same host cell, but in the first case in the host cell without glycoengineering, and in the second case in the same host cell but engineered

a) by integration of a GDP-6-deoxy-D-lyxo-4-hexulose reductase into the host cell (a eukaryotic cell, e.g. a CHO cell line), inhibiting therefore the de novo synthesis of fucose from D-mannose, in one embodiment in addition by production cells cultivation in fucose free medium, or
b) by targeted disruption of the FUT8 gene or
c) eby expressing a polynucleotide encoding a polypeptide having GnTIII activity under standard conditions (see above).

As used herein, the term "human immune effector cells" refers to a population of leukocytes that display Fc receptors on their surfaces, through which they bind to the Fc-region of antigen binding molecules or of Fc-fusion proteins and perform effector functions. Such a population may include, but is not limited to, peripheral blood mononuclear cells (PBMC) and/or natural killer (NK) cells and/or macrophages.

As used herein, the term "increased Fc-mediated cellular cytotoxicity" is defined as either an increase in the number of "targeted cells" that are lysed in a given time, at a given concentration of the bispecific antibody of the invention in the medium surrounding the target cells, by the mechanism of Fc-mediated cellular cytotoxicity defined above, and/or a reduction in the concentration of the bispecific antibody of the invention, in the medium surrounding the target cells, required to achieve the lysis of a given number of "targeted cells" in a given time, by the mechanism of Fc-mediated cellular cytotoxicity. The increase in Fc-mediated cellular cytotoxicity is relative to the cellular cytotoxicity mediated by the same bispecific antibody of the invention produced by the same type of host cells, using the same standard conditions, but that has not been produced by host cells engineered to have an altered pattern of glycosylation (e.g., by inhibiting fucose synthesis, by expressing glycosyltransferase, GnTIII, or other glycosyltransferases or FUT8 disruption) by the methods described herein.

As used herein the term "expression vector" refers to one or more vectors which comprise the heavy and light chains of the antibody according to the invention in an appropriate manner as known from the state of the art.

As used herein "host cells engineered by targeted disruption of the FUT8 gene" refers to host cells capable of expressing an antibody according to the invention and being in addition glycoengineered by targeted disruption of the FUT8 gene as described e.g. in US8067232, US7425446, US6946292 (each of which is incorporated by reference in its entirety), and Yamane-Ohnuki N. et al., Biotech. Bioeng.; 87 (2004) 614-622. An antibody according to the invention expressed in such host cell comprises a Fc region comprising complex N-glycoside-linked sugar chains bound to the Fc region, which comprise a reducing end which contains an N-acetylglucosamine, wherein the sugar chains do not contain fucose bound to the 6 position of N-acetylglucosamine in the reducing end of the sugar chains.

The present invention is further directed to a method for the production of a bispecific antibody according to the present invention characterized in comprising nonfucosylation of 50% to 100%, 60% to 100%, 70% to 100%, 80% to 100%, or 90% to 100%, that are produced by a host cell, comprising expressing in said host cell a nucleic acid encoding a bispecific antibody of the invention and a nucleic acid encoding a polypeptide with a glycosyltransferase activity, or a vector comprising such nucleic acids. Genes with glycosyltransferase activity include (1,4)-N-acetylglucosaminyltransferase III (GnTIII), α-mannosidase II (ManII), (1,4)-galactosyltransferase (GalT), (1,2)-N-acetylglucosaminyltransferase I (GnTI), and β(1,2)-N-acetylglucosaminyltransferase II (GnTII). In one embodiment, a combination of genes with glycosyltransferase activity is expressed in the host cell (e.g., GnTIII and Man II). Likewise, the method also encompasses expression of one or more polynucleotide(s) encoding the bispecific antibody in a host cell in which a glycosyltransferase gene has been disrupted or otherwise deactivated (e.g., a host cell in which the activity of the gene encoding al-6 core fucosyltransferase has been knocked out). In another embodiment, the bispecific antibodies of the present invention can be produced in a host cell that further expresses a polynucleotide encoding a polypeptide having GnTIII activity to modify the glycosylation pattern. In a specific embodiment, the polypeptide having GnTIII activity is a fusion polypeptide comprising the Golgi localization domain of a Golgi resident polypeptide. In another preferred embodiment, the expression of the bispecific antibodies of the present invention in a host cell that expresses a polynucleotide encoding a polypeptide having GnTIII activity results in bispecific antibodies with increased Fc receptor binding affinity and increased effector function. The present invention is further directed to a method for the production of a bispecific antibody according to the present invention characterized in comprising non-fucosylation of 50% to 100%, 60% to 100%, 70% to 100%, 80% to 100%, or 90% to 100%, that are produced by a host cell, comprising expressing in said host cell a nucleic acid encoding a bispecific antibody of the invention and a disrupted FUT8 gene.

The present invention is further directed to a method for the production of a bispecific antibody according to the present invention characterized in comprising non-fucosylation of 80% to 100%, or 90% to 100%, that are produced by a host cell, comprising expressing in said host cell a nucleic acid encoding a bispecific antibody of the invention by inhibiting fucose synthesis in said host cell.

In one embodiment the bispecific antibodies with altered glycosylation produced by the host cells of the invention exhibit increased Fc receptor binding affinity and/or increased effector function as a result of the modification of the host cell (e.g., by inhibiting fucose synthesis in said host cell or by expression of a glycosyltransferase gene). Preferably, the increased Fc receptor binding affinity is increased binding to a Fcγ activating receptor, such as the FcγRIIIa receptor.

In one embodiment, the percentage of nonfucosylated oligosaccharides is 50% to 100%, specifically 60% to 100%, 70% to 100%, and more specifically, 80% to 100%. The nonfucosylated oligosaccharides may be of the hybrid or complex type. In yet another embodiment, the bispecific antibody produced by the methods of the invention has an increased proportion of bisected oligosaccharides in the Fc region as a result of the modification of its oligosaccharides by the methods of the present invention. In one embodiment, the percentage of bisected oligosaccharides is 50% to 100%, specifically 50%, 60% to 70%, and more specifically, 80%. In a particularly preferred embodiment, the bispecific antibody produced by the host cells and methods of the invention has an increased proportion of bisected, nonfucosylated oligosaccharides in the Fc region. The bisected, nonfucosylated oligosaccharides may be either hybrid or complex.

As used herein, the term "host cell" covers any kind of cellular system which can be engineered to generate the bispecific antibodies of the present invention. In one embodiment, the host cell is engineered to allow the production of an antigen binding molecule with modified glycoforms. In certain embodiments, the host cells have been further manipulated to express increased levels of one or more polypeptides having GnTIII activity. Host cells include cultured cells, e.g., mammalian cultured cells, such as CHO cells (see above), BHK cells, NSO cells, SP2/0 cells, YO myeloma cells, P3X63 mouse myeloma cells, PER cells, PER.C6 cells or hybridoma cells, yeast cells, insect cells, and plant cells, to name only a few, but also cells comprised within a transgenic animal, transgenic plant or cultured plant or animal tissue. Host cells for the production of glycoengineered bispecific antibodies of the present invention have been described e.g. in US6602684, US20040241817, US20030175884; and WO 2004065540. The bispecific antibodies of the present invention can alternatively be glycoengineered to have reduced fucose residues in the Fc region according to the techniques disclosed in US2003/0157108, EP1176195, WO2003084570, WO2003085119 and US2003/0115614, US2004/093621, US2004/110282, US2004/110704, US2004/132140 (each of which is incorporated by reference in its entirety). Glycoengineered bispecific antibodies of the invention may also be produced in expression systems that produce modified glycoproteins, such as those described in WO2003/056914, WO2004/057002, and WO2004/024927(each of which is incorporated by reference in its entirety).

In a further embodiment of the invention the antibody according to the invention comprises one or two or three amino acid substitutions in the Fc region ("Fc amino acid substitution") selected from the group consisting of mono-substitutions S239D, I332E, G236A, of bi-substitutions I332E and G236A, S239D and I332E ("DE substitution"), S239D and G236A, and triple-substitution S329D and I332E and G236A ("DEA substitution"); (Richards JO, et al., Mol. Cancer Ther. 7 (2008) 2517-2527). Due to different counting of a heavy chain, these amino acid numbers can be different for +/one, two or three amino acids, but with the same shift for all three. In case of the heavy chain of SEQ ID NO:6 there is a one amino acid shift and S329D and I332E and G236A therefore denotes S328D and I331E and G235A. ADCC and/or ADCP activity of the bispecific antibody can be increased by such amino acid modification of the Fc part.

As used herein, the term "parent antibody, parent bispecific antibody" in the context of Fc substitution refers to a bispecific antibody according to the invention which comprises the same amino acid composition as the Fc substituted antibody, but without said substitution(s). For such comparison the parent antibody and the Fc substituted antibody are produced - as in the case of glycoengineered antibodies - in the same host cell under the same conditions, but in the first case in the host cell without Fc substitution, and in the second case in the same host cell but with such Fc substitution(s). A useful host cell line is e.g. CHO-K1.

As used herein, the term "parent antibody, parent bispecific antibody" in the context of a bispecific antibody according to the invention which comprises Fc substitution and is glycoengineered, such parent antibody therefore is the respective bispecific antibody which comprises the same amino acid composition as the Fc substituted antibody, but without said substitution(s) and is not glycoengineered.

In a further embodiment of the invention ADCC and/or ADCP activity of the bispecific antibody is increased by amino acid substitution of the Fc part in combination with glycoengineering of the Fc part compared ADCC and/or ADCP activity of the respective parent antibody.

The invention comprises therefore in one embodiment a bispecific antibody specifically binding to human CEACAM5 and human CD47, characterized in comprising one or two or three amino acid substitutions in the Fc region ("Fc amino acid substitution") selected from the group consisting of mono-substitutions S239D, I332E, G236A, of bi-substitutions I332E and G236A, S239D and I332E, of triple-substitutions S329D and I332E and G236A and comprising non-fucosylation of the Fc part of 50% to 100%, 60% to 100%, 70% to 100%, 80% to 100%, or 90% to 100%.

Example 9 describes assays used for the determination of ADCC activity and also of ADCP activity ADCC can be measured by an in vitro ADCC assay as follows:

1) the assay uses target cells that are known to express CEA recognized by the CEA-binding region of the bispecific antibody;
2) the assay uses human peripheral blood mononuclear cells (PBMCs), isolated from blood of a randomly chosen healthy donor, as effector cells;
3) the assay is carried out according to following protocol:

   i) the PBMCs are isolated using standard density centrifugation procedures and are suspended at 6.25 x $10^6$ cells/ml in RPMI cell culture medium;
   ii) the target cells are grown by standard tissue culture methods, harvested from the exponential growth phase with a viability higher than 90%, washed in RPMI cell culture medium, labelled with 100 micro-Curies of 51Cr for 1 x $10^6$ cells, washed twice with cell culture medium, and resuspended in cell culture medium at a density of 0.25 x $10^6$ cells/ml;
   iii) 20 microliters of the final target cell suspension above are transferred to each well of a 96-well microtiter plate;
   iv) the bispecific antibody is serially-diluted from 4000 ng/ml to 0.12ng/ml in cell culture medium and 20 microliters of the resulting antibody solutions are added to the target cells in the 96-well microtiter plate, testing in triplicate various antibody concentrations covering the whole concentration range above;
   v) for the maximum release (MR) controls, 3 additional wells in the plate containing the labelled target cells, receive 50 microliters of a 5% (V/V) aqueous solution of non-ionic detergent (Triton, Sigma, St. Louis), instead of the bispecific antibody solution (point iv above);
   vi) for the spontaneous release (SR) controls, 3 additional wells in the plate containing the labelled target cells, receive 20 microliters of RPMI cell culture medium instead of the bispecific antibody solution (point iv above);
   vii) the 96-well microtiter plate is then centrifuged at 50 x g for 1 minute and incubated for 1 hour at 4°C;
   viii) 40 microliters of the PBMC suspension (point i above) are added to each well to yield an effector:target (E:T) cell ratio of 50:1 and the plates are placed in an incubator under 5% $CO_2$ atmosphere at 37°C for 4 hours;
   ix) the cell-free supernatant from each well is harvested and the experimentally released radioactivity (ER) is quantified using a gamma counter;
   x) the percentage of specific lysis is calculated for each bispecific antibody concentration according to the formula (ER-MR)/(MR-SR) x 100, where ER is the average radioactivity quantified (see point ix above) for that antibody concentration, MR is the average radioactivity quantified (see point ix above) for the MR controls (see point v above), and SR is the average radioactivity quantified (see point ix above) for the SR controls (see point vi above);

As used herein "increased ADCC" is defined as either an increase in the maximum percentage of specific lysis observed within the bispecific antibody concentration range tested above, and/or a reduction in the concentration of bispecific antibody required to achieve one half of the maximum percentage of specific lysis (EC50) observed within the bispecific antibody concentration range tested above. The increase in ADCC is relative to the ADCC, measured with the above assay, mediated by the same bispecific antibody, produced by the same type of host cells, using the same standard production, purification, formulation and storage methods, but that has not been produced by host cells engineered a) to inhibit fucose synthesis in said host cell, b) to overexpress GnTIII or c) by host cells engineered by targeted disruption of the FUT8 gene ("parent antibody"). In case of amino acid substitutions in the Fc, the increase in ADCC is relative to the ADCC measured with the parent bispecific antibody not carrying the substitution(s). In case of a bispecific antibody comprising amino acid substitutions in the Fc part and being glycoengineered, the increase in ADCC is relative to the ADCC measured with the parent non glycoengineered, bispecific antibody not carrying the substitution(s).

*Therapeutic Applications and Methods of Using Anti-CEA Antigen Binding Molecules*

The CEACAM x CD47 bispecific antibodies according to the invention are optimized for treatment of solid tumors mainly by macrophages mediated phagocytosis of the tumor cells, either in monotherapy or in combination therapy especially together with a CEAxCD3 T-cell bispecific antibody like CEA-TCB or CEA-TCB1 and/or PD-1 axis antagonist. The antibody according to the invention and the CEAxCD3 T-cell bispecific antibody can be administered as described below.

In a particular embodiment, the disease resp. solid tumor is a cancer that expresses or even overexpresses CEACAM5 or CEACAM5 and CEACAM6, including but not limited to the group of colorectal tumors, non-small cell lung tumors, gastric tumors, pancreatic tumors and breast tumors. In a particular embodiment, the tumor is a colorectal tumor. In a particular embodiment the tumor is a gastric tumor. In a particular embodiment the tumor is a gastric tumor expressing CEACAM5 or CEACAM5 and CEACAM6 and HER-2. All therapeutic applications methods of use, uses, combinations, etc. described herein are especially embodiments for the treatment of these tumors/diseases.

The inventors recognize that the antibodies according to the invention show low or no ADA formation potential respectively loss of exposure due to neutralizing ADA respectively loss of efficacy.

In one embodiment, the invention provides a method of treating carcinomas (cancer, tumors, for example, human carcinomas), especially CEACAM5 or CEACAM5 and CEACAM6 expressing tumors, in vivo. This method comprises administering to a subject a pharmaceutically effective amount of a composition containing a bispecific antibody of the invention. By "subject" is meant a human subject, in one embodiment a patient suffering from cancer/tumor/carcinoma.

CEACAM5 or CEACAM5 and CEACAM6 expression in various tumor entities is generally very high, especially in colorectal carcinoma, pancreatic adenocarcinoma, gastric cancer, non-small cell lung cancer, breast cancer, head and neck carcinoma, uterine and bladder cancers among others. In healthy, normal glandular epithelia in the gastrointestinal tract, CEACAM5 or CEACAM5 and CEACAM6 is mainly expressed in a polarized pattern on the apical surface of the cells. This polarized expression pattern limits the accessibility by anti-CEA mono or bispecific antibodies which are administered systemically and therefore potential toxicity. Together with the low affinity CD47 binding of the antibody of the invention this leads to no or limited phagocytosis of such normal cells by the antibody of the invention. This polarized expression pattern gets lost in the cells of gastrointestinal and other malignant tumors. CEACAM5 or CEACAM5 and CEACAM6 is expressed equally over the whole cell surface of the cancer cells that means cancer cells are much better accessible to an antibody of the invention than normal, healthy cells and can be selectively killed by the CEAxCD47 bispecific antibodies of the invention respectively by the combinations mentioned above.

In one embodiment the bispecific antibodies of this invention can be used in monotherapy for the treatment of advanced solid tumors, in one embodiment CEACAM5 or CEACAM5 and CEACAM6 expressing tumors. In one embodiment a bispecific antibody according to the invention is used in combination with a CEAxCD3 Mab in simultaneous, separate, or sequential combination. In one embodiment a bispecific antibody according to the invention is used in combination with a CEAxCD3 Mab and/or a PD-1 axis antagonist in simultaneous, separate, or sequential combination. In one embodiment a bispecific antibody according to the invention is used in combination with a PD-1 axis antagonist in simultaneous, separate, or sequential combination. Such PD-1 axis antagonists are described e.g. in WO2017118675. Such combinations attack the solid cancer by macrophages and T-cells. Two CEAxCD3 Mabs are in clinical development (CEA-TCB and CEA-TCB1; see clinicaltrials.gov; RO6958688 in NCT3866239 and RO7172508 in NCT03539484). MEDI-565 was in clinical development but no active clinical trial could be identified in clinicaltrials.gov. In one embodiment as bispecific antibody against CEA and CD3, antibody CEA-TCB or CEA-TCB 1 is used.

The binder to CEA used in CEA-TCB has been derived from anti-CEA antibody PR1A3 (see e.g. EP2681244B1). This antibody binds to the so called B3 domain of CEA. CEA-TCB has a low nM binding affinity to CEA and shows efficacy in high doses (between 40 and 600 mg per dose and patient; (see e.g. J.Tabernero et.al., J. Clin. Oncol. 35, 2017 (suppl. Abstr. 3002)). At highest doses nearly all CEA targets on the cell surfaces are occupied by the CEA-TCB. Combination of CEA-TCB or CEA-TCB 1 and CEAxCD47 generates therapeutic plasma levels of both drugs at the same time and achieves best results (additive or even synergistic), if both drugs are non-competitive for the CEA antigen.

As used herein the terms "combination, simultaneous, separate, or sequential combination" of a an antibody according to the invention and a second bispecific antibody, binding to human CEA and human CD3ε refer to any administration of the two antibodies (or three antibodies in case of the combination of an antibody of the invention, a CEAxCD3 Mab and a PD-1 axis antagonist), either separately or together, where the two or three antibodies are administered as part of an appropriate dose regimen designed to obtain the benefit of the combination therapy, for example in separate, sequential, simultaneous, concurrent, chronologically staggered or alternating administration. Thus, the two or three antibodies can be administered either as part of the same pharmaceutical composition or in separate pharmaceutical compositions. The antibody according to the invention can be administered prior to, at the same time as, or subsequent to the administration of the second bispecific antibody, or in some combination thereof. Where the antibody according to the invention is administered to the patient at repeated intervals, e.g., during a standard course of treatment, the second bispecific antibody can be administered prior to, at the same time as, or subsequent to, each administration of the antibody of the invention or some combination thereof, or at different intervals in relation to the treatment with the antibody of the invention, or in a single dose prior to, at any time during, or subsequent to the course of treatment with the antibody of the invention. In one embodiment the antibody according to the invention and the second bispecific antibody are administered in alternating administration, in one embodiment in intervals of 6 to 15 days between administration of the antibody of the invention and the second antibody. In such alternating administration the first dose can be the antibody of the invention or the second antibody.

The term "PD-1 axis antagonist" refers to an anti-PD-1 antibody or an anti-PD-Ll antibody. Anti-PD-1 antibodies are e.g. pembrolizumab (Keytruda®, MK-3475), nivolumab, pidilizumab, lambrolizumab, MEDI-0680, PDR001, and REGN2810. Anti-PD-1 antibodies are described e.g. in 5 WO200815671, WO2013173223, WO2015026634, US7521051, US8008449, US8354509, WO20091 14335, WO2015026634, WO2008156712, WO2015026634, WO2003099196, WO2009101611, WO2010/027423, WO2010/027827, WO2010/027828, WO2008/156712, and WO2008/156712 (each of which is incorporated by reference in its entirety).

Anti-PD-Ll antibodies are e.g. atezolizumab, MDX-1 105, durvalumab and avelumab. Anti-PD-Ll antibodies are e.g. described in WO2015026634, WO2013/019906, WO2010077634, US8383796, WO2010077634, WO2007005874, and WO2016007235 (each of which is incorporated by reference in its entirety).

With regard to combined administration of the antibody according to the invention and the second bispecific antibody, both compounds may be present in one single dosage form or in separate dosage forms, for example in two different or identical dosage forms.

If the antibody of the invention and the second antibody are not competing in regard to CEACAM5, in one embodiment both antibodies if desired by the physician, can be administered simultaneously. If the antibody of the invention and the second antibody are competing in regard to CEACAM5, in one embodiment both antibodies are administered in alternating administration.

The antibody of the invention will typically be administered to the patient in a dose regimen that provides for the most effective treatment of the cancer (from both efficacy and safety perspectives) for which the patient is being treated, as known in the art. Preferably tumor cells are attacked at the same time by T-cells and macrophages, to achieve full therapeutic potential of this approach, CEA-CD3 and CEAxCD47 bispecific antibody have to be non-competitive regarding binding to CEA on cell surface.

As discussed above, the amount of the antibody administered and the timing of the administration of the antibody of the invention can depend on the type (e.g. gender, age, weight) and condition of the patient being treated, the severity of the disease or condition being treated, and on the route of administration. For example, the antibody of the invention and the second antibody can be administered to a patient in doses ranging from 0.1 to 100 mg/kg of body weight per day or per week in single or divided doses, or by continuous infusion. In one embodiment each of the antibodies of the invention and the second antibody is administered to a patient in doses ranging from 0.1 to 20 mg/kg. In some instances, dosage levels below the lower limit of the aforesaid range may be adequate, while in other cases still larger doses may be employed without causing any harmful side effect.

As used herein, the term "half-life of the antibody" refers to the half-life of said antibody as measured in a usual pharmacokinetic assay, e.g. as described in example 17. An antibody according to the invention and the second bispecific antibody against CEA and CD3 have elimination half-life of 3-14 days.

In another aspect, the invention is also directed to use of the bispecific antibody according to the invention in the treatment of disease, particularly cell proliferation disorders wherein CEACAM5 or CEACAM5 and CEACAM6 is expressed, particularly wherein CEACAM5 or CEACAM5 and CEACAM6 is abnormally expressed (e.g., overexpressed or expressed in a different pattern on the cell surface) compared to normal tissue of the same cell type. Such disorders include, but are not limited to colorectal cancer, NSCLC (non-small cell lung cancer), gastric cancer, pancreatic cancer and breast cancer. CEACAM5 or CEACAM5 and CEACAM6 expression levels may be determined by methods known in the art (e.g., via immunohistochemistry assay, immunofluorescence assay, immunoenzyme assay, ELISA, flow cytometry, radioimmunoassay etc.).

In one aspect, bispecific antibodies of the present invention can be used for targeting cells in vivo or in vitro that expresses CEACAM5 or CEACAM5 and CEACAM6. The bispecific antibodies of the invention are particularly useful in the prevention of tumor formation, eradication of tumors and inhibition of tumor growth or metastasis via the induction of ADCP and ADCC of tumor cells. The bispecific antibodies of the invention can be used to treat any tumor expressing CEACAM5 or CEACAM5 and CEACAM6 A. Particular malignancies that can be treated with the bispecific antibodies of the invention include, but are not limited to, colorectal cancer, non- small cell lung cancer, gastric cancer, pancreatic cancer and breast cancer.

The bispecific antibodies of the invention are administered to a mammal, preferably a human, in a pharmaceutically acceptable dosage form such as those discussed below, including those that may be administered to a human intravenously as a bolus or by continuous infusion over a period of time, by intramuscular, intraperitoneal, intra-cerebrospinal, subcutaneous, intra-articular, intrasynovial, intrathecal, oral, topical, or inhalation routes. The bispecific antibodies of the invention also are suitably administered by intra tumoral, peritumoral, intralesional, or perilesional routes, to exert local as well as systemic therapeutic effects.

For the treatment of disease, the appropriate dosage of bispecific antibodies of the invention will depend on the type of disease to be treated, the severity and course of the disease, previous therapy, the patient’s clinical history and response to the antibody, and the discretion of the attending physician. The bispecific antibody of the invention is suitably administered to the patient at one time or over a series of treatments. The present invention provides a method for selectively killing tumor cells expressing CEACAM5 or CEACAM5 and CEACAM6.

This method comprises interaction of the bispecific antibodies of the invention with said tumor cells. These tumor cells may be from a human carcinoma including colorectal carcinoma, non-small cell lung carcinoma (NSCLC), gastric carcinoma, pancreatic carcinoma and breast carcinoma.

In another aspect, the invention is directed to the use of the bispecific antibodies of the invention for the manufacture of a medicament for treating a disease related to abnormal CEACAM5 or CEACAM5 and CEACAM6 expression. In a particular embodiment, the disease is a cancer that expresses or even overexpresses CEACAM5 or CEACAM5 and CEACAM6, including but not limited to colorectal tumor, non-small cell lung tumor, gastric tumor, pancreatic tumor and breast tumor. In a particular embodiment, the tumor is a colorectal tumor.

*Compositions, Formulations, Dosages, and Routes of Administration*

In one aspect, the present invention is directed to pharmaceutical compositions comprising the bispecific antibodies of the present invention and a pharmaceutically acceptable carrier. The present invention is further directed to the use of such pharmaceutical compositions in the method of treatment of disease, such as cancer, or in the manufacture of a medicament for the treatment of disease, such as cancer. Specifically, the present invention is directed to a method for the treatment of disease, and more particularly, for the treatment of cancer, the method comprising administering a therapeutically effective amount of the pharmaceutical composition of the invention.

In one aspect, the present invention encompasses pharmaceutical compositions, combinations and methods for treating human carcinomas, tumors, as defined above. For example, the invention includes pharmaceutical compositions for use in the treatment of human carcinomas comprising a pharmaceutically effective amount of an antibody of the present invention and a pharmaceutically acceptable carrier.

The bispecific antibody compositions of the invention can be administered using conventional modes of administration including, but not limited to, intravenous, intraperitoneal, oral, intralymphatic or direct intratumoral administration. Intravenous administration or subcutaneous administration are preferred.

In one aspect of the invention, therapeutic formulations containing the bispecific antibodies of the invention are prepared for storage by mixing an antibody having the desired degree of purity with optional pharmaceutically acceptable carriers, excipients or stabilizers (Remington's Pharmaceutical Sciences 16th edition, Osol, A. Ed. (1980)), in the form of lyophilized formulations or liquid formulations. Acceptable carriers, excipients, or stabilizers are nontoxic to recipients at the dosages and concentrations employed. The formulations to be used for in vivo administration must be sterile. This is readily accomplished by filtration through sterile filtration membranes. The most effective mode of administration and dosage regimen for the pharmaceutical compositions of this invention depends upon the severity and course of the disease, the patient's condition and response to treatment and the judgment of the treating physician. Accordingly, the dosages of the compositions may be flat doses or may be adapted to the individual patient, e.g. the body weight. Nevertheless, an effective dose of the compositions of this invention will generally be in a range from 0.1 to 20 mg/kg.

The bispecific antibodies of this invention have a molecular weight in a magnitude of 150kD per Mol. They carry in one embodiment a Fc part. The elimination half-life in patients is in a range of 3 to 14 days. This half-life allows for, but not limited to administration once a day, once a week, or once every two weeks.

The bispecific antibodies of the present invention and their respective compositions may be in a variety of dosage forms which include, but are not limited to, liquid solutions or suspensions, tablets, pills, powders, suppositories, polymeric microcapsules or microvesicles, liposomes, and injectable or infusible solutions. The preferred form depends upon the mode of administration and the therapeutic application.

The composition comprising a bispecific antibody of the present invention will be formulated, dosed, and administered in a fashion consistent with good medical practice. Factors for consideration in this context include the particular disease or disorder being treated, the particular mammal being treated, the clinic condition of the individual patient, the cause of the disease or disorder, the site of delivery of the agent, the method of administration, the scheduling of administration, and other factors known to medical practitioners.

*Articles of Manufacture*

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is a bispecific antibody of the invention. The label or package insert indicates that the composition is used for treating the condition of choice. Moreover, the article of manufacture may comprise (a) a first container with a composition contained therein, wherein the composition comprises a bispecific antibody of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

**FURTHER EMBODIMENTS OF THE INVENTION**

1. A bispecific antibody comprising a first binding part specifically binding to human CEACAM5 (further named also as "CEA") and a second binding part specifically binding to human CD47 (further named also as "CD47") characterized in that:

   a) the first binding part comprises as heavy chain variable region a heavy chain variable region comprising a CDRH1 of SEQ ID NO:1, a CDRH2 of SEQ ID NO:2 and a CDRH3 of SEQ ID NO:3,
   b) the first binding part comprises as light chain variable region a light chain variable region comprising a CDRL set selected from the group consisting of

      b1) a CDRL1 of SEQ ID NO:17, CDRL2 of SEQ ID NO:18, and CDRL3 of SEQ ID NO:19,
      b2) a CDRL1 of SEQ ID NO:20, CDRL2 of SEQ ID NO:21, and CDRL3 of SEQ ID NO:22,
      b3) a CDRL1 of SEQ ID NO:23, CDRL2 of SEQ ID NO:24, and CDRL3 of SEQ ID NO:25,
      b4) a CDRL1 of SEQ ID NO:26, CDRL2 of SEQ ID NO:27, and CDRL3 of SEQ ID NO:28,
      b5) a CDRL1 of SEQ ID NO:29, CDRL2 of SEQ ID NO:30, and CDRL3 of SEQ ID NO:31,
      b6) a CDRL1 of SEQ ID NO:32, CDRL2 of SEQ ID NO:33, and CDRL3 of SEQ ID NO:34,
      b7) a CDRL1 of SEQ ID NO:35, CDRL2 of SEQ ID NO:36, and CDRL3 of SEQ ID NO:37,
      b8) a CDRL1 of SEQ ID NO:38, CDRL2 of SEQ ID NO:39, and CDRL3 of SEQ ID NO:40,
      b9) a CDRL1 of SEQ ID NO:41, CDRL2 of SEQ ID NO:42, and CDRL3 of SEQ ID NO:43,
      b10) a CDRL1 of SEQ ID NO:44, CDRL2 of SEQ ID NO:45, and CDRL3 of SEQ ID NO:46,
      b11) a CDRL1 of SEQ ID NO:47, CDRL2 of SEQ ID NO:48, and CDRL3 of SEQ ID NO:49,
      b12) a CDRL1 of SEQ ID NO:50, CDRL2 of SEQ ID NO:51, and CDRL3 of SEQ ID NO:52,
      b13) a CDRL1 of SEQ ID NO:53, CDRL2 of SEQ ID NO:54, and CDRL3 of SEQ ID NO:55,
      b14) a CDRL1 of SEQ ID NO:56, CDRL2 of SEQ ID NO:57, and CDRL3 of SEQ ID NO:58,
      b15) a CDRL1 of SEQ ID NO:59, CDRL2 of SEQ ID NO:60, and CDRL3 of SEQ ID NO:61,
      b16) a CDRL1 of SEQ ID NO:62, CDRL2 of SEQ ID NO:63, and CDRL3 of SEQ ID NO:64,
      b17) a CDRL1 of SEQ ID NO:71, CDRL2 of SEQ ID NO:72, and CDRL3 of SEQ ID NO:73,
      b18) a CDRL1 of SEQ ID NO:142, CDRL2 of SEQ ID NO:143, and CDRL3 of SEQ ID NO:144,
      b19) a CDRL1 of SEQ ID NO:145, CDRL2 of SEQ ID NO:146, and CDRL3 of SEQ ID NO:147,
      b20) a CDRL1 of SEQ ID NO:148, CDRL2 of SEQ ID NO:149, and CDRL3 of SEQ ID NO:150,
      b21) a CDRL1 of SEQ ID NO:151, CDRL2 of SEQ ID NO:152, and CDRL3 of SEQ ID NO:153,
      b22) a CDRL1 of SEQ ID NO:154, CDRL2 of SEQ ID NO:155, and CDRL3 of SEQ ID NO:156, and
      b23) a CDRL1 of SEQ ID NO:157, CDRL2 of SEQ ID NO:158, and CDRL3 of SEQ ID NO:159, and

   c) the second binding part comprises as heavy chain variable region a heavy chain variable region comprising a CDRH1 of SEQ ID NO:1, a CDRH2 of SEQ ID NO:2 and a CDRH3 of SEQ ID NO:3, and as light chain variable region a light chain variable region comprising a CDRL1 of SEQ ID NO:7, a CDRL2 of SEQ ID NO:8, and a CDRL3 of SEQ ID NO:9.

2. The bispecific antibody according to embodiment 1, characterized in comprising a first binding part specific for CEA, comprising a lambda light chain variable domain and a lambda light chain constant domain and a second binding part specific for CD47, comprising a kappa light chain variable domain and a kappa light chain constant domain.

3. The bispecific antibody according to embodiment 1, characterized in comprising a first binding part specifically binding to CEA, comprising a kappa light chain variable domain and a lambda light chain constant domain (hybrid light chain) and a second binding part specifically binding to CD47, comprising a kappa light chain variable domain and a kappa light chain constant domain.

4. The bispecific antibody according to embodiment 1, characterized in comprising a first binding part specifically binding to CEA, comprising a kappa light chain variable domain and a kappa light chain constant domain and a second binding part specifically binding to CD47, comprising a kappa light chain variable domain and a lambda light chain constant domain (hybrid light chain).

5. The bispecific antibody according to embodiment 1, characterized in comprising

a) in the first binding part as heavy chain region a heavy chain region of SEQ ID NO:5, comprising a CDRH1 of SEQ ID NO:1, a CDRH2 of SEQ ID NO:2, and a CDRH3 of SEQ ID NO:3, and
b) as light chain a light chain selected from the group consisting of

b1) a light chain of SEQ ID NO:77, and comprising
a CDRL1 of SEQ ID NO:17, CDRL2 of SEQ ID NO:18, and CDRL3 of SEQ ID NO:19,
b2) a light chain of SEQ ID NO:78, and comprising a CDRL1 of SEQ ID NO:20, CDRL2 of SEQ ID NO:21, and CDRL3 of SEQ ID NO:22,
b3) a light chain of SEQ ID NO:79, and comprising a CDRL1 of SEQ ID NO:23, CDRL2 of SEQ ID NO:24, and CDRL3 of SEQ ID NO:25,
b4) a light chain of SEQ ID NO:80, and comprising a CDRL1 of SEQ ID NO:26, CDRL2 of SEQ ID NO:27, and CDRL3 of SEQ ID NO:28,
b5) a light chain of SEQ ID NO:81, and comprising a CDRL1 of SEQ ID NO:29, CDRL2 of SEQ ID NO:30, and CDRL3 of SEQ ID NO:31,
b6) a light chain of SEQ ID NO:82, and comprising a CDRL1 of SEQ ID NO:32, CDRL2 of SEQ ID NO:33, and CDRL3 of SEQ ID NO:34,
b7) a light chain of SEQ ID NO:83, and comprising a CDRL1 of SEQ ID NO:35, CDRL2 of SEQ ID NO:36, and CDRL3 of SEQ ID NO:37,
b8) a light chain of SEQ ID NO:84, and comprising a CDRL1 of SEQ ID NO:38, CDRL2 of SEQ ID NO:39, and CDRL3 of SEQ ID NO:40,
b9) a light chain of SEQ ID NO:85, and comprising a CDRL1 of SEQ ID NO:41, CDRL2 of SEQ ID NO:42, and CDRL3 of SEQ ID NO:43,
b10) a light chain of SEQ ID NO:86, and comprising a CDRL1 of SEQ ID NO:44, CDRL2 of SEQ ID NO:45, and CDRL3 of SEQ ID NO:46,
b11) a light chain of SEQ ID NO:87, and comprising a CDRL1 of SEQ ID NO:47, CDRL2 of SEQ ID NO:48, and CDRL3 of SEQ ID NO:49,
b12) a light chain of SEQ ID NO:88, and comprising a CDRL1 of SEQ ID NO:50, CDRL2 of SEQ ID NO:51, and CDRL3 of SEQ ID NO:52,
b13) a light chain of SEQ ID NO:89, and comprising a CDRL1 of SEQ ID NO:53, CDRL2 of SEQ ID NO:54, and CDRL3 of SEQ ID NO:55,
b14) a light chain of SEQ ID NO:90, and comprising a CDRL1 of SEQ ID NO:56, CDRL2 of SEQ ID NO:57, and CDRL3 of SEQ ID NO:58,
b15) a light chain of SEQ ID NO:91, and comprising a CDRL1 of SEQ ID NO:59, CDRL2 of SEQ ID NO:60, and CDRL3 of SEQ ID NO:61,
b16) a light chain of SEQ ID NO:92, and comprising a CDRL1 of SEQ ID NO:62, CDRL2 of SEQ ID NO:63, and CDRL3 of SEQ ID NO:64,
b17) a light chain of SEQ ID NO:174, and comprising a CDRL1 of SEQ ID NO:71, CDRL2 of SEQ ID NO:72, and CDRL3 of SEQ ID NO:73,
b18) a light chain of SEQ ID NO:175, and comprising a CDRL1 of SEQ ID NO:142, CDRL2 of SEQ ID NO:143, and CDRL3 of SEQ ID NO:144
b19) a light chain of SEQ ID NO:176, and comprising a CDRL1 of SEQ ID NO:145, CDRL2 of SEQ ID NO:146, and CDRL3 of SEQ ID NO:147,
b20) a light chain of SEQ ID NO:177, and comprising a CDRL1 of SEQ ID NO:148, CDRL2 of SEQ ID NO:149, and CDRL3 of SEQ ID NO:150,
b21) a light chain of SEQ ID NO:178, and comprising a CDRL1 of SEQ ID NO:151, CDRL2 of SEQ ID NO:152, and CDRL3 of SEQ ID NO:153,

b22) a light chain of SEQ ID NO:179, and comprising a CDRL1 of SEQ ID NO:154, CDRL2 of SEQ ID NO:155, and CDRL3 of SEQ ID NO:156, and
b23) a light chain of SEQ ID NO:180, and comprising a CDRL1 of SEQ ID NO:157, CDRL2 of SEQ ID NO:158, and CDRL3 of SEQ ID NO:159,
and

c) in the second binding part as heavy chain a heavy chain region of SEQ ID NO:5, comprising a CDRH1 of SEQ ID NO:1, a CDRH2 of SEQ ID NO:2, and a CDRH3 of SEQ ID NO:3, and as light chain a light chain of SEQ ID NO:11, comprising a CDRL1 of SEQ ID NO:7, a CDRL2 of SEQ ID NO:8, and a CDRL3 of SEQ ID NO:9.

6. The bispecific antibody according to embodiment 1, characterized in comprising

a) in the first binding part as heavy chain a heavy chain region of SEQ ID NO:5, comprising a CDRH1 of SEQ ID NO:1, a CDRH2 of SEQ ID NO:2, and a CDRH3 of SEQ ID NO:3, and
b) as light chain a light chain selected from the group consisting of

b1) a light chain of SEQ ID NO:74, and comprising
a CDRL1 of SEQ ID NO:17, CDRL2 of SEQ ID NO:18, and CDRL3 of SEQ ID NO:19,
b2) a light chain of SEQ ID NO:75, and comprising a CDRL1 of SEQ ID NO:20, CDRL2 of SEQ ID NO:21, and CDRL3 of SEQ ID NO:22,
b3) a light chain of SEQ ID NO:76, and comprising a CDRL1 of SEQ ID NO:23, CDRL2 of SEQ ID NO:24, and CDRL3 of SEQ ID NO:25,
b20) a light chain of SEQ ID NO:167, and comprising a CDRL1 of SEQ ID NO:71, CDRL2 of SEQ ID NO:72, and CDRL3 of SEQ ID NO:73,
b21) a light chain of SEQ ID NO:168, and comprising a CDRL1 of SEQ ID NO:142, CDRL2 of SEQ ID NO:143, and CDRL3 of SEQ ID NO:144
b22) a light chain of SEQ ID NO:169, and comprising a CDRL1 of SEQ ID NO:145, CDRL2 of SEQ ID NO:146, and CDRL3 of SEQ ID NO:147,
b23) a light chain of SEQ ID NO:170, and comprising a CDRL1 of SEQ ID NO:148, CDRL2 of SEQ ID NO:149, and CDRL3 of SEQ ID NO:150,
b24) a light chain of SEQ ID NO:171, and comprising a CDRL1 of SEQ ID NO:151, CDRL2 of SEQ ID NO:152, and CDRL3 of SEQ ID NO:153,
b25) a light chain of SEQ ID NO:172, and comprising a CDRL1 of SEQ ID NO:154, CDRL2 of SEQ ID NO:155, and CDRL3 of SEQ ID NO:156, and
b26) a light chain of SEQ ID NO:173, and comprising a CDRL1 of SEQ ID NO:157, CDRL2 of SEQ ID NO:158, and CDRL3 of SEQ ID NO:159,
and

c) in the second binding part as heavy chain a heavy chain region of SEQ ID NO:5, comprising a CDRH1 of SEQ ID NO:1, a CDRH2 of SEQ ID NO:2, and a CDRH3 of SEQ ID NO:3, and as light chain a light chain of SEQ ID NO:181, comprising a CDRL1 of SEQ ID NO:7, a CDRL2 of SEQ ID NO:8, and a CDRL3 of SEQ ID NO:9.

7. The bispecific antibody according to embodiment 1, characterized in comprising in the first binding part as heavy chain region a heavy chain region of SEQ ID NO:5 and as light chain a light chain selected from the group of

a) the light chain of SEQ ID NO:77,
b) the light chain of SEQ ID NO:78,
c) the light chain of SEQ ID NO:79,
d) the light chain of SEQ ID NO:80,
e) the light chain of SEQ ID NO:81,
f) the light chain of SEQ ID NO:82,
g) the light chain of SEQ ID NO:83,
h) the light chain of SEQ ID NO:84,
i) the light chain of SEQ ID NO:85,
k) the light chain of SEQ ID NO:86,
l) the light chain of SEQ ID NO:87,
m) the light chain of SEQ ID NO:88,
n) the light chain of SEQ ID NO:89,

o) the light chain of SEQ ID NO:90,
p) the light chain of SEQ ID NO:91,
r) the light chain of SEQ ID NO:92,
s) the light chain of SEQ ID NO:174,
t) the light chain of SEQ ID NO:175,
u) the light chain of SEQ ID NO:176,
v) the light chain of SEQ ID NO:177,
w) the light chain of SEQ ID NO:178,
x) the light chain of SEQ ID NO:179, and
y) the light chain of SEQ ID NO:180, and
b) comprising in the second binding part as heavy chain variable region a heavy chain variable region of SEQ ID NO:4 and as light chain a light chain of SEQ ID NO:11.

8. The bispecific antibody according to embodiment 1, characterized in comprising in the first binding part as heavy chain region a heavy chain region of SEQ ID NO:5 and as light chain a light chain selected from the group of

a) the light chain of SEQ ID NO:74,
b) the light chain of SEQ ID NO:75,
c) the light chain of SEQ ID NO:76,
d) the light chain of SEQ ID NO:167,
e) the light chain of SEQ ID NO:168,
f) the light chain of SEQ ID NO:169,
g) the light chain of SEQ ID NO:170,
h) the light chain of SEQ ID NO:171,
i) the light chain of SEQ ID NO:172,
k) the light chain of SEQ ID NO:173,
and
b) comprising in the second binding part as heavy chain region a heavy chain region of SEQ ID NO:5 and as light chain a light chain of SEQ ID NO:181.

9. The bispecific antibody according to embodiment 1, characterized in specifically binding to human CEACAM5 and human CEACAM6 in the first binding part and human CD47 in the second binding part, characterized in

a) that the first binding part comprises as heavy chain variable region a heavy chain variable region comprising as CDRs a CDRH1 of SEQ ID NO:1, CDRH2 of SEQ ID NO:2 and CDRH3 of SEQ ID NO:3 and as light chain variable region a light chain variable region comprising as CDRs a CDRL1 of SEQ ID NO: 44, a CDRL2 of SEQ ID NO: 45, and a CDRL3 of SEQ ID NO: 46 and
b) that the second binding part comprises as heavy chain variable region a heavy chain variable region comprising as CDRs a CDRH1 of SEQ ID NO:1, CDRH2 of SEQ ID NO:2 and CDRH3 of SEQ ID NO:3 and as light chain variable region a light chain variable region comprising as CDRs a CDRL1 of SEQ ID NO:7, CDRL2 of SEQ ID NO:8, and CDRL3 of SEQ ID NO:9.

10. The bispecific antibody according to embodiment 1, characterized in specifically binding to human CEACAM5 and cynomolgus CEACAM5 in the first binding part and human CD47 in the second binding part, characterized in

b) a) that the first binding part comprises as heavy chain variable region a heavy chain variable region comprising as CDRs a CDRH1 of SEQ ID NO:1, CDRH2 of SEQ ID NO:2 and CDRH3 of SEQ ID NO:3 and as light chain variable region a light chain variable region comprising as CDRs a CDRL1 of SEQ ID NO: 53, a CDRL2 of SEQ ID NO: 54, and a CDRL3 of SEQ ID NO: 55, and
b) that the second binding part comprises as heavy chain variable region a heavy chain variable region comprising as CDRs a CDRH1 of SEQ ID NO:1, CDRH2 of SEQ ID NO:2 and CDRH3 of SEQ ID NO:3 and as light chain variable region a light chain variable region comprising as CDRs a CDRL1 of SEQ ID NO:7, CDRL2 of SEQ ID NO:8, and CDRL3 of SEQ ID NO:9.

11. The bispecific antibody according to embodiment 9 or 10, characterized in comprising a first binding part comprising a lambda light chain variable domain and a lambda light chain constant domain and a second binding part specific for CD47, comprising a kappa light chain variable domain and a kappa light chain constant domain.

12. The bispecific antibody according to embodiment 9, characterized in comprising in the first binding part as heavy chain a heavy chain of SEQ ID NO:5 or of SEQ ID NO:6 and as light chain a light chain of SEQ ID NO:86 and b) in the second binding part as heavy chain a heavy chain of SEQ ID NO:5 or of SEQ ID NO:6 and as light chain a light chain of SEQ ID NO:11.

13. The bispecific antibody according to embodiment 10, characterized in comprising in the first binding part as heavy chain a heavy chain of SEQ ID NO:5 or of SEQ ID NO:6 and as light chain a light chain of SEQ ID NO:89 and in the second binding part as heavy chain a heavy chain of SEQ ID NO:5 or of SEQ ID NO:6 and as light chain a light chain of SEQ ID NO:11.

14. The bispecific antibody of any one of the preceding embodiments, characterized in comprising in the first binding part as variable heavy chain region a variable heavy chain region of SEQ ID NO:4.

15 The bispecific antibody of any one of the preceding embodiments, characterized in comprising in the second binding part as variable heavy chain region a variable heavy chain region of SEQ ID NO:4.

16. The bispecific antibody according to embodiment 1, characterized in comprising as heavy chain variable region a heavy chain sequence of SEQ ID NO:5 or SEQ ID NO:6.

17. The bispecific antibody of any one of the preceding embodiments, characterized in comprising in the second binding part as variable light chain region a variable light chain region of SEQ ID NO:10.

18. The bispecific antibody of any one of the preceding embodiments, characterized in that said bispecific antibody competes for binding to CEACAM5 with the anti-CEACAM5 antibody SM3E, which comprises as VK and VH domains VK and VH of sequences SEQ ID NO:110 and 111.

19. The bispecific antibody of any one of the preceding embodiments, characterized in that said bispecific antibody competes for binding to CEACAM5 with the anti-CEACAM5 antibody SM3E, which comprises as VK and VH domains VK and VH of sequences SEQ ID NO:110 and 111.

20. The bispecific antibody of any one of the preceding embodiments, wherein said antibody is monovalent for the first binding part and monovalent for the second binding part.

21. The bispecific antibody of any one of the preceding embodiments, wherein the constant and variable framework region sequences are human.

22. The bispecific antibody of any one of the preceding embodiments, wherein each of the first and second binding parts comprises an immunoglobulin heavy chain and an immunoglobulin light chain.

23. The bispecific antibody of any one of the preceding embodiments, wherein the bispecific antibody is a full-length antibody.

24. The bispecific antibody according to embodiment 23, wherein the antibody is human IgG1 type.

25. The bispecific antibody according to embodiment 24, characterized in comprising as heavy chain a common heavy chain of SEQ ID NO:6.

26. The bispecific antibody of any one of the preceding embodiments, wherein said antibody comprises a Fc region that has been glycoengineered to have a reduced number of fucose residues as compared to the same bispecific antibody that has not been glycoengineered.

27. The bispecific antibody of any one of the preceding embodiments, wherein said first binding part specifically binds to human CEACAM5 and cynomolgus CEACAM5.

28. The bispecific antibody of any one of the preceding embodiments, wherein said first binding part specifically binds to human CEACAM5 and CEACAM 6.

29. The bispecific antibody according to embodiment 28, wherein the EC50 values of binding to human CEACAM5 and human CEACAM6 differ by not more than a factor of 10.

30. The bispecific antibody according to embodiment 29, wherein the EC50 values of binding to human CEACAM5 and human CEACAM6 differ by less than a factor of 5.

31. The bispecific antibody according to embodiment 27, wherein the EC50 values of binding to human CEACAM5 and cynomolgus CEACAM6 differ by not more than a factor of 10.

32.. The bispecific antibody according to embodiment 27, wherein the EC50 values of binding to human CEACAM5 and cynomolgus CEACAM5 differ by less than a factor of 5.

33. The bispecific antibody of any one of the preceding embodiments, characterized in a concentration dependent phagocytosis of CEACAM5 and/or CEACAM6 expressing tumor cell lines like MKN-45 cells by human macrophages at an EC50 of the bispecific antibody below 40 nM.

34. The bispecific antibody of any one of the preceding embodiments, characterized in a concentration dependent phagocytosis (ADCP) of CEACAM5 and/or CEACAM6 expressing tumor cell lines like MKN-45 cells by human macrophages at an EC50 of the bispecific antibody below 10 nM.

35. The bispecific antibody of any one of the preceding embodiments, wherein the EC50 value of phagocytosis index curve of said bispecific antibody is in the range of 0.1 to 10 times of the E50 value of reference antibody K2AC54 under the same experimental conditions.

36. The bispecific antibody of embodiment 35, wherein the EC50 range of phagocytosis is 0.01 to 10, 0.2 to 10, 0.3 to 10, or 0.5 to 10.

37. The bispecific antibody of any one of the preceding embodiments, characterized in binding to human CD47 with a binding affinity of 100 nM to 600 nM.

38. The bispecific antibody of embodiment 37, wherein said bispecific antibody binds to MKN-45 cells with an EC50 value of 1 to 250 nM.

39. The bispecific antibody of embodiment 37, wherein said bispecific antibody binds to MKN-45 cells with an EC50 value of 1 to 200 nM.

40. The bispecific antibody of embodiment 37, wherein said bispecific antibody binds to MKN-45 cells with an EC50 value of 50 to 100 nM.

41. The bispecific antibody of embodiment 37, wherein said bispecific antibody binds to MKN-45 cells with an EC50 value of 100 to 50 nM.

42. The bispecific antibody of any one of the preceding embodiments, wherein the bispecific antibody does not cross-react with human CEACAM1.

43. The bispecific antibody of any one of the preceding embodiments, wherein said bispecific antibody inhibits the interaction between CD47 and SIRP$\alpha$ on MKN-45 cells with an IC50 of 0.1 to 10 nM.

44. The bispecific antibody of any one of the preceding embodiments, wherein said bispecific antibody specifically binds to CEACAM5 but does not compete with CEA-TCB1 for binding to CEACAM5 on MKN-45 tumor cells.

45. The bispecific antibody of any one of the preceding embodiments, wherein the EC50 value for the binding to MKN-45 cells (EC50 between 1 and 250 nM) is increased by less than a factor of three in the presence of CEA-TCB at a concentration of 300 nM or in the presence of CEA-TCB1 at a concentration of 30 nM.

46. The bispecific antibody of any one of the preceding embodiments, wherein the bispecific antibody has a 100 or more times higher EC50 for RBC phagocytosis compared to the EC50 measured in the same assay with B6H12.2 (ATCC® HB9771™).

47. The bispecific antibody of embodiment 46, wherein the EC50 for the red blood cell (RBC) phagocytosis index of sais bispecific antibody is 5 to 10 times lower, or 10 to 30 times lower as for B6H12.2 (ATCC® HB9771™).

48. The bispecific antibody of any one of the preceding embodiments, wherein the bispecific antibody does not show significant platelet activation in concentrations up to 200 μg/mL.

49. The bispecific antibody of any one of the preceding embodiments, wherein the addition of 1 mg /mL of human IgG to the imaging based phagocytosis assay causes a less than a factor of 0.7 reduction of the maximum of the concentration/phagocytosis index curve and/or a less than a factor of 5 shift of the EC50 towards higher concentrations.

50. The bispecific antibody of any one of the preceding embodiments, wherein the bispecific antibody binds to human CEACAM5 and cynomolgus monkey CEACAM5 with an EC50 ratio which is between 0.1 to 10.

51. The bispecific antibody of any one of the preceding embodiments, wherein the bispecific antibody has been glycoengineered to have an Fc region with modified oligosaccharides.

52. The bispecific antibody of embodiment 51, wherein the glycoengineered bispecific antibody has at least 3 times lower EC50 value for the phagocytosis index curve measured by the imaging based assay compared to the same bispecific antibody that has not been glycoengineered if measured under the same experimental conditions.

53. The bispecific antibody of any one of the preceding embodiments, wherein the bispecific antibody has a Fc region that has been modified to have a reduced number of fucose residues as compared to the bispecific antibody that has not been glycoengineered.

54. The bispecific antibody of any one of the preceding embodiments, wherein said bispecific antibody has an Fc region in which 50% to 100% of the N-linked oligosaccharides are nonfucosylated.

55. The bispecific antibody of any one of the preceding embodiments, wherein said bispecific antibody has an Fc region in which 80% to 100% of the N-linked oligosaccharides are nonfucosylated.

56. The bispecific antibody of any one of the preceding embodiments, wherein said bispecific antibody has an Fc region in which 90% to 100% of the N-linked oligosaccharides are nonfucosylated.

57. The bispecific antibody of any one of the preceding embodiments, wherein said bispecific antibody has an Fc region in which 95% to 100% of the N-linked oligosaccharides are nonfucosylated.

58. The bispecific antibody of any one of the preceding embodiments, wherein said bispecific antibody has been glycoengineered and the concentration ADCC curve (maximum and/or EC50) induced by said glycoengineered antibody is increased by at least a factor of 1.2 compared to the ADCC induced by the same bispecific antibody that has not been glycoengineered.

59. The bispecific antibody of embodiment 58, wherein ADCC maximum and/or EC50 value of ADCC curve are/is increased by a factor of 1.2 to 2.0.

60. The bispecific antibody of any one of embodiments 51-59, wherein said bispecific antibody has an at least 3 times lower EC50 value for the phagocytosis index curve measured by the imaging based assay as compared to the same bispecific antibody that has not been glycoengineered if measured under the same experimental conditions.

61. The bispecific antibody of embodiment 60, wherein the EC50 for the phagocytosis index is 5 to 10 times lower.

62. The bispecific antibody of embodiment 60, wherein the EC50 for the phagocytosis index is 10 to 30 times lower.

63. The bispecific antibody of embodiment any one of embodiments 51-62, wherein flow cytometry determined maximal ADCP function induced by said glycoengineered antibody is increased by at least a factor of 1.2 compared to the ADCP induced by the same bispecific antibody that has not been glycoengineered.

64. The bispecific antibody of embodiment 63, wherein the ADCP is increased by a factor of 1.2 to 2.0.

The bispecific antibody of any one of the preceding embodiments, wherein the glycoengineered bispecific antibody comprises increased effector functions compared to the bispecific antibody that has not been glycoengineered comprising as common heavy chain SEQ ID NO:6.

65. The bispecific antibody of embodiment 61-64, wherein the bispecific antibody shows an increase in one or more of the following effector functions:

increased binding affinity to FcγRs,
increased binding of macrophages, including increased antibody dependent cellular phagocytosis (ADCP),
increased binding of NK cells, including increased antibody-mediated cellular cytotoxicity (ADCC), and
increased binding of monocytes.

66. An isolated polynucleotide encoding a bispecific antibody according to any one of the preceding embodiments.

67. An expression vector comprising the polynucleotide of embodiment 66.

68. A host cell comprising the expression vector of embodiment 67.

69. A method for the production of a bispecific antibody according to the invention, comprising a) culturing a host cell of embodiment 68 under conditions which permit the production of said bispecific antibody, and b) isolating said antibody.

70. The method of embodiment 69, wherein said antibody is capable of specifically binding to human CEACAM5 and cynomolgus CEACAM5 and CD47.

71. The method of embodiment 69, wherein said antibody is capable of specifically binding to human CEACAM5 and human CEACAM6 and CD47.

72. A method for producing a glycoengineered bispecific antibody according to any one of embodiments 1-65, comprising an Fc region in which 80% to 100% of the N-linked oligosaccharides are nonfucosylated, in a host cell of embodiment 68, cultured in a fucose free medium,
said host cell comprising a first polynucleotide F-4-hexylose reductase and a second polynucleotide encoding the bispecific antibody according to the invention, said method comprises:

i) expressing GDP-6-deoxy-D-lyxo-4-hexylose reductase encoded by the first polynucleotide and the bispecific antibody according to the invention encoded by the second polynucleotide in said cell, and
i) isolating said bispecific antibody from said cell under conditions which permit the production of said bispecific antibody of the invention, and which permit that the oligosaccharides of the Fc region of said bispecific antibody are lacking fucose in an amount of 80% to 100%; and

b) isolating said glycoengineered bispecific antibody wherein said glycoengineered bispecific antibody is capable of specifically binding to CEA and CD47.

73. A method according to embodiment 72, characterized in that the in the Fc region 90% to 100% of the N-linked oligosaccharides are nonfucosylated.

74. A method according to embodiment 72, characterized in that the in the Fc region 95% to 100% of the N-linked oligosaccharides are nonfucosylated

75. A method according to embodiment 72, characterized in that the in the Fc region 100% of the N-linked oligosaccharides are nonfucosylated.

76. A method of inducing cell lysis of a tumor cell comprising contacting the tumor cell with the bispecific antibody of any one of embodiments 1-65.

77. The method of embodiment 76, wherein the tumor cell is a human tumor cell.

78. The method of embodiment 76 or embodiment 77, wherein the tumor cell is in a patient.

79. The method of any one of embodiments 76 to 78, characterized in that the tumor cell is a colorectal cancer cell, NSCLC (non-small cell lung cancer) cell, gastric cancer cell, pancreatic cancer cell, breast cancer cell, or another tumor cell expressing CEACAM5 or CEACAM5 and CEACAM6.

80. A method of treating a subject having a cancer that expresses CEACAM5 or CEACAM5 and CEACAM6, the method comprising administering to the subject a therapeutically effective amount of the bispecific antibody of any one of embodiments 1-65

81. A method of increasing survival time in a subject having a cancer that expresses CEACAM5 or CEACAM5 and CEACAM6, said method comprising administering to said subject a therapeutically effective amount of the bispecific antibody of any one of embodiments 1-65.

82. The method of embodiment 80 or embodiment 81, characterized in that the cancer is colorectal cancer, non-small cell lung cancer (NSCLC), gastric cancer, pancreatic cancer or breast cancer.

83. The method of any one of embodiments 80 to 82, wherein the bispecific antibody is administered in combination with chemotherapy and/or radiation therapy to a human subject.

84. A method for treating a subject having a cancer that expresses CEACAM5 or CEACAM5 and CEACAM6, the method comprising administering to the subject a therapeutically effective amount of the bispecific antibody of any one of embodiments 1-65 characterized in that the EC50 value of phagocytosis of said bispecific antibody is in the range of 0.01 to 10 times of the E50 value of reference antibody K2AC54 under the same experimental conditions.

85. The method of embodiment 84, wherein the EC50 range is 0.01 to 10, 0.2 to 10, 0.3 to 10, or 0.5 to 10

86. The bispecific antibody of any one of embodiments 1-65 for use in a method of treating a subject having a cancer that expresses CEACAM5 or CEACAM5 and CEACAM6, the method comprising administering to the subject a therapeutically effective amount of a bispecific antibody according to the invention, characterized in that the EC50 value of phagocytosis of said bispecific antibody is in the range of 0.01 to 10 times of the E50 value of reference antibody K2AC54 under the same experimental conditions.

87. The bispecific antibody for the use of embodiment 86, wherein the EC50 range is 0.01 to 10, 0.2 to 10, 0.3 to 310, or 0.5 to 10.

88. The bispecific antibody for the use of embodiment 86 or embodiment 87, wherein the bispecific antibody is characterized in binding to human CD47 with a binding affinity of 100 nM to 600nM.

89. Use of the bispecific antibody of any one of embodiments 1-65 in the manufacture of a medicament for treating a subject having a cancer that expresses CEACAM5 or CEACAM5 and CEACAM6.

90. The use of the bispecific antibody according to embodiment 89, wherein the cancer is selected from the group consisting of colorectal cancer, non-small cell lung cancer (NSCLC), gastric cancer, pancreatic cancer and breast cancer.

91. The bispecific antibody of any one of embodiments 1-65 for use in simultaneous, separate, or sequential combination with a second bispecific antibody comprising a third binding part specifically binding to human CEACAM5, and a fourth binding part specifically binding to human CD3ε in the treatment of a subject having a cancer that expresses CEACAM5 or CEACAM5 and CEACAM6.

92. The bispecific antibody of any one of embodiments 1-65 for use in simultaneous, separate, or sequential combination with a second bispecific antibody comprising a third binding part specifically binding to human CEACAM5 and a fourth binding part specifically binding to an epitope of human CD3ε, said epitope comprising the amino acid sequence of SEQ ID NO:118, for the treatment of a subject having a cancer that expresses CEACAM5 or CEACAM5 and CEACAM6.

93. The bispecific antibody of any one of embodiments 1-65 for use in simultaneous, separate, or sequential combination with CEA-TCB and/or CEA-TCB1 in the treatment of a subject having a cancer that expresses CEACAM5 or CEACAM5 and CEACAM6.

94. The bispecific antibody of any one of embodiments 1-65, for use in simultaneous, separate, or sequential combination with a second bispecific antibody in the treatment of a subject having a cancer that expresses CEACAM5 or CEACAM5 and CEACAM6 wherein said bispecific antibody of the invention does not compete with said second bispecific antibody.

95. The bispecific antibody of any one of embodiments 1-65, for use in simultaneous, separate, or sequential combination with CEA-TCB or CEA-TCB 1 in the treatment of a subject having a cancer that expresses CEACAM5 or CEACAM5 and CEACAM6, whereby said CEA-TCB in a concentration of 300 nM or CEA-TCB1in a concentration of 30 nM does not shift the EC50 of the binding curve to MKN-45 cells of the bispecific antibody according to the invention by more than a factor of 3 towards higher concentrations.

96. A bispecific antibody, characterized in specifically binding to human CEACAM5 and human CEACAM6 in the first binding part and human CD47 in the second binding part, characterized in

a) that the first binding part comprises as heavy chain variable region a heavy chain variable region comprising as CDRs a CDRH1 of SEQ ID NO:1, CDRH2 of SEQ ID NO:2 and CDRH3 of SEQ ID NO:3 and as light chain variable region a light chain variable region comprising as CDRs a CDRL1 of SEQ ID NO: 44, a CDRL2 of SEQ ID NO: 45, and a CDRL3 of SEQ ID NO: 46 and
b) that the second binding part comprises as heavy chain variable region a heavy chain variable region comprising as CDRs a CDRH1 of SEQ ID NO:1, CDRH2 of SEQ ID NO:2 and CDRH3 of SEQ ID NO:3 and as light chain variable region a light chain variable region comprising as CDRs a CDRL1 of SEQ ID NO:7, CDRL2 of SEQ ID NO:8, and CDRL3 of SEQ ID NO:9, for use in simultaneous, separate, or sequential combination with CEA-TCB or CEA-TCB 1 in the treatment of a subject having a cancer that expresses CEACAM5 or CEACAM5 and CEACAM6, wherein the bispecific antibody is characterized in not competing with CEA-TCB or CEA-TCB 1.

97. A bispecific antibody characterized in specifically binding to human CEACAM5 and cynomolgus CEACAM5 in the first binding part and human CD47 in the second binding part, characterized in

b) a) that the first binding part comprises as heavy chain variable region a heavy chain variable region comprising as CDRs a CDRH1 of SEQ ID NO:1, CDRH2 of SEQ ID NO:2 and CDRH3 of SEQ ID NO:3 and as light chain variable region a light chain variable region comprising as CDRs a CDRL1 of SEQ ID NO: 53, a CDRL2 of SEQ ID NO: 54, and a CDRL3 of SEQ ID NO: 55, and
b) that the second binding part comprises as heavy chain variable region a heavy chain variable region comprising as CDRs a CDRH1 of SEQ ID NO:1, CDRH2 of SEQ ID NO:2 and CDRH3 of SEQ ID NO:3 and as light chain variable region a light chain variable region comprising as CDRs a CDRL1 of SEQ ID NO:7, CDRL2 of SEQ ID NO:8, and CDRL3 of SEQ ID NO:9, for use in simultaneous, separate, or sequential combination with CEA-TCB or CEA-TCB 1 in the treatment of a subject having a cancer that expresses CEACAM5 or CEACAM5 and CEACAM6, wherein the bispecific antibody is characterized in not competing with CEA-TCB or CEA-TCB 1.

98. The bispecific antibody for use according to embodiment 96 or 97, characterized in comprising in the first binding part as heavy chain a heavy chain of SEQ ID NO:5 or of SEQ ID NO:6 and as light chain a light chain of SEQ ID NO:86 and
b) in the second binding part as heavy chain a heavy chain of SEQ ID NO:5 or of SEQ ID NO:6 and as light chain a light chain of SEQ ID NO:11.

99. The bispecific antibody for use according to any one of embodiments 96 to 98, characterized in comprising in the first binding part as heavy chain a heavy chain of SEQ ID NO:5 or of SEQ ID NO:6 and as light chain a light chain of SEQ ID NO:89 and in the second binding part as heavy chain a heavy chain of SEQ ID NO:5 or of SEQ ID NO:6 and as light chain a light chain of SEQ ID NO:11.

100. The bispecific antibody for use according to any one of embodiments 96 to 99, wherein said bispecific antibody does not compete with said second bispecific antibody.

101. The bispecific antibody for use according to any one of embodiments 96 to 100, whereby said CEA-TCB in a concentration of 300 nM or CEA-TCB1in a concentration of 30 nM does not shift the EC50 of the binding curve to MKN-45 cells of the bispecific antibody according to the invention by more than a factor of 3 towards higher concentrations.

102. The bispecific antibody for the use according to any one of embodiments 91-101, characterized in that the bispecific antibody according to the invention and the second bispecific antibody are administered to said subject simultaneously in 6 to 15 day intervals.

103. The bispecific antibody for the use of any one of embodiments 91-102, wherein said cancer is colorectal cancer, non-small cell lung cancer (NSCLC), gastric cancer, pancreatic cancer and breast cancer.

104. The bispecific antibody for the use of any one of embodiments 91-103, wherein the bispecific antibody and the second bispecific antibody show an additive % killing of tumor cells in an assay containing e.g. MKN-45 tumor cells and human macrophages and T-cells derived from the same human donor.

105. The bispecific antibody for the use of any one of embodiments 91-104, wherein the bispecific antibody and the second bispecific antibody show a synergistic % killing of tumor cells in an assay containing e.g. MKN-45 tumor cells and human macrophages and T-cells derived from the same human donor.

106. A composition comprising the bispecific antibody of any one of embodiments 1-65, wherein the bispecific antibody is characterized in not competing with a second bispecific antibody for use in the treatment of a subject having a cancer that expresses CEACAM5 or CEACAM5 and CEACAM6.

107. A composition comprising the bispecific antibody of any one of embodiments 1-65, wherein the bispecific antibody is characterized in not competing with a second bispecific antibody comprising a third binding part specifically binding to human CEACAM5, comprising as heavy chain variable region a heavy chain variable region of SEQ ID NO:116 and a light chain variable region of SEQ ID NO:117, and a fourth binding part specifically binding to an epitope of human CD3ε, comprising the amino acid sequence of SEQ ID NO:118, wherein the composition is for use in the treatment of a subject having a cancer that expresses CEACAM5 or CEACAM5 and CEACAM6.

108. A composition comprising the bispecific antibody of any one of embodiments 1-65, wherein the bispecific antibody is characterized in not competing with a second bispecific antibody comprising a third binding part specifically binding to human CEACAM5, comprising as heavy chain variable region a heavy chain variable region of SEQ ID NO:98 and a light chain variable region of SEQ ID NO:99 and a fourth binding part specifically binding to human CD3ε, comprising a as heavy chain variable region heavy chain variable region of SEQ ID NO:100 and a light chain variable region of SEQ ID NO:101, wherein the composition is for use in the treatment of a subject having a cancer that expresses CEACAM5 or CEACAM5 and CEACAM6.

109. The composition of any one of embodiments 106-108, wherein the bispecific antibody is characterized in not competing with CEA-TCB and/or CEA-TCB1.

110. A method for the treatment of a human patient with a tumor, comprising administering an effective amount of the CEACAM5 x CD47 bispecific antibody of any one of embodiments 1-65 and a second bispecific antibody against CEACAM5 and CD3, to the human patient, the method comprising subsequently:
administering to the patient a dose of 0.1 to 10 mg/kg, in a further embodiment of 0.5 to 10 mg/kg, in a further embodiment of 1 to 2 mg/kg of said second anti CEAxCD3 antibody, e.g. weekly over 4 to 12 weeks or q2w, over 4 to 12 weeks and administering after these 4 to 12 weeks and after waiting for additional 2 or 3 or 4 elimination half-lives of said anti CEAxCD3 antibody to the patient a dose of 0.1 to 20 mg/kg of an antibody according to the invention, administering to the patient said antibody according to the invention q1, q2w, q3w or optionally q4w,for e.g. 12 or more weeks, waiting 2 or 3 or 4 elimination half-lives of said antibody according to the invention and then optionally repeating said cycle of CEA x CD3 bispecific antibody administration followed by CEA x CD47 bispecific antibody administration and optionally repeat again that cycle etc.

111. The method of embodiment 110, wherein the tumor is cancer.

112. The method of embodiment 111, wherein the tumor is a solid tumor.

113. The method of embodiment 112, wherein the cancer is a solid cancer that expresses CEACAM5 or CEACAM5 and CEACAM6.

114. The method of embodiments 111 or embodiment 112, wherein the cancer is colorectal cancer, non-small cell lung cancer (NSCLC), gastric cancer, pancreatic cancer or breast cancer.

115. The method of any one of embodiments 110 -114, wherein the second antibody is CEA-TCB.

116. The method of any one of embodiments 110-114, wherein the second antibody is CEA-TCB 1.

117. The method of any one of embodiments 110- 116, wherein the CEACAM5 x CD47 bispecific antibody and the second bispecific antibody are competitive.

118. A method for the treatment of a human patient with a tumor, administering an effective amount of the CEACAM5 x CD47 bispecific antibody of any one of embodiments 1-65 and a second bispecific antibody against CEACAM5 and CD3.

119. The method of embodiment 118, wherein the CEACAM5 x CD47 bispecific antibody and the CEACAM5 and CD3 antibodies are not competitive.

120. The method of embodiment 118 or embodiment 119, wherein the antibodies are administered simultaneously.

121. The method of any one of embodiments 118 -120, wherein the patient is administered at about the same time at doses of 0.01 to 10 mg/kg of the CEACAM5 x CD3 bispecific antibody and 1 to 20 mg/kg of the CEACAM5 x CD47 bispecific antibody, followed by one or more of these combined administrations at a frequency of q1w or q2w or q3w or optionally q4w.

122. The method of embodiment 121, wherein the CEACAM5 x CD3 bispecific antibody is administered at 0.5 to 10 mg/kg.

123. The method of any one of embodiments 118-122, wherein the tumor is cancer.

124. The method of any one of embodiments 118-123, wherein the tumor is a solid tumor.

125. The method of embodiment 124, wherein the cancer is a solid cancer that expresses CEACAM5 or CEACAM5 and CEACAM6.

126. The method of embodiment 124, wherein the cancer is a solid cancer that expresses CEACAM5 and CEACAM6.

127. The method of embodiment 123 to 126, wherein the cancer is colorectal cancer, non-small cell lung cancer (NSCLC), gastric cancer, pancreatic cancer or breast cancer.

128. The method of any one of embodiments 118-127, wherein the second antibody is CEA-TCB.

129. The method of any one of embodiments 118-127, wherein the second antibody is CEA-TCB 1.

130. The method of any one of embodiments 118-129, wherein the bispecific antibody and the second bispecific antibody show an additive efficacy.

131. The method of any one of embodiments 118-129, wherein the bispecific antibody and the second bispecific antibody show a synergistic efficacy.

132. A pharmaceutical composition comprising a bispecific antibody of any one of embodiments 1-65 and a pharmaceutically acceptable excipient or carrier.

133. The pharmaceutical composition of embodiment 132, for use as a medicament.

134. The pharmaceutical composition of embodiment 132 or embodiment 133, for use as a medicament in the treatment of solid tumor disorders.

135. The pharmaceutical composition of embodiment 134, for use as a medicament in the treatment of colorectal cancer, NSCLC (non-small cell lung cancer), gastric cancer, pancreatic cancer or breast cancer.

136. A pharmaceutical composition comprising a first bispecific antibody of any one of embodiments 1-65, for use in simultaneous, separate, or sequential combination in the treatment of a subject having a cancer that expresses CEACAM5 or CEACAM5 and CEACAM6, with a second bispecific antibody, said second bispecific antibody comprising a third binding part specifically binding to human CEACAM5, comprising as heavy chain variable region a heavy chain variable region of SEQ ID NO:116 and a light chain variable region of SEQ ID NO:117, and a fourth binding part specifically binding to an epitope of human CD3ε, comprising the amino acid sequence of SEQ ID NO:118, wherein said second bispecific antibody in a concentration of 300 nM does not shift the EC50 of the binding curve to MKN-45 cells and/or the phagocytosis index curve of the first bispecific antibody by more than a factor of 3 towards higher concentrations.

137. A pharmaceutical composition comprising a first bispecific antibody of any one of embodiments 1-65, for use in simultaneous, separate, or sequential combination in the treatment of a subject having a cancer that expresses CEACAM5 or CEACAM5 and CEACAM6, with a second bispecific antibody comprising a third binding part specifically binding to human CEACAM5, comprising as heavy chain variable region a heavy chain variable region of SEQ ID NO:98 and a light chain variable region of SEQ ID NO:99 and a fourth binding part specifically binding to human CD3ε, comprising as heavy chain variable region a heavy chain variable region of SEQ ID NO:100 and a light chain variable region of SEQ ID NO:101, whereby said second bispecific antibody in a concentration of 30 nM does not shift the EC50 of the binding curve to MKN-45 cells and/or the phagocytosis index curve of the first bispecific antibody by more than a factor of 3 towards higher concentrations.

138. The pharmaceutical composition of embodiment 136 or 137, wherein the second bispecific antibody does not shift the EC50 of the binding curve and/or the phagocytosis index curve of the first bispecific antibody by more than a factor of 3 towards higher concentrations.

139. The pharmaceutical composition of any one of embodiments 136 to 138, wherein the cancer is colorectal cancer, non-small cell lung cancer (NSCLC), gastric cancer, pancreatic cancer, or breast cancer.

140. The use of an antibody of any one of embodiments 1-65, for the manufacture of a pharmaceutical composition.

141. The use of an antibody of any one of embodiments 1-65 and a pharmaceutically acceptable excipient or carrier for the manufacture of a pharmaceutical composition.

142. The use of an antibody of any one of embodiments 1-65 for the manufacture of a medicament in the treatment of solid tumor disorders.

143. The use of an antibody of any one of embodiments 1-65 in the treatment of colorectal cancer, NSCLC (non-small cell lung cancer), gastric cancer, pancreatic cancer or breast cancer.

144. A method of inducing cell lysis of a tumor cell comprising contacting the tumor cell with the bispecific antibody of any of any one of embodiments 1-65.

145. The method of embodiment 144, wherein the tumor cell is a colorectal cancer cell, NSCLC (non-small cell lung cancer), gastric cancer cell, pancreatic cancer cell or breast cancer cell.

146. The method of embodiments 144 or 145, wherein the cell lysis is induced by antibody dependent cellular phagocytosis and/or antibody dependent cellular cytotoxicity of the bispecific antibody.

147. A method of treating a subject having a cancer that abnormally expresses CEACAM5 or CEACAM5 and CEACAM6, the method comprising administering to the subject a therapeutically effective amount of the bispecific antibody of any one of embodiments 1-65.

148. A method of treating a subject having a cancer that abnormally expresses CEACAM5 or CEACAM5 and CEACAM6, the method comprising administering to the subject a therapeutically effective amount of the bispecific antibody of any one of embodiments 1-65 in combination with a second bispecific antibody binding to human CEACAM5 and human CD3.

149. The method of embodiment 148, wherein the CEACAM5xCD3 and CEACAM5xCD47 bispecific antibodies are not competing and the two bispecific antibodies are administered parallel/simultaneously.

150. The method of embodiment 148 or 149, wherein the bispecific antibody and the second bispecific antibody show an additive efficacy.

151. The method of embodiment 148 or 149, wherein the bispecific antibody and the second bispecific antibody show a synergistic efficacy.

152. A method of increasing progression free survival and/or overall survival time in a subject having a cancer that abnormally expresses CEACAM5 or CEACAM5 and CEACAM6, said method comprising administering to said subject a therapeutically effective amount of the bispecific antibody of any one of embodiments 1-65.

153. The method of embodiment 152, wherein the cancer is colorectal cancer, non-small cell lung cancer (NSCLC), gastric cancer, pancreatic cancer or breast cancer or another cancer expressing CEACAM5.

154. The method of any one of embodiments 147-153, wherein the bispecific antibody is administered in combination with chemotherapy and/or radiation therapy.

155. The method of any one of embodiments 147-154, wherein the subject is a patient suffering from colorectal cancer or lung cancer or gastric cancer or pancreatic cancer or breast cancer or another cancer expressing CEACAM5.

156. A method of treating a subject having a cancer that abnormally expresses CEACAM5 or CEACAM5 and CEACAM6, the method comprising administering to the subject a therapeutically effective amount of the bispecific antibody of any one of embodiments 1-65 in combination with a second bispecific antibody against human CEACAM5 and human CD3ε.

157. The method of embodiment 156, wherein the bispecific antibody and the second bispecific antibody show an additive efficacy.

158. The method of embodiment 156, wherein the bispecific antibody and the second bispecific antibody show a synergistic efficacy.

159. A method of increasing progression free survival time and/or overall survival time in a subject having a cancer that abnormally expresses CEACAM5 or CEACAM5 and CEACAM6, said method comprising administering to said subject a therapeutically effective amount of the bispecific antibody of any one of embodiments 1-65.
The method of any one of embodiments 162-175, wherein the cancer is colorectal cancer, non-small cell lung cancer (NSCLC), gastric cancer, pancreatic cancer or breast cancer.

160. The method of any one of embodiments 147-159, wherein the bispecific antibody is administered in combination with chemotherapy or radiation therapy.

161. The method of any one of embodiments 147-160, the subject is a cancer patient with colorectal cancer or lung cancer or gastric cancer or pancreatic cancer or breast cancer or another CEACAM5 or CEACAM5 and CEACAM6.

162. The use of a bispecific antibody according to any one of embodiments 1-65 in the method of treatment of any one of embodiments 147-161.

163. The use of embodiment 162, characterized in that the cancer is selected from the group consisting of: colorectal cancer, non-small cell lung cancer (NSCLC), gastric cancer, pancreatic cancer and breast cancer.

164. A monoclonal antibody specifically binding to human CEACAM5, characterized in comprising

a) as heavy chain variable region a heavy chain variable region comprising a CDRH1 of SEQ ID NO:1, a CDRH2 of SEQ ID NO:2 and a CDRH3 of SEQ ID NO:3, and
b) as light chain variable region a light chain variable region comprising a CDRL set selected from the group consisting of

b1) a CDRL1 of SEQ ID NO:17, CDRL2 of SEQ ID NO:18, and CDRL3 of SEQ ID NO:19,
b2) a CDRL1 of SEQ ID NO:20, CDRL2 of SEQ ID NO:21, and CDRL3 of SEQ ID NO:22,

b3) a CDRL1 of SEQ ID NO:23, CDRL2 of SEQ ID NO:24, and CDRL3 of SEQ ID NO:25,
b4) a CDRL1 of SEQ ID NO:26, CDRL2 of SEQ ID NO:27, and CDRL3 of SEQ ID NO:28,
b5) a CDRL1 of SEQ ID NO:29, CDRL2 of SEQ ID NO:30, and CDRL3 of SEQ ID NO:31,
b6) a CDRL1 of SEQ ID NO:32, CDRL2 of SEQ ID NO:33, and CDRL3 of SEQ ID NO:34,
b7) a CDRL1 of SEQ ID NO:35, CDRL2 of SEQ ID NO:36, and CDRL3 of SEQ ID NO:37,
b8) a CDRL1 of SEQ ID NO:38, CDRL2 of SEQ ID NO:39, and CDRL3 of SEQ ID NO:40,
b9) a CDRL1 of SEQ ID NO:41, CDRL2 of SEQ ID NO:42, and CDRL3 of SEQ ID NO:43,
b10) a CDRL1 of SEQ ID NO:44, CDRL2 of SEQ ID NO:45, and CDRL3 of SEQ ID NO:46,
b11) a CDRL1 of SEQ ID NO:47, CDRL2 of SEQ ID NO:48, and CDRL3 of SEQ ID NO:49,
b12) a CDRL1 of SEQ ID NO:50, CDRL2 of SEQ ID NO:51, and CDRL3 of SEQ ID NO:52,
b13) a CDRL1 of SEQ ID NO:53, CDRL2 of SEQ ID NO:54, and CDRL3 of SEQ ID NO:55,
b14) a CDRL1 of SEQ ID NO:56, CDRL2 of SEQ ID NO:57, and CDRL3 of SEQ ID NO:58,
b15) a CDRL1 of SEQ ID NO:59, CDRL2 of SEQ ID NO:60, and CDRL3 of SEQ ID NO:61,
b16) a CDRL1 of SEQ ID NO:62, CDRL2 of SEQ ID NO:63, and CDRL3 of SEQ ID NO:64,
b17) a CDRL1 of SEQ ID NO:71, CDRL2 of SEQ ID NO:72, and CDRL3 of SEQ ID NO:73,
b18) a CDRL1 of SEQ ID NO:142, CDRL2 of SEQ ID NO:143, and CDRL3 of SEQ ID NO:144,
b19) a CDRL1 of SEQ ID NO:145, CDRL2 of SEQ ID NO:146, and CDRL3 of SEQ ID NO:147,
b20) a CDRL1 of SEQ ID NO:148, CDRL2 of SEQ ID NO:149, and CDRL3 of SEQ ID NO:150,
b21) a CDRL1 of SEQ ID NO:151, CDRL2 of SEQ ID NO:152, and CDRL3 of SEQ ID NO:153,
b22) a CDRL1 of SEQ ID NO:154, CDRL2 of SEQ ID NO:155, and CDRL3 of SEQ ID NO:156, and
b23) a CDRL1 of SEQ ID NO:157, CDRL2 of SEQ ID NO:158, and CDRL3 of SEQ ID NO:159.

165. The antibody according to embodiment 164, characterized in comprising

a) as heavy chain variable region a heavy chain region of SEQ ID NO:4, comprising a CDRH1 of SEQ ID NO:1, a CDRH2 of SEQ ID NO:2, and a CDRH3 of SEQ ID NO:3, and as light chain
b) a light chain selected from the group consisting of

b1) a light chain of SEQ ID NO:74, and comprising a CDRL1 of SEQ ID NO:17, CDRL2 of SEQ ID NO:18, and CDRL3 of SEQ ID NO:19,
b2) a light chain of SEQ ID NO:75, and comprising a CDRL1 of SEQ ID NO:20, CDRL2 of SEQ ID NO:21, and CDRL3 of SEQ ID NO:22,
b3) a light chain of SEQ ID NO:76, and comprising a CDRL1 of SEQ ID NO:23, CDRL2 of SEQ ID NO:24, and CDRL3 of SEQ ID NO:25,
b4) a light chain of SEQ ID NO:77, and comprising a CDRL1 of SEQ ID NO:17, CDRL2 of SEQ ID NO:18, and CDRL3 of SEQ ID NO:19,
b5) a light chain of SEQ ID NO:78, and comprising a CDRL1 of SEQ ID NO:20, CDRL2 of SEQ ID NO:21, and CDRL3 of SEQ ID NO:22,
b6) a light chain of SEQ ID NO:79, and comprising a CDRL1 of SEQ ID NO:23, CDRL2 of SEQ ID NO:24, and CDRL3 of SEQ ID NO:25,
b7) a light chain of SEQ ID NO:80, and comprising a CDRL1 of SEQ ID NO:26, CDRL2 of SEQ ID NO:27, and CDRL3 of SEQ ID NO:28,
b8) a light chain of SEQ ID NO:81, and comprising a CDRL1 of SEQ ID NO:29, CDRL2 of SEQ ID NO:30, and CDRL3 of SEQ ID NO:31,
b9) a light chain of SEQ ID NO:82, and comprising a CDRL1 of SEQ ID NO:32, CDRL2 of SEQ ID NO:33, and CDRL3 of SEQ ID NO:34,
b10) a light chain of SEQ ID NO:83, and comprising a CDRL1 of SEQ ID NO:35, CDRL2 of SEQ ID NO:36, and CDRL3 of SEQ ID NO:37,
b11) a light chain of SEQ ID NO:84, and comprising a CDRL1 of SEQ ID NO:38, CDRL2 of SEQ ID NO:39, and CDRL3 of SEQ ID NO:40,
b12) a light chain of SEQ ID NO:85, and comprising a CDRL1 of SEQ ID NO:41, CDRL2 of SEQ ID NO:42, and CDRL3 of SEQ ID NO:43,
b13) a light chain of SEQ ID NO:86, and comprising a CDRL1 of SEQ ID NO:44, CDRL2 of SEQ ID NO:45, and CDRL3 of SEQ ID NO:46,
b14) a light chain of SEQ ID NO:87, and comprising a CDRL1 of SEQ ID NO:47, CDRL2 of SEQ ID NO:48, and CDRL3 of SEQ ID NO:49,
b15) a light chain of SEQ ID NO:88, and comprising a CDRL1 of SEQ ID NO:50, CDRL2 of SEQ ID NO:51, and CDRL3 of SEQ ID NO:52,

b16) a light chain of SEQ ID NO:89, and comprising a CDRL1 of SEQ ID NO:53, CDRL2 of SEQ ID NO:54, and CDRL3 of SEQ ID NO:55,
b17) a light chain of SEQ ID NO:90, and comprising a CDRL1 of SEQ ID NO:56, CDRL2 of SEQ ID NO:57, and CDRL3 of SEQ ID NO:58,
b18) a light chain of SEQ ID NO:91, and comprising a CDRL1 of SEQ ID NO:59, CDRL2 of SEQ ID NO:60, and CDRL3 of SEQ ID NO:61,
b19) a light chain of SEQ ID NO:92, and comprising a CDRL1 of SEQ ID NO:62, CDRL2 of SEQ ID NO:63, and CDRL3 of SEQ ID NO:64,
b20) a light chain of SEQ ID NO:167, and comprising a CDRL1 of SEQ ID NO:71, CDRL2 of SEQ ID NO:72, and CDRL3 of SEQ ID NO:73,
b21) a light chain of SEQ ID NO:168, and comprising a CDRL1 of SEQ ID NO:142, CDRL2 of SEQ ID NO:143, and CDRL3 of SEQ ID NO:144
b22) a light chain of SEQ ID NO:169, and comprising a CDRL1 of SEQ ID NO:145, CDRL2 of SEQ ID NO:146, and CDRL3 of SEQ ID NO:147,
b23) a light chain of SEQ ID NO:170, and comprising a CDRL1 of SEQ ID NO:148, CDRL2 of SEQ ID NO:149, and CDRL3 of SEQ ID NO:150,
b24) a light chain of SEQ ID NO:171, and comprising a CDRL1 of SEQ ID NO:151, CDRL2 of SEQ ID NO:152, and CDRL3 of SEQ ID NO:153,
b25) a light chain of SEQ ID NO:172, and comprising a CDRL1 of SEQ ID NO:154, CDRL2 of SEQ ID NO:155, and CDRL3 of SEQ ID NO:156, and
b26) a light chain of SEQ ID NO:173, and comprising a CDRL1 of SEQ ID NO:157, CDRL2 of SEQ ID NO:158, and CDRL3 of SEQ ID NO:159.

166. The antibody according to embodiment 164, characterized in comprising as heavy chain variable region a heavy chain region of SEQ ID NO:5 or of SEQ ID NO:6 and as light chain a light chain selected from the group of

a) the light chain of SEQ ID NO:74,
b) the light chain of SEQ ID NO:75,
c) the light chain of SEQ ID NO:76,
d) the light chain of SEQ ID NO:77,
e) the light chain of SEQ ID NO:78,
f) the light chain of SEQ ID NO:79,
g) the light chain of SEQ ID NO:80,
h) the light chain of SEQ ID NO:81,
i) the light chain of SEQ ID NO:82,
k) the light chain of SEQ ID NO:83,
l) the light chain of SEQ ID NO:84,
m) the light chain of SEQ ID NO:85,
n) the light chain of SEQ ID NO:86,
o) the light chain of SEQ ID NO:87,
p) the light chain of SEQ ID NO:88,
q) the light chain of SEQ ID NO:89,
r) the light chain of SEQ ID NO:90,
s) the light chain of SEQ ID NO:91,
t) the light chain of SEQ ID NO:92,
u) the light chain of SEQ ID NO:167,
v) the light chain of SEQ ID NO:168,
w) the light chain of SEQ ID NO:169,
x) the light chain of SEQ ID NO:170,
y) the light chain of SEQ ID NO:171,
z) the light chain of SEQ ID NO:172,
aa) the light chain of SEQ ID NO:173,
ab) the light chain of SEQ ID NO:174,
ac) the light chain of SEQ ID NO:175,
ad) the light chain of SEQ ID NO:176,
ae) the light chain of SEQ ID NO:177,
af) the light chain of SEQ ID NO:178,
ag) the light chain of SEQ ID NO:179, and

ah) the light chain of SEQ ID NO:180.

167. The antibody according to embodiment 164, characterized in comprising as heavy chain variable region a heavy chain region of SEQ ID NO:5 or of SEQ ID NO:6 and as light chain a light chain selected from the group of

a) the light chain of SEQ ID NO:74,
b) the light chain of SEQ ID NO:75,
c) the light chain of SEQ ID NO:76,
d) the light chain of SEQ ID NO:77,
e) the light chain of SEQ ID NO:78,
f) the light chain of SEQ ID NO:79
g) the light chain of SEQ ID NO:80,
h) the light chain of SEQ ID NO:81,
i) the light chain of SEQ ID NO:82,
k) the light chain of SEQ ID NO:83,
l) the light chain of SEQ ID NO:84,
m) the light chain of SEQ ID NO:85,
n) the light chain of SEQ ID NO:86,
o) the light chain of SEQ ID NO:87,
p) the light chain of SEQ ID NO:88,
q) the light chain of SEQ ID NO:89,
r) the light chain of SEQ ID NO:90,
s) the light chain of SEQ ID NO:91,
t) the light chain of SEQ ID NO:92,
u) the light chain of SEQ ID NO:167,
v) the light chain of SEQ ID NO:168,
w) the light chain of SEQ ID NO:169,
x) the light chain of SEQ ID NO:170,
y) the light chain of SEQ ID NO:171,
z) the light chain of SEQ ID NO:172,
aa) the light chain of SEQ ID NO:173,
ab) the light chain of SEQ ID NO:174,
ac) the light chain of SEQ ID NO:175,
ad) the light chain of SEQ ID NO:176,
ae) the light chain of SEQ ID NO:177,
af) the light chain of SEQ ID NO:178,
ag) the light chain of SEQ ID NO:179, and
ah) the light chain of SEQ ID NO:180.

168. The antibody according to any one of embodiments 164 to 167, characterized in binding to human CEACAM5 and cynomolgus CEACAM5.

169. The antibody according to any one of embodiments 164 to 168, characterized in binding to human CEACAM5 and human CEACAM6.

170. The antibody according to any one of embodiments 164 to 169, characterized in being a Fab or a $F(ab)_2$ fragment.

171. The antibody according to any one of embodiments 164 to 170, characterized in being of human IgG1 type.

172. The antibody according to any one of embodiments 164 to 171, characterized in competing for binding to CEACAM5 with the anti-CEACAM5 antibody SM3E, said antibody SM3E comprises as VK and VH domains VK and VH of sequences SEQ ID NO:110 and 111.

173. The antibody according to any one of embodiments 164 to 172, characterized in being a bispecific antibody.

174. The antibody according to embodiment 173, characterized in being a bispecific antibody, specifically binding in first binding part to human CEACAM5 and in the second binding part to human CD47.

**Sequence List**

**Table 2**

| Sequence Number | Relates to |
|---|---|
| SEQ ID NO:1 | Mab CD47 CDRH1 |
| SEQ ID NO:2 | Mab CD47 CDRH2 |
| SEQ ID NO:3 | Mab CD47 CDRH3 |
| SEQ ID NO:4 | Mab CD47 VH |
| SEQ ID NO:5 | Mab CD47 heavy chain (VH-CH1) |
| SEQ ID NO:6 | Mab CD47 heavy chain (VH-CH1-CH2-CH3) |
| SEQ ID NO:7 | Mab CD47 CDRL1 |
| SEQ ID NO:8 | Mab CD47 CDRL2 |
| SEQ ID NO:9 | Mab CD47 CDRL3 |
| SEQ ID NO:10 | Mab CD47 VK |
| SEQ ID NO:11 | Mab CD47 light chain (VKCK; K2) |
| SEQ ID NO:12 | Mab CD47 light chain (VKCK; nucleic acid); (K2) |
| SEQ ID NO:13 | Mab CD47 constant light chain kappa (CK) |
| SEQ ID NO:14 | Mab CD47 constant light chain lambda (CL) |
| SEQ ID NO:15 | CEACAM antibody AC constant light chain lambda (CL) |
| SEQ ID NO:16 | CEACAM antibody AC constant light chain kappa (CK) |
| SEQ ID NO:17 | AC41 CDRL1; |
| SEQ ID NO:18 | AC41 CDRL2 |
| SEQ ID NO:19 | AC41 CDRL3 |
| SEQ ID NO:20 | AC42 CDRL1 |
| SEQ ID NO:21 | AC42 CDRL2 |
| SEQ ID NO:22 | AC42 CDRL3 |
| SEQ ID NO:23 | AC43 CDRL1 |
| SEQ ID NO:24 | AC43 CDRL2 |
| SEQ ID NO:25 | AC43 CDRL3 |
| SEQ ID NO:26 | AC44 CDRL1 |
| SEQ ID NO:27 | AC44 CDRL2 |
| SEQ ID NO:28 | AC44 CDRL3 |
| SEQ ID NO:29 | AC45 CDRL1 |
| SEQ ID NO:30 | AC45 CDRL2 |
| SEQ ID NO:31 | AC45 CDRL3 |
| SEQ ID NO:32 | AC46 CDRL1 |
| SEQ ID NO:33 | AC46 CDRL2 |
| SEQ ID NO:34 | AC46 CDRL3 |
| SEQ ID NO:35 | AC47 CDRL1 |

(continued)

| Sequence Number | Relates to |
|---|---|
| SEQ ID NO:36 | AC47 CDRL2 |
| SEQ ID NO:37 | AC47 CDRL3 |
| SEQ ID NO:38 | AC48 CDRL1 |
| SEQ ID NO:39 | AC48 CDRL2 |
| SEQ ID NO:40 | AC48 CDRL3 |
| SEQ ID NO:41 | AC49 CDRL1 |
| SEQ ID NO:42 | AC49 CDRL2 |
| SEQ ID NO:43 | AC49 CDRL3 |
| SEQ ID NO:44 | AC50 CDRL1 |
| SEQ ID NO:45 | AC50 CDRL2 |
| SEQ ID NO:46 | AC50 CDRL3 |
| SEQ ID NO:47 | AC52 CDRL1 |
| SEQ ID NO:48 | AC52 CDRL2 |
| SEQ ID NO:49 | AC52 CDRL3 |
| SEQ ID NO:50 | AC53 CDRL1 |
| SEQ ID NO:51 | AC53 CDRL2 |
| SEQ ID NO:52 | AC53 CDRL3 |
| SEQ ID NO:53 | AC54 CDRL1 |
| SEQ ID NO:54 | AC54 CDRL2 |
| SEQ ID NO:55 | AC54 CDRL3 |
| SEQ ID NO:56 | AC55 CDRL1 |
| SEQ ID NO:57 | AC55 CDRL2 |
| SEQ ID NO:58 | AC55 CDRL3 |
| SEQ ID NO:59 | AC56 CDRL1 |
| SEQ ID NO:60 | AC56 CDRL2 |
| SEQ ID NO:61 | AC56 CDRL3 |
| SEQ ID NO:62 | AC57 CDRL1 |
| SEQ ID NO:63 | AC57 CDRL2 |
| SEQ ID NO:64 | AC57 CDRL3 |
| SEQ ID NO:65 | AC58 CDRL1 |
| SEQ ID NO:66 | AC58 CDRL2 |
| SEQ ID NO:67 | AC58 CDRL3 |
| SEQ ID NO:68 | AC59 CDRL1 |
| SEQ ID NO:69 | AC59 CDRL2 |
| SEQ ID NO:70 | AC59 CDRL3 |
| SEQ ID NO:71 | AC60 CDRL1 |
| SEQ ID NO:72 | AC60 CDRL2 |
| SEQ ID NO:73 | AC60 CDRL3 |

(continued)

| Sequence Number | Relates to |
|---|---|
| SEQ ID NO:74 | AC41 VKCK |
| SEQ ID NO:75 | AC42 VKCK |
| SEQ ID NO:76 | AC43 VKCK |
| SEQ ID NO:77 | AC41 VK-H-CL1; hybrid |
| SEQ ID NO:78 | AC42 VK-H-CL1; hybrid |
| SEQ ID NO:79 | AC43 VK-H-CL1; hybrid |
| SEQ ID NO:80 | AC44 VLCL |
| SEQ ID NO:81 | AC45 VLCL |
| SEQ ID NO:82 | AC46 VLCL |
| SEQ ID NO:83 | AC47 VLCL |
| SEQ ID NO:84 | AC48 VLCL |
| SEQ ID NO:85 | AC49 VLCL |
| SEQ ID NO:86 | AC50 VLCL |
| SEQ ID NO:87 | AC52 VLCL |
| SEQ ID NO:88 | AC53 VLCL |
| SEQ ID NO:89 | AC54 VLCL |
| SEQ ID NO:90 | AC55 VLCL |
| SEQ ID NO:91 | AC56 VLCL |
| SEQ ID NO:92 | AC57 VLCL |
| SEQ ID NO:93 | AC58 VLCL |
| SEQ ID NO:94 | AC59 VLCL |
| SEQ ID NO:95 | AC60 VKCK |
| SEQ ID NO:96 | Human CEA (CEACAM5) |
| SEQ ID NO:97 | Human CEA (CEACAM6) |
| SEQ ID NO:98 | MAB CEA1 VH (SEQ31) and part of CEA VH-CH(EE)-Fc (hole, P329G LALA) [SEQ ID 36] aa TCB WO2017055389) and SEQ37 |
| SEQ ID NO:99 | MAB CEA1 VL (SEQ32) and part of Hum. CEA VL-CL(RK) [SEQ ID 38] aa TCB WO201705538 |
| SEQ ID NO:100 | MAB CD3 VH (SEQ33) and part of CD3 VH-CL(CK) aa TCB WO2017055389 [SEQ ID 34]) |
| SEQ ID NO:101 | MAB CD3 VL, (SEQ 34) and part of CEA VH-CH(EE)-CD3 VL-CH1-Fc (knob, P329G LALA) [SEQ ID 37] aa TCB WO2017055389) |
| SEQ ID NO:102 | CD3 VH-CL(CK) |
| SEQ ID NO:103 | CEA VH-CH(EE)-Fc (hole, P329G LALA) |
| SEQ ID NO:104 | CEAVH-CH(EE)-CD3 VL-CH1-Fc (knob, P329G) LALA) |
| SEQ ID NO:105 | CEA VL-CL(RK) |
| SEQ ID NO:106 | CD3 CH2527 Cross Fab VL-CH1 |
| SEQ ID NO:107 | CH1A10 VH CH1 FC Hole P329G LALA |
| SEQ ID NO:108 | CH1A1A CD3 CH2527 Cross Fab VH-CK FC Knob P329G LALA |

(continued)

| Sequence Number | Relates to |
|---|---|
| SEQ ID NO:109 | LC CEA |
| SEQ ID NO:110 | VK_SM3E |
| SEQ ID NO:111 | VH_SM3E |
| SEQ ID NO:112 | VK_SAR |
| SEQ ID NO:113 | VH_SAR |
| SEQ ID NO:114 | VK_CH1A1A |
| SEQ ID NO:115 | VH_ CH1A1A |
| SEQ ID NO:116 | CEA-TCB VH |
| SEQ ID NO:117 | CEA-TCB 1 VH |
| SEQ ID NO:118 | Epitope of CD3 epsilon |
| SEQ ID NO:119 | Mab CD47 Heavy chain (only VHCH); nucleic acid |
| SEQ ID NO:120 | Mab CD47 Light Chain K2 VKCK |
| SEQ ID NO:121 | AC41 VKCK |
| SEQ ID NO:122 | AC41 hybrid VK-H-CL1 |
| SEQ ID NO:123 | AC42 VKCK |
| SEQ ID NO:124 | AC42 hybrid VK-H-CL1 |
| SEQ ID NO:125 | AC43VKCK |
| SEQ ID NO:126 | AC43 hybrid VK-H-CL1 |
| SEQ ID NO:127 | AC44 VLCL |
| SEQ ID NO:128 | AC45 VLCL |
| SEQ ID NO:129 | AC46 VLCL |
| SEQ ID NO:130 | AC47VLCL |
| SEQ ID NO:131 | AC48 VLCL |
| SEQ ID NO:132 | AC49 VLCL |
| SEQ ID NO:133 | AC50 VLCL |
| SEQ ID NO:134 | AC52VLCL |
| SEQ ID NO:135 | AC53 VLCL |
| SEQ ID NO:136 | AC54 VLCL |
| SEQ ID NO:137 | AC55 VLCL |
| SEQ ID NO:138 | AC56 VLCL |
| SEQ ID NO:139 | AC57VLCL |
| SEQ ID NO:140 | AC58 VLCL |
| SEQ ID NO:141 | AC59 VLCL |
| SEQ ID NO:142 | AC61 CDRL1 |
| SEQ ID NO:143 | AC61 CDRL2 |
| SEQ ID NO:144 | AC61 CDRL3 |
| SEQ ID NO:145 | AC62 CDRL1 |
| SEQ ID NO:146 | AC62 CDRL2 |

(continued)

| Sequence Number | Relates to |
|---|---|
| SEQ ID NO:147 | AC62 CDRL3 |
| SEQ ID NO:148 | AC63 CDRL1 |
| SEQ ID NO:149 | AC63 CDRL2 |
| SEQ ID NO:150 | AC63 CDRL3 |
| SEQ ID NO:151 | AC64 CDRL1 |
| SEQ ID NO:152 | AC64 CDRL2 |
| SEQ ID NO:153 | AC64 CDRL3 |
| SEQ ID NO:154 | AC65 CDRL1 |
| SEQ ID NO:155 | AC65 CDRL2 |
| SEQ ID NO:156 | AC65 CDRL3 |
| SEQ ID NO:157 | AC66 CDRL1 |
| SEQ ID NO:158 | AC66 CDRL3 |
| SEQ ID NO:159 | AC66 CDRL3; |
| SEQ ID NO:160 | AC60 VKCK; DNA |
| SEQ ID NO:161 | AC61 VKCK; DNA |
| SEQ ID NO:162 | AC62 VKCK; DNA |
| SEQ ID NO:163 | AC63 VKCK; DNA |
| SEQ ID NO:164 | AC64 VKCK; DNA |
| SEQ ID NO:165 | AC65 VKCK; DNA |
| SEQ ID NO:166 | AC66 VKCK; DNA |
| SEQ ID NO:167 | AC60 VKCK |
| SEQ ID NO:168 | AC61 VKCK |
| SEQ ID NO:169 | AC62 VKCK |
| SEQ ID NO:170 | AC63 VKCK |
| SEQ ID NO:171 | AC64 VKCK |
| SEQ ID NO:172 | AC65 VKCK |
| SEQ ID NO:173 | AC66 VKCK |
| SEQ ID NO:174 | AC60 hybrid; VK-H-CL1 |
| SEQ ID NO:175 | AC61 hybrid; VK-H-CL1 |
| SEQ ID NO:176 | AC62 hybrid; VK-H-CL1 |
| SEQ ID NO:177 | AC63 hybrid; VK-H-CL1 |
| SEQ ID NO:178 | AC64 hybrid; VK-H-CL1 |
| SEQ ID NO:179 | AC65 hybrid; VK-H-CL1 |
| SEQ ID NO:180 | AC66 hybrid; VK-H-CL1 |
| SEQ ID NO:181 | MAB CD47 VKCL (kappa/lambda hybrid); VK H-CL1 (K2) |
| SEQ ID NO:182 | AC21 CDRL1 |
| SEQ ID NO:183 | AC21 CDRL2 |
| SEQ ID NO:184 | AC21 CDRL3 |

(continued)

| Sequence Number | Relates to |
|---|---|
| SEQ ID NO:185 | AC21 VLCL |
| SEQ ID NO:186 | AC21 VLCL DNA |
| SEQ ID NO:187 | Primer |
| SEQ ID NO:188 | Primer |
| SEQ ID NO:189 | Primer |
| SEQ ID NO:190 | Primer |
| SEQ ID NO:191 | Primer |
| SEQ ID NO:192 | Primer |

## EXAMPLES

### Example 1 Cloning, Expression and Purification of Human CD47

Cloning

The sequence corresponding to the extracellular domain of human CD47 (hCD47), is amplified from human cDNA by polymerase chain reaction (PCR) using specific oligonucleotides. The amplification product is gel-purified and cloned into the pEAK8 mammalian expression vector (Edge Biosystems, Gaithersburg, Md.). The vector is further modified to introduce an Avitag™ (Avidity, Denver Colo.) and a hexa-histidine tag at the C-terminus allowing for single site biotinylation of the protein and purification by IMAC (Immobilized Metal Ion Affinity Chromatography), respectively. The constructs are verified by DNA sequencing.

Expression

The plasmid is then transfected into mammalian cells using a liposome-based transfection reagent such as Lipofectamine2000 (Thermofisher Scientific). The transfection step requires only small quantities of DNA and cells, typically $2x10^5$ cells and 2 μg of plasmid DNA per well and the transfection carried out in a 6-well plate. Although different mammalian cell lines can be used, in the examples given below, transformed human embryo kidney monolayer epithelial cells (PEAK cells) are transfected. These cells stably express the EBNA-1 gene, further supporting the episomal replication process, are semi-adherent and can be grown under standard cell culture conditions (5% $CO_2$; 37°C in DMEM medium supplemented with 10% fetal calf serum). After 24 h, cells are placed under selective conditions by adding medium containing 0.5-2 μg/mL puromycin: cells harboring the episomal vector are resistant to this antibiotic.

Two to three weeks after transfection, amplified and selected cells were injected in disposable CELLine™ bioreactors for the production step. The CELLine™ is a two-compartment bioreactor that can be used in a standard cell culture incubator. The smaller compartment (15 ml) contains the cells and is separated from a larger (one liter) medium containing compartment by a semi-permeable membrane with a cut-off size of 10 kDa (Bruce et al. 2002, McDonald et al. 2005). This system allows for the diffusion of nutrients, gazes and metabolic waste products, while retaining cells and secreted proteins in the smaller compartment. The culture is maintained for 7-10 days before harvest of the supernatant. As the medium contains serum, the cells maintain good viability and several production runs can be generated using the same cells and containers.

Purification

After harvest, the cell culture supernatants are clarified by centrifugation. The supernatant is then supplemented with 100 mM imidazole and loaded on Ni-NTA affinity chromatography resin (Qiagen). The relatively high concentration of imidazole minimizes binding of contaminants to the resin. After washing of the column, proteins are eluted at a flow rate of 2 mL/min using a 30 mL imidazole gradient (20-400 mM imidazole) on an AKTA Prime chromatography system (Amersham Pharmacia Biotech). The elution gradient further improves the purity of the recombinant protein but can be replaced by a step elution approach if a chromatography system is not available. The eluted fractions can be analyzed by SDS-PAGE or ELISA to determine their content in recombinant protein. The fractions of interest are pooled and desalted on Amicon 10KD columns (Millipore) equilibrated with phosphate buffered saline or another appropriate buffer.

The desalted proteins can then be quantified using various techniques and their purity analyzed by SDS-PAGE. Recombinant CD47 is biotinylated in vitro using biotin ligase (Avidity, Denver Colo.) according to manufacturer's instructions. After desalting the biotinylation level is evaluated by pull-down assays using streptavidin magnetic beads and SDS-PAGE analysis.

**Example 2 Cloning, Expression and Purification of Human CEACAM family members**

Cloning

The sequence corresponding to the complete extracellular domain (ECD) and A3-B3 domains of CEACAM5 were synthesized by Eurofins and Twist Bioscience. These synthetic genes were subcloned into the pEAK8 mammalian expression vector (Edge Biosystems, Gaithersburg, Md.). The vectors were modified to introduce an Avitag™ (Avidity, Denver Colo.) and either a hexa-histidine tag, a human FC region or a mouse FC region at the C-terminus. Constructs were verified by DNA sequencing. Purification of recombinant soluble protein was carried out by IMAC (Immobilized Metal Ion Affinity Chromatography), FcXL or CaptureSelect™ IgG-Fc (ms) Affinity Matrix (Thermofisher Scientific).

Vectors encoding for the full-length version of human CEACAM 1, 3, 4, 5, 6, 7, 8, 18, 19, 20, 21 and cynomolgus CEACAM5 were also generated for expression at the cell surface of PEAK and/or CHO cells. The soluble, full-length human CEACAM16 was also similarly cloned.

Expression and Purification

The expression, purification and biotinylation of the above-mentioned recombinant proteins was carried out as detailed in Example 1.

**Example 3 Phage Display Selection of CEACAM5 Fvs Using Human scFv Libraries Containing Fixed Variable heavy domain**

General procedures for construction and handling of human scFv libraries displayed on M13 bacteriophage are described in Vaughan et al., (Nat. Biotech. 1996, 14:309-314), hereby incorporated by reference in its entirety. The libraries for selection and screening encode scFv that all share the same VH domain and are solely diversified in the VL domain. Methods for the generation of fixed VH libraries and their use for the identification and assembly of bispecific antibodies are described in US 2012/0184716 and WO 2012/023053, each of which is hereby incorporated by reference in its entirety. The procedures to identify scFv binding to human CECAM5 are described below.

Protein Selections

Aliquots of scFv phage libraries ($10^{12}$ Pfu) are blocked with PBS containing 3% (w/v) skimmed milk for one hour at room temperature on a rotary mixer. Blocked phage is deselected on streptavidin magnetic beads (Dynabeads™ M-280) for one hour at room temperature on a rotary mixer. Deselected phage is incubated with 100 nM of either biotinylated human CEACAM5 or the A3-B3 domain captured on streptavidin magnetic beads for two hours at room temperature on a rotary mixer. Beads are captured using a magnetic stand followed by five washes with PBS/0.1% Tween 20 and two washes with PBS. Phage is eluted with 100 nM TEA for 30 minutes at room temperature on a rotary mixer. Eluted phage and beads are neutralized with Tris-HCl 1M pH 7.4 and directly added to 10 ml of exponentially growing TG1 cells and incubated for one hour at 37°C with slow shaking (90 rpm). An aliquot of the infected TG1 is serial diluted to titer the selection output. The remaining infected TG1 are spun at 3800 rpm for 10 minutes and resuspended in 2 ml 2xTY and spread on 2xTYAG (2xTY medium containing 100 μg/ml ampicillin and 2% glucose) agar Bioassay plates. After overnight incubation at 30°C, 10 ml of 2xTY is added to the plates and the cells are scraped from the surface and transferred to a 50 ml polypropylene tube. 50% glycerol solution is added to the cell suspension to obtain a final concentration of 17% glycerol. Aliquots of the selection rounds are kept at -80°C.

Phage Rescue

50 μl of cell suspension obtained from previous selection rounds are added to 50 ml of 2xTYAG and grown at 37°C with agitation (240 rpm) until an $OD_{600}$ of 0.3 to 0.5 is reached. The culture is then super-infected with $1.2x10^{11}$ M13K07 helper phage and incubated for one hour at 37°C (90 rpm). The medium is changed by centrifuging the cells at 3800 rpm for 10 minutes, removing the medium and resuspending the pellet in 50 ml of 2xTYAK (2xTY medium containing 100 μg/ml ampicillin; 50 μg/ml kanamycin). The culture is then grown overnight at 30°C (240 rpm). The next day, the phage containing supernatant is used for the next round of selection.

Cell Surface Selections

Phage containing supernatants are blocked with PBS containing 3% (w/v) skimmed milk for one hour at room temperature on a rotary mixer. Blocked phage is then deselected for one hour on MKN45 $CEACAM5^{KO}$ that do not express human CEACAM5. Deselected phage is incubated with $2x10^7$ MKN45 cells expressing CEACAM5 (blocked in PBS 3% BSA 0.1% $NaN_3$) for two hours at room temperature with gentle shaking. Cells are pelleted and washed six times with PBS. Bound phage is eluted with 76 mM citric acid and shaking for 10 minutes. After neutralization with Tris-HCl 1M pH 8 the cells are added directly to 10 ml of exponentially growing TG1 and incubated for one hour at 37°C with slow shaking. An aliquot of the infected TG1 is serial diluted to titer the selection output. Infected TG1 are spun at 3800 rpm for 10 minutes and resuspended in 2 ml 2xTY medium and spread on a 2xTYAG agar Bioassay plate. After overnight incubation at 30°C 10 ml of 2xTY is added to the plate and the cells are scraped from the surface and transferred to a 50 ml polypropylene tube. 50% glycerol solution is added to the cell suspension to obtain a final concentration of 17% glycerol. Aliquots of the selection rounds are kept at -80°C.

**Example 4 Screening for scFv Binding/Non-binding to CEACAM5, CEACAM6, and CEACAM1**

scFv Periplasmic Preparation for Binding and Functional Tests

Individual clones are inoculated into a deep-well microtiter plate containing 0.9 ml per well of 2xTYAG medium (2xTY medium containing 100 μg/ml ampicillin, 0.1% glucose) and grown at 37°C for 5-6 hours (240 rpm). 100 μl per well of 0.2 mM IPTG in 2xTY medium are then added to give a final concentration of 0.02 mM IPTG. The plate is incubated overnight at 30°C with shaking at 240 rpm. The deep-well plate is centrifuged at 3200 rpm for 10 minutes at 4°C and the supernatant carefully removed. The pellets are resuspended in 150 μl TES buffer (50 mM Tris-HCl (pH 8), 1 mM EDTA (pH 8), 20% sucrose, complemented with Complete protease inhibitor, Roche). A hypotonic shock is produced by adding 150 μl of diluted TES buffer (1:5 TES:water dilution) and incubation on ice for 30 minutes. The plate is centrifuged at 4000 rpm for 10 minutes at 4°C to pellet cells and debris. The supernatants are carefully transferred into another microtiter plate and kept on ice for immediate testing in functional assays or binding assays.

Binding

Screening of scFv for binding to CEACAM5 is tested in a homogenous assay using CellInsight™ technology. The following reagents are mixed in each well of a 384 clear bottom well plate (Corning): 30 μl of a streptavidin polystyrene bead suspension (Polysciences; 3000 beads/well) coated with either biotinylated CEACAM5, biotinylated domain A3-B3 or biotinylated NusA for a control protein; 60 μl of blocked scFv periplasmic preparation; 10 μl of detection buffer (PBS containing mouse anti-c-myc antibody at 5 μg/ml; anti-mouse Fc AlexaFluor® 647 diluted 1:200). After mixing at 600 rpm for 5 minutes, the 384-well plate is incubated at room temperature and read after 2 hours on a CellInsight™ CX5 High-Content Screening platform (ThermoFisher Scientific). Clones expressing scFv giving a specific signal for CEACAM5 and not NusA are selected for further analysis or sequencing.

Binding to CEACAM1, CEACAM6 and other CEACAMs can be measured in the same manner.

Phage Clone Sequencing

Single clones are inoculated into a 96-deep-well microtiter plate containing 1 ml LBAG medium (LB medium with 100 μg/ml ampicillin and 2% glucose) per well and grown overnight at 37°C, 240 rpm. DNA is extracted using the Zyppy-96 Plamis Miniprep kit (Zymo Research) and sequenced.

**Example 5 Fixed VH Candidates Reformatting into IgG and Transient Expression in Mammalian Cells and further optimization of antibodies according to the invention (Lead Optimization LO)**

1. Reformatting of scFv antibodies into IgG1 and production in PEAK cells

After screening and sequencing, scFv candidates with the desired binding properties are reformatted into IgG1 antibodies and expressed by transient transfection into PEAK cells. The VH and VL sequences of selected scFv are amplified with specific oligonucleotides and cloned into an expression vector containing the heavy and light chain constant regions and the constructions are verified by sequencing. The expression vectors are transfected into mammalian cells using Lipofectamine 2000 (Thermofisher Scientific) according to manufacturer's instructions. Briefly, $3.5x10^6$ PEAK cells are cultured in T75 flasks in 25 ml culture media containing fetal bovine serum. Transfected cells are cultured for 5-6 days at 37°C, IgG1 production is quantified by OctetRED96 instrument. The supernatant is harvested for IgG1 purification on FcXL affinity resin (Thermofisher Scientific) according to manufacturer's instructions. Briefly, supernatants from transfected cells are incubated overnight at 4°C with an appropriate amount of FcXL resin. After resin wash by PBS, samples are loaded on Amicon Pro column and the IgG consequently eluted in 50 mM Glycine pH3.5. The eluted IgG fraction is then dialyzed by Amicon 50kDa against Histidine NaCl pH6.0 buffer and the IgG1 content is quantified by absorption at 280 nm. Purity and IgG1 integrity are verified by Agilent Bioanalyzer manufacturer (Agilent Technologies, Santa Clara, Calif., USA).

2. Further Optimization of promising IgG1 lead candidates (Lead Optimization LO)

Mutations are introduced in some positions in the CDR1, CDR2 and/or in the CDR3 of the variable light chain (VL) of the IgG1 candidate (parenteral VL) to be optimized, thereby creating lead optimization phage libraries. Using the phage display technology (see Example 3), variants with improved binding affinities are selected.

The diversity in the CDRs is introduced in the parental VL sequence by using synthetic degenerated oligonucleotides and PCR assembling to generate VL fragments that are cloned into the expression vector pNDS containing the fixed variable heavy chain (VH). The plasmids thus created are transformed into TG1 bacteria by electroporation. Lead optimization libraries have typically diversities of ~108-109 variants. The transformed TG1 are then rescued by the helper phage M13KO7 to produce phage displaying scFv at the surface. These phage are used for rounds of phage display selections (for Screening/Sélections see Example 4) where the stringency was maintained relatively high using target concentrations in the 1-10 nM range in order to enable enrichment for candidates with higher affinities.

**Example 6 Characterization of CEACAM5 Antibodies**

a) Binding of CEACAM5 antibodies to cells transfected with different members of the CEACAM family

According to the knowledge of the inventors specificity of CEACAM5 monoclonal antibodies (mAbs) can be shown by flow cytometry using PEAK and/or CHO cells transfected with different members of the CEACAM family. Vectors encoding the full-length version of human CEACAM 1, 3, 4, 5, 6, 7, 8, 18, 19, 20 and 21 and 20 are used to express these proteins at the surface of PEAK and/or CHO cells as described in Example 2. Non-transfected PEAK and/or CHO cells are used as negative control. Cells are harvested, counted, checked for viability and resuspended at $3\times10^6$ cells/ml in FACS buffer (PBS 2% BSA, 0.1% $NaN_3$). 100 μl of the cell suspension are distributed in V-bottom 96-well plates ($3\times10^5$ cells/well). The supernatant is removed by centrifugation 3 minutes at 4°C, 1300 rpm and the cells incubated for 15 minutes at 4°C with increasing concentrations of the antibody according to the invention. The antibodies are diluted in FACS buffer and the concentration range is 30 pM-500 nM. Cells are washed twice with cold FACS buffer and re-incubated for further 15 minutes at 4°C with the PE (R-phycoerythrin)-conjugated mouse anti-human IgG Fc secondary antibody (SouthernBiotech, pre-diluted 1:100 in FACS buffer). Cells are washed twice with cold FACS buffer and resuspended in 300 μl FACS buffer with 1:1500-diluted TOPRO-3 (Invitrogen). Fluorescence is measured using a FACSCalibur™ (BD Biosciences). Dose-response binding curves are fitted using GraphPad Prism7 software. In the same manner, CEACAM1, CEACAM6 and other CEACAMs can be characterized. In brief, purified Mabs are incubated with cells expressing one of the CEACAM family proteins at a final concentration of 10 μg/ml for 30 minutes. After two washes, bound antibodies are detected using a Cy-5 conjugated anti-human Fc secondary antibody (BD biosciences).

An antibody according to the invention is found as non-binding to said CEACAM, if no bound antibody is detected by the PE-conjugated anti-human IgG Fc secondary antibody.

b) Binding of the antibodies of the invention to recombinant human CEACAM5 and cynomolgus CEACAM5 to determine cross-reactivity (ELISA based assay)

Biotinylated recombinant human CEACAM5 or CEACAM6 proteins or recombinant cynomolgus monkey CEACAM5 are captured at 0.5 μg/mL in a streptavidin coated 96-well microplate. The plate is washed and monoclonal anti-CEA bivalent antibodies of the present invention are added as a broad concentration-range (e.g. from 5x10-4 to 10 μg/mL) and incubated during 1 hr. The plate is washed and bound antibodies are detected with an anti-human IgG(Fc)-HRP (Jackson ImmunoResearch). After washing, the plate is revealed with Amplex Red reagent (Molecular Probes). The fluorescence signal is measured on a Synergy HT plate reader (Biotek). Results are shown in table 3 and figure 6.

**Table 3. EC50 for binding on human CEACAM5 and cyno CEACAM5 (determined by ELISA using recombinant proteins) for 22 anti-human CEACAM5 mAbs and the 2 anti-human CEACAM5/CEACAM6 mAbs AC49 and AC50.**

| Antibody name | EC50 (nM) binding to human CEACAM5 | EC50 (nM) binding to cyno CEACAM5 |
|---|---|---|
| AC41 | 0.3 | 0.6 |
| AC42 | 0.1 | 0.6 |
| AC43 | 0.08 | 0.7 |
| AC44 | 0.09 | 0.07 |
| AC45 | 0.1 | 0.03 |
| AC46 | 0.07 | 0.04 |
| AC47 | 0.03 | 0.06 |
| AC48 | 0.05 | 0.04 |
| AC49 | 0.1 | 0.03 |
| AC50 | 0.09 | 0.05 |
| AC52 | 0.03 | 0.02 |
| AC53 | 0.03 | 0.02 |
| AC54 | 0.03 | 0.02 |
| AC55 | 0.03 | 0.02 |
| AC56 | 0.02 | 0.02 |
| AC57 | 0.01 | 0.02 |
| AC58 | 0.04 | 0.04 |
| AC59 | 0.05 | 0.05 |
| AC60 | 0.013 | 0.06 |
| AC61 | 0.008 | 0.02 |
| AC62 | 0.012 | 0.01 |
| AC63 | 0.016 | 0.02 |
| AC64 | 0.016 | 0.03 |
| AC65 | 0.014 | 0.03 |
| AC66 | 0.012 | 0.01 |

**Example 7 Expression and Purification of Bispecific Antibodies Carrying a Lambda and a Kappa Light Chain**

The simultaneous expression of one heavy chain and two lights chain in the same cell can lead to the assembly of three different antibodies. Simultaneous expression can be achieved in different ways such as that the transfection of multiple vectors expressing one of the chains to be co-expressed or by using vectors that drive multiple gene expression. The vector encoding the different anti-CEACAM5 antibodies are co-transfected with another vector expressing the heavy and light chain of anti-CD47 antibody K2 (SEQ ID NO:5 and 11), an anti-CD47 antibody bearing the same common heavy chain and that is described in US 2014/0303354. Alternatively, the two light chains are cloned into the vector pNovi κHλ that is previously generated to allow for the co-expression of one heavy chain, one Kappa light chain and one Lambda light chain as described in US 2012/0184716 and WO 2012/023053, each of which is hereby incorporated by reference in its entirety. The expression of the three genes is driven by human cytomegalovirus promoters (hCMV) and the vector also contains a glutamine synthetase gene (GS) that enables the selection and establishment of stable cell lines. The common VH and the VL genes of the anti- CEACAM5 IgG and of the anti-CD47 IgG are cloned in the vector pNovi κHλ, for transient expression in mammalian cells. Peak cells are cultured in appropriate Flask with suitable cells number and culture medium volume (containing fetal bovine serum). Plasmid DNA is transfected into the cells using Lipofectamine 2000) according to manufacturer's instructions. Antibody concentration in the supernatant of transfected cells is measured during the production using OctetRED96. According to antibody concentration, supernatants are harvested 5 to 7 days after transfection and clarified by centrifugation at 1300 g for 10 min. The purification process is composed of three affinity steps. First, the FcXL affinity matrix (Thermofisher Scientific) is washed with PBS and then added in the clarified supernatant. After incubation overnight at +4°C, supernatants are centrifuged at 2000 g for 10 min, flow through is stored and resin washed twice with PBS. Then, the resin is transferred on Amicon Pro columns and a solution containing 50 mM glycine at pH 3.0is used for elution. Several elution fractions are generated, pooled and desalted against PBS using 50 kDa Amicon™ Ultra Centrifugal filter units (Merck KGaA, Darmstadt, Germany). The elueted product, containing total human IgGs from the supernatant, is quantified using a Nanodrop spectrophotometer (NanoDrop Technologies, Wilmington, Del.) and incubated for 15 min at RT and 20 rpm with the appropriate volume of Kappa select affinity matrix (GE Healthcare). Incubation, resin recovery, elution and desalting steps are performed as described previously. The last affinity purification step is performed using the lambda Fab select affinity matrix (GE Healthcare) applying the same process as for the two previous purifications. The final product is quantified using the Nanodrop. Purified bispecific antibodies are analyzed by electrophoresis in denaturing and reducing conditions. The Agilent 2100 Bioanalyzer is used with the Protein 80 kit as described by the manufacturer (Agilent Technologies, Santa Clara, Calif., USA). 4 μL of purified samples are mixed with sample buffer supplemented with dithiothreitol (DTT; Sigma Aldrich, St. Louis, Mo.). Samples are heated at 95°C for 5 min and then loaded on the chip. All samples are tested for endotoxin contamination using the Limulus Amebocyte Lysate test (LAL; Charles River Laboratories, Wilmington, Mass.).

**Example 8: Characterization of Monovalent and Bispecific Antibodies**

a) Dual-targeting bispecific antibodies bind to two different antigens on the surface of the same cell.

According to the knowledge of the inventors simultaneous binding of the two antibody arms to two antigens on the surface of the cell (termed co-engagement) may result in additive or synergistic increase of affinity due to avidity mechanism. As a consequence, co-engagement confers high selectivity towards cells expressing both antigens as compared to cells that express just one single antigen. In addition, the affinities of the two arms of a bispecific antibody to their respective targets can be set up in a way that binding to target cells is principally driven by one of the antibody arms. For instance, a dual targeting κλ antibody composed of one arm binding with high affinity to CEACAM5 or to CEACAM5 and CEACAM6, and a second arm binding with lower affinity to CD47-but sufficient to inhibit CD47/SIRPα upon CEACAM5 or CEACAM5 and CEACAM6 co-engagement with CD47 should allow preferential inhibition of CD47 in cancer versus normal cells.

b) Affinity Measurement to human CD47

According to the knowledge of the inventors the binding affinity of the antibodies according to the invention to human CD47 can be evaluated by surface plasmon resonance technology using a Biacore T200 instrument. The biotinylated human CD47 soluble recombinant protein can be captured on a streptavidin coated sensor chip (Series S Sensor Chip SA). Then a concentration series of the test antibody can be injected over the surface, with regeneration of the surface between each injection.

Such measurements were performed with a CD19xCD47 κλ bispecific antibody. The binding affinity measured in repeated determinations was between 400 and 500 nM. The CD47 binding arm of this antibody is the same as the CD47 binding arm of the CEAxCD47 bispecific antibodies of this invention (in one embodiment the sequences SEQ ID NO:1 to 14, 119, 120 and 181 of table 2, sequence list). According to the knowledge of the inventors that same experiments performed with the CEAxCD47 bispecific antibodies of the invention will provide similar results within the standard deviation of such experiments. This hold especially also true for the reference bispecific antibody K2AC54.

c) SIRPα Blocking Activity of Monovalent and Bispecific Antibodies to demonstrate co-engagement of CEACAM5 and CD47 on surface of target tumor cells

According to the knowledge of the inventors another series of experiments can be performed which can provide a proof of co-engagement of CEACAM5 and CD47 on the surface of the target cell are experiments showing that the neutralization of CD47-SIRPα interaction by CD47x CEACAM5 κλ antibodies is CEACAM5 dependent. In such experiments, the activity of CD47x CEACAM5 κλ bodies and the corresponding monovalent antibodies can be tested in the CD47-SIRPα inhibition assay.

d) SIRPα Blocking Activity of CD47 Antibodies

Experimental set-up for the measurement of the SIRPα inhibition potency data shown for bispecific antibodies of this invention (results see table 4):
The detection of bound SIRPα cell-based assay monitoring the interaction of soluble SIRPα with human CD47 expressed at the surface of MKN45 is used for the detection of the blocking activity. Dose-response experiments with bispecific antibodies according to the invention allow determination of an IC50 value.

MKN45 cancer cells, expressing both CD47 and CEACAM5, are stained with CFSE violet to allow the imaging system (CX5) to detect the cells. Briefly, 3'000 stained MKN45 cells per well are seeded in a 384 optical well plate (Costar) and incubated for 50 minutes with increased concentrations of bispecific antibodies of the invention (1.9 pM to 333 nM, in quadruplicates). Then, a fixe concentration of SIRPα mouseFc premixed with anti-mouse IgG-Fc AF647 coupled antibody (Jackson Immunoresearch diluted 1:2000) is added at 50ng/mL final. After an incubation of 3H30 plates are acquired with the imaging system (CX5, Thermofisher) and fluorescence signals emitted by the detected bound SIRPα is recorded by the software dedicated to the imaging system. Fluorescence signals are plotted according to the dose range tested and IC50 are calculated by the software (Prism, Graphpad).

Table 4 shows the potency of several CEAxCD47 bispecific antibodies at inhibiting CD47/SIRPα binding displaying a range of IC50, from 0.16nM to 9.4nM.

e) Epitope binning of CEACAM5 antibodies by competition with reference antibodies

Epitope binning is a competitive immunoassay used to characterize the binding of antibodies according to the invention or e.g. the binding of the related anti-CEA (target protein) antibodies of the first binding part. A competitive blocking profile of an antibody binding to the target protein is created against antibodies also binding to this target protein and for which the binding epitope has already been established/published. Competition to one of these reference antibodies indicate that the antibody has the same or a closely located epitope and they are "binned" together. The ability of CEACAM5 mAbs, which are part of the bispecific antibodies of the present invention to compete with CEACAM5 reference antibodies is tested by ELISA on recombinant human CEACAM5 with the following reference antibodies carrying a mouse Fc region: SM3E, sequences of mAb derived from SM3E described in patent US20050147614A1, mAb produced using standard methods; MEDI, mAb derived from MEDI-565 described in patent WO2016036678A1; SAR, mAb derived from Mab2_VLg5VHg2 described in patent EP3199552A1; CH1A1A, mAb derived from CH1A1A-2F1 described in patent US20120251529 and by Klein et al in Oncoimmunology, 2017 Jan 11;6(3); humanized T84.66 mAb derived from variant 1 described in patent WO2017055389; LAB mAb derived from hMN14 described in patent US 2002/0165360 A1. SM3E binds e.g. more to the N-terminal, cell membrane distal part of CEA, MEDI to the middle part and CH1A1A binds close to the membrane.

Biotinylated human CEACAM5 is coated at 0.5 μg/ml in a Streptavidin-coated 96-well plate and incubated with 10 μg/ml of the reference mAbs or an irrelevant mAb carrying a mouse Fc region for 1 hour. The CEACAM5 mAbs (as bivalent monoclonal anti-CEA antibodies and not as respective CEAxCD47 bispecific antibodies) are added at 0.2 μg/ml for 1 hour at room temperature. The plate is washed and the bound CEACAM5 mAbs are detected with an anti-human IgG(Fc)-HRP (Jackson ImmunoResearch). After washing, the plate is revealed with Amplex Red reagent. The fluorescence signal is measured on a Synergy HT plate reader (Biotek).

The competition experiments were for all of the CEAxCD47 bispecific antibodies according to the invention performed with the respective anti-CEA bivalent monoclonal antibodies. In case binding of such a monoclonal antibody to CEACAM5 was reduced by the respective tool antibody by 80% or more, it was concluded that the CEAxCD47 bispecific antibody is classified to bind competitively with the tool antibody. A CEAxCD47 antibody is identified as non-competitive with a tool antibody in case binding of the respective anti-CEA bivalent mAb to CEACAM5 is reduced by 20% or less if the results with and w/o addition of a tool antibody are compared. Results: All the antibodies listed in Table 3 above with the only exception of AC58 and 59 are competitive with SM3E (binding to N-terminal domain of CEACAM5). Antibodies AC58 and AC59 are competitive with SAR binding to A3B3 domain of CEACAM5 close to the cell membrane).

Results for Bin characterization, EC50 values as well as maximum of binding to CEACAM5 expressed on MKN-45wt cells, SIRPα inhibition potency, and EC50 as well as maximal. index of phagocytosis for bispecific antibodies according to the invention are shown in tables 4 and 5.

**Table 4: *In vitro* characteristics of CEAxCD47 bispecific antibodies**

| Antibody name | Domain binding | BiAb format | Binding on cells# | | SIRPα inhibition potency (nM)# |
|---|---|---|---|---|---|
| | | | EC50 (nM) | Emax (MFI*; x10$^6$) | |
| K2AC41 | SM3E | Hybrid | 145 | 1.41 | 3.7 |
| AC41K2 H-CL1 | SM3E | Hybrid | 146 | 1.52 | 6.8 |
| K2AC42 | SM3E | Hybrid | 123 | 1.52 | 2.9 |
| AC42K2 H-CL1 | SM3E | Hybrid | 129 | 1.84 | 3 |
| K2AC43 | SM3E | Hybrid | 135 | 1.77 | 3.9 |
| AC43K2 H-CL1 | SM3E | Hybrid | 121 | 2.08 | 3.2 |
| K2AC44 | SM3E | KL | 140 | 1.62 | 3.3 |
| K2AC45 | SM3E | KL | 256 | 1.65 | 9.4 |
| K2AC46 | SM3E | KL | 243 | 1.14 | 7.9 |
| K2AC47 | SM3E | KL | 11 | 2.27 | 0.7 |
| K2AC48 | SM3E | KL | 201 | 2.19 | 4.4 |
| K2AC52 | SM3E | KL | 219 | 3.30 | 1.1 |
| K2AC53 | SM3E | KL | 38 | 2.63 | 0.3 |
| K2AC54 | SM3E | KL | 19 | 2.75 | 0.16 |
| K2AC55 | SM3E | KL | 221 | 3.90 | 1.6 |
| K2AC56 | SM3E | KL | 47 | 1.59 | 2.8 |
| K2AC57 | SM3E | KL | 91 | 2.26 | 1.1 |
| K2AC58[1] | SAR | KL | 20 | 0.3 | NOT TESTED |
| K2AC59[1] | SAR | KL | 26 | 0.4 | NOT TESTED |
| # using MKN45 wt cancer cell line, #Mean Fluorescence Intensity<br>[1]: which means that binding of K2AC58 and K2AC59, respectively AC58 and 59, is in the A3B3 domain of CEACAM5, which is proximal to the cell membrane (if CEACAM5 is bound to the membrane) and therefore far away from SM3E bin, which is proximal to N-terminus. | | | | | |

AC58, AC59 and the bispecifics antibodies K2AC58 and K2AC59, resulting from a Lead Optimization approach as described in Example 5 (2.) of parenteral antibody AC21 (identified in a phage display library approach), are much weaker in binding to MKN-45 cells and in phagocytosis of MKN-45 cells (see table 4 - low Emax for binding and figure 7 for phagocytosis compared to reference biAb K2AC54) as compared to the SM3E binding anti CEA antibodies according to the invention and the respective SM3E binding bispecific antibodies according to the invention. K2AC41 means that there is a fully kappa LC (VL and CL as kappa) in the K2 anti CD47 arm and in the AC41 arm the VL is kappa and therefore a lambda hybrid CL is introduced (or in other words AC41 LC is hybrid). In contrast in AC41K2 H-CL1 the AC41 LC is fully kappa, therefore in the CD47 arm the LC is hybrid with kappa VL and lambda CL (same for K2AC42 and K2AC43 and also the CEAxCD47 bispecific antibodies based on AC60 to AC66).

**Table 5: *In vitro* functional activity CEAxCD47 bispecific antibodies***

**Table 5A.** *In vitro* functional activity characteristics of CEACAM5xCD47 bispecific antibodies K2AC52 - K2AC57.

| Antibody name | EC50 (μg/mL) of phagocytosis | Max Index of phagocytosis (±SD) |
|---|---|---|
| K2AC52 | 1 | 31.5 (±0.7) |

(continued)

| Antibody name | EC50 (μg/mL) of phagocytosis | Max Index of phagocytosis (±SD) |
|---|---|---|
| K2AC53 | 0.8 | 31 |
| K2AC54 | 0.3 | 31.5 (±2.1) |
| K2AC55 | 0.9 | 29 (±1.4) |
| K2AC56 | 5 | 30 (±1.4) |
| K2AC57 | 1.6 | 30 (±1.4) |

**Table 5B.** *In vitro* functional activity of characteristics of CEACAM6 cross-reactive CEACAM5xCD47 bispecific antibodies K2AC49 and K2AC50 (measured with imaging based ADCP assay, Example 9e).

| Antibody name | EC50 (μg/mL) of phagocytosis | Max Index of phagocytosis (±SD) |
|---|---|---|
| K2AC49 | 0.3 | 50.5 (±3.5) |
| K2AC50 | 0.07 | 43 (±4.2) |

f) Binding of anti-CEACAM antibodies to human CEACAM5 and human CEACAM6 (ELISA based assay)

Biotinylated recombinant human CEACAM5 or CEACAM6 proteins are captured at 0.5μg/mL in a streptavidin coated 96-well microplate. The plate is washed and monoclonal anti-CEA bivalent antibodies of the present invention are added as a broad concentration-range (e.g. from $5x10^{-4}$ to 1μg/mL) and incubated during 1 hr. The plate is washed and bound antibodies are detected with an anti-human IgG(Fc)-HRP (Jackson ImmunoResearch). After washing, the plate is revealed with Amplex Red reagent (Molecular Probes). The fluorescence signal is measured on a Synergy HT plate reader (Biotek).

Results obtained for the monoclonal antibodies 49 and AC50 are contained in table 6 and figures 4 and 5; these antibodies show balanced CEACAM5 and CEACAM6 binding, that means EC50 for binding to CEACAM5 and CEACAM6 are similar (range of the ratio of the EC50 for CEACAM5 binding to CEACAM6 binding of balanced antibodies from 0.2 to 5). Antibodies with a ratio outside such ranges are considered as not balanced.

**Table 6. EC50 binding on human CEACAM5 and human CEACAM6 by ELISA using recombinant proteins for two anti-CEA mAbs.**

| Antibody name | EC50 (nM) binding to human CEACAM5 | EC50 (nM) binding to human CEACAM6 |
|---|---|---|
| AC49 | 0.1 | 0.2 |
| AC50 | 0.09 | 0.03 |

**Example 9: ADCC and ADCP Mediated by Bispecific Antibodies**

a) ADCP and ADCC mediated by CEAxCD47 bispecific antibodies is CEA dependent

The ability of dual targeting CEAxCD47 κλ antibodies to co-engage CD47 and CEACAM5 results in a significant increase in the affinity of binding to CEA-positive cells as compared to CEACAM5-negative cells and in CEACAM5 -dependent neutralization of the CD47-SIRPα interaction. This, in turn, could translate into efficient and selective cancer cell killing mediated by CEAxCD47 κλ antibodies.

b) $Cr51^+$ release assay, measured with CEACAM5xCD47 antibodies

According to the knowledge of the inventors, healthy PBMC were activated overnight at 37°C with RPMI/10% heat inactivated FCS supplemented with 10 ng/mL of recombinant hIL-2. The next day, targets cells (i.e. cancer cells expressing the CEACAM5) were incubated with 100 μCi Cr51 (Perkin Elmer, 37°C, 1h). After washing, cells were opsonized with test antibodies (30 min, 37°C). Cr51-loaded cancer cells were then mixed with PBMC cells to obtain the final 80:1 or 50:1 ratio between effector (PBMC) and target cells (CEACAM5-expressing cells). The cell mixture was incubated for 4h at 37°C before being centrifuged for 10 min at 1500 rpm. Supernatant was transferred into a LumaPlate (coated with scintillant) and counted in a γ-counter. Negative controls (spontaneous Cr51 release) consisted of Cr51-loaded target cells incubated with medium in the absence of effector cells. Total lysis control consisted of Cr51-loaded target cells incubated with 5 μL of cell lysis solution (Triton X-100). Nonspecific lysis control (baseline) consisted of Cr51-loaded target cells incubated with effector cells, without Ab. The ADCC percentage was calculated using the following formula: % specific ADCC = ((sample counts per minute (cpm) - nonspecific lysis control cpm)/(total lysis control cpm - negative control cpm)) x 100%.

c) ADCC measured by LDH release assay

According to the knowledge of the inventors, ADCC of the CEAxCD47 bispecific antibodies was tested in the following assay:
Healthy PBMC were activated overnight at 37°C with RPMI/10% heat inactivated FCS supplemented with 10 ng/mL of recombinant hIL-2. The next day, target cells (e.g. MKN45 cancer cells) are opsonized with different concentrations of tested antibodies. The PBMCs and the opsonized target cells are co-incubated at a ratio effector/target 50/1 in round bottom plates for 6 hours at 37°c in a cell culture incubator. After this incubation, supernatants are transferred into optical flat bottom plate and the LDH release is quantified with a commercial kit from Roche by measuring OD with a microplate reader. The % of specific lysis is calculated with the following formula:

$$\text{Specific lysis} = \left(\frac{\text{LDH Sample} - (\text{LDH Effector} + \text{Target cells})}{\text{Maximum LDH} - \text{LDH Target cells alone}}\right) \times 100$$

d) ADCP assay

Two methods are used. In the FACS based method the percentage of phagocytosis (representing the percentage of macrophages which have engulfed at least one tumor cell) is determined. With the imaging-based method, which makes use of the CellInsight CX5 High Content Screening Platform, the phagocytosis index, defined as the average number of target cells engulfed by 100 macrophages, is determined (see figure 3).

e) Phagocytosis Assays: 1. Imaging assay based on CellInsight CX5 High Content Screening Platform and 2. Flow cytometry based assay

Preparation of the macrophages: Human peripheral blood mononuclear cells (PBMCs) are isolated from buffy coats by Ficoll gradient. Macrophages are generated by culturing PBMCs for 7 days in complete medium (RPMI 1640, 10% heat-inactivated fetal calf serum [Invitrogen]), 2 mM L-glutamine, 1 mM sodium pyruvate, 10 mM HEPES buffer, 25 mg/mL gentamicin (all from Sigma-Aldrich), and 50 mM 2-mercaptoethanol (Thermo Fisher Scientific) in the presence of 20 ng/mL of human macrophage colony-stimulating factor (M-CSF) (PeproTech). Non-adherent cells are subsequently eliminated in the differentiation phase (day+1) by exchanging the cell culture medium, and adherent cells representing macrophages are detached using cell dissociation buffer (Sigma-Aldrich) and washed in complete medium the day of use (day8 or day9) for ADCP experiment based on cytometry. For ADCP based on cell imaging, macrophages are detached at day6 using cell dissociation buffer and seeded at 30'000 per well in 96 optical plate (costar).

1. CellInsight™ based assay

Macrophages (stained with calcein red orange) adhering to microplate wells are co-incubated with Calcein AM-labeled target tumor cells at an effector: target cells ratio of 1:3 for 2.5 hours at 37 degree C in the presence of different concentrations of the to be tested antibody. At the end of the incubation period, supernatants are replaced by complete culture medium and the microplates are imaged with the CellInsight™ CX5 High Content Screening Platform. 1500 macrophages are acquired and analyzed per well. Phagocytosis is evidenced as double-positive events (macrophage + target tumor cell) and the phagocytosis indexes are calculated by the CellInsight™ manufacturers' software.

All the results in figure 3 and table 5a and 5b are obtained with MKN-45 cells expressing CEACAM5 and CEACAM6 and with an effector cell to target/tumor cell ratio of 1:3. Strongest ADCP (lowest EC50 and highest max. index of phagocytosis) is achieved with the CEACAM5 and CEACAM6 expressing biAb K2AC49 and 50. According to the inventor's knowledge, this can be seen as a result of the triple co-engagement, that means binding of the biAb to CEACAM5, CEACAM6 and CD47 (all 3 receptors/targets are expressed on MKN-45 cells and also on most of human cancer primary cells); strongest ADCP of CEACAM5 specific antibodies was achieved with K2AC54

All ADCP (phagocytosis) values, ranges and the like in the present invention are based on the imaging based assay if not otherwise and explicitly stated.

2. Flow cytometry based ADCP assay

According to the knowledge of the inventors ADCP can also be measured by a method as described as follows: The macrophages are co-incubated with CSFE-labeled target tumor cells (e.g. MKN-45, LS174T or HPAC tumor cells) at an effector: target cells ratio of e.g. 3:1 for 2.5 hours at 37 degree C in the presence of different concentrations of to be tested antibody. At the end of the incubation period, biotinylated anti-human CD14 antibody and Strep-Cy5 are added to label the macrophages. The cells are then washed and subjected to flow cytometry analysis. Phagocytosis is evidenced by double-positive events CD14+ and CFSE+. Percentage of phagocytosis is presented as the ratio between CD14+/CSFE+ double positive events and total target cells multiplied by 100.

**Example 10: Binding of CEAxCD47 bispecific antibodies of the invention to MNK-45 cells; measurement of competition of binding with CEAxCD3 bispecific antibodies**

a) The binding of CD47xCEACAM5 bispecific antibody is tested on e.g. CEA-expressing human gastric adenocarcinoma cells (MKN-45, DSMZ ACC 409).

Cells are harvested, counted, checked for viability and resuspended at $3 \times 10^6$ cells/ml in FACS buffer (PBS 2% BSA, 0.1% NaN3). 100 $\mu$l of the cell suspension are distributed in V-bottom 96-well plates ($3 \times 10^5$ cells/well). The supernatant is removed by centrifugation 3 minutes at 4°C, 1300 rpm. Increasing concentrations of the antibody according to the invention are then added into the wells and incubated for 15 minutes at 4°C. Cells are washed twice with cold FACS buffer and re-incubated for further 15 minutes at 4°C with the PE (R-phycoerythrin)-conjugated mouse anti-human IgG Fc secondary antibody (SouthernBiotech, pre-diluted 1:100 in FACS buffer). Cells are washed twice with cold FACS buffer and resuspended in 300 $\mu$l FACS buffer with 1:15000-diluted SytoxBlue (Life Technologies). Fluorescence is measured using a Cytoflex (Millipore) flow cytometer. Binding curves and EC50 and Emax values are obtained and calculated using GraphPad Prism7 software. Results are shown in Table 4 and Figure 2.

b) Shift of binding curve of a CEAxCD47 antibody to CEA positive tumor cell-line (MKN-45) by addition of a CEAxCD3 T-cell bispecific antibody.

According to the knowledge of the inventors for competition experiments of CD47xCEACAM5 bispecific antibody according to the invention and CEAxCD3 T-cell bispecific antibodies like CEA-TCB or CEA-TCB1, the binding of the CEACAM5xCD47 to MKN-45 cells can be determined as described above, but with and w/o addition of the CEAxCD3 T-cell bispecific antibody to study if a CEAxCD3 T-cell bispecific antibody as combination partner for the CEAxCD47 bispecific antibodies of this invention is competitive for binding to CEA or not.

**Example 11: Production and Purification of fucosylated and afucosylated bispecific antibodies, e.g. K2AC50 and K2AC54**

Production of fucosylated and afucosylated K2AC50 and K2AC54 bispecific antibodies:

According to the knowledge of the inventors a CHO pool (one for K2AC50 and one for K2AC54) is inoculated at a viable cell concentration of 0.3 x $10^6$ cells/mL in a Thomson erlen device with a working volume of 700 mL or 100 mL for the production of fucosylated and afucosylated antibodies, respectively. All the pools are operated in a 15 days duration fed-batch mode using CDACF medium CDCHO and an adapted feeding regime. For the production of afucosylated antibodies, bolus of 200$\mu$M fucose inhibitor (1,3,4-Tri-O-acetyl-2-deoxy-2-fluoro-L-fucose) are added at day 0, 5, 8 and 11 during the fed batch process based on afucosylation strategy described by Rillahan et al. Nature Chem. Biol. 2012 Jul;8(7):661-8 and based on EP2282773. Harvest of the K2AC50 AND K2AC54 pools supernatants containing fucosylated or afucosylated antibodies is performed after 15 days of Fed batch culture. Harvests of *CHO* pools *supernatants* are clarified using the Sartoclear Dynamics® Lab V Cell Harvesting Sartorius system (see supplier instructions).

Purification of fucosylated and afucosylated K2AC50 AND K2AC54 bispecific antibodies

According to the knowledge of the inventors purification of fucosylated and afucosylated bispecific antibodies according to the invention is a three affinity step purification process. Before starting purification, antibody concentration in the supernatant of bispecific antibody pools is measured using OctetRED96 in order to use columns with appropriate volume of affinity matrix. Each clarified CHO pool supernatant containing fucosylated or afucosylated bispecific antibodies, is loaded onto a MabSelect SuRe (MSS) column (GE Healthcare) without prior adjustment, to remove a major part of cell culture contaminants. The MSS eluate is then treated by low pH hold to inactivate viruses, and neutralized at pH 6 with Tris 1M pH9. The MSS eluate's is then loaded onto the LambdaFabSelect (LFS) column (GE Healthcare) to remove monospecific κ (mono κ). The LFS eluate is then pH adjusted at pH 6. The LFS is loaded onto the Capto L (CL) column (GE Healthcare) to remove monospecific λ (mono λ). The CL Eluate is pH adjusted before storage. The final material is then concentrated and diafiltered into the final formulation buffer, its concentration adjusted using the Nanodrop. Fucosylated and afucosylated

Bispecific antibodies according to the invention will be aliquoted and stored at -80°C until delivery. Purified bispecific antibodies are analyzed for sizing by electrophoresis in denaturing and reducing conditions with the Agilent 2100 Bioanalyzer using the Protein 80 kit as described by the manufacturer (Agilent Technologies, Santa Clara, Calif., USA). Aggregation level is assessed by size exclusion chromatography (SEC-UPLC) using the ACQUITY UPLC H-Class Bio System (Waters). Charge variant analysis of purified *bispecific antibodies* is achieved by isoelectric focusing technique (*IEF*) using the Multiphor II Electrophoresis System (GE Healthcare). The relative distribution of *N*-linked complex biantennary glycoforms of fucosylated and afucosylated antibodies will be determined using the throughput microchip-CE method on the *LabChip GXII* Touch (Perkin Elmer). All antibodies are tested for endotoxin contamination using the Limulus Amebocyte Lysate test (LAL; Charles River Laboratories, Wilmington, Mass). Typical afucosylation achieved by this method is expected to be in the range of 70 to 90%.

**Example 12: Production of afucosylated bispecific antibodies of the invention**

1. By using FUT 8 negative production cell line

Alternatively, and according to the knowledge of the inventors, afucosylated bispecific antibodies according to the invention can be produced also according to the method as follows:
Material and Methods are according to Naoko Yamane-Ohnuki et al., Biotech. Bioeng.; 87 (2004) 614-622.

<u>Isolation of Chinese Hamster FUT8 cDNA</u>

Total RNA is isolated from CHO/DG44 cells using the RNeasy® Mini Kit (Qiagen, Hilden, Germany) and reverse transcribed with oligo-dT using a Superscript first-strand synthesis system for reverse transcript-polymerase chain reaction (RT-PCR) (Invitrogen, Carlsbad, CA). A Chinese hamster FUT8 cDNA is amplified from single-stranded CHO/DG44 cell cDNAs by PCR using primers
5V-GTCTGAAGCATTATGTGTTGAAGC-3V (SEQ ID NO:187) and
5V-GTGAGTACATTCATTGTACTGTG-3V (SEQ ID NO:188), designed from the murine FUT8 cDNA (Hayashi, 2000; DNA Seq 11:91-96).

<u>Targeting Construct of FUT8 Locus</u>

The targeted disruption of the FUT8 gene in CHO/DG44 cells is carried out using two replacement vectors, pKOFUT8Neo and pKOFUT8Puro. The 9.0-kb fragment of the FUT8 gene including the first coding exon is isolated by screening the CHO-K1 cell E-genomic library (Stratagene, La Jolla, CA) with the Chinese hamster FUT8 cDNA as a probe to establish the targeting constructs. A 234-bp segment containing the translation initiation site is replaced with the neomycin-resistance gene (Neor) cassette or the puromycin-resistance gene (Puror) cassette from plasmid pKOSelectNeo or pKOSelectPuro (Lexicon, TX), respectively, flanked by loxP sites. The diphtheria toxin gene (DT) cassette from plasmid pKOSelectDT (Lexicon) is inserted at the 5V homologous region. The resulting targeting constructs, pKOFUT8Neo and pKOFUT8Puro, included the 1.5-kb 5V homologous sequence and the 5.3-kb 3V homologous sequence. Before transfection, the targeting constructs are linearized at a unique SalI site.

<u>Transfection and Screening for Homologous Recombinants</u>

Subconfluent CHO/DG44 cells (1.6 106) are electroporated with 4 Ag of linearized pKOFUT8Neo at 350 V and 250 AF using a Bio-Rad GenePulser® II. After electroporation, transfectants are selected with 600 Ag/mL G418 (Nacalai Tesque, Kyoto, Japan). Genomic PCR is performed in 96-well plates by the modified microextraction method reported previously (Ramirez-Solis et al., 1992; Anal Biochem 201:331- 335.) using the following primers:

5V-TTGTGTGACTCTTAACTCTCAGAG-3V (SEQ ID NO:189) and
5V-GAGGCCACTTGTGTAGCGCCAAGTG-3V (SEQ ID NO:190).

Homologous recombinants are identified by the 1.7-kb fragment obtained using genomic PCR and confirmed by Southern blot analysis using the 221-bp fragment amplified with the following primers:

5V-GTGAGTCCATGGCTGTCACTG-3V (SEQ ID NO:191) and
5V-CCTGACTTGGCTATTCTCAG-3V (SEQ ID NO:192).

The hemizygous clone is subject to a second round of homologous recombination using linearized pKOFUT8Puro and drug selection with 15 Ag/mL puromycin (Sigma-Aldrich, St. Louis, MO) as described earlier. The identified homozygous disruptants are electroporated with the Crerecombinase expression vector pBS185 (Invitrogen) to remove drug-resistance gene cassettes from both FUT8 alleles.

Monoclonal Antibody Production by FUT8(-) Cells

FUT8(-) cell lines are electroporated with an expression vector encoding an bispecific antibody according to the invention and selected in media lacking hypoxanthine and thymidine. The confluent transfectants are cultured in Ex-Cell® 301 Medium (JRH Biosciences, Lenexa, KS) for 1 week. The antibody is purified from culture supernatants using MabSelect™ (Amersham Biosciences, Piscataway, NJ). Further purification steps can be anion/cation exchange chromatography, size exclusion chromatography and especially purification using kappa respectively lambda selective resins as described above.

2. By retrieval of extracellular fucose from production cell medium plus enzymatic intervention with the intracellular fucose biosynthesis

Preferably, and according to the knowledge of the inventors, afucosylated bispecific antibodies of the invention can be produced also according to the method/technology as follows and described in, US8642292. This technology is designed to configure the stable integration of a heterologous bacterial enzyme into an antibody producer cell line like a CHO cell line or others. By this, the de novo synthesis of fucuse from D-mannose is blocked. If in addition production cells are cultivated in fucose free medium, as a result antibodies with a stable level of afucosylation are produced.

In eucaryotic cells fucose is generated through two routes,

a) from the extracellular space or lysosome through the salvage pathway and

b) by de novo synthesis of fucose from D-mannose in the de novo synthesis pathway of fucose.

The salvage pathway can be completely blocked by omission of fucose from the culture medium. The de novo biosynthesis pathway can be blocked by converting the intermediate GDP-4-keto-6-deoxy-D-mannose of this pathway to GDP-D-rhamnose instead of GDP-4-keto-6-deoxy-D-galactose. This is achieved by bringing the bacterial enzyme GDP-6-deoxy-D-lyxo-4-hexulose reductase (RMD) into the production cell line, respectively by stable integration of the gene encoding for RMD into the production cell line. Even rather low amounts of RMD expressed in the production cell line completely block the de novo synthesis pathway of the production cell.

This technology will be used to construct production cell lines, e.g. CHO based cell lines, designed for the production of afucosylated antibodies of the invention as well as to existing production cell lines which already produce antibodies of the invention and are engineered to produce the antibodies with fucose content reduced by 80% to 100%.

**EXAMPLE 13: in vivo Antitumor Activity of Bispecific Antibodies**

According to the knowledge of the inventors the anti-tumor activity of a bispecific antibody according to the invention can be evaluated in Xenograft models, e.g. by the following model: 1 to $3x10^6$ CEA positive tumor cells like MKN-45, LS174T, or SNUC-1 cells are implanted subcutaneously in e.g. NOD/SCID mice. Tumor volumes are measured 3 times per week. After 3 or 5 or 7 or 9 days after the tumor cell implantation or alternatively when the tumor graft reached a volume of approx. 100 to $300mm^3$, mice are randomized into groups (e.g. 4 to 6 mice per group) and the antibody treatment is initiated. This experiment could e.g. compare the effect of the bispecific antibody according to the invention and positive control Mabs, e.g. the CD47 Mab B6H12.2. Antibody is injected e.g. i.v. every week until the end of the experiment (approximately d25). Antibodies are administered at e.g. daily or 3 times a week or weekly etc doses of e.g. 1 or 2.5 or 5 or 10 or 20 mg/kg.

Combinations of a bispecific antibody of this invention with a CEAxCD3 bispecific antibody can be tested in an appropriate model. Models, in which the combination of an antibody according to the invention together with CEA-TCB or CEA-TCB1 can be tested, are e.g. described by Bacac et al (Clin. Cancer Res., 22(13);3286-97;2016) and are also used, especially for combination studies of CEACAM5xCD47 or CEACAM5/6xCD47 and CEA-TCB or CEA-TCB 1. Usually human PBMC have to be engrafted in such a model.

**Example 14: Cytokine release tested in whole blood and PBMCs from healthy human donors human blood**

According to the knowledge of the inventors an in vitro cytokine release assay can be performed using whole blood (WB CRA) with minimal dilution by the test antibodies (95% v/v blood) in aqueous presentation. This assay format is considered to mimic more closely the *in vivo* environment, containing factors at physiological concentrations that may influence mechanisms of cytokine release. However, this format is thought to be poorly predictive of T cell-mediated cytokine release (e.g., anti-CD28).

The assay can be also performed using peripheral blood mononuclear cells (PBMCs) from healthy human donors and with an immobilized mAb (Solid Phase, SP) presentation to assess T cell-mediated cytokine release (PBMC SP CRA). This assay format simulates cross-linking and high density presentation of mAbs, which may occur in vivo (e.g. clustering of the target via the interaction of the Fc part of the antibody with Fcγ receptors on other immune cells or the cross-linking of mAbs by anti-drug antibodies). This format is predictive of T cell-mediated cytokine release.

**Example 15: Antibody Binding to Erythrocytes, Phagocytosis of Erythrocytes, and Platelet activation and aggregation**

Whole blood binding

According to the knowledge of the inventors human whole blood samples collected from healthy donors in citrate can be mixed with 3 μg/mL of AF488-coupled CEA x CD47 bispecific antibodies of this invention, B6H12.2 or isotype control and surface staining antibodies (PE-Cy7 anti-hCD45 and PE anti-hCD41a, for platelets only) for 30 min at 4°C. After the incubation, whole blood is divided in two samples: 5 μL are diluted and washed in PBS for erythrocyte analysis while 150 μL are incubated with erythrocyte lysing solution and washed for platelet analysis. Samples are acquired on a CytoFLEX instrument and analyzed with the FlowJo software to determine MFI values.

Erythrophagocytosis

According to the knowledge of the inventors human red blood cells (RBCs) can be isolated from human whole blood by centrifugation at 300xg, washed twice in PBS, labeled with CFSE-(Carboxyfluorescein succinimidyl ester) and pre-incubated with the test antibody for 1 hour at 37° C before the addition of macrophages. Labeled RBCs can be cultured with human macrophages in the presence of an antibody according to the invention or control (non-binding IgG1 antibody) for one hour at a target-to-effector ratio of 200:1. After culture, cells are stained with anti-CD14-APC and analyzed by flow cytometry. Phagocytosis was quantitated as the percent of CD14+ events (macrophages) that are also CFSE+ and had therefore engulfed at least one RBC (events are gated on singlets). Phagocytosis and FACS analysis is done as described in example 9, except that the erythrocytes were lysed with FACS lysing solution after macrophage staining.

*In vitro* platelet activation and aggregation

In a standard flow cytometry experiment the ability of CEAxCD47 bispecific antibodies to induce human platelet activation in whole blood of seven human healthy donors was measured by the upregulation of surface marker CD62P. Briefly, 5 μL of whole blood is incubated with 10 μL of each sample (prepared at 2X) for 15 minutes at room temperature. Each tested antibody is added at different concentrations (0, 0.02, 0.2, 2, 20 and 200 μg/mL). Adenosine diphosphate (ADP) and anti-CD9 (ALB6), included as positive control reagents known to induce platelet activation, are added at a concentration of 10μM and 10 μg/mL, respectively. Then, 10 μL of anti-CD41a-PE and 10 μL of anti-CD62P-APC were added and incubated for 15 min. in the dark at room temperature. Finally, 500 μL of CellFix (BD Biosciences, diluted 1/10 in water) were added and 200 μL of each sample is transferred in a U-bottom 96-well plate suitable for CytoFLEX acquisition. Platelets are identifed by the CD41a-PE positive staining. Platelet activation is assessed by the expression of CD62P marker.

According to the knowledge of the inventors, the potential for aggregation in the presence of CD47/CEA bispecific antibody could be assessed on platelet rich plasma (PRP). PRP is challenged with ADP at 10 μM and 5μM or with the test articles at 200, 100, 20, 25, and 12.5 μg/mL, as well as with saline or the isotype control. Platelet aggregation can be evaluated throughout platelet stimulation (i.e. 10 min) with a Thrombo-aggregometer TA 4V under constant stirring. Thrombosoft 1.6 software (SD Innovation, Frouard, France) can be used for analysis of the data.

**Example 16: Hematology assessment in Cynomolgus**

According to the knowledge of the inventors cynomolgus monkey cross-reactive antibodies could be tested *in vivo* in Cynomolgus Monkeys for any effect on hematology parameters (including RBC and platelets). An antibody according to the invention is e.g. given to cynomolgus monkeys per intravenous route, at doses up to 100 mg/kg, on a weekly basis. Hematology parameters, including red blood cell and platelet counts, are monitored over time and compared to control values in monkeys (pre-dose values). Hematology parameters are determined by routine methods.

**Example 17: Determination of Pharmacokinetics properties in cynomolgus monkeys**

According to the knowledge of the inventors in single dose pharmacokinetic studies, animals can be randomized to 2 to 5 treatment groups of n=2 to 4 monkeys per group (including males and females). Animals are administered with single IV doses of the bispecific antibodies of this invention (infusion over 15 to 30 minutes). Doses in the treatment groups are ranging from 0.01 mg/kg to 100 mg/kg. Administration volumes are up to 5 mL/kg. Blood withdrawals are scheduled according to the experimental protocol at multiple time points, e,g, 0.25, 1, 4, 8, 24, 48, 72, 96, 120, 168, 240, 336, 504 (day 22), 672 (day 29), 840 (day 36), 1008 (day 43), 1176 (day 50) and 1344h (day 57) after the intravenous administration of the bispecific antibody. Blood samples of approximately 2 mL per animal and time-point are collected. Concentrations of the antibodies were either measured in serum or in plasma. An ELISA test is developed and validated to measure the concentrations. Each sample is measured in duplicates.

From the concentration time curves PK parameters like Cmax, clearance, elimination half-life, area under the curve etc. can be determined by using industry standard software (Phoenix WinNonlin; non-compartmental analysis).

Elimination half-lives of the CEA x CD47 kappa-lambda bispecific antibodies are expected to be in the range of 3 to 14 days, suggesting q1w or q2w or q3w or q4w administrations to patients.

**Example 18: ADCP Mediated by Bispecific Antibodies in presence of CEA-TCB and CEA-TCB1**

According to the knowledge of the inventors calcein AM-labeled MKN45 cells used as target cells are pre incubated or not with a fixed dose of CEA-TCB (300nM) or CEA-TCB1 (30nM) for 20 min at RT. After this incubation different concentrations of tested antibody are added in appropriate well for 20 min Then macrophages (stained with calcein red orange) adhering to microplate wells are co-incubated with the opsonized labeled target tumor cells at an effector:target cells ratio of 1:3 for 2.5 hours at 37 °C. The ADCP is performed in a presence of 1mg/mL of human hIgG. At the end of the incubation period, supernatants are replaced by complete culture medium and the microplates are imaged with the CellInsight™ CX5 High Content Screening Platform. 1500 macrophages are acquired and analyzed per well. Phagocytosis is evidenced as double-positive events (macrophage + engulfed target tumor cell) and the phagocytosis indexes are calculated by the CellInsight™ manufacturers' software.

**Example 19: Killing assay by Combination of CD47xCEA and CEAxCD3**

According to the knowledge of the inventors human peripheral blood mononuclear cells (PBMCs) were isolated from buffy coats. Part of these PBMCs were frozen in freezing medium (90% FCS 10% DMSO) (in order to be used as source of T cells) and part were used to prepare macrophages (as explained in Phagocytosis section). After 6 days of macrophage differentiation, cells were plated in 96 well-plates and incubated at 37°C. On the day of the assay (2 days after macrophage plating), frozen PBMCs from the corresponding macrophage donor were thawed and added to the macrophage plates. Target cells (MKN45 engineered to express Luciferase) were opsonized with a combination of antibodies, i.e. with a CEAxCD3 T-cell bispecific antibody at certain concentrations together with certain concentrations of of aCEAxCD47 bispecific antibody. Opsonized targets were added to the plates containing macrophages and autologous PBMCs; and the plates were incubated at 37°C for 48h. After 48h, half of the well medium was removed and a solution of 2X Luciferin was added to the plates to obtain a final concentration of 150μg/mL. After 5 minutes incubation at RT, plates were read using a Synergy NEO. Percentage of viability was calculated dividing the luminescence value (minus background) by the control containing only target cells and multiplying by 100. Percentage of killing was then extrapolated by subtracting the percentage of viability to 100.

All publications, patents, patent applications, internet sites, and accession numbers/database sequences including both polynucleotide and polypeptide sequences cited herein are hereby incorporated by reference herein in their entirety for all purposes to the same extent as if each individual publication, patent, patent application, internet site, or accession number/database sequence were specifically and individually indicated to be so incorporated by reference.

## Claims

1. A bispecific antibody comprising a first binding part specifically binding to human CEACAM5 (further named also as "CEA") and a second binding part specifically binding to human CD47 (further named also as "CD47") **characterized in that**:

    a) the first binding part comprises as heavy chain variable region a heavy chain variable region comprising a CDRH1 of SEQ ID NO:1, a CDRH2 of SEQ ID NO:2 and a CDRH3 of SEQ ID NO:3,
    b) the first binding part comprises as light chain variable region a light chain variable region comprising a CDRL set selected from the group consisting of

        b1) a CDRL1 of SEQ ID NO:17, CDRL2 of SEQ ID NO:18, and CDRL3 of SEQ ID NO:19,
        b2) a CDRL1 of SEQ ID NO:20, CDRL2 of SEQ ID NO:21, and CDRL3 of SEQ ID NO:22,
        b3) a CDRL1 of SEQ ID NO:23, CDRL2 of SEQ ID NO:24, and CDRL3 of SEQ ID NO:25,
        b4) a CDRL1 of SEQ ID NO:26, CDRL2 of SEQ ID NO:27, and CDRL3 of SEQ ID NO:28,
        b5) a CDRL1 of SEQ ID NO:29, CDRL2 of SEQ ID NO:30, and CDRL3 of SEQ ID NO:31,
        b6) a CDRL1 of SEQ ID NO:32, CDRL2 of SEQ ID NO:33, and CDRL3 of SEQ ID NO:34,
        b7) a CDRL1 of SEQ ID NO:35, CDRL2 of SEQ ID NO:36, and CDRL3 of SEQ ID NO:37,
        b8) a CDRL1 of SEQ ID NO:38, CDRL2 of SEQ ID NO:39, and CDRL3 of SEQ ID NO:40,
        b9) a CDRL1 of SEQ ID NO:41, CDRL2 of SEQ ID NO:42, and CDRL3 of SEQ ID NO:43,
        b10) a CDRL1 of SEQ ID NO:44, CDRL2 of SEQ ID NO:45, and CDRL3 of SEQ ID NO:46,
        b11) a CDRL1 of SEQ ID NO:47, CDRL2 of SEQ ID NO:48, and CDRL3 of SEQ ID NO:49,
        b12) a CDRL1 of SEQ ID NO:50, CDRL2 of SEQ ID NO:51, and CDRL3 of SEQ ID NO:52,
        b13) a CDRL1 of SEQ ID NO:53, CDRL2 of SEQ ID NO:54, and CDRL3 of SEQ ID NO:55,
        b14) a CDRL1 of SEQ ID NO:56, CDRL2 of SEQ ID NO:57, and CDRL3 of SEQ ID NO:58,
        b15) a CDRL1 of SEQ ID NO:59, CDRL2 of SEQ ID NO:60, and CDRL3 of SEQ ID NO:61,
        b16) a CDRL1 of SEQ ID NO:62, CDRL2 of SEQ ID NO:63, and CDRL3 of SEQ ID NO:64,
        b17) a CDRL1 of SEQ ID NO:71, CDRL2 of SEQ ID NO:72, and CDRL3 of SEQ ID NO:73,
        b18) a CDRL1 of SEQ ID NO:142, CDRL2 of SEQ ID NO:143, and CDRL3 of SEQ ID NO:144,
        b19) a CDRL1 of SEQ ID NO:145, CDRL2 of SEQ ID NO:146, and CDRL3 of SEQ ID NO:147,
        b20) a CDRL1 of SEQ ID NO:148, CDRL2 of SEQ ID NO:149, and CDRL3 of SEQ ID NO:150,
        b21) a CDRL1 of SEQ ID NO:151, CDRL2 of SEQ ID NO:152, and CDRL3 of SEQ ID NO:153,
        b22) a CDRL1 of SEQ ID NO:154, CDRL2 of SEQ ID NO:155, and CDRL3 of SEQ ID NO:156, and
        b23) a CDRL1 of SEQ ID NO:157, CDRL2 of SEQ ID NO:158, and CDRL3 of SEQ ID NO:159, and

    c) the second binding part comprises as heavy chain variable region a heavy chain variable region comprising a CDRH1 of SEQ ID NO:1, a CDRH2 of SEQ ID NO:2 and a CDRH3 of SEQ ID NO:3, and as light chain variable region a light chain variable region comprising a CDRL1 of SEQ ID NO:7, a CDRL2 of SEQ ID NO:8, and a CDRL3 of SEQ ID NO:9.

2. The bispecific antibody according to claim 1, **characterized in** comprising in the first binding part as heavy chain region a heavy chain region of SEQ ID NO:5 and as light chain a light chain selected from the group of

    a) the light chain of SEQ ID NO:77,
    b) the light chain of SEQ ID NO:78,
    c) the light chain of SEQ ID NO:79,

d) the light chain of SEQ ID NO:80,
e) the light chain of SEQ ID NO:81,
f) the light chain of SEQ ID NO:82,
g) the light chain of SEQ ID NO:83,
h) the light chain of SEQ ID NO:84,
i) the light chain of SEQ ID NO:85,
k) the light chain of SEQ ID NO:86,
l) the light chain of SEQ ID NO:87,
m) the light chain of SEQ ID NO:88,
n) the light chain of SEQ ID NO:89,
o) the light chain of SEQ ID NO:90,
p) the light chain of SEQ ID NO:91,
r) the light chain of SEQ ID NO:92,
s) the light chain of SEQ ID NO:174,
t) the light chain of SEQ ID NO:175,
u) the light chain of SEQ ID NO:176,
v) the light chain of SEQ ID NO:177,
w) the light chain of SEQ ID NO:178,
x) the light chain of SEQ ID NO:179, and
y) the light chain of SEQ ID NO:180, and
b) comprising in the second binding part as heavy chain variable region a heavy chain variable region of SEQ ID NO:4 and as light chain a light chain of SEQ ID NO:11.

**3.** The bispecific antibody according to claim 1, **characterized in** comprising in the first binding part as heavy chain region a heavy chain region of SEQ ID NO:5 and as light chain a light chain selected from the group of

a) the light chain of SEQ ID NO:74,
b) the light chain of SEQ ID NO:75,
c) the light chain of SEQ ID NO:76,
d) the light chain of SEQ ID NO:167,
e) the light chain of SEQ ID NO:168,
f) the light chain of SEQ ID NO:169,
g) the light chain of SEQ ID NO:170,
h) the light chain of SEQ ID NO:171,
i) the light chain of SEQ ID NO:172,
k) the light chain of SEQ ID NO:173,
and
b) comprising in the second binding part as heavy chain region a heavy chain region of SEQ ID NO:5 and as light chain a light chain of SEQ ID NO:181.

**4.** The bispecific antibody according to claim 1, **characterized in** specifically binding to human CEACAM5 and human CEACAM6 in the first binding part and human CD47 in the second binding part, **characterized in**

a) **that** the first binding part comprises as heavy chain variable region a heavy chain variable region comprising as CDRs a CDRH1 of SEQ ID NO:1, CDRH2 of SEQ ID NO:2 and CDRH3 of SEQ ID NO:3 and as light chain variable region a light chain variable region comprising as CDRs a CDRL1 of SEQ ID NO: 44, a CDRL2 of SEQ ID NO: 45, and a CDRL3 of SEQ ID NO: 46 and
b) **that** the second binding part comprises as heavy chain variable region a heavy chain variable region comprising as CDRs a CDRH1 of SEQ ID NO:1, CDRH2 of SEQ ID NO:2 and CDRH3 of SEQ ID NO:3 and as light chain variable region a light chain variable region comprising as CDRs a CDRL1 of SEQ ID NO:7, CDRL2 of SEQ ID NO:8, and CDRL3 of SEQ ID NO:9.

**5.** The bispecific antibody according to claim 1, **characterized in** specifically binding to human CEACAM5 and cynomolgus CEACAM5 in the first binding part and human CD47 in the second binding part, **characterized in**

b) a) that the first binding part comprises as heavy chain variable region a heavy chain variable region comprising as CDRs a CDRH1 of SEQ ID NO:1, CDRH2 of SEQ ID NO:2 and CDRH3 of SEQ ID NO:3 and as light chain variable region a light chain variable region comprising as CDRs a CDRL1 of SEQ ID NO: 53, a CDRL2 of SEQ ID NO: 54, and a CDRL3 of SEQ ID NO: 55, and
b) that the second binding part comprises as heavy chain variable region a heavy chain variable region comprising as CDRs a CDRH1 of SEQ ID NO:1, CDRH2 of SEQ ID NO:2 and CDRH3 of SEQ ID NO:3 and as light chain variable region a light chain variable region comprising as CDRs a CDRL1 of SEQ ID NO:7, CDRL2 of SEQ ID NO:8, and CDRL3 of SEQ ID NO:9.

**6.** The bispecific antibody according to claim 4, **characterized in** comprising in the first binding part as heavy chain a heavy chain of SEQ ID NO:5 or of SEQ ID NO:6 and as light chain a light chain of SEQ ID NO:86 and
b) in the second binding part as heavy chain a heavy chain of SEQ ID NO:5 or of SEQ ID NO:6 and as light chain a light chain of SEQ ID NO:11.

**7.** The bispecific antibody according to claim 5, **characterized in** comprising in the first binding part as heavy chain a heavy chain of SEQ ID NO:5 or of SEQ ID NO:6 and as light chain a light chain of SEQ ID NO:89 and in the second binding part as heavy chain a heavy chain of SEQ ID NO:5 or of SEQ ID NO:6 and as light chain a light chain of SEQ ID NO:11.

**8.** The bispecific antibody of any one of the preceding claims, **characterized in that** said bispecific antibody competes for binding to CEACAM5 with the anti-CEACAM5 antibody SM3E, which comprises as VK and VH domains VK and VH of sequences SEQ ID NO:110 and 111.

**9.** The bispecific antibody of any one of the preceding claims, wherein said antibody comprises a Fc region that has been glycoengineered to have a reduced number of fucose residues as compared to the same bispecific antibody that has not been glycoengineered, wherein said bispecific antibody has an Fc region in which 95% to 100% of the N-linked oligosaccharides are nonfucosylated.

**10.** The bispecific antibody of any one of the preceding claims, wherein said first binding part specifically binds to human CEACAM5 and cynomolgus CEACAM5.

**11.** The bispecific antibody of any one of the preceding claims, wherein said first binding part specifically binds to human CEACAM5 and CEACAM 6, and wherein the EC50 values of binding to human CEACAM5 and human CEACAM6 differ by not more than a factor of 10.

**12.** The bispecific antibody of any one of the preceding claims, **characterized in** a concentration dependent phagocytosis of CEACAM5 and/or CEACAM6 expressing tumor cell lines like MKN-45 cells by human macrophages at an EC50 of the bispecific antibody below 40 nM.

**13.** The bispecific antibody of any one of the preceding claims, **characterized in** binding to human CD47 with a binding affinity of 100 nM to 600 nM.

**14.** A method for producing a glycoengineered bispecific antibody according to any one of claims 1-65, comprising an Fc region in which 80% to 100% of the N-linked oligosaccharides are nonfucosylated, in a host cell of claim 68, cultured in a fucose free medium, said host cell comprising a first polynucleotide F-4-hexylose reductase and a second polynucleotide encoding the bispecific antibody according to the invention, said method comprises:

i) expressing GDP-6-deoxy-D-lyxo-4-hexylose reductase encoded by the first polynucleotide and the bispecific antibody according to the invention encoded by the second polynucleotide in said cell, and
i) isolating said bispecific antibody from said cell under conditions which permit the production of said bispecific antibody of the invention, and which permit that the oligosaccharides of the Fc region of said bispecific antibody are lacking fucose in an amount of 80% to 100%; and

b) isolating said glycoengineered bispecific antibody wherein said glycoengineered bispecific antibody is capable of specifically binding to CEA and CD47.

**15.** A bispecific antibody, **characterized in** specifically binding to human CEACAM5 and human CEACAM6 in the first binding part and human CD47 in the second binding part, **characterized in**

a) **that** the first binding part comprises as heavy chain variable region a heavy chain variable region comprising as CDRs a CDRH1 of SEQ ID NO:1, CDRH2 of SEQ ID NO:2 and CDRH3 of SEQ ID NO:3 and as light chain variable region a light chain variable region comprising as CDRs a CDRL1 of SEQ ID NO: 44, a CDRL2 of SEQ ID NO: 45, and a CDRL3 of SEQ ID NO: 46 and

b) **that** the second binding part comprises as heavy chain variable region a heavy chain variable region comprising as CDRs a CDRH1 of SEQ ID NO:1, CDRH2 of SEQ ID NO:2 and CDRH3 of SEQ ID NO:3 and as light chain variable region a light chain variable region comprising as CDRs a CDRL1 of SEQ ID NO:7, CDRL2 of SEQ ID NO:8, and CDRL3 of SEQ ID NO:9, for use in simultaneous, separate, or sequential combination with CEA-TCB or CEA-TCB 1 in the treatment of a subject having a cancer that expresses CEACAM5 or CEACAM5 and CEACAM6, wherein the bispecific antibody is **characterized in** not competing with CEA-TCB or CEA-TCB 1.

**16.** A bispecific antibody **characterized in** specifically binding to human CEACAM5 and cynomolgus CEACAM5 in the first binding part and human CD47 in the second binding part, **characterized in**

b) a) that the first binding part comprises as heavy chain variable region a heavy chain variable region comprising as CDRs a CDRH1 of SEQ ID NO:1, CDRH2 of SEQ ID NO:2 and CDRH3 of SEQ ID NO:3 and as light chain variable region a light chain variable region comprising as CDRs a CDRL1 of SEQ ID NO: 53, a CDRL2 of SEQ ID NO: 54, and a CDRL3 of SEQ ID NO: 55, and

b) that the second binding part comprises as heavy chain variable region a heavy chain variable region comprising as CDRs a CDRH1 of SEQ ID NO:1, CDRH2 of SEQ ID NO:2 and CDRH3 of SEQ ID NO:3 and as light chain variable region a light chain variable region comprising as CDRs a CDRL1 of SEQ ID NO:7, CDRL2 of SEQ ID NO:8, and CDRL3 of SEQ ID NO:9, for use in simultaneous, separate, or sequential combination with CEA-TCB or CEA-TCB 1 in the treatment of a subject having a cancer that expresses CEACAM5 or CEACAM5 and CEACAM6, wherein the bispecific antibody is **characterized in** not competing with CEA-TCB or CEA-TCB1.

**17.** A monoclonal antibody specifically binding to human CEACAM5 and in specifically binding to cynomolgus CEACAM5, **characterized**

i) **in** competing for binding to CEACAM5 with the anti-CEACAM5 antibody SM3E, said antibody SM3E comprises as VK and VH domains VK and VH of sequences SEQ ID NO:110 and 111, and

ii) in comprising as heavy chain region a heavy chain region of SEQ ID NO:5 or of SEQ ID NO:6 and as light chain a light chain selected from the group of

a) the light chain of SEQ ID NO:74,
b) the light chain of SEQ ID NO:75,
c) the light chain of SEQ ID NO:76,
d) the light chain of SEQ ID NO:77,
e) the light chain of SEQ ID NO:78,
f) the light chain of SEQ ID NO:79,
g) the light chain of SEQ ID NO:80,
h) the light chain of SEQ ID NO:81,
i) the light chain of SEQ ID NO:82,
k) the light chain of SEQ ID NO:83,
l) the light chain of SEQ ID NO:84,
m) the light chain of SEQ ID NO:85,
n) the light chain of SEQ ID NO:86,
o) the light chain of SEQ ID NO:87,
p) the light chain of SEQ ID NO:88,
q) the light chain of SEQ ID NO:89,
r) the light chain of SEQ ID NO:90,
s) the light chain of SEQ ID NO:91,
t) the light chain of SEQ ID NO:92,
u) the light chain of SEQ ID NO:167,
v) the light chain of SEQ ID NO:168,
w) the light chain of SEQ ID NO:169,
x) the light chain of SEQ ID NO:170,
y) the light chain of SEQ ID NO:171,
z) the light chain of SEQ ID NO:172,
aa) the light chain of SEQ ID NO:173,
ab) the light chain of SEQ ID NO:174,

ac) the light chain of SEQ ID NO:175,
ad) the light chain of SEQ ID NO:176,
ae) the light chain of SEQ ID NO:177,
af) the light chain of SEQ ID NO:178,
ag) the light chain of SEQ ID NO:179, and
ah) the light chain of SEQ ID NO:180.

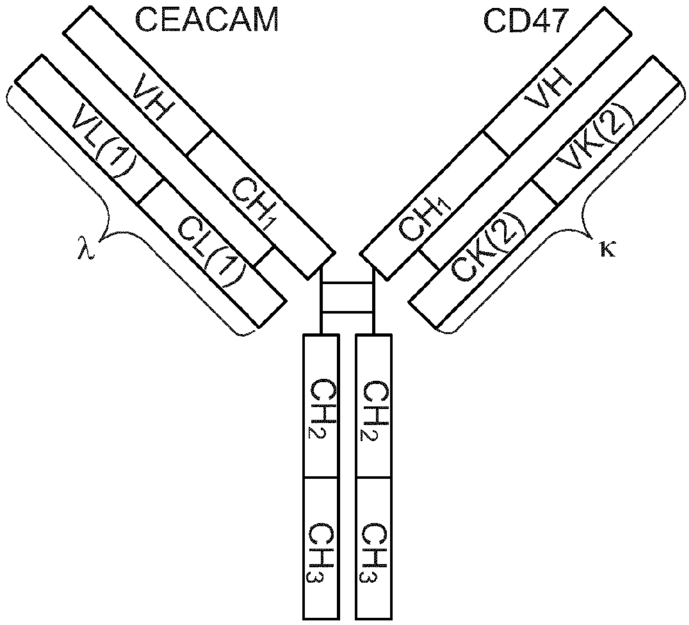


Fig. 1A

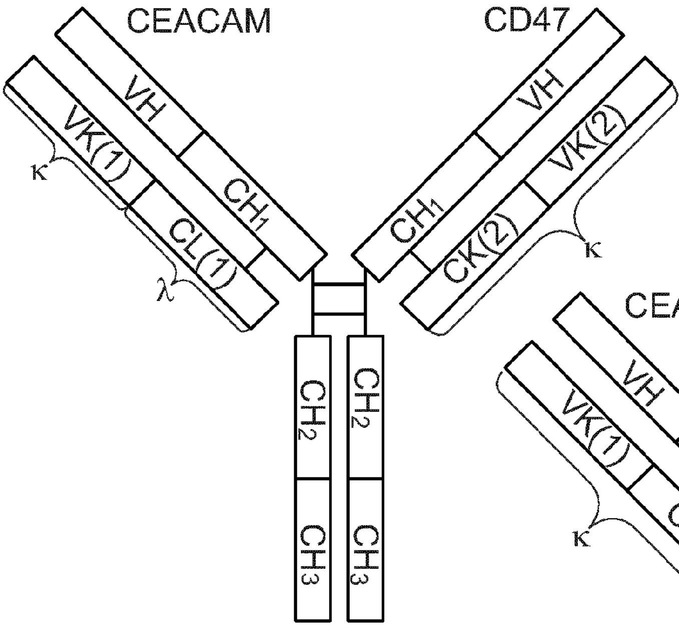


Fig. 1B

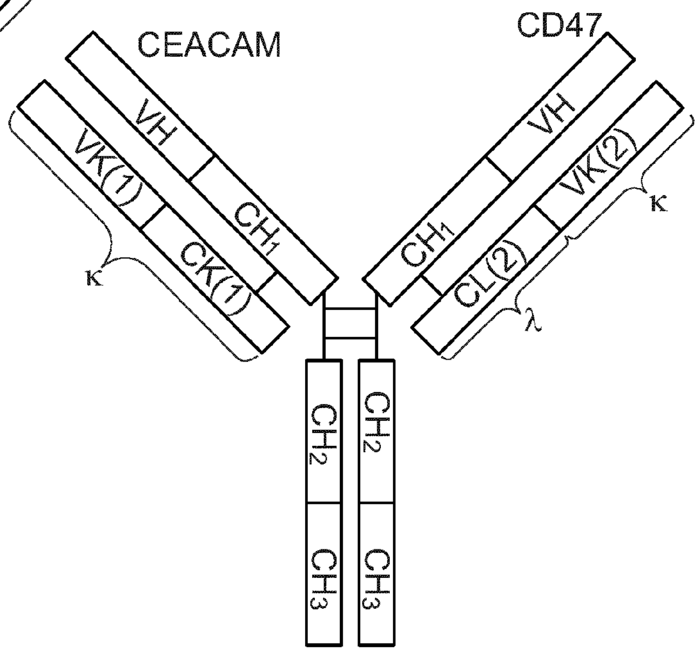


Fig. 1C

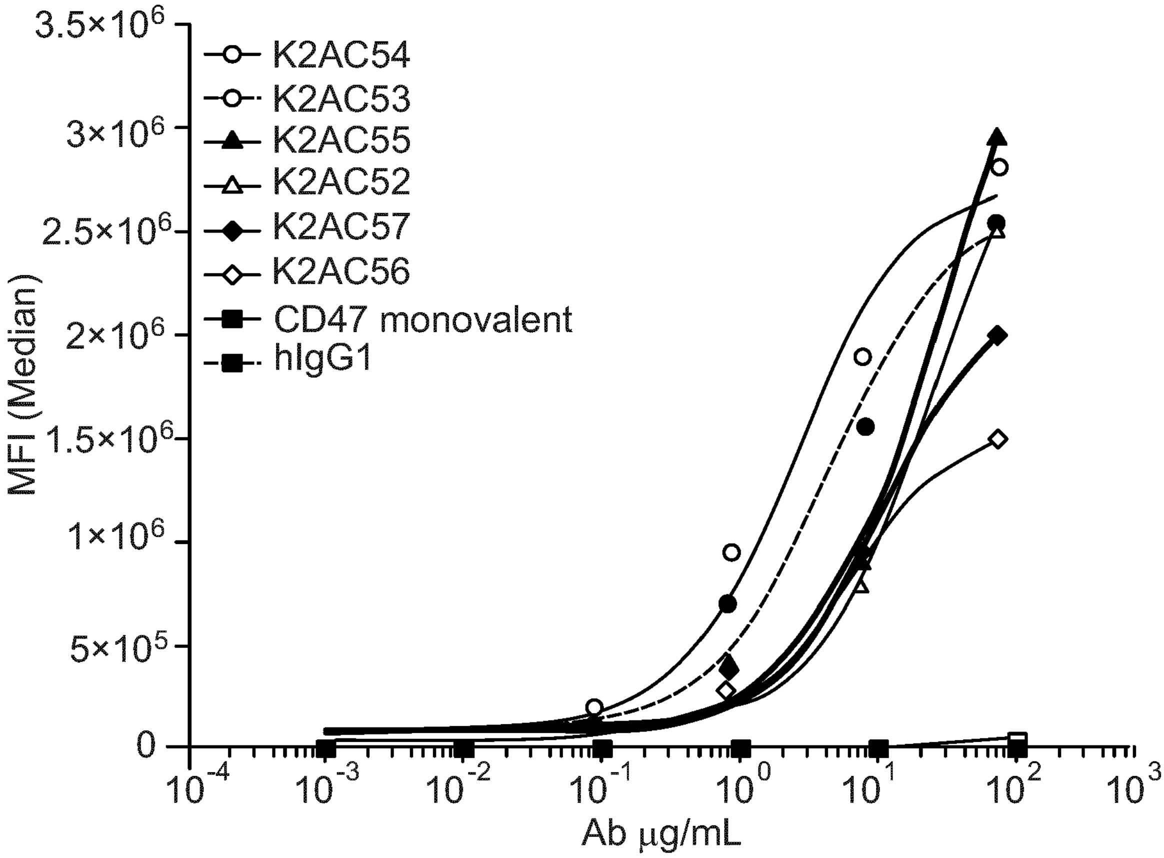
3.5×10^6
3×10^6
2.5×10^6
2×10^6
1.5×10^6
1×10^6
5×10^5
0
MFI (Median)
K2AC54
K2AC53
K2AC55
K2AC52
K2AC57
K2AC56
CD47 monovalent
hIgG1
10^-4
10^-3
10^-2
10^-1
10^0
10^1
10^2
10^3
Ab μg/mL

Fig. 2

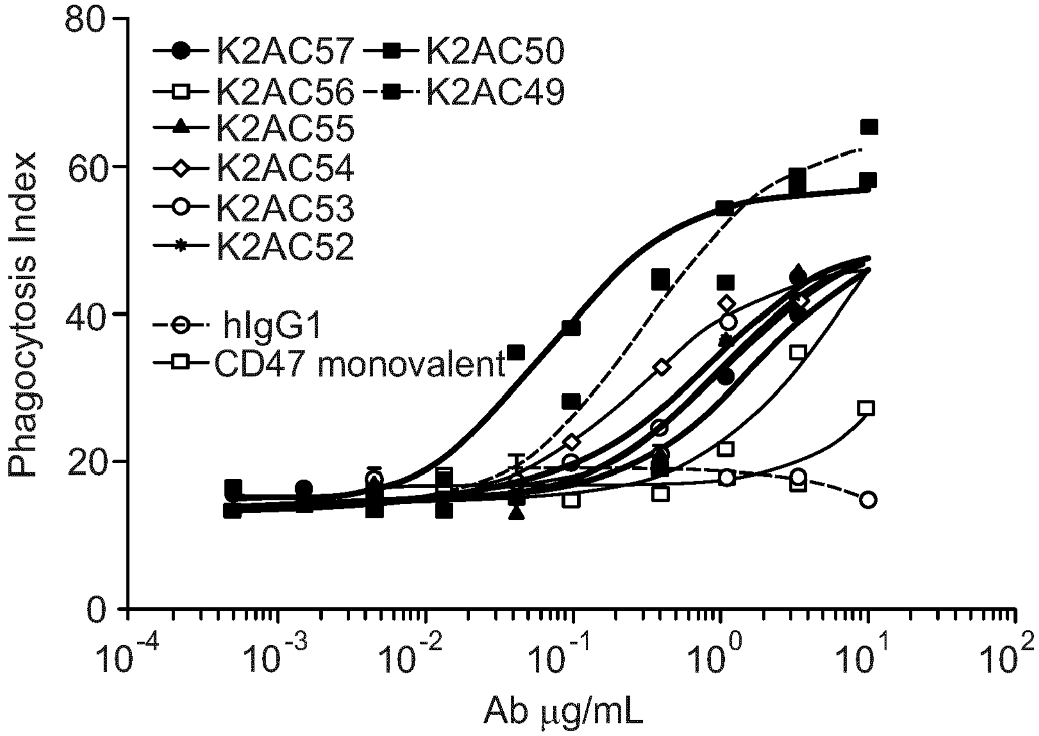
80
60
40
20
0
Phagocytosis Index
K2AC57
K2AC50
K2AC56
K2AC49
K2AC55
K2AC54
K2AC53
K2AC52
hIgG1
CD47 monovalent
$10^{-4}$
$10^{-3}$
$10^{-2}$
$10^{-1}$
$10^{0}$
$10^{1}$
$10^{2}$
Ab μg/mL

Fig. 3

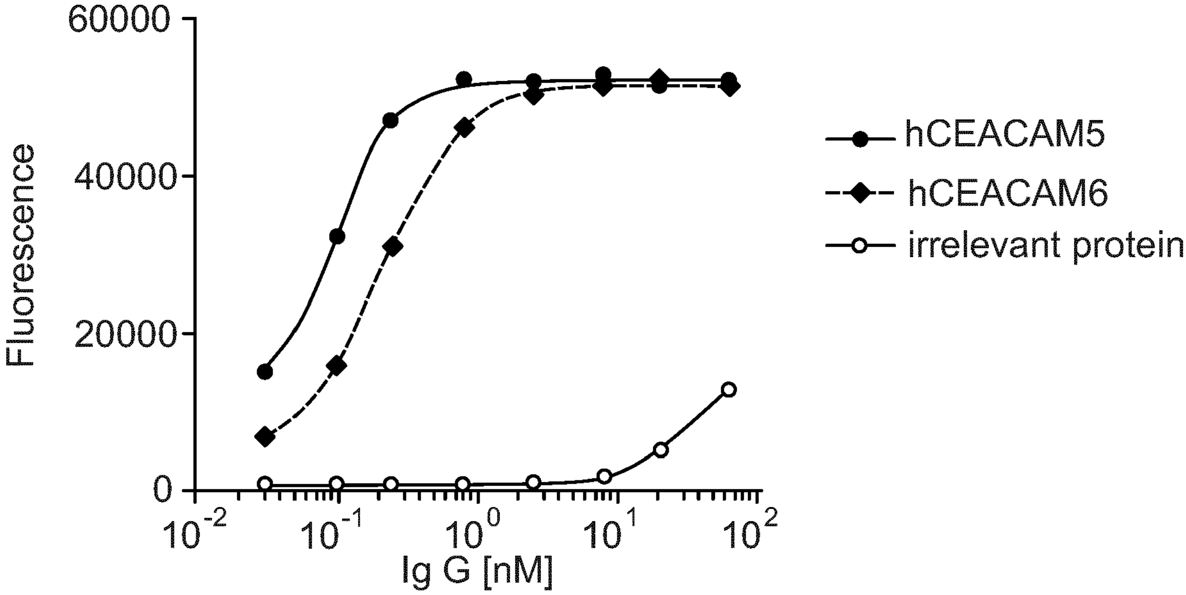
60000
40000
20000
0
Fluorescence
$10^{-2}$
$10^{-1}$
$10^{0}$
$10^{1}$
$10^{2}$
Ig G [nM]
hCEACAM5
hCEACAM6
irrelevant protein

Fig. 4

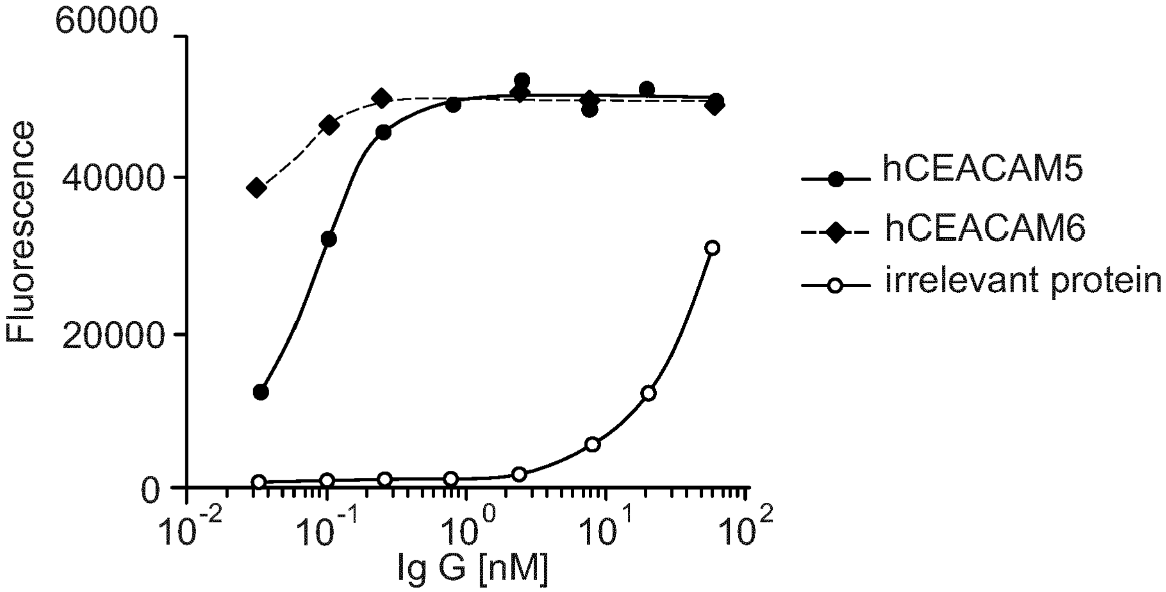
60000
40000
20000
0
Fluorescence
$10^{-2}$
$10^{-1}$
$10^{0}$
$10^{1}$
$10^{2}$
Ig G [nM]
hCEACAM5
hCEACAM6
irrelevant protein

Fig. 5

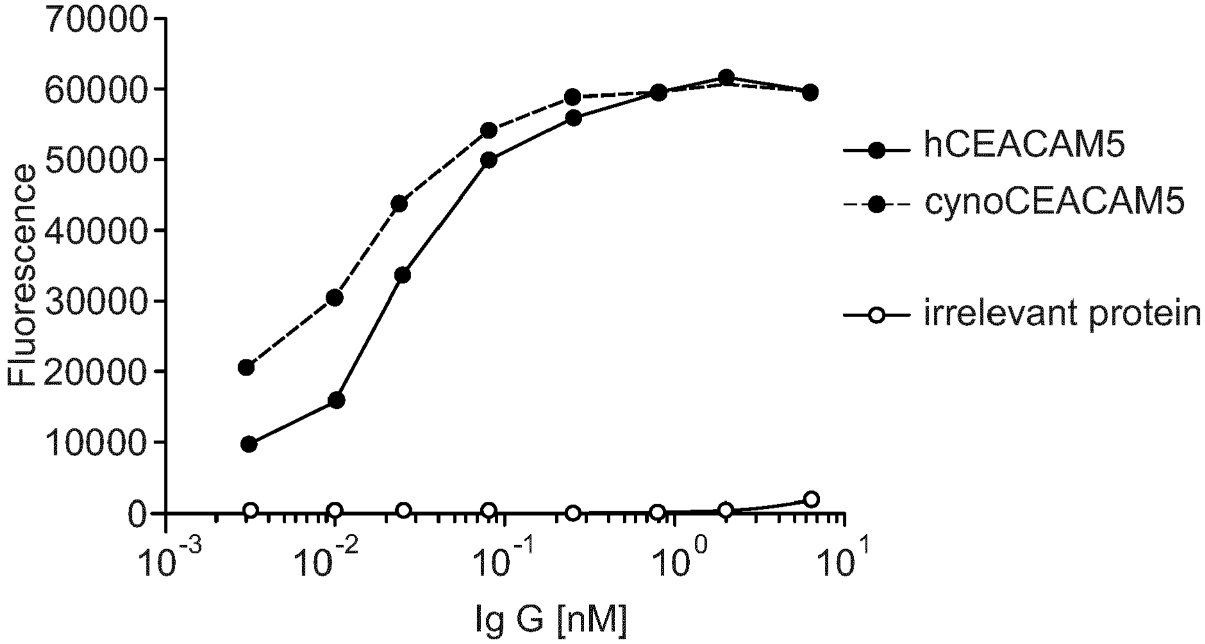
70000
60000
50000
40000
30000
20000
10000
0
Fluorescence
$10^{-3}$
$10^{-2}$
$10^{-1}$
$10^{0}$
$10^{1}$
Ig G [nM]
hCEACAM5
cynoCEACAM5
irrelevant protein

Fig. 6

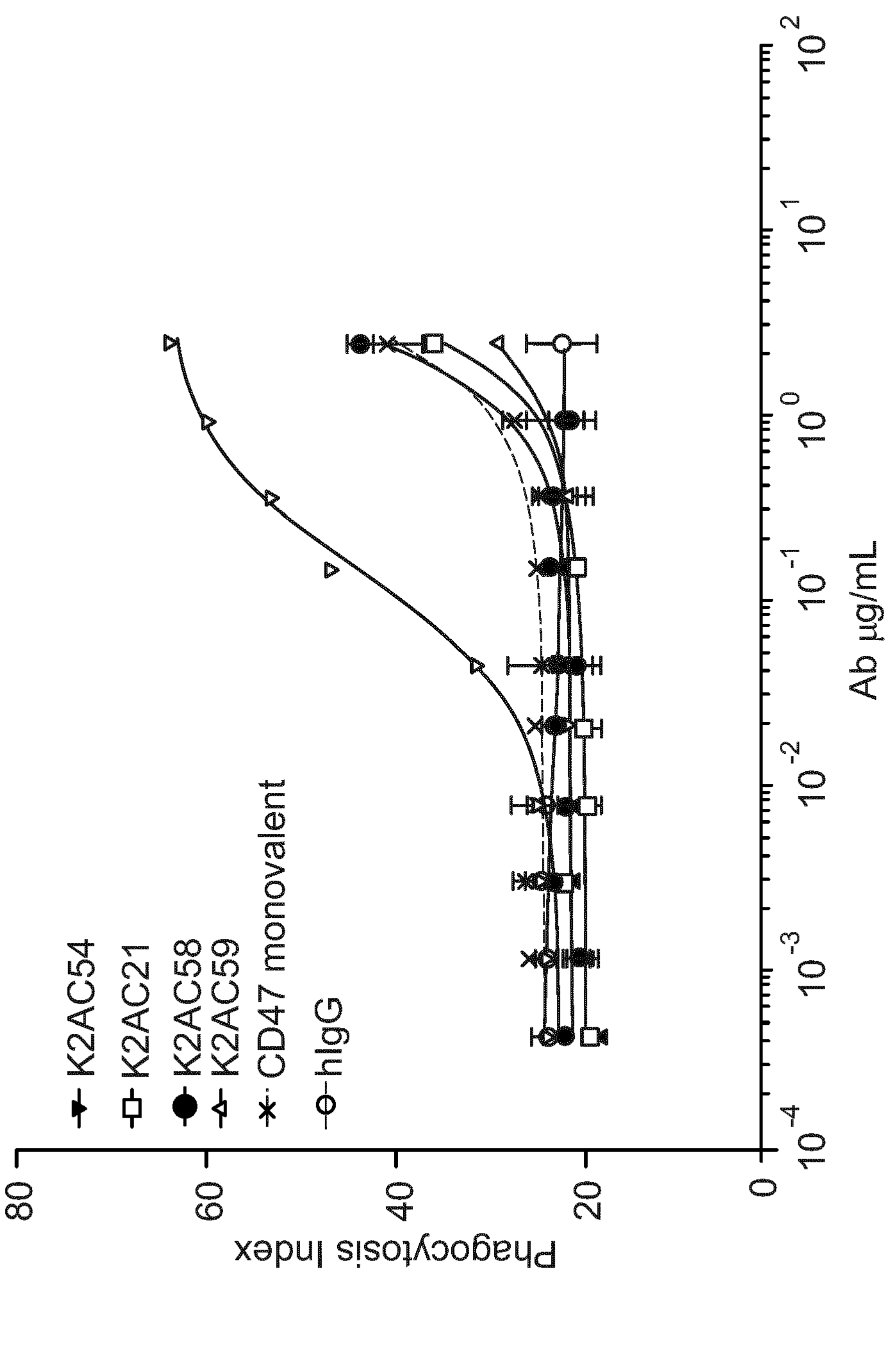
K2AC54
K2AC21
K2AC58
K2AC59
CD47 monovalent
hIgG
Phagocytosis Index
80
60
40
20
0
$10^{-4}$
$10^{-3}$
$10^{-2}$
$10^{-1}$
$10^{0}$
$10^{1}$
$10^{2}$
Ab µg/mL

Fig. 7

Europäisches Patentamt
European Patent Office
Office européen des brevets

# EUROPEAN SEARCH REPORT

Application Number
EP 19 21 3002

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 2014/087248 A2 (NOVIMMUNE SA [CH]) 12 June 2014 (2014-06-12) * paragraphs [0153], [0154], [0240]; claims 1-29 * | 1-16 | INV. C07K16/28 C07K16/30 |
| Y | WO 2018/098384 A1 (UNIV CALIFORNIA [US]) 31 May 2018 (2018-05-31) * paragraph [0037] * | 1-16 | |
| A | WO 2018/215835 A1 (NOVIMMUNE SA [CH]) 29 November 2018 (2018-11-29) * claims 1-30 * | 1-16 | |
| A | WO 2018/057955 A1 (ELSTAR THERAPEUTICS INC [US]) 29 March 2018 (2018-03-29) * page 26, line 9 - line 19; claims 1-127 * | 1-16 | |
| A | WO 2019/016411 A1 (NOVIMMUNE SA [CH]) 24 January 2019 (2019-01-24) * claims 1-23 * | 1-16 | |

TECHNICAL FIELDS SEARCHED (IPC)

C07K

~~The present search report has been drawn up for all claims~~

10

| Place of search | Date of completion of the search | Examiner |
|---|---|---|
| The Hague | 18 May 2020 | Le Flao, Katell |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number
EP 19 21 3002

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-16

☐ The present supplementary European search report has been drawn up for those parts of the European patent application which relate to the invention first mentioned in the claims (Rule 164 (1) EPC).

Europäisches Patentamt
European Patent Office
Office européen des brevets

## LACK OF UNITY OF INVENTION SHEET B

Application Number
EP 19 21 3002

The Search Division considers that the present European patent application does not comply with the requirements of unity of invention and relates to several inventions or groups of inventions, namely:

1. claims: 1-16

A bispecific antibody comprising a first binding part, binding to human CEACAM5 and a second binding part, binding to human CD47, characterized in comprising a) a heavy chain in both the first and second binding part characterised by CDRH1, CDRH2, and CDRH3 having amino acid sequences as in SEQ ID NO : 1-3, respectively ; b) a light chain in the first binding part characterised by CDRH1, CDRH2, and CDRH3 as defined in claim 1 as one of the 23 alternatives b1) to b23) : and c) a light chain in the second binding part characterised by CDRL1, CDRL2, and CDRL3 of SEQ ID NO:7-9.

---

2. claim: 17

A monoclonal antibody binding to CEACAM5 as defined in claim 17

---

## ANNEX TO THE EUROPEAN SEARCH REPORT ON EUROPEAN PATENT APPLICATION NO. EP 19 21 3002

This annex lists the patent family members relating to the patent documents cited in the above-mentioned European search report. The members are as contained in the European Patent Office EDP file on
The European Patent Office is in no way liable for these particulars which are merely given for the purpose of information.

18-05-2020

| Patent document cited in search report | Publication date | Patent family member(s) | Publication date |
|---|---|---|---|
| WO 2014087248 A2 | 12-06-2014 | AU 2013353763 A1 | 11-06-2015 |
| | | AU 2018220150 A1 | 13-09-2018 |
| | | CA 2892585 A1 | 12-06-2014 |
| | | CN 105121467 A | 02-12-2015 |
| | | CN 110183534 A | 30-08-2019 |
| | | DK 2925782 T3 | 06-04-2020 |
| | | EP 2925782 A2 | 07-10-2015 |
| | | JP 6392770 B2 | 19-09-2018 |
| | | JP 2015536351 A | 21-12-2015 |
| | | JP 2019023187 A | 14-02-2019 |
| | | PT 2925782 T | 22-04-2020 |
| | | RU 2015126533 A | 12-01-2017 |
| | | RU 2019118257 A | 24-06-2019 |
| | | US 2014303354 A1 | 09-10-2014 |
| | | US 2018291115 A1 | 11-10-2018 |
| | | US 2018291116 A1 | 11-10-2018 |
| | | US 2018355065 A1 | 13-12-2018 |
| | | WO 2014087248 A2 | 12-06-2014 |
| WO 2018098384 A1 | 31-05-2018 | US 2019276537 A1 | 12-09-2019 |
| | | WO 2018098384 A1 | 31-05-2018 |
| WO 2018215835 A1 | 29-11-2018 | AU 2018272311 A1 | 19-12-2019 |
| | | CA 3065008 A1 | 29-11-2018 |
| | | EP 3630167 A1 | 08-04-2020 |
| | | US 2018339031 A1 | 29-11-2018 |
| | | WO 2018215835 A1 | 29-11-2018 |
| WO 2018057955 A1 | 29-03-2018 | AU 2017331277 A1 | 28-03-2019 |
| | | CA 3036564 A1 | 29-03-2018 |
| | | CN 109906232 A | 18-06-2019 |
| | | EP 3515936 A1 | 31-07-2019 |
| | | JP 2019531725 A | 07-11-2019 |
| | | US 2019218311 A1 | 18-07-2019 |
| | | WO 2018057955 A1 | 29-03-2018 |
| WO 2019016411 A1 | 24-01-2019 | AU 2018303254 A1 | 05-03-2020 |
| | | CA 3070519 A1 | 24-01-2019 |
| | | CN 111201238 A | 26-05-2020 |
| | | EP 3655429 A1 | 27-05-2020 |
| | | US 2019031714 A1 | 31-01-2019 |
| | | WO 2019016411 A1 | 24-01-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012117002 A
- US 20140242079 A
- WO 2017118657 A
- WO 2015112534 A
- WO 2016116907 A
- US 20110064653 A
- WO 2000041474 A
- WO 2007042261 A
- WO 2008119565 A
- WO 2008119566 A
- WO 2008119567 A
- WO 2010037836 A
- WO 2010037837 A
- WO 2010037838 A
- US 8236308 B
- US 20140242079 A1
- US 20140140989 A
- WO 2017196793 A
- WO 2014087248 A
- US 6946292 B
- US 7425446 B
- US 8067232 B
- WO 199954342 A
- US 20030175884 A
- US 8642292 B
- WO 2004063351 A
- WO 2004099249 A
- WO 2005018669 A
- WO 2005063815 A
- WO 2005110474 A
- WO 2005056759 A
- WO 2005092925 A
- WO 2005018572 A
- WO 2006019447 A
- WO 2006116260 A
- WO 2006023420 A
- WO 2006047350 A
- WO 2006085967 A
- WO 2006105338 A
- WO 2007021841 A
- WO 2007008943 A
- WO 2007024249 A
- WO 2007041635 A
- WO 2007048077 A
- WO 2007044616 A
- WO 2007106707 A
- WO 2008022152 A
- WO 2008140603 A
- WO 2008036688 A
- WO 2008091798 A
- WO 2008091954 A
- WO 2008092117 A
- WO 2008098115 A
- WO 2008121160 A
- WO 2008150494 A
- WO 2010033736 A
- WO 2014113510 A
- WO 2017055389 A
- WO 201705389 A
- US 20160144009 A
- WO 2012023053 A
- WO 2013088259 A
- WO 2016156537 A
- WO 2007071426 A
- WO 2013012414 A
- WO 2017118675 A
- US 6602684 B
- US 20040241817 A
- WO 2004065540 A
- US 20030157108 A
- EP 1176195 A
- WO 2003084570 A
- WO 2003085119 A
- US 20030115614 A
- US 2004093621 A
- US 2004110282 A
- US 2004110704 A
- US 2004132140 A
- WO 2003056914 A
- WO 2004057002 A
- WO 2004024927 A
- EP 2681244 B1
- WO 200815671 A
- WO 2013173223 A
- WO 2015026634 A
- US 7521051 B
- US 8008449 B
- US 8354509 B
- WO 2009114335 A
- WO 2008156712 A
- WO 2003099196 A
- WO 2009101611 A
- WO 2010027423 A
- WO 2010027827 A
- WO 2010027828 A

- WO 2013019906 A
- WO 2010077634 A
- US 8383796 B
- WO 2007005874 A
- WO 2016007235 A
- WO 201705538 A
- US 20120184716 A
- US 20140303354 A
- US 20050147614 A1
- WO 2016036678 A1
- EP 3199552 A1
- US 20120251529 A
- US 20020165360 A1
- EP 2282773 A

**Non-patent literature cited in the description**


- **HAMMARSTRÖM S.** *Semin Cancer Biol.,* 1999, vol. 9 (2), 67-81
- **GOLD ; FREEDMAN.** *J Exp. Med.,* 1965, vol. 121, 439-462
- **BERINSTEIN N. L.** *J Clin Oncol.,* 2002, vol. 20, 2197-2207
- **SANDLER B. et al.** *Anticancer Res,* 1999, vol. 19 (5B), 4229-33
- **HAO C. ; ZHANG G.** *Progress in Molecular Biology and Translational Science,* 2019
- **WANEBO.** *New Eng. J. Med.,* 1978
- **JURGENSMERIER et al.** *Br. J. Cancer,* 2013
- **RICHMAN P. I. ; BODMER W. F.** *Int. J. Cancer,* 1987, vol. 39, 317-328
- **DURBIN H. et al.** *Proc. Natl. Scad. Sci. USA,* 1994, vol. 91, 4313-4317
- **STEWART et al.** *Cancer Immunol Immunother,* 1999, vol. 47, 299-06
- **CONAGHHAN P. J. et al.** *Br. J. Cancer,* 2008, vol. 98, 1217-1225
- **BLUMENTHAL RD.** *BMC Cancer,* 03 January 2007, vol. 7, 2
- **TABERNERO et al.** *J Clin Oncol,* 2017, vol. 35
- **YANG SJ.** *The Journal of Immunology,* 1986, vol. 137, 1097-1100
- **ANASETTI et al.** *Transplantation,* 1992, vol. 54, 844
- **SALMERON et al.** *J. Immunol.,* 1991, vol. 147, 3047
- **WEISKOPF K.** *European Journal of Cancer,* 2017, vol. 76, 100-109
- **HUANG Y et al.** *J Thorac Dis,* 2017, vol. 9 (2), E168-E174
- **VON BOMMEL PE et al.** *Oncoimmunol.,* 2018, vol. 7, e386361
- **PICCIONE EC et al.** *mAbs,* 2015, vol. 7, 946-956
- **DHEILLY E. et al.** *Mol. Thera.,* 2017, vol. 25, 523-533
- **L. CASSARD ; F. JOENSSON ; S. ARNAUD ; M. DAERON.** *J. Immunol.,* 2012, vol. 189, 2995-3006
- **BRUHNS P.** *Blood,* 2012, vol. 119, 5640
- **BRUHNS P.** *Blood,* 2009, vol. 113, 3716
- **SHIELDS, R. L. et al.** *J Biol. Chem.,* 2002, vol. 277, 26733-26740
- *J Biol. Chem.,* 2002, vol. 8, 8
- **OKAZAKI, A. et al.** *J Mol. Biol.,* 2004, vol. 336, 1239-1249
- **WORMALD et al.** *Biochemistry,* 1997, vol. 36, 1370-1380
- **LUND et al.** *J. Immunol.,* 1996, vol. 157, 4963-69
- **NIWA R et al.** *Cancer Res,* 2004, vol. 64, 2127-33
- **UMAÑA, P. et al.** *Nature Biotechnol.,* 1999, vol. 17, 176-180
- **XU.** *Expert Review of Anticancer Therapy,* 2017, vol. 17, 1099-1106
- **BACAC et al.** *Clin. Cancer Res.,* 2016, vol. 22 (13), 3286-97
- **J.TABERNERO.** *J. Clin. Oncol.,* 2017, vol. 35
- **KABAT et al.** Sequences of Proteins of Immunological Interest. Public Health Service, National Institutes of Health, 1991
- **CHOTHIA ; LESK.** *J. Mol. Biol.,* 1987, vol. 196, 901-917
- **MACCALLUM et al.** *J. Mol. Biol.,* 1996, vol. 262, 732-745
- **W. R. PEARSON ; D. J. LIPMAN.** Improved Tools for Biological Sequence Analysis. *PNAS,* 1988, vol. 85, 2444-2448
- **W. R. PEARSON.** Effective protein sequence comparison. *Meth. Enzymol.,* 1996, vol. 266, 227-258
- **PEARSON.** *Genomics,* 1997, vol. 46, 24-36, http://fasta.bioch.virginia.edu/fasta_www2/fasta_down.shtml.
- **JOHNSON, G. ; WU, T.T.** *Nucleic Acids Res.,* 2000, vol. 28, 214-218
- **KABAT, E.A. et al.** *Proc. Natl. Acad. Sci. USA,* 1975, vol. 72, 2785-2788
- **BERINSTEIN NL.** *J Clin Oncol.,* 2002, vol. 20, 2197-2207
- **KUESPERT K.** *Current opinion in Cell Biol,* 2006, vol. 18, 565-571
- **KABAT et al.** Sequences of Proteins of Immunological Interest. U.S. Dept. of Health and Human Services, 1983
- **CHOTHIA et al.** *J. Mol. Biol.,* 1987, vol. 196, 901-917

- **KABAT et al.** Sequence of Proteins of Immunological Interest. U.S. Dept. of Health and Human Services, 1983
- **DAVIS J. et al.** *Biotechnol. Bioeng.,* 2001, vol. 74, 288-294
- **SABA JA et al.** *Anal. Biochem.,* 2002, vol. 305, 16-31
- **KANDA Y et al.** *J. Biotechnol.,* 2007, vol. 130, 300-310
- **MORI K et al.** *Biotechnol. Bioeng.,* 2004, vol. 88, 901-908
- **YAMANE-OHNUKI N. et al.** *Biotech. Bioeng.,* 2004, vol. 87, 614-622
- **RICHARDS JO et al.** *Mol. Cancer Ther.,* 2008, vol. 7, 2517-2527
- Remington's Pharmaceutical Sciences. 1980
- **VAUGHAN et al.** *Nat. Biotech.,* 1996, vol. 14, 309-314
- **KLEIN et al.** *Oncoimmunology,* 11 January 2017, vol. 6 (3
- **RILLAHAN et al.** *Nature Chem. Biol.,* July 2012, vol. 8 (7), 661-8
- **NAOKO YAMANE-OHNUKI et al.** *Biotech. Bioeng.,* 2004, vol. 87, 614-622
- **HAYASHI.** *DNA Seq,* 2000, vol. 11, 91-96
- **RAMIREZ-SOLIS et al.** *Anal Biochem,* 1992, vol. 201, 331-335

SEQUENCE LISTING

<110> LamKap Bio beta AG

<120> BISPECIFIC ANTIBODIES AGAINST CEACAM5 AND CD47

<130> LCD47_1

<160> 192

<170> BiSSAP 1.3.6

<210> 1
<211> 5
<212> PRT
<213> Artificial Sequence

<220>
<223> MAB CD47 CDRH1

<400> 1
Ser Tyr Ala Met Ser
1               5

<210> 2
<211> 17
<212> PRT
<213> Artificial Sequence

<220>
<223> MAB CD47 CDRH2

<400> 2
Ala Ile Ser Gly Ser Gly Gly Ser Thr Tyr Tyr Ala Asp Ser Val Lys
1               5                   10                  15
Gly

<210> 3
<211> 7
<212> PRT
<213> Artificial Sequence

<220>
<223> MAB CD47 CDRH3

<400> 3
Ser Tyr Gly Ala Phe Asp Tyr
1               5

<210> 4
<211> 116
<212> PRT
<213> Artificial Sequence

<220>
<223> MAB CD47 VH (kappa)

<400> 4

```
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Ala Met Ser Trp Val Arg Gln Ala Pro Gly Lys Gly Leu Glu Trp Val
        35                  40                  45
Ser Ala Ile Ser Gly Ser Gly Gly Ser Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys Ser Tyr Gly Ala Phe Asp Tyr Trp Gly Gln Gly Thr Leu Val
            100                 105                 110
Thr Val Ser Ser
        115

<210> 5
<211> 229
<212> PRT
<213> Artificial Sequence


<220>
<223> MAB CD47 heavy chain VH-CH1

<400> 5
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Ala Met Ser Trp Val Arg Gln Ala Pro Gly Lys Gly Leu Glu Trp Val
        35                  40                  45
Ser Ala Ile Ser Gly Ser Gly Gly Ser Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys Ser Tyr Gly Ala Phe Asp Tyr Trp Gly Gln Gly Thr Leu Val
            100                 105                 110
Thr Val Ser Ser Ala Ser Thr Lys Gly Pro Ser Val Phe Pro Leu Ala
        115                 120                 125
Pro Ser Ser Lys Ser Thr Ser Gly Gly Thr Ala Ala Leu Gly Cys Leu
    130                 135                 140
Val Lys Asp Tyr Phe Pro Glu Pro Val Thr Val Ser Trp Asn Ser Gly
145                 150                 155                 160
Ala Leu Thr Ser Gly Val His Thr Phe Pro Ala Val Leu Gln Ser Ser
                165                 170                 175
Gly Leu Tyr Ser Leu Ser Ser Val Val Thr Val Pro Ser Ser Ser Leu
            180                 185                 190
Gly Thr Gln Thr Tyr Ile Cys Asn Val Asn His Lys Pro Ser Asn Thr
        195                 200                 205
Lys Val Asp Lys Arg Val Glu Pro Lys Ser Cys Asp Lys Thr His Thr
    210                 215                 220
Cys Pro Pro Cys Pro
225

<210> 6
<211> 445
<212> PRT
<213> Artificial Sequence
```

<220>
<223> MAB CD47 heavy chain with Fc

<400> 6
```
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Ala Met Ser Trp Val Arg Gln Ala Pro Gly Lys Gly Leu Glu Trp Val
        35                  40                  45
Ser Ala Ile Ser Gly Ser Gly Gly Ser Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys Ser Tyr Gly Ala Phe Asp Tyr Trp Gly Gln Gly Thr Leu Val
            100                 105                 110
Thr Val Ser Ser Ala Ser Thr Lys Gly Pro Ser Val Phe Pro Leu Ala
        115                 120                 125
Pro Ser Ser Lys Ser Thr Ser Gly Gly Thr Ala Ala Leu Gly Cys Leu
    130                 135                 140
Val Lys Asp Tyr Phe Pro Glu Pro Val Thr Val Ser Trp Asn Ser Gly
145                 150                 155                 160
Ala Leu Thr Ser Gly Val His Thr Phe Pro Ala Val Leu Gln Ser Ser
                165                 170                 175
Gly Leu Tyr Ser Leu Ser Ser Val Val Thr Val Pro Ser Ser Ser Leu
            180                 185                 190
Gly Thr Gln Thr Tyr Ile Cys Asn Val Asn His Lys Pro Ser Asn Thr
        195                 200                 205
Lys Val Asp Lys Arg Val Glu Pro Lys Ser Cys Asp Lys Thr His Thr
    210                 215                 220
Cys Pro Pro Cys Pro Ala Pro Glu Leu Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser His Glu Asp Pro Glu Val
            260                 265                 270
Lys Phe Asn Trp Tyr Val Asp Gly Val Glu Val His Asn Ala Lys Thr
        275                 280                 285
Lys Pro Arg Glu Glu Gln Tyr Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Ala Leu Pro Ala Pro Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Arg Glu Glu Met Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Lys Leu Thr Val Asp Lys Ser Arg Trp
                405                 410                 415
Gln Gln Gly Asn Val Phe Ser Cys Ser Val Met His Glu Ala Leu His
            420                 425                 430
Asn His Tyr Thr Gln Lys Ser Leu Ser Leu Ser Pro Gly
        435                 440                 445
```

<210> 7

<211> 11
<212> PRT
<213> Artificial Sequence

<220>
<223> MAB CD47 CDRL1

<400> 7
Arg Ala Ser Gln Ser Ile Ser Ser Tyr Leu Asn
1               5                   10

<210> 8
<211> 7
<212> PRT
<213> Artificial Sequence

<220>
<223> MAB CD47 CDRL2

<400> 8
Ala Ala Ser Ser Leu Gln Ser
1               5

<210> 9
<211> 10
<212> PRT
<213> Artificial Sequence

<220>
<223> MAB CD47 CDRL3

<400> 9
Gln Gln Met His Pro Arg Ala Pro Lys Thr
1               5                   10

<210> 10
<211> 108
<212> PRT
<213> Artificial Sequence

<220>
<223> MAB CD47 VK

<400> 10
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Ser Ile Ser Ser Tyr
            20                  25                  30
Leu Asn Trp Tyr Gln Gln Lys Pro Gly Lys Ala Pro Lys Leu Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Met His Pro Arg Ala Pro
                85                  90                  95
Lys Thr Phe Gly Gln Gly Thr Lys Val Glu Ile Lys
            100                 105

<210> 11
<211> 215
<212> PRT
<213> Artificial Sequence

<220>
<223> Mab CD47 light chain (VKCK;K2)

<400> 11
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Ser Ile Ser Ser Tyr
            20                  25                  30
Leu Asn Trp Tyr Gln Gln Lys Pro Gly Lys Ala Pro Lys Leu Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Met His Pro Arg Ala Pro
                85                  90                  95
Lys Thr Phe Gly Gln Gly Thr Lys Val Glu Ile Lys Arg Thr Val Ala
            100                 105                 110
Ala Pro Ser Val Phe Ile Phe Pro Pro Ser Asp Glu Gln Leu Lys Ser
        115                 120                 125
Gly Thr Ala Ser Val Val Cys Leu Leu Asn Asn Phe Tyr Pro Arg Glu
    130                 135                 140
Ala Lys Val Gln Trp Lys Val Asp Asn Ala Leu Gln Ser Gly Asn Ser
145                 150                 155                 160
Gln Glu Ser Val Thr Glu Gln Asp Ser Lys Asp Ser Thr Tyr Ser Leu
                165                 170                 175
Ser Ser Thr Leu Thr Leu Ser Lys Ala Asp Tyr Glu Lys His Lys Val
            180                 185                 190
Tyr Ala Cys Glu Val Thr His Gln Gly Leu Ser Ser Pro Val Thr Lys
        195                 200                 205
Ser Phe Asn Arg Gly Glu Cys
    210                 215

<210> 12
<211> 648
<212> DNA
<213> Artificial Sequence

<220>
<223> Mab CD47 light chain (VKCK; nucleic acid) K2

<400> 12
gacatccaga tgacccagtc tccatcctcc ctgtctgcat ctgtaggaga cagagtcacc 60

atcacttgcc gggcaagtca gagcattagc agctatttaa attggtatca gcagaaacca 120

gggaaagccc ctaagctcct gatctatgct gcatccagtt tgcaaagtgg ggtcccatca 180

aggttcagtg gcagtggatc tgggacagat ttcactctca ccatcagcag tctgcaacct 240

gaagattttg caacttacta ctgtcagcag atgcacccgc gcgccccgaa gaccttcggc 300

caagggacca aggtggaaat caaacgtacg gtggctgcac catctgtctt catcttcccg 360

ccatctgatg agcagttgaa atctggaact gcctctgttg tgtgcctgct gaataacttc 420

tatcccagag aggccaaagt acagtggaag gtggataacg ccctccaatc gggtaactcc 480

caggagagtg tcacagagca ggacagcaag gacagcacct acagcctcag cagcaccctg 540

acgctgagca aagcagacta cgagaaacac aaagtctacg cctgcgaagt cacccatcag 600

ggcctgagct cgcccgtcac aaagagcttc aacaggggag agtgttaa 648

<210> 13
<211> 107
<212> PRT
<213> Artificial Sequence

<220>
<223> Mab CD47 CK constant light chain kappa

<400> 13
Arg Thr Val Ala Ala Pro Ser Val Phe Ile Phe Pro Pro Ser Asp Glu
1               5                   10                  15
Gln Leu Lys Ser Gly Thr Ala Ser Val Val Cys Leu Leu Asn Asn Phe
            20                  25                  30
Tyr Pro Arg Glu Ala Lys Val Gln Trp Lys Val Asp Asn Ala Leu Gln
        35                  40                  45
Ser Gly Asn Ser Gln Glu Ser Val Thr Glu Gln Asp Ser Lys Asp Ser
    50                  55                  60
Thr Tyr Ser Leu Ser Ser Thr Leu Thr Leu Ser Lys Ala Asp Tyr Glu
65                  70                  75                  80
Lys His Lys Val Tyr Ala Cys Glu Val Thr His Gln Gly Leu Ser Ser
                85                  90                  95
Pro Val Thr Lys Ser Phe Asn Arg Gly Glu Cys
            100                 105

<210> 14
<211> 106
<212> PRT
<213> Artificial Sequence

<220>
<223> Mab CD47 CL constant light chain lambda

<400> 14
Gly Gln Pro Lys Ala Ala Pro Ser Val Thr Leu Phe Pro Pro Ser Ser
1               5                   10                  15
Glu Glu Leu Gln Ala Asn Lys Ala Thr Leu Val Cys Leu Ile Ser Asp
            20                  25                  30
Phe Tyr Pro Gly Ala Val Thr Val Ala Trp Lys Ala Asp Ser Ser Pro
        35                  40                  45
Val Lys Ala Gly Val Glu Thr Thr Thr Pro Ser Lys Gln Ser Asn Asn
    50                  55                  60
Lys Tyr Ala Ala Ser Ser Tyr Leu Ser Leu Thr Pro Glu Gln Trp Lys
65                  70                  75                  80
Ser His Arg Ser Tyr Ser Cys Gln Val Thr His Glu Gly Ser Thr Val
                85                  90                  95
Glu Lys Thr Val Ala Pro Thr Glu Cys Ser
            100                 105

<210> 15
<211> 106
<212> PRT
<213> Artificial Sequence

<220>
<223> AC CEA CL constant light chain lambda

<400> 15
Gly Gln Pro Lys Ala Ala Pro Ser Val Thr Leu Phe Pro Pro Ser Ser
1               5                   10                  15
Glu Glu Leu Gln Ala Asn Lys Ala Thr Leu Val Cys Leu Ile Ser Asp
            20                  25                  30
Phe Tyr Pro Gly Ala Val Thr Val Ala Trp Lys Ala Asp Ser Ser Pro
        35                  40                  45
Val Lys Ala Gly Val Glu Thr Thr Thr Pro Ser Lys Gln Ser Asn Asn
    50                  55                  60
Lys Tyr Ala Ala Ser Ser Tyr Leu Ser Leu Thr Pro Glu Gln Trp Lys
65                  70                  75                  80
Ser His Arg Ser Tyr Ser Cys Gln Val Thr His Glu Gly Ser Thr Val
                85                  90                  95
Glu Lys Thr Val Ala Pro Thr Glu Cys Ser
            100                 105

<210> 16
<211> 107
<212> PRT
<213> Artificial Sequence

<220>
<223> AC CEA CK constant light chain kappa

<400> 16
Arg Thr Val Ala Ala Pro Ser Val Phe Ile Phe Pro Pro Ser Asp Glu
1               5                   10                  15
Gln Leu Lys Ser Gly Thr Ala Ser Val Val Cys Leu Leu Asn Asn Phe
            20                  25                  30
Tyr Pro Arg Glu Ala Lys Val Gln Trp Lys Val Asp Asn Ala Leu Gln
        35                  40                  45
Ser Gly Asn Ser Gln Glu Ser Val Thr Glu Gln Asp Ser Lys Asp Ser
    50                  55                  60
Thr Tyr Ser Leu Ser Ser Thr Leu Thr Leu Ser Lys Ala Asp Tyr Glu
65                  70                  75                  80
Lys His Lys Val Tyr Ala Cys Glu Val Thr His Gln Gly Leu Ser Ser
                85                  90                  95
Pro Val Thr Lys Ser Phe Asn Arg Gly Glu Cys
            100                 105

<210> 17
<211> 11
<212> PRT
<213> Artificial Sequence

<220>
<223> AC41 CDRL1

<400> 17
Gln Ala Ser Gln Glu Ile Ala Ala Thr Leu Asn
1               5                   10

<210> 18
<211> 7
<212> PRT
<213> Artificial Sequence

<220>
<223> AC41 CDRL2

<400> 18
Asn Tyr Ser Thr Leu Glu Thr
1 5

<210> 19
<211> 11
<212> PRT
<213> Artificial Sequence

<220>
<223> AC41 CDRL3

<400> 19
Gln Gln Val Gly Leu Asn Pro Glu Asn Thr Thr
1 5 10

<210> 20
<211> 10
<212> PRT
<213> Artificial Sequence

<220>
<223> AC42 CDRL1

<400> 20
Ala Ser Gln Arg Ile Ala Arg Thr Leu Asn
1 5 10

<210> 21
<211> 7
<212> PRT
<213> Artificial Sequence

<220>
<223> AC42 CDRL2

<400> 21
Asn Phe Ser Ala Leu Glu Thr
1 5

<210> 22
<211> 11
<212> PRT
<213> Artificial Sequence

<220>
<223> AC42 CDRL3

<400> 22
Gln Gln Val Gly Leu Asp Pro Asn Leu Thr Thr
1 5 10

<210> 23
<211> 11

<212> PRT
<213> Artificial Sequence

<220>
<223> AC43 CDRL1

<400> 23
Gln Ala Ser Gln Asn Ile Pro Arg Thr Ile Asn
1               5                   10

<210> 24
<211> 7
<212> PRT
<213> Artificial Sequence

<220>
<223> AC43 CDRL2

<400> 24
Asp Ile Ser Asn Leu Glu Thr
1               5

<210> 25
<211> 11
<212> PRT
<213> Artificial Sequence

<220>
<223> AC43 CDRL3

<400> 25
Gln Gln Val Gly Leu Asn Pro Glu Leu Thr Thr
1               5                   10

<210> 26
<211> 13
<212> PRT
<213> Artificial Sequence

<220>
<223> AC44 CDRL1

<400> 26
Ser Gly Ser Ser Ser Asn Ile Val Gly Val Gly Val Asn
1               5                   10

<210> 27
<211> 7
<212> PRT
<213> Artificial Sequence

<220>
<223> AC44 CDRL2

<400> 27
Ser Tyr Asn Lys Arg Pro Ser
1               5

<210> 28
<211> 13
<212> PRT
<213> Artificial Sequence

<220>
<223> AC44 CDRL3

<400> 28
Ala Ala Tyr Asp Phe Leu Pro Val Leu Pro Ala Pro Val
1 5 10

<210> 29
<211> 13
<212> PRT
<213> Artificial Sequence

<220>
<223> AC45 CDRL1

<400> 29
Ser Gly Ser Ser Ser Asn Ile Val Gly Val Gly Val Asn
1 5 10

<210> 30
<211> 7
<212> PRT
<213> Artificial Sequence

<220>
<223> AC45 CDRL2

<400> 30
Ser Tyr Asn Lys Arg Pro Ser
1 5

<210> 31
<211> 13
<212> PRT
<213> Artificial Sequence

<220>
<223> AC45 CDRL3

<400> 31
Ala Ala Tyr Asp Ala Asp Met Ile Val Pro Ala Pro Val
1 5 10

<210> 32
<211> 13
<212> PRT
<213> Artificial Sequence

<220>
<223> AC46 CDRL1

<400> 32
Ser Gly Ser Ser Ser Asn Ile Val Gly Val Gly Val Asn

1 5 10

<210> 33
<211> 7
<212> PRT
<213> Artificial Sequence

<220>
<223> AC46 CDRL2

<400> 33
Ser Tyr Asn Lys Arg Pro Ser
1 5

<210> 34
<211> 13
<212> PRT
<213> Artificial Sequence

<220>
<223> AC46 CDRL3

<400> 34
Ala Ala Tyr Asp Phe Ser Leu Leu Leu Pro Ala Pro Val
1 5 10

<210> 35
<211> 13
<212> PRT
<213> Artificial Sequence

<220>
<223> AC47 CDRL1

<400> 35
Ser Gly Ser Ser Ser Asn Ile Asn Gly Val Gly Val Asn
1 5 10

<210> 36
<211> 7
<212> PRT
<213> Artificial Sequence

<220>
<223> AC47 CDRL2

<400> 36
Gly Phe Asn Ser Arg Pro Ser
1 5

<210> 37
<211> 13
<212> PRT
<213> Artificial Sequence

<220>
<223> AC47 CDRL3

<400> 37
Ala Ala Tyr Asp Met Ser Thr Tyr Val Pro Ala Pro Val
1 5 10

<210> 38
<211> 13
<212> PRT
<213> Artificial Sequence

<220>
<223> AC48 CDRL1

<400> 38
Ser Gly Ser Ser Ser Asn Ile Asn Gly Val Gly Val Asn
1 5 10

<210> 39
<211> 7
<212> PRT
<213> Artificial Sequence

<220>
<223> AC48 CDRL2

<400> 39
Glu Phe Asn Lys Arg Pro Ser
1 5

<210> 40
<211> 13
<212> PRT
<213> Artificial Sequence

<220>
<223> AC48 CDRL3

<400> 40
Ala Ala Tyr Asp Ala Ala Arg Tyr Val Pro Ala Pro Val
1 5 10

<210> 41
<211> 13
<212> PRT
<213> Artificial Sequence

<220>
<223> AC49 CDRL1

<400> 41
Ser Gly Ser Ser Ser Asn Ile Phe Asp Ser Gly Val Ser
1 5 10

<210> 42
<211> 7
<212> PRT
<213> Artificial Sequence

<220>

<223> AC49 CDRL2

<400> 42
Asn His Asn Glu Arg Pro Ser
1               5

<210> 43
<211> 11
<212> PRT
<213> Artificial Sequence

<220>
<223> AC49 CDRL3

<400> 43
Gly Thr Trp Asp Phe Pro Pro Ser Arg Phe Val
1               5                   10

<210> 44
<211> 14
<212> PRT
<213> Artificial Sequence

<220>
<223> AC50 CDRL1

<400> 44
Thr Gly Thr Ser Ser Asp Val Tyr Glu Phe Arg Leu Val Ser
1               5                   10

<210> 45
<211> 7
<212> PRT
<213> Artificial Sequence

<220>
<223> AC50 CDRL2

<400> 45
Phe Asp Ser Leu Arg Pro Ser
1               5

<210> 46
<211> 13
<212> PRT
<213> Artificial Sequence

<220>
<223> AC50 CDRL3

<400> 46
Ser Ser Trp Asp Lys Phe Gly Tyr Ala Phe Asn Ser Val
1               5                   10

<210> 47
<211> 14
<212> PRT
<213> Artificial Sequence

<220>
<223> AC52 CDRL1

<400> 47
Thr Gly Ser Ser Ser Asn Ile His Asp Val Gly Tyr Val His
1 5 10

<210> 48
<211> 7
<212> PRT
<213> Artificial Sequence

<220>
<223> AC52 CDRL2

<400> 48
Gly Ala Ser Gly Arg Pro Ser
1 5

<210> 49
<211> 13
<212> PRT
<213> Artificial Sequence

<220>
<223> AC52 CDRL3

<400> 49
Gln Ser Tyr Asp Thr Lys Arg Glu Phe Pro Thr Pro Val
1 5 10

<210> 50
<211> 14
<212> PRT
<213> Artificial Sequence

<220>
<223> AC53 CDRL1

<400> 50
Thr Gly Ser Ser Ser Asn Ile His Asp Val Gly Tyr Val His
1 5 10

<210> 51
<211> 7
<212> PRT
<213> Artificial Sequence

<220>
<223> AC53 CDRL2

<400> 51
Gly Ala Ser Gly Arg Pro Ser
1 5

<210> 52
<211> 13
<212> PRT

<213> Artificial Sequence

<220>
<223> AC53 CDRL3

<400> 52
Gln Ser Tyr Asp Gly Arg Ala Val Phe Pro Thr Pro Val
1               5                   10

<210> 53
<211> 14
<212> PRT
<213> Artificial Sequence

<220>
<223> AC54 CDRL1

<400> 53
Thr Gly Ser Ser Ser Asn Ile His Asp Val Gly Tyr Val His
1               5                   10

<210> 54
<211> 7
<212> PRT
<213> Artificial Sequence

<220>
<223> AC54 CDRL2

<400> 54
Gly Ala Ser Gly Arg Pro Ser
1               5

<210> 55
<211> 13
<212> PRT
<213> Artificial Sequence

<220>
<223> AC54 CDRL3

<400> 55
Gln Ser Tyr Asp Thr Arg Gln Val Val Pro Thr Pro Val
1               5                   10

<210> 56
<211> 14
<212> PRT
<213> Artificial Sequence

<220>
<223> AC55 CDRL1

<400> 56
Thr Gly Ser Ser Ser Asn Ile His Asp Val Gly Tyr Val His
1               5                   10

<210> 57

<211> 7
<212> PRT
<213> Artificial Sequence

<220>
<223> AC55 CDRL2

<400> 57
Gly Ala Ser Gly Arg Pro Ser
1 5

<210> 58
<211> 13
<212> PRT
<213> Artificial Sequence

<220>
<223> AC55 CDRL3

<400> 58
Gln Ser Tyr Asp Thr Ser Ala Val Phe Pro Thr Pro Val
1 5 10

<210> 59
<211> 14
<212> PRT
<213> Artificial Sequence

<220>
<223> AC56 CDRL1

<400> 59
Thr Gly Ser Ser Ser Asn Ile Ile Asn Asn Ser Tyr Val His
1 5 10

<210> 60
<211> 7
<212> PRT
<213> Artificial Sequence

<220>
<223> AC56 CDRL2

<400> 60
Ala Pro Ser Asn Arg Pro Ser
1 5

<210> 61
<211> 13
<212> PRT
<213> Artificial Sequence

<220>
<223> AC56 CDRL3

<400> 61
Gln Ser Tyr Asp Thr Thr Gly Val Met Pro Thr Pro Val
1 5 10

<210> 62
<211> 14
<212> PRT
<213> Artificial Sequence

<220>
<223> AC57 CDRL1

<400> 62
Thr Gly Ser Ser Ser Asn Ile Ile Asn Asn Ser Tyr Val His
1 5 10

<210> 63
<211> 7
<212> PRT
<213> Artificial Sequence

<220>
<223> AC57 CDRL2

<400> 63
Ala Pro Ser Asn Arg Pro Ser
1 5

<210> 64
<211> 13
<212> PRT
<213> Artificial Sequence

<220>
<223> AC57 CDRL3

<400> 64
Gln Ser Tyr Asp Thr Arg Phe Val Val Pro Thr Pro Val
1 5 10

<210> 65
<211> 14
<212> PRT
<213> Artificial Sequence

<220>
<223> AC58 CDRL1

<400> 65
Thr Gly Ser Ser Ser Asn Ile Gly Val Ala His Asp Val His
1 5 10

<210> 66
<211> 7
<212> PRT
<213> Artificial Sequence

<220>
<223> AC58 CDRL2

<400> 66

Asp Tyr Ser Glu Arg Pro Ser
1 5

<210> 67
<211> 13
<212> PRT
<213> Artificial Sequence

<220>
<223> AC58 CDRL3

<400> 67
Gln Ser Trp Asp Asn Gly Pro Asn Gly Leu Val Trp Val
1 5 10

<210> 68
<211> 14
<212> PRT
<213> Artificial Sequence

<220>
<223> AC59 CDRL1

<400> 68
Thr Gly Ser Ser Ser Asn Ile Gly Val Ala His Asp Val His
1 5 10

<210> 69
<211> 7
<212> PRT
<213> Artificial Sequence

<220>
<223> AC59 CDRL2

<400> 69
Asp Tyr Ser Glu Arg Pro Ser
1 5

<210> 70
<211> 13
<212> PRT
<213> Artificial Sequence

<220>
<223> AC59 CDRL3

<400> 70
Gln Ser Trp Asp Asn Ser Glu Asn Gly Leu Ile Tyr Val
1 5 10

<210> 71
<211> 11
<212> PRT
<213> Artificial Sequence

<220>
<223> AC60 CDRL1

<400> 71
Gln Ala Ser Gln Asn Ile Pro Arg Thr Ile Asn
1 5 10

<210> 72
<211> 7
<212> PRT
<213> Artificial Sequence

<220>
<223> AC60 CDRL2

<400> 72
Asp Ile Ser Asn Leu Glu Thr
1 5

<210> 73
<211> 11
<212> PRT
<213> Artificial Sequence

<220>
<223> AC60 CDRL3

<400> 73
Gln Gln Val Gly Leu Asn Trp Asp Asp Arg Ala
1 5 10

<210> 74
<211> 216
<212> PRT
<213> Artificial Sequence

<220>
<223> AC41 VKCK

<400> 74
Asp Ile Gln Met Thr Gln Ser Pro Ser Ser Leu Ser Ala Ser Val Gly
1 5 10 15
Asp Arg Val Thr Ile Thr Cys Gln Ala Ser Gln Glu Ile Ala Ala Thr
20 25 30
Leu Asn Trp Tyr Gln Gln Lys Pro Gly Lys Ala Pro Lys Leu Leu Ile
35 40 45
Tyr Asn Tyr Ser Thr Leu Glu Thr Gly Val Pro Ser Arg Phe Ser Gly
50 55 60
Ser Gly Ser Gly Thr Asp Phe Thr Phe Thr Ile Ser Ser Leu Gln Pro
65 70 75 80
Glu Asp Ile Ala Thr Tyr Tyr Cys Gln Gln Val Gly Leu Asn Pro Glu
85 90 95
Asn Thr Thr Phe Gly Gln Gly Thr Lys Val Glu Ile Lys Arg Thr Val
100 105 110
Ala Ala Pro Ser Val Phe Ile Phe Pro Pro Ser Asp Glu Gln Leu Lys
115 120 125
Ser Gly Thr Ala Ser Val Val Cys Leu Leu Asn Asn Phe Tyr Pro Arg
130 135 140
Glu Ala Lys Val Gln Trp Lys Val Asp Asn Ala Leu Gln Ser Gly Asn
145 150 155 160
Ser Gln Glu Ser Val Thr Glu Gln Asp Ser Lys Asp Ser Thr Tyr Ser
165 170 175

```
Leu Ser Ser Thr Leu Thr Leu Ser Lys Ala Asp Tyr Glu Lys His Lys
            180                 185                 190
Val Tyr Ala Cys Glu Val Thr His Gln Gly Leu Ser Ser Pro Val Thr
        195                 200                 205
Lys Ser Phe Asn Arg Gly Glu Cys
    210                 215
```

<210> 75
<211> 216
<212> PRT
<213> Artificial Sequence

<220>
<223> AC42 VKCK

<400> 75

```
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Arg Ile Ala Arg Thr
            20                  25                  30
Leu Asn Trp Tyr Gln Gln Lys Pro Gly Lys Ala Pro Lys Leu Leu Ile
        35                  40                  45
Tyr Asn Phe Ser Ala Leu Glu Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Val Gly Leu Asp Pro Asn
                85                  90                  95
Leu Thr Thr Phe Gly Gln Gly Thr Lys Val Glu Ile Lys Arg Thr Val
            100                 105                 110
Ala Ala Pro Ser Val Phe Ile Phe Pro Pro Ser Asp Glu Gln Leu Lys
        115                 120                 125
Ser Gly Thr Ala Ser Val Val Cys Leu Leu Asn Asn Phe Tyr Pro Arg
    130                 135                 140
Glu Ala Lys Val Gln Trp Lys Val Asp Asn Ala Leu Gln Ser Gly Asn
145                 150                 155                 160
Ser Gln Glu Ser Val Thr Glu Gln Asp Ser Lys Asp Ser Thr Tyr Ser
                165                 170                 175
Leu Ser Ser Thr Leu Thr Leu Ser Lys Ala Asp Tyr Glu Lys His Lys
            180                 185                 190
Val Tyr Ala Cys Glu Val Thr His Gln Gly Leu Ser Ser Pro Val Thr
        195                 200                 205
Lys Ser Phe Asn Arg Gly Glu Cys
    210                 215
```

<210> 76
<211> 216
<212> PRT
<213> Artificial Sequence

<220>
<223> AC3 VKCK

<400> 76

```
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asn Ile Pro Arg Thr
            20                  25                  30
Ile Asn Trp Tyr Gln Gln Lys Pro Gly Lys Ala Pro Lys Leu Leu Ile
        35                  40                  45
Tyr Asp Ile Ser Asn Leu Glu Thr Gly Val Pro Ser Arg Phe Ser Gly
```

```
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Val Gly Leu Asn Pro Glu
                85                  90                  95
Leu Thr Thr Phe Gly Gln Gly Thr Lys Val Glu Ile Lys Arg Thr Val
            100                 105                 110
Ala Ala Pro Ser Val Phe Ile Phe Pro Pro Ser Asp Glu Gln Leu Lys
        115                 120                 125
Ser Gly Thr Ala Ser Val Val Cys Leu Leu Asn Asn Phe Tyr Pro Arg
    130                 135                 140
Glu Ala Lys Val Gln Trp Lys Val Asp Asn Ala Leu Gln Ser Gly Asn
145                 150                 155                 160
Ser Gln Glu Ser Val Thr Glu Gln Asp Ser Lys Asp Ser Thr Tyr Ser
                165                 170                 175
Leu Ser Ser Thr Leu Thr Leu Ser Lys Ala Asp Tyr Glu Lys His Lys
            180                 185                 190
Val Tyr Ala Cys Glu Val Thr His Gln Gly Leu Ser Ser Pro Val Thr
        195                 200                 205
Lys Ser Phe Asn Arg Gly Glu Cys
    210                 215

<210> 77
<211> 215
<212> PRT
<213> Artificial Sequence


<220>
<223> AC41 VK-H-CL1

<400> 77
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Glu Ile Ala Ala Thr
            20                  25                  30
Leu Asn Trp Tyr Gln Gln Lys Pro Gly Lys Ala Pro Lys Leu Leu Ile
        35                  40                  45
Tyr Asn Tyr Ser Thr Leu Glu Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Val Gly Leu Asn Pro Glu
                85                  90                  95
Asn Thr Thr Phe Gly Gly Gly Thr Lys Leu Thr Val Leu Gly Gln Pro
            100                 105                 110
Lys Ala Ala Pro Ser Val Thr Leu Phe Pro Pro Ser Ser Glu Glu Leu
        115                 120                 125
Gln Ala Asn Lys Ala Thr Leu Val Cys Leu Ile Ser Asp Phe Tyr Pro
    130                 135                 140
Gly Ala Val Thr Val Ala Trp Lys Ala Asp Ser Ser Pro Val Lys Ala
145                 150                 155                 160
Gly Val Glu Thr Thr Thr Pro Ser Lys Gln Ser Asn Asn Lys Tyr Ala
                165                 170                 175
Ala Ser Ser Tyr Leu Ser Leu Thr Pro Glu Gln Trp Lys Ser His Arg
            180                 185                 190
Ser Tyr Ser Cys Gln Val Thr His Glu Gly Ser Thr Val Glu Lys Thr
        195                 200                 205
Val Ala Pro Thr Glu Cys Ser
    210                 215

<210> 78
<211> 215
```

```
<212> PRT
<213> Artificial Sequence


<220>
<223> AC42 VK-H-CL1

<400> 78
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Arg Ile Ala Arg Thr
            20                  25                  30
Leu Asn Trp Tyr Gln Gln Lys Pro Gly Lys Ala Pro Lys Leu Leu Ile
        35                  40                  45
Tyr Asn Phe Ser Ala Leu Glu Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Val Gly Leu Asp Pro Asn
                85                  90                  95
Leu Thr Thr Phe Gly Gly Gly Thr Lys Leu Thr Val Leu Gly Gln Pro
            100                 105                 110
Lys Ala Ala Pro Ser Val Thr Leu Phe Pro Pro Ser Ser Glu Glu Leu
        115                 120                 125
Gln Ala Asn Lys Ala Thr Leu Val Cys Leu Ile Ser Asp Phe Tyr Pro
    130                 135                 140
Gly Ala Val Thr Val Ala Trp Lys Ala Asp Ser Ser Pro Val Lys Ala
145                 150                 155                 160
Gly Val Glu Thr Thr Thr Pro Ser Lys Gln Ser Asn Asn Lys Tyr Ala
                165                 170                 175
Ala Ser Ser Tyr Leu Ser Leu Thr Pro Glu Gln Trp Lys Ser His Arg
            180                 185                 190
Ser Tyr Ser Cys Gln Val Thr His Glu Gly Ser Thr Val Glu Lys Thr
        195                 200                 205
Val Ala Pro Thr Glu Cys Ser
    210                 215

<210> 79
<211> 215
<212> PRT
<213> Artificial Sequence


<220>
<223> AC43 VK-H-CL1

<400> 79
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asn Ile Pro Arg Thr
            20                  25                  30
Ile Asn Trp Tyr Gln Gln Lys Pro Gly Lys Ala Pro Lys Leu Leu Ile
        35                  40                  45
Tyr Asp Ile Ser Asn Leu Glu Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Val Gly Leu Asn Pro Glu
                85                  90                  95
Leu Thr Thr Phe Gly Gly Gly Thr Lys Leu Thr Val Leu Gly Gln Pro
            100                 105                 110
Lys Ala Ala Pro Ser Val Thr Leu Phe Pro Pro Ser Ser Glu Glu Leu
        115                 120                 125
```

```
Gln Ala Asn Lys Ala Thr Leu Val Cys Leu Ile Ser Asp Phe Tyr Pro
    130                 135                 140
Gly Ala Val Thr Val Ala Trp Lys Ala Asp Ser Ser Pro Val Lys Ala
145                 150                 155                 160
Gly Val Glu Thr Thr Thr Pro Ser Lys Gln Ser Asn Asn Lys Tyr Ala
                165                 170                 175
Ala Ser Ser Tyr Leu Ser Leu Thr Pro Glu Gln Trp Lys Ser His Arg
            180                 185                 190
Ser Tyr Ser Cys Gln Val Thr His Glu Gly Ser Thr Val Glu Lys Thr
        195                 200                 205
Val Ala Pro Thr Glu Cys Ser
    210                 215
```

<210> 80
<211> 218
<212> PRT
<213> Artificial Sequence

<220>
<223> AC44 VLCL

<400> 80

```
Gln Ser Val Leu Thr Gln Pro Pro Ser Ala Ser Gly Thr Pro Gly Gln
1               5                   10                  15
Arg Val Thr Ile Ser Cys Ser Gly Ser Ser Ser Asn Ile Val Gly Val
            20                  25                  30
Gly Val Asn Trp Tyr Gln Gln Leu Pro Gly Thr Ala Pro Lys Leu Leu
        35                  40                  45
Ile Tyr Ser Tyr Asn Lys Arg Pro Ser Gly Val Pro Asp Arg Phe Ser
    50                  55                  60
Gly Ser Lys Ser Gly Thr Ser Ala Ser Leu Ala Ile Ser Gly Leu Gln
65                  70                  75                  80
Ser Glu Asp Glu Ala Asp Tyr Tyr Cys Ala Ala Tyr Asp Phe Leu Pro
                85                  90                  95
Val Leu Pro Ala Pro Val Phe Gly Gly Gly Thr Lys Leu Thr Val Leu
            100                 105                 110
Gly Gln Pro Lys Ala Ala Pro Ser Val Thr Leu Phe Pro Pro Ser Ser
        115                 120                 125
Glu Glu Leu Gln Ala Asn Lys Ala Thr Leu Val Cys Leu Ile Ser Asp
    130                 135                 140
Phe Tyr Pro Gly Ala Val Thr Val Ala Trp Lys Ala Asp Ser Ser Pro
145                 150                 155                 160
Val Lys Ala Gly Val Glu Thr Thr Thr Pro Ser Lys Gln Ser Asn Asn
                165                 170                 175
Lys Tyr Ala Ala Ser Ser Tyr Leu Ser Leu Thr Pro Glu Gln Trp Lys
            180                 185                 190
Ser His Arg Ser Tyr Ser Cys Gln Val Thr His Glu Gly Ser Thr Val
        195                 200                 205
Glu Lys Thr Val Ala Pro Thr Glu Cys Ser
    210                 215
```

<210> 81
<211> 218
<212> PRT
<213> Artificial Sequence

<220>
<223> AC45 VLCL

<400> 81

```
Gln Ser Val Leu Thr Gln Pro Pro Ser Ala Ser Gly Thr Pro Gly Gln
```

```
1               5                   10                  15
Arg Val Thr Ile Ser Cys Ser Gly Ser Ser Ser Asn Ile Val Gly Val
            20                  25                  30
Gly Val Asn Trp Tyr Gln Gln Leu Pro Gly Thr Ala Pro Lys Leu Leu
        35                  40                  45
Ile Tyr Ser Tyr Asn Lys Arg Pro Ser Gly Val Pro Asp Arg Phe Ser
    50                  55                  60
Gly Ser Lys Ser Gly Thr Ser Ala Ser Leu Ala Ile Ser Gly Leu Gln
65                  70                  75                  80
Ser Glu Asp Glu Ala Asp Tyr Tyr Cys Ala Ala Tyr Asp Ala Asp Met
                85                  90                  95
Ile Val Pro Ala Pro Val Phe Gly Gly Gly Thr Lys Leu Thr Val Leu
            100                 105                 110
Gly Gln Pro Lys Ala Ala Pro Ser Val Thr Leu Phe Pro Pro Ser Ser
        115                 120                 125
Glu Glu Leu Gln Ala Asn Lys Ala Thr Leu Val Cys Leu Ile Ser Asp
    130                 135                 140
Phe Tyr Pro Gly Ala Val Thr Val Ala Trp Lys Ala Asp Ser Ser Pro
145                 150                 155                 160
Val Lys Ala Gly Val Glu Thr Thr Thr Pro Ser Lys Gln Ser Asn Asn
                165                 170                 175
Lys Tyr Ala Ala Ser Ser Tyr Leu Ser Leu Thr Pro Glu Gln Trp Lys
            180                 185                 190
Ser His Arg Ser Tyr Ser Cys Gln Val Thr His Glu Gly Ser Thr Val
        195                 200                 205
Glu Lys Thr Val Ala Pro Thr Glu Cys Ser
    210                 215
```

<210> 82
<211> 218
<212> PRT
<213> Artificial Sequence

<220>
<223> AC46 VLCL

<400> 82

```
Gln Ser Val Leu Thr Gln Pro Pro Ser Ala Ser Gly Thr Pro Gly Gln
1               5                   10                  15
Arg Val Thr Ile Ser Cys Ser Gly Ser Ser Ser Asn Ile Val Gly Val
            20                  25                  30
Gly Val Asn Trp Tyr Gln Gln Leu Pro Gly Thr Ala Pro Lys Leu Leu
        35                  40                  45
Ile Tyr Ser Tyr Asn Lys Arg Pro Ser Gly Val Pro Asp Arg Phe Ser
    50                  55                  60
Gly Ser Lys Ser Gly Thr Ser Ala Ser Leu Ala Ile Ser Gly Leu Gln
65                  70                  75                  80
Ser Glu Asp Glu Ala Asp Tyr Tyr Cys Ala Ala Tyr Asp Phe Ser Leu
                85                  90                  95
Leu Leu Pro Ala Pro Val Phe Gly Gly Gly Thr Lys Leu Thr Val Leu
            100                 105                 110
Gly Gln Pro Lys Ala Ala Pro Ser Val Thr Leu Phe Pro Pro Ser Ser
        115                 120                 125
Glu Glu Leu Gln Ala Asn Lys Ala Thr Leu Val Cys Leu Ile Ser Asp
    130                 135                 140
Phe Tyr Pro Gly Ala Val Thr Val Ala Trp Lys Ala Asp Ser Ser Pro
145                 150                 155                 160
Val Lys Ala Gly Val Glu Thr Thr Thr Pro Ser Lys Gln Ser Asn Asn
                165                 170                 175
Lys Tyr Ala Ala Ser Ser Tyr Leu Ser Leu Thr Pro Glu Gln Trp Lys
            180                 185                 190
Ser His Arg Ser Tyr Ser Cys Gln Val Thr His Glu Gly Ser Thr Val
```

```
        195                 200                 205
Glu Lys Thr Val Ala Pro Thr Glu Cys Ser
    210                 215
```

<210> 83
<211> 218
<212> PRT
<213> Artificial Sequence

<220>
<223> AC47 VLCL

<400> 83

```
Gln Ser Val Leu Thr Gln Pro Pro Ser Ala Ser Gly Thr Pro Gly Gln
1               5                   10                  15
Arg Val Thr Ile Ser Cys Ser Gly Ser Ser Ser Asn Ile Asn Gly Val
            20                  25                  30
Gly Val Asn Trp Tyr Gln Gln Leu Pro Gly Thr Ala Pro Lys Leu Leu
        35                  40                  45
Ile Tyr Gly Phe Asn Ser Arg Pro Ser Gly Val Pro Asp Arg Phe Ser
    50                  55                  60
Gly Ser Lys Ser Gly Thr Ser Ala Ser Leu Ala Ile Ser Gly Leu Gln
65                  70                  75                  80
Ser Glu Asp Glu Ala Asp Tyr Tyr Cys Ala Ala Tyr Asp Met Ser Thr
                85                  90                  95
Tyr Val Pro Ala Pro Val Phe Gly Gly Gly Thr Lys Leu Thr Val Leu
            100                 105                 110
Gly Gln Pro Lys Ala Ala Pro Ser Val Thr Leu Phe Pro Pro Ser Ser
        115                 120                 125
Glu Glu Leu Gln Ala Asn Lys Ala Thr Leu Val Cys Leu Ile Ser Asp
    130                 135                 140
Phe Tyr Pro Gly Ala Val Thr Val Ala Trp Lys Ala Asp Ser Ser Pro
145                 150                 155                 160
Val Lys Ala Gly Val Glu Thr Thr Thr Pro Ser Lys Gln Ser Asn Asn
                165                 170                 175
Lys Tyr Ala Ala Ser Ser Tyr Leu Ser Leu Thr Pro Glu Gln Trp Lys
            180                 185                 190
Ser His Arg Ser Tyr Ser Cys Gln Val Thr His Glu Gly Ser Thr Val
        195                 200                 205
Glu Lys Thr Val Ala Pro Thr Glu Cys Ser
    210                 215
```

<210> 84
<211> 218
<212> PRT
<213> Artificial Sequence

<220>
<223> AC48 VLCL

<400> 84

```
Gln Ser Val Leu Thr Gln Pro Pro Ser Ala Ser Gly Thr Pro Gly Gln
1               5                   10                  15
Arg Val Thr Ile Ser Cys Ser Gly Ser Ser Ser Asn Ile Asn Gly Val
            20                  25                  30
Gly Val Asn Trp Tyr Gln Gln Leu Pro Gly Thr Ala Pro Lys Leu Leu
        35                  40                  45
Ile Tyr Glu Phe Asn Lys Arg Pro Ser Gly Val Pro Asp Arg Phe Ser
    50                  55                  60
Gly Ser Lys Ser Gly Thr Ser Ala Ser Leu Ala Ile Ser Gly Leu Gln
65                  70                  75                  80
```

Ser Glu Asp Glu Ala Asp Tyr Tyr Cys Ala Ala Tyr Asp Ala Ala Arg
85 90 95
Tyr Val Pro Ala Pro Val Phe Gly Gly Gly Thr Lys Leu Thr Val Leu
100 105 110
Gly Gln Pro Lys Ala Ala Pro Ser Val Thr Leu Phe Pro Pro Ser Ser
115 120 125
Glu Glu Leu Gln Ala Asn Lys Ala Thr Leu Val Cys Leu Ile Ser Asp
130 135 140
Phe Tyr Pro Gly Ala Val Thr Val Ala Trp Lys Ala Asp Ser Ser Pro
145 150 155 160
Val Lys Ala Gly Val Glu Thr Thr Thr Pro Ser Lys Gln Ser Asn Asn
165 170 175
Lys Tyr Ala Ala Ser Ser Tyr Leu Ser Leu Thr Pro Glu Gln Trp Lys
180 185 190
Ser His Arg Ser Tyr Ser Cys Gln Val Thr His Glu Gly Ser Thr Val
195 200 205
Glu Lys Thr Val Ala Pro Thr Glu Cys Ser
210 215

<210> 85
<211> 216
<212> PRT
<213> Artificial Sequence

<220>
<223> AC49 VLCL

<400> 85
Gln Ser Val Leu Thr Gln Pro Pro Ser Val Ser Ala Ala Pro Gly Gln
1 5 10 15
Lys Val Thr Ile Ser Cys Ser Gly Ser Ser Ser Asn Ile Phe Asp Ser
20 25 30
Gly Val Ser Trp Tyr Gln Gln Leu Pro Gly Thr Ala Pro Lys Leu Leu
35 40 45
Ile Tyr Asn His Asn Glu Arg Pro Ser Gly Ile Pro Asp Arg Phe Ser
50 55 60
Gly Ser Lys Ser Gly Thr Ser Ala Thr Leu Gly Ile Thr Gly Leu Gln
65 70 75 80
Thr Gly Asp Glu Ala Asp Tyr Tyr Cys Gly Thr Trp Asp Phe Pro Pro
85 90 95
Ser Arg Phe Val Phe Gly Gly Gly Thr Lys Leu Thr Val Leu Gly Gln
100 105 110
Pro Lys Ala Ala Pro Ser Val Thr Leu Phe Pro Pro Ser Ser Glu Glu
115 120 125
Leu Gln Ala Asn Lys Ala Thr Leu Val Cys Leu Ile Ser Asp Phe Tyr
130 135 140
Pro Gly Ala Val Thr Val Ala Trp Lys Ala Asp Ser Ser Pro Val Lys
145 150 155 160
Ala Gly Val Glu Thr Thr Thr Pro Ser Lys Gln Ser Asn Asn Lys Tyr
165 170 175
Ala Ala Ser Ser Tyr Leu Ser Leu Thr Pro Glu Gln Trp Lys Ser His
180 185 190
Arg Ser Tyr Ser Cys Gln Val Thr His Glu Gly Ser Thr Val Glu Lys
195 200 205
Thr Val Ala Pro Thr Glu Cys Ser
210 215

<210> 86
<211> 219
<212> PRT
<213> Artificial Sequence

<220>
<223> AC50 VLCL

<400> 86
```
Gln Ser Ala Leu Thr Gln Pro Ala Ser Val Ser Gly Ser Pro Gly Gln
1               5                   10                  15
Ser Ile Thr Ile Ser Cys Thr Gly Thr Ser Ser Asp Val Tyr Glu Phe
            20                  25                  30
Arg Leu Val Ser Trp Tyr Gln Gln His Pro Gly Lys Ala Pro Lys Leu
        35                  40                  45
Met Ile Tyr Phe Asp Ser Leu Arg Pro Ser Gly Val Ser Asn Arg Phe
    50                  55                  60
Ser Gly Ser Lys Ser Gly Asn Thr Ala Ser Leu Thr Ile Ser Gly Leu
65                  70                  75                  80
Gln Ala Glu Asp Glu Ala Asp Tyr Tyr Cys Ser Ser Trp Asp Lys Phe
                85                  90                  95
Gly Tyr Ala Phe Asn Ser Val Phe Gly Gly Gly Thr Lys Leu Thr Val
            100                 105                 110
Leu Gly Gln Pro Lys Ala Ala Pro Ser Val Thr Leu Phe Pro Pro Ser
        115                 120                 125
Ser Glu Glu Leu Gln Ala Asn Lys Ala Thr Leu Val Cys Leu Ile Ser
    130                 135                 140
Asp Phe Tyr Pro Gly Ala Val Thr Val Ala Trp Lys Ala Asp Ser Ser
145                 150                 155                 160
Pro Val Lys Ala Gly Val Glu Thr Thr Thr Pro Ser Lys Gln Ser Asn
                165                 170                 175
Asn Lys Tyr Ala Ala Ser Ser Tyr Leu Ser Leu Thr Pro Glu Gln Trp
            180                 185                 190
Lys Ser His Arg Ser Tyr Ser Cys Gln Val Thr His Glu Gly Ser Thr
        195                 200                 205
Val Glu Lys Thr Val Ala Pro Thr Glu Cys Ser
    210                 215
```

<210> 87
<211> 219
<212> PRT
<213> Artificial Sequence

<220>
<223> AC52 VLCL

<400> 87
```
Gln Ser Val Leu Thr Gln Pro Pro Ser Val Ser Gly Ala Pro Gly Gln
1               5                   10                  15
Arg Val Thr Ile Ser Cys Thr Gly Ser Ser Ser Asn Ile His Asp Val
            20                  25                  30
Gly Tyr Val His Trp Tyr Gln Gln Leu Pro Gly Thr Ala Pro Lys Leu
        35                  40                  45
Leu Ile Tyr Gly Ala Ser Gly Arg Pro Ser Gly Val Pro Asp Arg Phe
    50                  55                  60
Ser Gly Ser Lys Ser Gly Thr Ser Ala Ser Leu Ala Ile Thr Gly Leu
65                  70                  75                  80
Gln Ala Glu Asp Glu Ala Asp Tyr Tyr Cys Gln Ser Tyr Asp Thr Lys
                85                  90                  95
Arg Glu Phe Pro Thr Pro Val Phe Gly Gly Gly Thr Lys Leu Thr Val
            100                 105                 110
Leu Gly Gln Pro Lys Ala Ala Pro Ser Val Thr Leu Phe Pro Pro Ser
        115                 120                 125
Ser Glu Glu Leu Gln Ala Asn Lys Ala Thr Leu Val Cys Leu Ile Ser
    130                 135                 140
Asp Phe Tyr Pro Gly Ala Val Thr Val Ala Trp Lys Ala Asp Ser Ser
```

```
145                 150                 155                 160
Pro Val Lys Ala Gly Val Glu Thr Thr Thr Pro Ser Lys Gln Ser Asn
                165                 170                 175
Asn Lys Tyr Ala Ala Ser Ser Tyr Leu Ser Leu Thr Pro Glu Gln Trp
            180                 185                 190
Lys Ser His Arg Ser Tyr Ser Cys Gln Val Thr His Glu Gly Ser Thr
        195                 200                 205
Val Glu Lys Thr Val Ala Pro Thr Glu Cys Ser
    210                 215

<210> 88
<211> 219
<212> PRT
<213> Artificial Sequence


<220>
<223> AC53 VLCL

<400> 88
Gln Ser Val Leu Thr Gln Pro Pro Ser Val Ser Gly Ala Pro Gly Gln
1               5                   10                  15
Arg Val Thr Ile Ser Cys Thr Gly Ser Ser Ser Asn Ile His Asp Val
            20                  25                  30
Gly Tyr Val His Trp Tyr Gln Gln Leu Pro Gly Thr Ala Pro Lys Leu
        35                  40                  45
Leu Ile Tyr Gly Ala Ser Gly Arg Pro Ser Gly Val Pro Asp Arg Phe
    50                  55                  60
Ser Gly Ser Lys Ser Gly Thr Ser Ala Ser Leu Ala Ile Thr Gly Leu
65                  70                  75                  80
Gln Ala Glu Asp Glu Ala Asp Tyr Tyr Cys Gln Ser Tyr Asp Gly Arg
                85                  90                  95
Ala Val Phe Pro Thr Pro Val Phe Gly Gly Gly Thr Lys Leu Thr Val
            100                 105                 110
Leu Gly Gln Pro Lys Ala Ala Pro Ser Val Thr Leu Phe Pro Pro Ser
        115                 120                 125
Ser Glu Glu Leu Gln Ala Asn Lys Ala Thr Leu Val Cys Leu Ile Ser
    130                 135                 140
Asp Phe Tyr Pro Gly Ala Val Thr Val Ala Trp Lys Ala Asp Ser Ser
145                 150                 155                 160
Pro Val Lys Ala Gly Val Glu Thr Thr Thr Pro Ser Lys Gln Ser Asn
                165                 170                 175
Asn Lys Tyr Ala Ala Ser Ser Tyr Leu Ser Leu Thr Pro Glu Gln Trp
            180                 185                 190
Lys Ser His Arg Ser Tyr Ser Cys Gln Val Thr His Glu Gly Ser Thr
        195                 200                 205
Val Glu Lys Thr Val Ala Pro Thr Glu Cys Ser
    210                 215

<210> 89
<211> 219
<212> PRT
<213> Artificial Sequence


<220>
<223> AC54

<400> 89
Gln Ser Val Leu Thr Gln Pro Pro Ser Val Ser Gly Ala Pro Gly Gln
1               5                   10                  15
Arg Val Thr Ile Ser Cys Thr Gly Ser Ser Ser Asn Ile His Asp Val
            20                  25                  30
```

Gly Tyr Val His Trp Tyr Gln Gln Leu Pro Gly Thr Ala Pro Lys Leu
35 40 45
Leu Ile Tyr Gly Ala Ser Gly Arg Pro Ser Gly Val Pro Asp Arg Phe
50 55 60
Ser Gly Ser Lys Ser Gly Thr Ser Ala Ser Leu Ala Ile Thr Gly Leu
65 70 75 80
Gln Ala Glu Asp Glu Ala Asp Tyr Tyr Cys Gln Ser Tyr Asp Thr Arg
85 90 95
Gln Val Val Pro Thr Pro Val Phe Gly Gly Gly Thr Lys Leu Thr Val
100 105 110
Leu Gly Gln Pro Lys Ala Ala Pro Ser Val Thr Leu Phe Pro Pro Ser
115 120 125
Ser Glu Glu Leu Gln Ala Asn Lys Ala Thr Leu Val Cys Leu Ile Ser
130 135 140
Asp Phe Tyr Pro Gly Ala Val Thr Val Ala Trp Lys Ala Asp Ser Ser
145 150 155 160
Pro Val Lys Ala Gly Val Glu Thr Thr Thr Pro Ser Lys Gln Ser Asn
165 170 175
Asn Lys Tyr Ala Ala Ser Ser Tyr Leu Ser Leu Thr Pro Glu Gln Trp
180 185 190
Lys Ser His Arg Ser Tyr Ser Cys Gln Val Thr His Glu Gly Ser Thr
195 200 205
Val Glu Lys Thr Val Ala Pro Thr Glu Cys Ser
210 215

<210> 90
<211> 219
<212> PRT
<213> Artificial Sequence

<220>
<223> AC55 VLCL

<400> 90
Gln Ser Val Leu Thr Gln Pro Pro Ser Val Ser Gly Ala Pro Gly Gln
1 5 10 15
Arg Val Thr Ile Ser Cys Thr Gly Ser Ser Ser Asn Ile His Asp Val
20 25 30
Gly Tyr Val His Trp Tyr Gln Gln Leu Pro Gly Thr Ala Pro Lys Leu
35 40 45
Leu Ile Tyr Gly Ala Ser Gly Arg Pro Ser Gly Val Pro Asp Arg Phe
50 55 60
Ser Gly Ser Lys Ser Gly Thr Ser Ala Ser Leu Ala Ile Thr Gly Leu
65 70 75 80
Gln Ala Glu Asp Glu Ala Asp Tyr Tyr Cys Gln Ser Tyr Asp Thr Ser
85 90 95
Ala Val Phe Pro Thr Pro Val Phe Gly Gly Gly Thr Lys Leu Thr Val
100 105 110
Leu Gly Gln Pro Lys Ala Ala Pro Ser Val Thr Leu Phe Pro Pro Ser
115 120 125
Ser Glu Glu Leu Gln Ala Asn Lys Ala Thr Leu Val Cys Leu Ile Ser
130 135 140
Asp Phe Tyr Pro Gly Ala Val Thr Val Ala Trp Lys Ala Asp Ser Ser
145 150 155 160
Pro Val Lys Ala Gly Val Glu Thr Thr Thr Pro Ser Lys Gln Ser Asn
165 170 175
Asn Lys Tyr Ala Ala Ser Ser Tyr Leu Ser Leu Thr Pro Glu Gln Trp
180 185 190
Lys Ser His Arg Ser Tyr Ser Cys Gln Val Thr His Glu Gly Ser Thr
195 200 205
Val Glu Lys Thr Val Ala Pro Thr Glu Cys Ser
210 215

<210> 91
<211> 219
<212> PRT
<213> Artificial Sequence

<220>
<223> AC56 VLCL

<400> 91
Gln Ser Val Leu Thr Gln Pro Pro Ser Val Ser Gly Ala Pro Gly Gln
1               5                   10                  15
Arg Val Thr Ile Ser Cys Thr Gly Ser Ser Ser Asn Ile Ile Asn Asn
            20                  25                  30
Ser Tyr Val His Trp Tyr Gln Gln Leu Pro Gly Thr Ala Pro Lys Leu
        35                  40                  45
Leu Ile Tyr Ala Pro Ser Asn Arg Pro Ser Gly Val Pro Asp Arg Phe
    50                  55                  60
Ser Gly Ser Lys Ser Gly Thr Ser Ala Ser Leu Ala Ile Thr Gly Leu
65                  70                  75                  80
Gln Ala Glu Asp Glu Ala Asp Tyr Tyr Cys Gln Ser Tyr Asp Thr Thr
                85                  90                  95
Gly Val Met Pro Thr Pro Val Phe Gly Gly Gly Thr Lys Leu Thr Val
            100                 105                 110
Leu Gly Gln Pro Lys Ala Ala Pro Ser Val Thr Leu Phe Pro Pro Ser
        115                 120                 125
Ser Glu Glu Leu Gln Ala Asn Lys Ala Thr Leu Val Cys Leu Ile Ser
    130                 135                 140
Asp Phe Tyr Pro Gly Ala Val Thr Val Ala Trp Lys Ala Asp Ser Ser
145                 150                 155                 160
Pro Val Lys Ala Gly Val Glu Thr Thr Thr Pro Ser Lys Gln Ser Asn
                165                 170                 175
Asn Lys Tyr Ala Ala Ser Ser Tyr Leu Ser Leu Thr Pro Glu Gln Trp
            180                 185                 190
Lys Ser His Arg Ser Tyr Ser Cys Gln Val Thr His Glu Gly Ser Thr
        195                 200                 205
Val Glu Lys Thr Val Ala Pro Thr Glu Cys Ser
    210                 215

<210> 92
<211> 219
<212> PRT
<213> Artificial Sequence

<220>
<223> AC57 VLCL

<400> 92
Gln Ser Val Leu Thr Gln Pro Pro Ser Val Ser Gly Ala Pro Gly Gln
1               5                   10                  15
Arg Val Thr Ile Ser Cys Thr Gly Ser Ser Ser Asn Ile Ile Asn Asn
            20                  25                  30
Ser Tyr Val His Trp Tyr Gln Gln Leu Pro Gly Thr Ala Pro Lys Leu
        35                  40                  45
Leu Ile Tyr Ala Pro Ser Asn Arg Pro Ser Gly Val Pro Asp Arg Phe
    50                  55                  60
Ser Gly Ser Lys Ser Gly Thr Ser Ala Ser Leu Ala Ile Thr Gly Leu
65                  70                  75                  80
Gln Ala Glu Asp Glu Ala Asp Tyr Tyr Cys Gln Ser Tyr Asp Thr Arg
                85                  90                  95
Phe Val Val Pro Thr Pro Val Phe Gly Gly Gly Thr Lys Leu Thr Val

```
            100                 105                 110
Leu Gly Gln Pro Lys Ala Ala Pro Ser Val Thr Leu Phe Pro Pro Ser
        115                 120                 125
Ser Glu Glu Leu Gln Ala Asn Lys Ala Thr Leu Val Cys Leu Ile Ser
    130                 135                 140
Asp Phe Tyr Pro Gly Ala Val Thr Val Ala Trp Lys Ala Asp Ser Ser
145                 150                 155                 160
Pro Val Lys Ala Gly Val Glu Thr Thr Thr Pro Ser Lys Gln Ser Asn
                165                 170                 175
Asn Lys Tyr Ala Ala Ser Ser Tyr Leu Ser Leu Thr Pro Glu Gln Trp
            180                 185                 190
Lys Ser His Arg Ser Tyr Ser Cys Gln Val Thr His Glu Gly Ser Thr
        195                 200                 205
Val Glu Lys Thr Val Ala Pro Thr Glu Cys Ser
    210                 215

<210> 93
<211> 219
<212> PRT
<213> Artificial Sequence


<220>
<223> AC58 VLCL

<400> 93
Gln Ser Val Leu Thr Gln Pro Pro Ser Val Ser Gly Ala Pro Gly Gln
1               5                   10                  15
Arg Val Thr Ile Ser Cys Thr Gly Ser Ser Ser Asn Ile Gly Val Ala
            20                  25                  30
His Asp Val His Trp Tyr Gln Gln Leu Pro Gly Thr Ala Pro Lys Leu
        35                  40                  45
Leu Ile Tyr Asp Tyr Ser Glu Arg Pro Ser Gly Val Pro Asp Arg Phe
    50                  55                  60
Ser Gly Ser Lys Ser Gly Thr Ser Ala Ser Leu Ala Ile Thr Gly Leu
65                  70                  75                  80
Gln Ala Glu Asp Glu Ala Asp Tyr Tyr Cys Gln Ser Trp Asp Asn Gly
                85                  90                  95
Pro Asn Gly Leu Val Trp Val Phe Gly Gly Gly Thr Lys Leu Thr Val
            100                 105                 110
Leu Gly Gln Pro Lys Ala Ala Pro Ser Val Thr Leu Phe Pro Pro Ser
        115                 120                 125
Ser Glu Glu Leu Gln Ala Asn Lys Ala Thr Leu Val Cys Leu Ile Ser
    130                 135                 140
Asp Phe Tyr Pro Gly Ala Val Thr Val Ala Trp Lys Ala Asp Ser Ser
145                 150                 155                 160
Pro Val Lys Ala Gly Val Glu Thr Thr Thr Pro Ser Lys Gln Ser Asn
                165                 170                 175
Asn Lys Tyr Ala Ala Ser Ser Tyr Leu Ser Leu Thr Pro Glu Gln Trp
            180                 185                 190
Lys Ser His Arg Ser Tyr Ser Cys Gln Val Thr His Glu Gly Ser Thr
        195                 200                 205
Val Glu Lys Thr Val Ala Pro Thr Glu Cys Ser
    210                 215

<210> 94
<211> 219
<212> PRT
<213> Artificial Sequence


<220>
<223> AC59 VLCL
```

<400> 94
Gln Ser Val Leu Thr Gln Pro Pro Ser Val Ser Gly Ala Pro Gly Gln
1 5 10 15
Arg Val Thr Ile Ser Cys Thr Gly Ser Ser Ser Asn Ile Gly Val Ala
20 25 30
His Asp Val His Trp Tyr Gln Gln Leu Pro Gly Thr Ala Pro Lys Leu
35 40 45
Leu Ile Tyr Asp Tyr Ser Glu Arg Pro Ser Gly Val Pro Asp Arg Phe
50 55 60
Ser Gly Ser Lys Ser Gly Thr Ser Ala Ser Leu Ala Ile Thr Gly Leu
65 70 75 80
Gln Ala Glu Asp Glu Ala Asp Tyr Tyr Cys Gln Ser Trp Asp Asn Ser
85 90 95
Glu Asn Gly Leu Ile Tyr Val Phe Gly Gly Gly Thr Lys Leu Thr Val
100 105 110
Leu Gly Gln Pro Lys Ala Ala Pro Ser Val Thr Leu Phe Pro Pro Ser
115 120 125
Ser Glu Glu Leu Gln Ala Asn Lys Ala Thr Leu Val Cys Leu Ile Ser
130 135 140
Asp Phe Tyr Pro Gly Ala Val Thr Val Ala Trp Lys Ala Asp Ser Ser
145 150 155 160
Pro Val Lys Ala Gly Val Glu Thr Thr Thr Pro Ser Lys Gln Ser Asn
165 170 175
Asn Lys Tyr Ala Ala Ser Ser Tyr Leu Ser Leu Thr Pro Glu Gln Trp
180 185 190
Lys Ser His Arg Ser Tyr Ser Cys Gln Val Thr His Glu Gly Ser Thr
195 200 205
Val Glu Lys Thr Val Ala Pro Thr Glu Cys Ser
210 215

<210> 95
<211> 216
<212> PRT
<213> Artificial Sequence

<220>
<223> AK60 VKCK

<400> 95
Asp Ile Gln Met Thr Gln Ser Pro Ser Ser Leu Ser Ala Ser Val Gly
1 5 10 15
Asp Arg Val Thr Ile Thr Cys Gln Ala Ser Gln Asn Ile Pro Arg Thr
20 25 30
Ile Asn Trp Tyr Gln Gln Lys Pro Gly Lys Ala Pro Lys Leu Leu Ile
35 40 45
Tyr Asp Ile Ser Asn Leu Glu Thr Gly Val Pro Ser Arg Phe Ser Gly
50 55 60
Ser Gly Ser Gly Thr Asp Phe Thr Phe Thr Ile Ser Ser Leu Gln Pro
65 70 75 80
Glu Asp Ile Ala Thr Tyr Tyr Cys Gln Gln Val Gly Leu Asn Trp Asp
85 90 95
Asp Arg Ala Phe Gly Gln Gly Thr Lys Val Glu Ile Lys Arg Thr Val
100 105 110
Ala Ala Pro Ser Val Phe Ile Phe Pro Pro Ser Asp Glu Gln Leu Lys
115 120 125
Ser Gly Thr Ala Ser Val Val Cys Leu Leu Asn Asn Phe Tyr Pro Arg
130 135 140
Glu Ala Lys Val Gln Trp Lys Val Asp Asn Ala Leu Gln Ser Gly Asn
145 150 155 160
Ser Gln Glu Ser Val Thr Glu Gln Asp Ser Lys Asp Ser Thr Tyr Ser
165 170 175

```
Leu Ser Ser Thr Leu Thr Leu Ser Lys Ala Asp Tyr Glu Lys His Lys
            180                 185                 190
Val Tyr Ala Cys Glu Val Thr His Gln Gly Leu Ser Ser Pro Val Thr
        195                 200                 205
Lys Ser Phe Asn Arg Gly Glu Cys
    210                 215
```

<210> 96
<211> 702
<212> PRT
<213> Homo sapiens

<220>
<223> human CEACAM5

<400> 96

```
Met Glu Ser Pro Ser Ala Pro Pro His Arg Trp Cys Ile Pro Trp Gln
1               5                   10                  15
Arg Leu Leu Leu Thr Ala Ser Leu Leu Thr Phe Trp Asn Pro Pro Thr
            20                  25                  30
Thr Ala Lys Leu Thr Ile Glu Ser Thr Pro Phe Asn Val Ala Glu Gly
        35                  40                  45
Lys Glu Val Leu Leu Leu Val His Asn Leu Pro Gln His Leu Phe Gly
    50                  55                  60
Tyr Ser Trp Tyr Lys Gly Glu Arg Val Asp Gly Asn Arg Gln Ile Ile
65                  70                  75                  80
Gly Tyr Val Ile Gly Thr Gln Gln Ala Thr Pro Gly Pro Ala Tyr Ser
                85                  90                  95
Gly Arg Glu Ile Ile Tyr Pro Asn Ala Ser Leu Leu Ile Gln Asn Ile
            100                 105                 110
Ile Gln Asn Asp Thr Gly Phe Tyr Thr Leu His Val Ile Lys Ser Asp
        115                 120                 125
Leu Val Asn Glu Glu Ala Thr Gly Gln Phe Arg Val Tyr Pro Glu Leu
    130                 135                 140
Pro Lys Pro Ser Ile Ser Ser Asn Asn Ser Lys Pro Val Glu Asp Lys
145                 150                 155                 160
Asp Ala Val Ala Phe Thr Cys Glu Pro Glu Thr Gln Asp Ala Thr Tyr
                165                 170                 175
Leu Trp Trp Val Asn Asn Gln Ser Leu Pro Val Ser Pro Arg Leu Gln
            180                 185                 190
Leu Ser Asn Gly Asn Arg Thr Leu Thr Leu Phe Asn Val Thr Arg Asn
        195                 200                 205
Asp Thr Ala Ser Tyr Lys Cys Glu Thr Gln Asn Pro Val Ser Ala Arg
    210                 215                 220
Arg Ser Asp Ser Val Ile Leu Asn Val Leu Tyr Gly Pro Asp Ala Pro
225                 230                 235                 240
Thr Ile Ser Pro Leu Asn Thr Ser Tyr Arg Ser Gly Glu Asn Leu Asn
                245                 250                 255
Leu Ser Cys His Ala Ala Ser Asn Pro Pro Ala Gln Tyr Ser Trp Phe
            260                 265                 270
Val Asn Gly Thr Phe Gln Gln Ser Thr Gln Glu Leu Phe Ile Pro Asn
        275                 280                 285
Ile Thr Val Asn Asn Ser Gly Ser Tyr Thr Cys Gln Ala His Asn Ser
    290                 295                 300
Asp Thr Gly Leu Asn Arg Thr Thr Val Thr Thr Ile Thr Val Tyr Ala
305                 310                 315                 320
Glu Pro Pro Lys Pro Phe Ile Thr Ser Asn Asn Ser Asn Pro Val Glu
                325                 330                 335
Asp Glu Asp Ala Val Ala Leu Thr Cys Glu Pro Glu Ile Gln Asn Thr
            340                 345                 350
Thr Tyr Leu Trp Trp Val Asn Asn Gln Ser Leu Pro Val Ser Pro Arg
        355                 360                 365
```

```
Leu Gln Leu Ser Asn Asp Asn Arg Thr Leu Thr Leu Leu Ser Val Thr
    370                 375                 380
Arg Asn Asp Val Gly Pro Tyr Glu Cys Gly Ile Gln Asn Lys Leu Ser
385                 390                 395                 400
Val Asp His Ser Asp Pro Val Ile Leu Asn Val Leu Tyr Gly Pro Asp
                405                 410                 415
Asp Pro Thr Ile Ser Pro Ser Tyr Thr Tyr Tyr Arg Pro Gly Val Asn
            420                 425                 430
Leu Ser Leu Ser Cys His Ala Ala Ser Asn Pro Pro Ala Gln Tyr Ser
        435                 440                 445
Trp Leu Ile Asp Gly Asn Ile Gln Gln His Thr Gln Glu Leu Phe Ile
    450                 455                 460
Ser Asn Ile Thr Glu Lys Asn Ser Gly Leu Tyr Thr Cys Gln Ala Asn
465                 470                 475                 480
Asn Ser Ala Ser Gly His Ser Arg Thr Thr Val Lys Thr Ile Thr Val
                485                 490                 495
Ser Ala Glu Leu Pro Lys Pro Ser Ile Ser Ser Asn Asn Ser Lys Pro
            500                 505                 510
Val Glu Asp Lys Asp Ala Val Ala Phe Thr Cys Glu Pro Glu Ala Gln
        515                 520                 525
Asn Thr Thr Tyr Leu Trp Trp Val Asn Gly Gln Ser Leu Pro Val Ser
    530                 535                 540
Pro Arg Leu Gln Leu Ser Asn Gly Asn Arg Thr Leu Thr Leu Phe Asn
545                 550                 555                 560
Val Thr Arg Asn Asp Ala Arg Ala Tyr Val Cys Gly Ile Gln Asn Ser
                565                 570                 575
Val Ser Ala Asn Arg Ser Asp Pro Val Thr Leu Asp Val Leu Tyr Gly
            580                 585                 590
Pro Asp Thr Pro Ile Ile Ser Pro Pro Asp Ser Ser Tyr Leu Ser Gly
        595                 600                 605
Ala Asn Leu Asn Leu Ser Cys His Ser Ala Ser Asn Pro Ser Pro Gln
    610                 615                 620
Tyr Ser Trp Arg Ile Asn Gly Ile Pro Gln Gln His Thr Gln Val Leu
625                 630                 635                 640
Phe Ile Ala Lys Ile Thr Pro Asn Asn Asn Gly Thr Tyr Ala Cys Phe
                645                 650                 655
Val Ser Asn Leu Ala Thr Gly Arg Asn Asn Ser Ile Val Lys Ser Ile
            660                 665                 670
Thr Val Ser Ala Ser Gly Thr Ser Pro Gly Leu Ser Ala Gly Ala Thr
        675                 680                 685
Val Gly Ile Met Ile Gly Val Leu Val Gly Val Ala Leu Ile
    690                 695                 700
```

<210> 97
<211> 702
<212> PRT
<213> Artificial Sequence

<220>
<223> human CEACAM5

<400> 97

```
Met Glu Ser Pro Ser Ala Pro Pro His Arg Trp Cys Ile Pro Trp Gln
1               5                   10                  15
Arg Leu Leu Leu Thr Ala Ser Leu Leu Thr Phe Trp Asn Pro Pro Thr
            20                  25                  30
Thr Ala Lys Leu Thr Ile Glu Ser Thr Pro Phe Asn Val Ala Glu Gly
        35                  40                  45
Lys Glu Val Leu Leu Leu Val His Asn Leu Pro Gln His Leu Phe Gly
    50                  55                  60
Tyr Ser Trp Tyr Lys Gly Glu Arg Val Asp Gly Asn Arg Gln Ile Ile
65                  70                  75                  80
```

```
Gly Tyr Val Ile Gly Thr Gln Gln Ala Thr Pro Gly Pro Ala Tyr Ser
                85                  90                  95
Gly Arg Glu Ile Ile Tyr Pro Asn Ala Ser Leu Leu Ile Gln Asn Ile
            100                 105                 110
Ile Gln Asn Asp Thr Gly Phe Tyr Thr Leu His Val Ile Lys Ser Asp
        115                 120                 125
Leu Val Asn Glu Glu Ala Thr Gly Gln Phe Arg Val Tyr Pro Glu Leu
    130                 135                 140
Pro Lys Pro Ser Ile Ser Ser Asn Asn Ser Lys Pro Val Glu Asp Lys
145                 150                 155                 160
Asp Ala Val Ala Phe Thr Cys Glu Pro Glu Thr Gln Asp Ala Thr Tyr
                165                 170                 175
Leu Trp Trp Val Asn Asn Gln Ser Leu Pro Val Ser Pro Arg Leu Gln
            180                 185                 190
Leu Ser Asn Gly Asn Arg Thr Leu Thr Leu Phe Asn Val Thr Arg Asn
        195                 200                 205
Asp Thr Ala Ser Tyr Lys Cys Glu Thr Gln Asn Pro Val Ser Ala Arg
    210                 215                 220
Arg Ser Asp Ser Val Ile Leu Asn Val Leu Tyr Gly Pro Asp Ala Pro
225                 230                 235                 240
Thr Ile Ser Pro Leu Asn Thr Ser Tyr Arg Ser Gly Glu Asn Leu Asn
                245                 250                 255
Leu Ser Cys His Ala Ala Ser Asn Pro Pro Ala Gln Tyr Ser Trp Phe
            260                 265                 270
Val Asn Gly Thr Phe Gln Gln Ser Thr Gln Glu Leu Phe Ile Pro Asn
        275                 280                 285
Ile Thr Val Asn Asn Ser Gly Ser Tyr Thr Cys Gln Ala His Asn Ser
    290                 295                 300
Asp Thr Gly Leu Asn Arg Thr Thr Val Thr Thr Ile Thr Val Tyr Ala
305                 310                 315                 320
Glu Pro Pro Lys Pro Phe Ile Thr Ser Asn Asn Ser Asn Pro Val Glu
                325                 330                 335
Asp Glu Asp Ala Val Ala Leu Thr Cys Glu Pro Glu Ile Gln Asn Thr
            340                 345                 350
Thr Tyr Leu Trp Trp Val Asn Asn Gln Ser Leu Pro Val Ser Pro Arg
        355                 360                 365
Leu Gln Leu Ser Asn Asp Asn Arg Thr Leu Thr Leu Leu Ser Val Thr
    370                 375                 380
Arg Asn Asp Val Gly Pro Tyr Glu Cys Gly Ile Gln Asn Lys Leu Ser
385                 390                 395                 400
Val Asp His Ser Asp Pro Val Ile Leu Asn Val Leu Tyr Gly Pro Asp
                405                 410                 415
Asp Pro Thr Ile Ser Pro Ser Tyr Thr Tyr Tyr Arg Pro Gly Val Asn
            420                 425                 430
Leu Ser Leu Ser Cys His Ala Ala Ser Asn Pro Pro Ala Gln Tyr Ser
        435                 440                 445
Trp Leu Ile Asp Gly Asn Ile Gln Gln His Thr Gln Glu Leu Phe Ile
    450                 455                 460
Ser Asn Ile Thr Glu Lys Asn Ser Gly Leu Tyr Thr Cys Gln Ala Asn
465                 470                 475                 480
Asn Ser Ala Ser Gly His Ser Arg Thr Thr Val Lys Thr Ile Thr Val
                485                 490                 495
Ser Ala Glu Leu Pro Lys Pro Ser Ile Ser Ser Asn Asn Ser Lys Pro
            500                 505                 510
Val Glu Asp Lys Asp Ala Val Ala Phe Thr Cys Glu Pro Glu Ala Gln
        515                 520                 525
Asn Thr Thr Tyr Leu Trp Trp Val Asn Gly Gln Ser Leu Pro Val Ser
    530                 535                 540
Pro Arg Leu Gln Leu Ser Asn Gly Asn Arg Thr Leu Thr Leu Phe Asn
545                 550                 555                 560
Val Thr Arg Asn Asp Ala Arg Ala Tyr Val Cys Gly Ile Gln Asn Ser
                565                 570                 575
Val Ser Ala Asn Arg Ser Asp Pro Val Thr Leu Asp Val Leu Tyr Gly
```

```
            580                 585                 590
Pro Asp Thr Pro Ile Ile Ser Pro Pro Asp Ser Ser Tyr Leu Ser Gly
        595                 600                 605
Ala Asn Leu Asn Leu Ser Cys His Ser Ala Ser Asn Pro Ser Pro Gln
    610                 615                 620
Tyr Ser Trp Arg Ile Asn Gly Ile Pro Gln Gln His Thr Gln Val Leu
625                 630                 635                 640
Phe Ile Ala Lys Ile Thr Pro Asn Asn Asn Gly Thr Tyr Ala Cys Phe
                645                 650                 655
Val Ser Asn Leu Ala Thr Gly Arg Asn Asn Ser Ile Val Lys Ser Ile
            660                 665                 670
Thr Val Ser Ala Ser Gly Thr Ser Pro Gly Leu Ser Ala Gly Ala Thr
        675                 680                 685
Val Gly Ile Met Ile Gly Val Leu Val Gly Val Ala Leu Ile
    690                 695                 700
```

<210> 98
<211> 121
<212> PRT
<213> Artificial Sequence

<220>
<223> MAB CEA1 VH SEQ31/36/37 of WO2017055389

<400> 98

```
Gln Val Gln Leu Val Gln Ser Gly Ala Glu Val Lys Lys Pro Gly Ser
1               5                   10                  15
Ser Val Lys Val Ser Cys Lys Ala Ser Gly Phe Asn Ile Lys Asp Thr
            20                  25                  30
Tyr Met His Trp Val Arg Gln Ala Pro Gly Gln Gly Leu Glu Trp Met
        35                  40                  45
Gly Arg Ile Asp Pro Ala Asn Gly Asn Ser Lys Tyr Val Pro Lys Phe
    50                  55                  60
Gln Gly Arg Val Thr Ile Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Pro Phe Gly Tyr Tyr Val Ser Asp Tyr Ala Met Ala Tyr Trp Gly
            100                 105                 110
Gln Gly Thr Leu Val Thr Val Ser Ser
        115                 120
```

<210> 99
<211> 111
<212> PRT
<213> Artificial Sequence

<220>
<223> MAB CEA1 VL SEQ32/38 of WO2017055389

<400> 99

```
Glu Ile Val Leu Thr Gln Ser Pro Ala Thr Leu Ser Leu Ser Pro Gly
1               5                   10                  15
Glu Arg Ala Thr Leu Ser Cys Arg Ala Gly Glu Ser Val Asp Ile Phe
            20                  25                  30
Gly Val Gly Phe Leu His Trp Tyr Gln Gln Lys Pro Gly Gln Ala Pro
        35                  40                  45
Arg Leu Leu Ile Tyr Arg Ala Ser Asn Arg Ala Thr Gly Ile Pro Ala
    50                  55                  60
Arg Phe Ser Gly Ser Gly Ser Gly Thr Asp Phe Thr Leu Thr Ile Ser
65                  70                  75                  80
```

Ser Leu Glu Pro Glu Asp Phe Ala Val Tyr Tyr Cys Gln Gln Thr Asn
85 90 95
Glu Asp Pro Tyr Thr Phe Gly Gln Gly Thr Lys Leu Glu Ile Lys
100 105 110

<210> 100
<211> 125
<212> PRT
<213> Artificial Sequence

<220>
<223> CD3 VH of WO2017055389, SEQ34

<400> 100
Glu Val Gln Leu Leu Glu Ser Gly Gly Gly Leu Val Gln Pro Gly Gly
1 5 10 15
Ser Leu Arg Leu Ser Cys Ala Ala Ser Gly Phe Thr Phe Ser Thr Tyr
20 25 30
Ala Met Asn Trp Val Arg Gln Ala Pro Gly Lys Gly Leu Glu Trp Val
35 40 45
Ser Arg Ile Arg Ser Lys Tyr Asn Asn Tyr Ala Thr Tyr Tyr Ala Asp
50 55 60
Ser Val Lys Gly Arg Phe Thr Ile Ser Arg Asp Asp Ser Lys Asn Thr
65 70 75 80
Leu Tyr Leu Gln Met Asn Ser Leu Arg Ala Glu Asp Thr Ala Val Tyr
85 90 95
Tyr Cys Val Arg His Gly Asn Phe Gly Asn Ser Tyr Val Ser Trp Phe
100 105 110
Ala Tyr Trp Gly Gln Gly Thr Leu Val Thr Val Ser Ser
115 120 125

<210> 101
<211> 109
<212> PRT
<213> Artificial Sequence

<220>
<223> CD3 VL part of WO2017055389; SEQ 34/37

<400> 101
Gln Ala Val Val Thr Gln Glu Pro Ser Leu Thr Val Ser Pro Gly Gly
1 5 10 15
Thr Val Thr Leu Thr Cys Gly Ser Ser Thr Gly Ala Val Thr Thr Ser
20 25 30
Asn Tyr Ala Asn Trp Val Gln Glu Lys Pro Gly Gln Ala Phe Arg Gly
35 40 45
Leu Ile Gly Gly Thr Asn Lys Arg Ala Pro Gly Thr Pro Ala Arg Phe
50 55 60
Ser Gly Ser Leu Leu Gly Gly Lys Ala Ala Leu Thr Leu Ser Gly Ala
65 70 75 80
Gln Pro Glu Asp Glu Ala Glu Tyr Tyr Cys Ala Leu Trp Tyr Ser Asn
85 90 95
Leu Trp Val Phe Gly Gly Gly Thr Lys Leu Thr Val Leu
100 105

<210> 102
<211> 232
<212> PRT
<213> Artificial Sequence

<220>
<223> CD3 VH-CL (CK)

<400> 102
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser Thr Tyr
            20                  25                  30
Ala Met Asn Trp Val Arg Gln Ala Pro Gly Lys Gly Leu Glu Trp Val
        35                  40                  45
Ser Arg Ile Arg Ser Lys Tyr Asn Asn Tyr Ala Thr Tyr Tyr Ala Asp
    50                  55                  60
Ser Val Lys Gly Arg Phe Thr Ile Ser Arg Asp Asp Ser Lys Asn Thr
65                  70                  75                  80
Leu Tyr Leu Gln Met Asn Ser Leu Arg Ala Glu Asp Thr Ala Val Tyr
                85                  90                  95
Tyr Cys Val Arg His Gly Asn Phe Gly Asn Ser Tyr Val Ser Trp Phe
            100                 105                 110
Ala Tyr Trp Gly Gln Gly Thr Leu Val Thr Val Ser Ser Ala Ser Val
        115                 120                 125
Ala Ala Pro Ser Val Phe Ile Phe Pro Pro Ser Asp Glu Gln Leu Lys
    130                 135                 140
Ser Gly Thr Ala Ser Val Val Cys Leu Leu Asn Asn Phe Tyr Pro Arg
145                 150                 155                 160
Glu Ala Lys Val Gln Trp Lys Val Asp Asn Ala Leu Gln Ser Gly Asn
                165                 170                 175
Ser Gln Glu Ser Val Thr Glu Gln Asp Ser Lys Asp Ser Thr Tyr Ser
            180                 185                 190
Leu Ser Ser Thr Leu Thr Leu Ser Lys Ala Asp Tyr Glu Lys His Lys
        195                 200                 205
Val Tyr Ala Cys Glu Val Thr His Gln Gly Leu Ser Ser Pro Val Thr
    210                 215                 220
Lys Ser Phe Asn Arg Gly Glu Cys
225                 230

<210> 103
<211> 449
<212> PRT
<213> Artificial Sequence

<220>
<223> CEA VH-CH1(EE)-Fc (hole, P329G LALA)

<400> 103
Gln Val Gln Leu Val Gln Ser Gly Ala Glu Val Lys Lys Pro Gly Ser
1               5                   10                  15
Ser Val Lys Val Ser Cys Lys Ala Ser Gly Phe Asn Ile Lys Asp Thr
            20                  25                  30
Tyr Met His Trp Val Arg Gln Ala Pro Gly Gln Gly Leu Glu Trp Met
        35                  40                  45
Gly Arg Ile Asp Pro Ala Asn Gly Asn Ser Lys Tyr Val Pro Lys Phe
    50                  55                  60
Gln Gly Arg Val Thr Ile Thr Ala Asp Thr Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Pro Phe Gly Tyr Tyr Val Ser Asp Tyr Ala Met Ala Tyr Trp Gly
            100                 105                 110
Gln Gly Thr Leu Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140

Ala Leu Gly Cys Leu Val Glu Asp Tyr Phe Pro Glu Pro Val Thr Val
145 150 155 160
Ser Trp Asn Ser Gly Ala Leu Thr Ser Gly Val His Thr Phe Pro Ala
165 170 175
Val Leu Gln Ser Ser Gly Leu Tyr Ser Leu Ser Ser Val Val Thr Val
180 185 190
Pro Ser Ser Ser Leu Gly Thr Gln Thr Tyr Ile Cys Asn Val Asn His
195 200 205
Lys Pro Ser Asn Thr Lys Val Asp Glu Lys Val Glu Pro Lys Ser Cys
210 215 220
Asp Lys Thr His Thr Cys Pro Pro Cys Pro Ala Pro Glu Ala Ala Gly
225 230 235 240
Gly Pro Ser Val Phe Leu Phe Pro Pro Lys Pro Lys Asp Thr Leu Met
245 250 255
Ile Ser Arg Thr Pro Glu Val Thr Cys Val Val Val Asp Val Ser His
260 265 270
Glu Asp Pro Glu Val Lys Phe Asn Trp Tyr Val Asp Gly Val Glu Val
275 280 285
His Asn Ala Lys Thr Lys Pro Arg Glu Glu Gln Tyr Asn Ser Thr Tyr
290 295 300
Arg Val Val Ser Val Leu Thr Val Leu His Gln Asp Trp Leu Asn Gly
305 310 315 320
Lys Glu Tyr Lys Cys Lys Val Ser Asn Lys Ala Leu Gly Ala Pro Ile
325 330 335
Glu Lys Thr Ile Ser Lys Ala Lys Gly Gln Pro Arg Glu Pro Gln Val
340 345 350
Cys Thr Leu Pro Pro Ser Arg Asp Glu Leu Thr Lys Asn Gln Val Ser
355 360 365
Leu Ser Cys Ala Val Lys Gly Phe Tyr Pro Ser Asp Ile Ala Val Glu
370 375 380
Trp Glu Ser Asn Gly Gln Pro Glu Asn Asn Tyr Lys Thr Thr Pro Pro
385 390 395 400
Val Leu Asp Ser Asp Gly Ser Phe Phe Leu Val Ser Lys Leu Thr Val
405 410 415
Asp Lys Ser Arg Trp Gln Gln Gly Asn Val Phe Ser Cys Ser Val Met
420 425 430
His Glu Ala Leu His Asn His Tyr Thr Gln Lys Ser Leu Ser Leu Ser
435 440 445
Pro

<210> 104
<211> 614
<212> PRT
<213> Artificial Sequence

<220>
<223> CEAVH-CH1(EE)-CD3 VL-CH1-Fc (knob, P329G) LALA)

<400> 104
Val Pro Lys Phe Gln Gly Arg Val Thr Ile Thr Ala Asp Thr Ser Thr
1 5 10 15
Ser Thr Ala Tyr Met Glu Leu Ser Ser Leu Arg Ser Glu Asp Thr Ala
20 25 30
Val Tyr Tyr Cys Ala Pro Phe Gly Tyr Tyr Val Ser Asp Tyr Ala Met
35 40 45
Ala Tyr Trp Gly Gln Gly Thr Leu Val Thr Val Ser Ser Ala Ser Thr
50 55 60
Lys Gly Pro Ser Val Phe Pro Leu Ala Pro Ser Ser Lys Ser Thr Ser
65 70 75 80
Gly Gly Thr Ala Ala Leu Gly Cys Leu Val Glu Asp Tyr Phe Pro Glu
85 90 95

```
Pro Val Thr Val Ser Trp Asn Ser Gly Ala Leu Thr Ser Gly Val His
            100                 105                 110
Thr Phe Pro Ala Val Leu Gln Ser Ser Gly Leu Tyr Ser Leu Ser Ser
        115                 120                 125
Val Val Thr Val Pro Ser Ser Ser Leu Gly Thr Gln Thr Tyr Ile Cys
    130                 135                 140
Asn Val Asn His Lys Pro Ser Asn Thr Lys Val Asp Glu Lys Val Glu
145                 150                 155                 160
Pro Lys Ser Cys Asp Gly Gly Gly Gly Ser Gly Gly Gly Gly Ser Gln
                165                 170                 175
Ala Val Val Thr Gln Glu Pro Ser Leu Thr Val Ser Pro Gly Gly Thr
            180                 185                 190
Val Thr Leu Thr Cys Gly Ser Ser Thr Gly Ala Val Thr Thr Ser Asn
        195                 200                 205
Tyr Ala Asn Trp Val Gln Glu Lys Pro Gly Gln Ala Phe Arg Gly Leu
    210                 215                 220
Ile Gly Gly Thr Asn Lys Arg Ala Pro Gly Thr Pro Ala Arg Phe Ser
225                 230                 235                 240
Gly Ser Leu Leu Gly Gly Lys Ala Ala Leu Thr Leu Ser Gly Ala Gln
                245                 250                 255
Pro Glu Asp Glu Ala Glu Tyr Tyr Cys Ala Leu Trp Tyr Ser Asn Leu
            260                 265                 270
Trp Val Phe Gly Gly Gly Thr Lys Leu Thr Val Leu Ser Ser Ala Ser
        275                 280                 285
Thr Lys Gly Pro Ser Val Phe Pro Leu Ala Pro Ser Ser Lys Ser Thr
    290                 295                 300
Ser Gly Gly Thr Ala Ala Leu Gly Cys Leu Val Lys Asp Tyr Phe Pro
305                 310                 315                 320
Glu Pro Val Thr Val Ser Trp Asn Ser Gly Ala Leu Thr Ser Gly Val
                325                 330                 335
His Thr Phe Pro Ala Val Leu Gln Ser Ser Gly Leu Tyr Ser Leu Ser
            340                 345                 350
Ser Val Val Thr Val Pro Ser Ser Ser Leu Gly Thr Gln Thr Tyr Ile
        355                 360                 365
Cys Asn Val Asn His Lys Pro Ser Asn Thr Lys Val Asp Lys Lys Val
    370                 375                 380
Glu Pro Lys Ser Cys Asp Lys Thr His Thr Cys Pro Pro Cys Pro Ala
385                 390                 395                 400
Pro Glu Ala Ala Gly Gly Pro Ser Val Phe Leu Phe Pro Pro Lys Pro
                405                 410                 415
Lys Asp Thr Leu Met Ile Ser Arg Thr Pro Glu Val Thr Cys Val Val
            420                 425                 430
Val Asp Val Ser His Glu Asp Pro Glu Val Lys Phe Asn Trp Tyr Val
        435                 440                 445
Asp Gly Val Glu Val His Asn Ala Lys Thr Lys Pro Arg Glu Glu Gln
    450                 455                 460
Tyr Asn Ser Thr Tyr Arg Val Val Ser Val Leu Thr Val Leu His Gln
465                 470                 475                 480
Asp Trp Leu Asn Gly Lys Glu Tyr Lys Cys Lys Val Ser Asn Lys Ala
                485                 490                 495
Leu Gly Ala Pro Ile Glu Lys Thr Ile Ser Lys Ala Lys Gly Gln Pro
            500                 505                 510
Arg Glu Pro Gln Val Tyr Thr Leu Pro Pro Cys Arg Asp Glu Leu Thr
        515                 520                 525
Lys Asn Gln Val Ser Leu Trp Cys Leu Val Lys Gly Phe Tyr Pro Ser
    530                 535                 540
Asp Ile Ala Val Glu Trp Glu Ser Asn Gly Gln Pro Glu Asn Asn Tyr
545                 550                 555                 560
Lys Thr Thr Pro Pro Val Leu Asp Ser Asp Gly Ser Phe Phe Leu Tyr
                565                 570                 575
Ser Lys Leu Thr Val Asp Lys Ser Arg Trp Gln Gln Gly Asn Val Phe
            580                 585                 590
Ser Cys Ser Val Met His Glu Ala Leu His Asn His Tyr Thr Gln Lys
```

595 600 605
Ser Leu Ser Leu Ser Pro
610

<210> 105
<211> 218
<212> PRT
<213> Artificial Sequence

<220>
<223> CEA VL-CL(RK)

<400> 105
Glu Ile Val Leu Thr Gln Ser Pro Ala Thr Leu Ser Leu Ser Pro Gly
1 5 10 15
Glu Arg Ala Thr Leu Ser Cys Arg Ala Gly Glu Ser Val Asp Ile Phe
20 25 30
Gly Val Gly Phe Leu His Trp Tyr Gln Gln Lys Pro Gly Gln Ala Pro
35 40 45
Arg Leu Leu Ile Tyr Arg Ala Ser Asn Arg Ala Thr Gly Ile Pro Ala
50 55 60
Arg Phe Ser Gly Ser Gly Ser Gly Thr Asp Phe Thr Leu Thr Ile Ser
65 70 75 80
Ser Leu Glu Pro Glu Asp Phe Ala Val Tyr Tyr Cys Gln Gln Thr Asn
85 90 95
Glu Asp Pro Tyr Thr Phe Gly Gln Gly Thr Lys Leu Glu Ile Lys Arg
100 105 110
Thr Val Ala Ala Pro Ser Val Phe Ile Phe Pro Pro Ser Asp Arg Lys
115 120 125
Leu Lys Ser Gly Thr Ala Ser Val Val Cys Leu Leu Asn Asn Phe Tyr
130 135 140
Pro Arg Glu Ala Lys Val Gln Trp Lys Val Asp Asn Ala Leu Gln Ser
145 150 155 160
Gly Asn Ser Gln Glu Ser Val Thr Glu Gln Asp Ser Lys Asp Ser Thr
165 170 175
Tyr Ser Leu Ser Ser Thr Leu Thr Leu Ser Lys Ala Asp Tyr Glu Lys
180 185 190
His Lys Val Tyr Ala Cys Glu Val Thr His Gln Gly Leu Ser Ser Pro
195 200 205
Val Thr Lys Ser Phe Asn Arg Gly Glu Cys
210 215

<210> 106
<211> 214
<212> PRT
<213> Artificial Sequence

<220>
<223> CD3 CH2527 Cross Fab VL-CH1

<400> 106
Gln Ala Val Val Thr Gln Glu Pro Ser Leu Thr Val Ser Pro Gly Gly
1 5 10 15
Thr Val Thr Leu Thr Cys Gly Ser Ser Thr Gly Ala Val Thr Thr Ser
20 25 30
Asn Tyr Ala Asn Trp Val Gln Glu Lys Pro Gly Gln Ala Phe Arg Gly
35 40 45
Leu Ile Gly Gly Thr Asn Lys Arg Ala Pro Gly Thr Pro Ala Arg Phe
50 55 60
Ser Gly Ser Leu Leu Gly Gly Lys Ala Ala Leu Thr Leu Ser Gly Ala
65 70 75 80

Gln Pro Glu Asp Glu Ala Glu Tyr Tyr Cys Ala Leu Trp Tyr Ser Asn
85 90 95
Leu Trp Val Phe Gly Gly Gly Thr Lys Leu Thr Val Leu Ser Ser Ala
100 105 110
Ser Thr Lys Gly Pro Ser Val Phe Pro Leu Ala Pro Ser Ser Lys Ser
115 120 125
Thr Ser Gly Gly Thr Ala Ala Leu Gly Cys Leu Val Lys Asp Tyr Phe
130 135 140
Pro Glu Pro Val Thr Val Ser Trp Asn Ser Gly Ala Leu Thr Ser Gly
145 150 155 160
Val His Thr Phe Pro Ala Val Leu Gln Ser Ser Gly Leu Tyr Ser Leu
165 170 175
Ser Ser Val Val Thr Val Pro Ser Ser Ser Leu Gly Thr Gln Thr Tyr
180 185 190
Ile Cys Asn Val Asn His Lys Pro Ser Asn Thr Lys Val Asp Lys Lys
195 200 205
Val Glu Pro Lys Ser Cys
210

<210> 107
<211> 451
<212> PRT
<213> Artificial Sequence

<220>
<223> CH1A10 VH CH1 FC Hole P329G LALA

<400> 107
Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Glu Phe
20 25 30
Gly Met Asn Trp Val Arg Gln Ala Pro Gly Gln Gly Leu Glu Trp Met
35 40 45
Gly Trp Ile Asn Thr Lys Thr Gly Glu Ala Thr Tyr Val Glu Glu Phe
50 55 60
Lys Gly Arg Val Thr Phe Thr Thr Asp Thr Ser Thr Ser Thr Ala Tyr
65 70 75 80
Met Glu Leu Arg Ser Leu Arg Ser Asp Asp Thr Ala Val Tyr Tyr Cys
85 90 95
Ala Arg Trp Asp Phe Ala Tyr Tyr Val Glu Ala Met Asp Tyr Trp Gly
100 105 110
Gln Gly Thr Thr Val Thr Val Ser Ser Ala Ser Thr Lys Gly Pro Ser
115 120 125
Val Phe Pro Leu Ala Pro Ser Ser Lys Ser Thr Ser Gly Gly Thr Ala
130 135 140
Ala Leu Gly Cys Leu Val Lys Asp Tyr Phe Pro Glu Pro Val Thr Val
145 150 155 160
Ser Trp Asn Ser Gly Ala Leu Thr Ser Gly Val His Thr Phe Pro Ala
165 170 175
Val Leu Gln Ser Ser Gly Leu Tyr Ser Leu Ser Ser Val Val Thr Val
180 185 190
Pro Ser Ser Ser Leu Gly Thr Gln Thr Tyr Ile Cys Asn Val Asn His
195 200 205
Lys Pro Ser Asn Thr Lys Val Asp Lys Lys Val Glu Pro Lys Ser Cys
210 215 220
Asp Lys Thr His Thr Cys Pro Pro Cys Pro Ala Pro Glu Ala Ala Gly
225 230 235 240
Gly Pro Ser Val Phe Leu Phe Pro Pro Lys Pro Lys Asp Thr Leu Met
245 250 255
Ile Ser Arg Thr Pro Glu Val Thr Cys Val Val Val Asp Val Ser His
260 265 270

```
Glu Asp Pro Glu Val Lys Phe Asn Trp Tyr Val Asp Gly Val Glu Val
        275                 280                 285
His Asn Ala Lys Thr Lys Pro Arg Glu Glu Gln Tyr Asn Ser Thr Tyr
    290                 295                 300
Arg Val Val Ser Val Leu Thr Val Leu His Gln Asp Trp Leu Asn Gly
305                 310                 315                 320
Lys Glu Tyr Lys Cys Lys Val Ser Asn Lys Ala Leu Gly Ala Pro Ile
                325                 330                 335
Glu Lys Thr Ile Ser Lys Ala Lys Gly Gln Pro Arg Glu Pro Gln Val
            340                 345                 350
Cys Thr Leu Pro Pro Ser Arg Asp Glu Leu Thr Lys Asn Gln Val Ser
        355                 360                 365
Leu Ser Cys Ala Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Val Leu Asp Ser Asp Gly Ser Phe Phe Leu Val Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly Lys
    450
```

<210> 108
<211> 694
<212> PRT
<213> Artificial Sequence

<220>
<223> CH1A1A CD3 CH2527 Cross Fab

<400> 108

```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Glu Phe
            20                  25                  30
Gly Met Asn Trp Val Arg Gln Ala Pro Gly Gln Gly Leu Glu Trp Met
        35                  40                  45
Gly Trp Ile Asn Thr Lys Thr Gly Glu Ala Thr Tyr Val Glu Glu Phe
    50                  55                  60
Lys Gly Arg Val Thr Phe Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Arg Trp Asp Phe Ala Tyr Tyr Val Glu Ala Met Asp Tyr Trp Gly
            100                 105                 110
Gln Gly Thr Thr Val Thr Val Ser Ser Ala Ser Thr Lys Gly Pro Ser
        115                 120                 125
Val Phe Pro Leu Ala Pro Ser Ser Lys Ser Thr Ser Gly Gly Thr Ala
    130                 135                 140
Ala Leu Gly Cys Leu Val Lys Asp Tyr Phe Pro Glu Pro Val Thr Val
145                 150                 155                 160
Ser Trp Asn Ser Gly Ala Leu Thr Ser Gly Val His Thr Phe Pro Ala
                165                 170                 175
Val Leu Gln Ser Ser Gly Leu Tyr Ser Leu Ser Ser Val Val Thr Val
            180                 185                 190
Pro Ser Ser Ser Leu Gly Thr Gln Thr Tyr Ile Cys Asn Val Asn His
        195                 200                 205
Lys Pro Ser Asn Thr Lys Val Asp Lys Lys Val Glu Pro Lys Ser Cys
    210                 215                 220
```

Asp Gly Gly Gly Gly Ser Gly Gly Gly Gly Ser Glu Val Gln Leu Leu
225                 230                 235                 240
Glu Ser Gly Gly Gly Leu Val Gln Pro Gly Gly Ser Leu Arg Leu Ser
                245                 250                 255
Cys Ala Ala Ser Gly Phe Thr Phe Ser Thr Tyr Ala Met Asn Trp Val
            260                 265                 270
Arg Gln Ala Pro Gly Lys Gly Leu Glu Trp Val Ser Arg Ile Arg Ser
        275                 280                 285
Lys Tyr Asn Asn Tyr Ala Thr Tyr Tyr Ala Asp Ser Val Lys Gly Arg
    290                 295                 300
Phe Thr Ile Ser Arg Asp Asp Ser Lys Asn Thr Leu Tyr Leu Gln Met
305                 310                 315                 320
Asn Ser Leu Arg Ala Glu Asp Thr Ala Val Tyr Tyr Cys Val Arg His
                325                 330                 335
Gly Asn Phe Gly Asn Ser Tyr Val Ser Trp Phe Ala Tyr Trp Gly Gln
            340                 345                 350
Gly Thr Leu Val Thr Val Ser Ser Ala Ser Val Ala Ala Pro Ser Val
        355                 360                 365
Phe Ile Phe Pro Pro Ser Asp Glu Gln Leu Lys Ser Gly Thr Ala Ser
    370                 375                 380
Val Val Cys Leu Leu Asn Asn Phe Tyr Pro Arg Glu Ala Lys Val Gln
385                 390                 395                 400
Trp Lys Val Asp Asn Ala Leu Gln Ser Gly Asn Ser Gln Glu Ser Val
                405                 410                 415
Thr Glu Gln Asp Ser Lys Asp Ser Thr Tyr Ser Leu Ser Ser Thr Leu
            420                 425                 430
Thr Leu Ser Lys Ala Asp Tyr Glu Lys His Lys Val Tyr Ala Cys Glu
        435                 440                 445
Val Thr His Gln Gly Leu Ser Ser Pro Val Thr Lys Ser Phe Asn Arg
    450                 455                 460
Gly Glu Cys Asp Lys Thr His Thr Cys Pro Pro Cys Pro Ala Pro Glu
465                 470                 475                 480
Ala Ala Gly Gly Pro Ser Val Phe Leu Phe Pro Pro Lys Pro Lys Asp
                485                 490                 495
Thr Leu Met Ile Ser Arg Thr Pro Glu Val Thr Cys Val Val Val Asp
            500                 505                 510
Val Ser His Glu Asp Pro Glu Val Lys Phe Asn Trp Tyr Val Asp Gly
        515                 520                 525
Val Glu Val His Asn Ala Lys Thr Lys Pro Arg Glu Glu Gln Tyr Asn
    530                 535                 540
Ser Thr Tyr Arg Val Val Ser Val Leu Thr Val Leu His Gln Asp Trp
545                 550                 555                 560
Leu Asn Gly Lys Glu Tyr Lys Cys Lys Val Ser Asn Lys Ala Leu Gly
                565                 570                 575
Ala Pro Ile Glu Lys Thr Ile Ser Lys Ala Lys Gly Gln Pro Arg Glu
            580                 585                 590
Pro Gln Val Tyr Thr Leu Pro Pro Cys Arg Asp Glu Leu Thr Lys Asn
        595                 600                 605
Gln Val Ser Leu Trp Cys Leu Val Lys Gly Phe Tyr Pro Ser Asp Ile
    610                 615                 620
Ala Val Glu Trp Glu Ser Asn Gly Gln Pro Glu Asn Asn Tyr Lys Thr
625                 630                 635                 640
Thr Pro Pro Val Leu Asp Ser Asp Gly Ser Phe Phe Leu Tyr Ser Lys
                645                 650                 655
Leu Thr Val Asp Lys Ser Arg Trp Gln Gln Gly Asn Val Phe Ser Cys
            660                 665                 670
Ser Val Met His Glu Ala Leu His Asn His Tyr Thr Gln Lys Ser Leu
        675                 680                 685
Ser Leu Ser Pro Gly Lys
    690

<210> 109
<211> 215

<212> PRT
<213> Artificial Sequence

<220>
<223> LC CEA

<400> 109
Asp Ile Gln Met Thr Gln Ser Pro Ser Ser Leu Ser Ala Ser Val Gly
1 5 10 15
Asp Arg Val Thr Ile Thr Cys Lys Ala Ser Ala Ala Val Gly Thr Tyr
20 25 30
Val Ala Trp Tyr Gln Gln Lys Pro Gly Lys Ala Pro Lys Leu Leu Ile
35 40 45
Tyr Ser Ala Ser Tyr Arg Lys Arg Gly Val Pro Ser Arg Phe Ser Gly
50 55 60
Ser Gly Ser Gly Thr Asp Phe Thr Leu Thr Ile Ser Ser Leu Gln Pro
65 70 75 80
Glu Asp Phe Ala Thr Tyr Tyr Cys His Gln Tyr Tyr Thr Tyr Pro Leu
85 90 95
Phe Thr Phe Gly Gln Gly Thr Lys Leu Glu Ile Lys Arg Thr Val Ala
100 105 110
Ala Pro Ser Val Phe Ile Phe Pro Pro Ser Asp Glu Gln Leu Lys Ser
115 120 125
Gly Thr Ala Ser Val Val Cys Leu Leu Asn Asn Phe Tyr Pro Arg Glu
130 135 140
Ala Lys Val Gln Trp Lys Val Asp Asn Ala Leu Gln Ser Gly Asn Ser
145 150 155 160
Gln Glu Ser Val Thr Glu Gln Asp Ser Lys Asp Ser Thr Tyr Ser Leu
165 170 175
Ser Ser Thr Leu Thr Leu Ser Lys Ala Asp Tyr Glu Lys His Lys Val
180 185 190
Tyr Ala Cys Glu Val Thr His Gln Gly Leu Ser Ser Pro Val Thr Lys
195 200 205
Ser Phe Asn Arg Gly Glu Cys
210 215

<210> 110
<211> 106
<212> PRT
<213> Artificial Sequence

<220>
<223> VK-SM3E

<400> 110
Glu Asn Val Leu Thr Gln Ser Pro Ser Ser Met Ser Val Ser Val Gly
1 5 10 15
Asp Arg Val Asn Ile Ala Cys Ser Ala Ser Ser Ser Val Pro Tyr Met
20 25 30
His Trp Leu Gln Gln Lys Pro Gly Lys Ser Pro Lys Leu Leu Ile Tyr
35 40 45
Leu Thr Ser Asn Leu Ala Ser Gly Val Pro Ser Arg Phe Ser Gly Ser
50 55 60
Gly Ser Gly Thr Asp Tyr Ser Leu Thr Ile Ser Ser Val Gln Pro Glu
65 70 75 80
Asp Ala Ala Thr Tyr Tyr Cys Gln Gln Arg Ser Ser Tyr Pro Leu Thr
85 90 95
Phe Gly Gly Gly Thr Lys Leu Glu Ile Lys
100 105

<210> 111

<211> 120
<212> PRT
<213> Artificial Sequence

<220>
<223> VH_SM3E

<400> 111
Gln Val Lys Leu Glu Gln Ser Gly Ala Glu Val Val Lys Pro Gly Ala
1 5 10 15
Ser Val Lys Leu Ser Cys Lys Ala Ser Gly Phe Asn Ile Lys Asp Ser
20 25 30
Tyr Met His Trp Leu Arg Gln Gly Pro Gly Gln Arg Leu Glu Trp Ile
35 40 45
Gly Trp Ile Asp Pro Glu Asn Gly Asp Thr Glu Tyr Ala Pro Lys Phe
50 55 60
Gln Gly Lys Ala Thr Phe Thr Thr Asp Thr Ser Ala Asn Thr Ala Tyr
65 70 75 80
Leu Gly Leu Ser Ser Leu Arg Pro Glu Asp Thr Ala Val Tyr Tyr Cys
85 90 95
Asn Glu Gly Thr Pro Thr Gly Pro Tyr Tyr Phe Asp Tyr Trp Gly Gln
100 105 110
Gly Thr Leu Val Thr Val Ser Ser
115 120

<210> 112
<211> 107
<212> PRT
<213> Artificial Sequence

<220>
<223> VK_SAR

<400> 112
Asp Ile Gln Met Thr Gln Ser Pro Ser Ser Leu Ser Ala Ser Val Gly
1 5 10 15
Asp Arg Val Thr Ile Thr Cys Arg Ala Ser Glu Asn Ile Phe Ser Tyr
20 25 30
Leu Ala Trp Tyr Gln Gln Lys Pro Gly Lys Ala Pro Lys Leu Leu Ile
35 40 45
Tyr Asn Thr Arg Thr Leu Gln Ser Gly Val Pro Ser Arg Phe Ser Gly
50 55 60
Ser Gly Ser Gly Thr Asp Phe Thr Leu Thr Ile Ser Ser Leu Gln Pro
65 70 75 80
Glu Asp Phe Ala Thr Tyr Tyr Cys Gln His His Tyr Gly Thr Pro Phe
85 90 95
Thr Phe Gly Gln Gly Thr Lys Leu Glu Ile Lys
100 105

<210> 113
<211> 120
<212> PRT
<213> Artificial Sequence

<220>
<223> VH_SAR

<400> 113
Glu Val Gln Leu Val Glu Ser Gly Gly Gly Leu Val Gln Pro Gly Gly
1 5 10 15

Ser Leu Arg Leu Ser Cys Ala Ala Ser Gly Phe Val Phe Ser Ser Tyr
            20                  25                  30
Asp Met Ser Trp Val Arg Gln Ala Pro Gly Lys Gly Leu Glu Trp Val
        35                  40                  45
Ser Tyr Ile Ser Ser Gly Gly Gly Ile Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Ala His Tyr Phe Gly Ser Ser Gly Pro Phe Ala Tyr Trp Gly Gln
            100                 105                 110
Gly Thr Leu Val Thr Val Ser Ser
        115                 120

<210> 114
<211> 108
<212> PRT
<213> Artificial Sequence

<220>
<223> VK_CH1A1A

<400> 114
Asp Ile Gln Met Thr Gln Ser Pro Ser Ser Leu Ser Ala Ser Val Gly
1               5                   10                  15
Asp Arg Val Thr Ile Thr Cys Lys Ala Ser Ala Ala Val Gly Thr Tyr
            20                  25                  30
Val Ala Trp Tyr Gln Gln Lys Pro Gly Lys Ala Pro Lys Leu Leu Ile
        35                  40                  45
Tyr Ser Ala Ser Tyr Arg Lys Arg Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys His Gln Tyr Tyr Thr Tyr Pro Leu
                85                  90                  95
Phe Thr Phe Gly Gln Gly Thr Lys Leu Glu Ile Lys
            100                 105

<210> 115
<211> 121
<212> PRT
<213> Artificial Sequence

<220>
<223> VH_ CH1A1A

<400> 115
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Glu Phe
            20                  25                  30
Gly Met Asn Trp Val Arg Gln Ala Pro Gly Gln Gly Leu Glu Trp Met
        35                  40                  45
Gly Trp Ile Asn Thr Lys Thr Gly Glu Ala Thr Tyr Val Glu Glu Phe
    50                  55                  60
Lys Gly Arg Val Thr Phe Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Arg Trp Asp Phe Ala Tyr Tyr Val Glu Ala Met Asp Tyr Trp Gly

100 105 110
Gln Gly Thr Thr Val Thr Val Ser Ser
115 120

<210> 116
<211> 121
<212> PRT
<213> Artificial Sequence

<220>
<223> MAB CEA variable heavy chain

<400> 116
Gln Val Gln Leu Val Gln Ser Gly Ala Glu Val Lys Lys Pro Gly Ala
1 5 10 15
Ser Val Lys Val Ser Cys Lys Ala Ser Gly Tyr Thr Phe Thr Glu Phe
20 25 30
Gly Met Asn Trp Val Arg Gln Ala Pro Gly Gln Gly Leu Glu Trp Met
35 40 45
Gly Trp Ile Asn Thr Lys Thr Gly Glu Ala Thr Tyr Val Glu Glu Phe
50 55 60
Lys Gly Arg Val Thr Phe Thr Thr Asp Thr Ser Thr Ser Thr Ala Tyr
65 70 75 80
Met Glu Leu Arg Ser Leu Arg Ser Asp Asp Thr Ala Val Tyr Tyr Cys
85 90 95
Ala Arg Trp Asp Phe Ala Tyr Tyr Val Glu Ala Met Asp Tyr Trp Gly
100 105 110
Gln Gly Thr Thr Val Thr Val Ser Ser
115 120

<210> 117
<211> 108
<212> PRT
<213> Artificial Sequence

<220>
<223> MAB CEA variable light chain

<400> 117
Asp Ile Gln Met Thr Gln Ser Pro Ser Ser Leu Ser Ala Ser Val Gly
1 5 10 15
Asp Arg Val Thr Ile Thr Cys Lys Ala Ser Ala Ala Val Gly Thr Tyr
20 25 30
Val Ala Trp Tyr Gln Gln Lys Pro Gly Lys Ala Pro Lys Leu Leu Ile
35 40 45
Tyr Ser Ala Ser Tyr Arg Lys Arg Gly Val Pro Ser Arg Phe Ser Gly
50 55 60
Ser Gly Ser Gly Thr Asp Phe Thr Leu Thr Ile Ser Ser Leu Gln Pro
65 70 75 80
Glu Asp Phe Ala Thr Tyr Tyr Cys His Gln Tyr Tyr Thr Tyr Pro Leu
85 90 95
Phe Thr Phe Gly Gln Gly Thr Lys Leu Glu Ile Lys
100 105

<210> 118
<211> 5
<212> PRT
<213> Artificial Sequence

<220>

<223> Epitope of CD3 epsilon

<400> 118
Gln Asp Gly Asn Glu
1 5

<210> 119
<211> 1338
<212> DNA
<213> Artificial Sequence

<220>
<223> Mab CD47 heavy chain VHCH

<400> 119
gaggtgcagc tgttggagtc tgggggaggc ttggtacagc ctggggggtc cctgagactc 60

tcctgtgcag cctctggatt cacctttagc agctatgcca tgagctgggt ccgccaggct 120

ccagggaagg ggctggagtg ggtctcagct attagtggta gtggtggtag cacatactac 180

gcagactccg tgaagggccg gttcaccatc tccagagaca attccaagaa cacgctgtat 240

ctgcaaatga acagcctgag agccgaggac acggccgtat attactgtgc gaaaagttat 300

ggtgcttttg actactgggg ccagggaacc ctggtcacag tctcgagcgc ctccaccaag 360

ggcccatcgg tcttccccct ggcaccctcc tccaagagca cctctggggg cacagcggcc 420

ctgggctgcc tggtcaagga ctacttcccc gaaccggtga cagtctcgtg gaactcagga 480

gccctgacca gcggcgtgca caccttcccg gctgtcctac agtcctcagg actctactcc 540

ctcagcagcg tggtgactgt gccctccagc agcttgggca cccagaccta catctgcaac 600

gtgaatcaca agcccagcaa caccaaggtg gacaagagag ttgagcccaa atcttgtgac 660

aaaactcaca catgcccacc gtgcccagca cctgaactcc tggggggacc gtcagtcttc 720

ctcttccccc caaaacccaa ggacaccctc atgatctccc ggacccctga ggtcacatgc 780

gtggtggtgg acgtgagcca cgaagaccct gaggtcaagt tcaactggta cgtggacggc 840

gtggaggtgc ataatgccaa gacaaagccg cgggaggagc agtacaacag cacgtaccgt 900

gtggtcagcg tcctcaccgt cctgcaccag gactggctga atggcaagga gtacaagtgc 960

aaggtctcca acaaagccct cccagccccc atcgagaaaa ccatctccaa agccaaaggg 1020

cagccccgag aaccacaggt gtacaccctg cccccatctc gggaggagat gaccaagaac 1080

caggtcagcc tgacttgcct ggtcaaaggc ttctatccca gcgacatcgc cgtggagtgg 1140

gagagcaacg ggcagccgga gaacaactac aagaccacgc ctcccgtgct ggactccgac 1200

ggctccttct tcctctatag caagctcacc gtggacaagt ccaggtggca gcaggggaac 1260

gtcttctcat gctccgtgat gcatgaggct ctgcacaacc actacacgca gaagagcctc 1320

tccctgtctc cgggttaa 1338

<210> 120
<211> 648
<212> DNA
<213> Artificial Sequence

<220>
<223> Mab CD47 Light Chain KA3 VKCK(K2)

<400> 120
gacatccaga tgacccagtc tccatcctcc ctgtctgcat ctgtaggaga cagagtcacc 60

atcacttgcc gggcaagtca gagcattagc agctatttaa attggtatca gcagaaacca 120

gggaaagccc ctaagctcct gatctatgct gcatccagtt tgcaaagtgg ggtcccatca 180

aggttcagtg gcagtggatc tgggacagat ttcactctca ccatcagcag tctgcaacct 240

gaagattttg caacttacta ctgtcagcag atgcacccgc gcgccccgaa gaccttcggc 300

caagggacca aggtggaaat caaacgtacg gtggctgcac catctgtctt catcttcccg 360

ccatctgatg agcagttgaa atctggaact gcctctgttg tgtgcctgct gaataacttc 420

tatcccagag aggccaaagt acagtggaag gtggataacg ccctccaatc gggtaactcc 480

caggagagtg tcacagagca ggacagcaag gacagcacct acagcctcag cagcaccctg 540

acgctgagca aagcagacta cgagaaacac aaagtctacg cctgcgaagt cacccatcag 600

ggcctgagct cgcccgtcac aaagagcttc aacaggggag agtgttaa 648

<210> 121
<211> 651
<212> DNA
<213> Artificial Sequence

<220>
<223> AC41 VKCK

<400> 121
gacatccaga tgacccagtc tccatcctcc ctgtctgcat ctgtaggaga cagagtcacc 60

atcacttgcc aggcgagtca ggagattgct gcgactttaa attggtatca gcagaaacca 120

gggaaagccc ctaagctcct gatctacaat tattccactt tggaaacagg ggtcccatca 180

aggttcagtg gaagtggatc tgggacagat tttactttca ccatcagcag cctgcagcct 240

gaagatattg caacatatta ctgtcagcag gttggtctta atcctgagaa tactaccttc 300

ggccaaggga ccaaggtgga aatcaaacgt acggtggctg caccatctgt cttcatcttc 360

ccgccatctg atgagcagtt gaaatctgga actgcctctg ttgtgtgcct gctgaataac 420

ttctatccca gagaggccaa agtacagtgg aaggtggata acgccctcca atcgggtaac 480

tcccaggaga gtgtcacaga gcaggacagc aaggacagca cctacagcct cagcagcacc 540

ctgacgctga gcaaagcaga ctacgagaaa cacaaagtct acgcctgcga agtcacccat 600

cagggcctga gctcgcccgt cacaaagagc ttcaacaggg gagagtgtta a 651

<210> 122
<211> 648
<212> DNA
<213> Artificial Sequence

<220>
<223> AC41 VK-H-CL1 hybrid

<400> 122
gacatccaga tgacccagtc tccatcctcc ctgtctgcat ctgtaggaga cagagtcacc 60

atcacttgcc aggcgagtca ggagattgct gcgactttaa attggtatca gcagaaacca 120

gggaaagccc ctaagctcct gatctacaat tattccactt tggaaacagg ggtcccatca 180

aggttcagtg gaagtggatc tgggacagat tttactttca ccatcagcag cctgcagcct 240

gaagatattg caacatatta ctgtcagcag gttggtctta atcctgagaa tactaccttc 300

ggcggaggga ccaagctgac cgtcctaggt cagcccaagg ctgccccctc ggtcactctg 360

ttcccgccct cctctgagga gcttcaagcc aacaaggcca cactggtgtg tctcataagt 420

gacttctacc cgggagccgt gacagtggct tggaaagcag atagcagccc cgtcaaggcg 480

ggagtggaga ccaccacacc ctccaaacaa agcaacaaca agtacgcggc cagcagctat 540

ctgagcctga cgcctgagca gtggaagtcc cacagaagct acagctgcca ggtcacgcat 600

gaagggagca ccgtggagaa gacagtggcc cctacagaat gttcataa 648

<210> 123
<211> 651
<212> DNA
<213> Artificial Sequence

<220>
<223> AC42 VKCK

<400> 123
gacatccaga tgacccagtc tccatcctcc ctgtctgcat ctgtaggaga cagagtcacc 60

atcacttgcc aggcgagtca gaggattgct aggactttaa attggtatca gcagaaacca 120

gggaaagccc ctaagctcct gatctacaat ttttccgcgt tggaaacagg ggtcccatca 180

aggttcagtg gaagtggatc tgggacagat tttactttca ccatcagcag cctgcagcct 240

gaagatattg caacatatta ctgtcagcag gttgggcttg atcctaatct tactaccttc 300

ggccaaggga ccaaggtgga aatcaaacgt acggtggctg caccatctgt cttcatcttc 360

ccgccatctg atgagcagtt gaaatctgga actgcctctg ttgtgtgcct gctgaataac 420

ttctatccca gagaggccaa agtacagtgg aaggtggata acgccctcca atcgggtaac 480

tcccaggaga gtgtcacaga gcaggacagc aaggacagca cctacagcct cagcagcacc 540

ctgacgctga gcaaagcaga ctacgagaaa cacaaagtct acgcctgcga agtcacccat 600

cagggcctga gctcgcccgt cacaaagagc ttcaacaggg gagagtgtta a 651

<210> 124
<211> 648
<212> DNA
<213> Artificial Sequence

<220>
<223> AC42 VK-H-CL1

<400> 124
gacatccaga tgacccagtc tccatcctcc ctgtctgcat ctgtaggaga cagagtcacc 60

atcacttgcc aggcgagtca gaggattgct aggactttaa attggtatca gcagaaacca 120

gggaaagccc ctaagctcct gatctacaat tttccgcgt tggaaacagg ggtcccatca 180

aggttcagtg gaagtggatc tgggacagat tttactttca ccatcagcag cctgcagcct 240

gaagatattg caacatatta ctgtcagcag gttgggcttg atcctaatct tactaccttc 300

ggcggaggga ccaagctgac cgtcctaggt cagcccaagg ctgccccctc ggtcactctg 360

ttcccgccct cctctgagga gcttcaagcc aacaaggcca cactggtgtg tctcataagt 420

gacttctacc cgggagccgt gacagtggct tggaaagcag atagcagccc cgtcaaggcg 480

ggagtggaga ccaccacacc ctccaaacaa agcaacaaca agtacgcggc cagcagctat 540

ctgagcctga cgcctgagca gtggaagtcc cacagaagct acagctgcca ggtcacgcat 600

gaagggagca ccgtggagaa gacagtggcc cctacagaat gttcataa 648

<210> 125
<211> 651
<212> DNA
<213> Artificial Sequence

<220>
<223> AC43 VKCK

<400> 125
gacatccaga tgacccagtc tccatcctcc ctgtctgcat ctgtaggaga cagagtcacc 60

atcacttgcc aggcgagtca gaatattcct aggactataa attggtatca gcagaaacca 120

gggaaagccc ctaagctcct gatctacgat atttccaatt tggaaacagg ggtcccatca 180

aggttcagtg gaagtggatc tgggacagat tttactttca ccatcagcag cctgcagcct 240

gaagatattg caacatatta ctgtcagcag gttggtctta atcctgagct tacgaccttc 300

ggccaaggga ccaaggtgga aatcaaacgt acggtggctg caccatctgt cttcatcttc 360

ccgccatctg atgagcagtt gaaatctgga actgcctctg ttgtgtgcct gctgaataac 420

ttctatccca gagaggccaa agtacagtgg aaggtggata acgccctcca atcgggtaac 480

tcccaggaga gtgtcacaga gcaggacagc aaggacagca cctacagcct cagcagcacc 540

ctgacgctga gcaaagcaga ctacgagaaa cacaaagtct acgcctgcga agtcacccat 600

cagggcctga gctcgcccgt cacaaagagc ttcaacaggg gagagtgtta a 651

<210> 126
<211> 648
<212> DNA
<213> Artificial Sequence

<220>
<223> AC43 VK-H-CL1

<400> 126
gacatccaga tgacccagtc tccatcctcc ctgtctgcat ctgtaggaga cagagtcacc 60

atcacttgcc aggcgagtca gaatattcct aggactataa attggtatca gcagaaacca 120

gggaaagccc ctaagctcct gatctacgat atttccaatt tggaaacagg ggtcccatca 180

aggttcagtg gaagtggatc tgggacagat tttactttca ccatcagcag cctgcagcct 240

gaagatattg caacatatta ctgtcagcag gttggtctta atcctgagct tacgaccttc 300

ggcggaggga ccaagctgac cgtcctaggt cagcccaagg ctgcccccctc ggtcactctg 360

ttcccgccct cctctgagga gcttcaagcc aacaaggcca cactggtgtg tctcataagt 420

gacttctacc cgggagccgt gacagtggct tggaaagcag atagcagccc cgtcaaggcg 480

ggagtggaga ccaccacacc ctccaaacaa agcaacaaca agtacgcggc cagcagctat 540

ctgagcctga cgcctgagca gtggaagtcc cacagaagct acagctgcca ggtcacgcat 600

gaagggagca ccgtggagaa gacagtggcc cctacagaat gttcataa 648

<210> 127
<211> 657
<212> DNA
<213> Artificial Sequence

<220>
<223> AC44 VLCL

<400> 127
cagtctgtgc tgactcagcc accctcagcg tctgggaccc ccgggcagag ggtcaccatc 60

tcttgttctg gaagcagctc caacatcgtt ggtgttggtg taaactggta ccagcagctc 120

ccaggaacgg cccccaaact cctcatctat agttataata agcggccctc aggggtccct 180

gaccgattct ctggctccaa gtctggcacc tcagcctccc tggccatcag tgggctccag 240

tctgaggatg aggctgatta ttactgtgca gcatatgatt tcttgcccgt gctgcctgcg 300

cctgtgttcg gcggagggac caagctgacc gtcctaggtc agcccaaggc tgcccccctcg 360

gtcactctgt tcccgccctc ctctgaggag cttcaagcca acaaggccac actggtgtgt 420

ctcataagtg acttctaccc gggagccgtg acagtggctt ggaaagcaga tagcagcccc 480

gtcaaggcgg gagtggagac caccacaccc tccaaacaaa gcaacaacaa gtacgcggcc 540

agcagctatc tgagcctgac gcctgagcag tggaagtccc acagaagcta cagctgccag 600

gtcacgcatg aagggagcac cgtggagaag acagtggccc ctacagaatg ttcataa 657

<210> 128
<211> 657
<212> DNA
<213> Artificial Sequence

<220>
<223> AC45 VLCL

<400> 128
cagtctgtgc tgactcagcc accctcagcg tctgggaccc ccgggcagag ggtcaccatc 60

tcttgttctg gaagcagctc caacatcgtt ggtgttggtg taaactggta ccagcagctc 120

ccaggaacgg cccccaaact cctcatctat agttataata agcggccctc aggggtccct 180

gaccgattct ctggctccaa gtctggcacc tcagcctccc tggccatcag tgggctccag 240

tctgaggatg aggctgatta ttactgtgca gcatatgatg cggacatgat cgtgcctgcg 300

cctgtgttcg gcggagggac caagctgacc gtcctaggtc agcccaaggc tgccccctcg 360

gtcactctgt tcccgccctc ctctgaggag cttcaagcca acaaggccac actggtgtgt 420

ctcataagtg acttctaccc gggagccgtg acagtggctt ggaaagcaga tagcagcccc 480

gtcaaggcgg gagtggagac caccacaccc tccaaacaaa gcaacaacaa gtacgcggcc 540

agcagctatc tgagcctgac gcctgagcag tggaagtccc acagaagcta cagctgccag 600

gtcacgcatg aagggagcac cgtggagaag acagtggccc ctacagaatg ttcataa 657

<210> 129
<211> 657
<212> DNA
<213> Artificial Sequence

<220>
<223> AC46VLCL

<400> 129
cagtctgtgc tgactcagcc accctcagcg tctgggaccc ccgggcagag ggtcaccatc 60

tcttgttctg gaagcagctc caacatcgtt ggtgttggtg taaactggta ccagcagctc 120

ccaggaacgg cccccaaact cctcatctat agttataata agcggccctc aggggtccct 180

gaccgattct ctggctccaa gtctggcacc tcagcctccc tggccatcag tgggctccag 240

tctgaggatg aggctgatta ttactgtgca gcatatgatt tctcgctgtt gctgcctgcg 300

cctgtgttcg gcggagggac caagctgacc gtcctaggtc agcccaaggc tgccccctcg 360

gtcactctgt tcccgccctc ctctgaggag cttcaagcca acaaggccac actggtgtgt 420

ctcataagtg acttctaccc gggagccgtg acagtggctt ggaaagcaga tagcagcccc 480

gtcaaggcgg gagtggagac caccacaccc tccaaacaaa gcaacaacaa gtacgcggcc 540

agcagctatc tgagcctgac gcctgagcag tggaagtccc acagaagcta cagctgccag 600

gtcacgcatg aagggagcac cgtggagaag acagtggccc ctacagaatg ttcataa 657

<210> 130
<211> 657
<212> DNA
<213> Artificial Sequence

<220>
<223> AC47 VLCL

<400> 130
cagtctgtgc tgactcagcc accctcagcg tctgggaccc ccgggcagag ggtcaccatc 60

tcttgttctg gaagcagctc caacatcaat ggggttgggg taaactggta ccagcagctc 120

ccaggaacgg cccccaaact cctcatctat gggtttaata gtcggccctc aggggtccct 180

gaccgattct ctggctccaa gtctggcacc tcagcctccc tggccatcag tgggctccag 240

tctgaggatg aggctgatta ttactgtgca gcatatgata tgtcgacgta tgtgcctgcg 300

cctgtgttcg gcggagggac caagctgacc gtcctaggtc agcccaaggc tgccccctcg 360

gtcactctgt tcccgccctc ctctgaggag cttcaagcca acaaggccac actggtgtgt 420

ctcataagtg acttctaccc gggagccgtg acagtggctt ggaaagcaga tagcagcccc 480

gtcaaggcgg gagtggagac caccacaccc tccaaacaaa gcaacaacaa gtacgcggcc 540

agcagctatc tgagcctgac gcctgagcag tggaagtccc acagaagcta cagctgccag 600

gtcacgcatg aagggagcac cgtggagaag acagtggccc ctacagaatg ttcataa 657

<210> 131
<211> 657
<212> DNA
<213> Artificial Sequence

<220>
<223> AC48 VLCL

<400> 131
cagtctgtgc tgactcagcc accctcagcg tctgggaccc ccgggcagag ggtcaccatc 60

tcttgttctg gaagcagctc caacatcaat ggggttgggg taaactggta ccagcagctc 120

ccaggaacgg cccccaaact cctcatctat gagtttaata agcggccctc aggggtccct 180

gaccgattct ctggctccaa gtctggcacc tcagcctccc tggccatcag tgggctccag 240

tctgaggatg aggctgatta ttactgtgca gcatatgatg cggcgcggta tgtgcctgcg 300

cctgtgttcg gcggagggac caagctgacc gtcctaggtc agcccaaggc tgccccctcg 360

gtcactctgt tcccgccctc ctctgaggag cttcaagcca acaaggccac actggtgtgt 420

ctcataagtg acttctaccc gggagccgtg acagtggctt ggaaagcaga tagcagcccc 480

gtcaaggcgg gagtggagac caccacaccc tccaaacaaa gcaacaacaa gtacgcggcc 540

agcagctatc tgagcctgac gcctgagcag tggaagtccc acagaagcta cagctgccag 600

gtcacgcatg aagggagcac cgtggagaag acagtggccc ctacagaatg ttcataa 657

<210> 132
<211> 651
<212> DNA
<213> Artificial Sequence

<220>
<223> AC49 VLCL

<400> 132
cagtctgtgt tgacgcagcc gccctcagtg tctgcggccc caggacagaa ggtcaccatc 60

tcctgctctg gaagcagctc caacattttt gattctgggg tatcctggta ccagcagctc 120

ccaggaacag cccccaaact cctcatttat aatcataatg agcgaccctc agggattcct 180

gaccgattct ctggctccaa gtctggcacg tcagccaccc tgggcatcac cggactccag 240

actggggacg aggccgatta ttactgcgga acatgggatt tcccgccgtc caggttcgtg 300

ttcggcggag ggaccaagct gaccgtccta ggtcagccca aggctgcccc ctcggtcact 360

ctgttcccgc cctcctctga ggagcttcaa gccaacaagg ccacactggt gtgtctcata 420

agtgacttct acccgggagc cgtgacagtg gcttggaaag cagatagcag ccccgtcaag 480

gcgggagtgg agaccaccac accctccaaa caaagcaaca acaagtacgc ggccagcagc 540

tatctgagcc tgacgcctga gcagtggaag tcccacagaa gctacagctg ccaggtcacg 600

catgaaggga gcaccgtgga gaagacagtg gcccctacag aatgttcata a 651

<210> 133
<211> 660
<212> DNA
<213> Artificial Sequence

<220>
<223> AC50 VLCL

<400> 133
cagtctgccc tgactcagcc tgcctccgtg tctgggtctc ctggacagtc gatcaccatc 60

tcctgcactg gaaccagcag tgacgtttat gagtttaggc ttgtctcctg gtaccaacag 120

cacccaggca aagcccccaa actcatgatt tattttgata gtcttcggcc ctcaggggtt 180

tctaatcgct tctctggctc caagtctggc aacacggcct ccctgaccat ctctgggctc 240

caggctgagg acgaggctga ttattactgc agctcatggg ataagtttgg gtatgcgttt 300

aattctgtgt tcggcggagg gaccaagctg accgtcctag gtcagcccaa ggctgccccc 360

tcggtcactc tgttcccgcc ctcctctgag gagcttcaag ccaacaaggc cacactggtg 420

tgtctcataa gtgacttcta cccgggagcc gtgacagtgg cttggaaagc agatagcagc 480

cccgtcaagg cgggagtgga gaccaccaca ccctccaaac aaagcaacaa caagtacgcg 540

gccagcagct atctgagcct gacgcctgag cagtggaagt cccacagaag ctacagctgc 600

caggtcacgc atgaagggag caccgtggag aagacagtgg cccctacaga atgttcataa 660

<210> 134
<211> 660
<212> DNA
<213> Artificial Sequence

<220>
<223> AC52 VLCL

<400> 134
cagtctgtgc tgacgcagcc gccctcagtg tctggggccc cagggcagag ggtcaccatc 60

tcctgcactg ggagcagctc caacatccat gatgttgggt atgtacactg gtaccagcag 120

cttccaggaa cagcccccaa actcctcatc tatggggcta gcgggcggcc ctcaggggtc 180

cctgaccgat tctctggctc caagtctggc acctcagcct ccctggccat cactgggctc 240

caggctgagg atgaggctga ttattactgt cagtcctatg atacgaagcg cgagttccct 300

actcctgtgt tcggcggagg gaccaagctg accgtcctag gtcagcccaa ggctgccccc 360

tcggtcactc tgttcccgcc ctcctctgag gagcttcaag ccaacaaggc cacactggtg 420

tgtctcataa gtgacttcta cccgggagcc gtgacagtgg cttggaaagc agatagcagc 480

cccgtcaagg cgggagtgga gaccaccaca ccctccaaac aaagcaacaa caagtacgcg 540

gccagcagct atctgagcct gacgcctgag cagtggaagt cccacagaag ctacagctgc 600

caggtcacgc atgaagggag caccgtggag aagacagtgg cccctacaga atgttcataa 660

<210> 135
<211> 660
<212> DNA
<213> Artificial Sequence

<220>
<223> AC53 VLCL

<400> 135
cagtctgtgc tgacgcagcc gccctcagtg tctggggccc cagggcagag ggtcaccatc 60
tcctgcactg ggagcagctc caacatccat gatgttgggt atgtacactg gtaccagcag 120
cttccaggaa cagcccccaa actcctcatc tatggggcta gcgggcggcc ctcaggggtc 180
cctgaccgat tctctggctc caagtctggc acctcagcct ccctggccat cactgggctc 240
caggctgagg atgaggctga ttattactgt cagtcctatg atgggagggc cgtcttccct 300
actcctgtgt tcggcggagg gaccaagctg accgtcctag gtcagcccaa ggctgccccc 360
tcggtcactc tgttcccgcc ctcctctgag gagcttcaag ccaacaaggc cacactggtg 420
tgtctcataa gtgacttcta cccgggagcc gtgacagtgg cttggaaagc agatagcagc 480
cccgtcaagg cgggagtgga gaccaccaca ccctccaaac aaagcaacaa caagtacgcg 540
gccagcagct atctgagcct gacgcctgag cagtggaagt cccacagaag ctacagctgc 600
caggtcacgc atgaagggag caccgtggag aagacagtgg cccctacaga atgttcataa 660

<210> 136
<211> 660
<212> DNA
<213> Artificial Sequence

<220>
<223> AC54 VLCL

<400> 136
cagtctgtgc tgacgcagcc gccctcagtg tctggggccc cagggcagag ggtcaccatc 60
tcctgcactg ggagcagctc caacatccat gatgttgggt atgtacactg gtaccagcag 120
cttccaggaa cagcccccaa actcctcatc tatggggcta gcgggcggcc ctcaggggtc 180
cctgaccgat tctctggctc caagtctggc acctcagcct ccctggccat cactgggctc 240
caggctgagg atgaggctga ttattactgt cagtcctatg atacgcggca ggtcgtccct 300
actcctgtgt tcggcggagg gaccaagctg accgtcctag gtcagcccaa ggctgccccc 360
tcggtcactc tgttcccgcc ctcctctgag gagcttcaag ccaacaaggc cacactggtg 420
tgtctcataa gtgacttcta cccgggagcc gtgacagtgg cttggaaagc agatagcagc 480
cccgtcaagg cgggagtgga gaccaccaca ccctccaaac aaagcaacaa caagtacgcg 540
gccagcagct atctgagcct gacgcctgag cagtggaagt cccacagaag ctacagctgc 600
caggtcacgc atgaagggag caccgtggag aagacagtgg cccctacaga atgttcataa 660

<210> 137
<211> 660
<212> DNA
<213> Artificial Sequence

<220>
<223> AC55 VLCL

<400> 137
cagtctgtgc tgacgcagcc gccctcagtg tctggggccc cagggcagag ggtcaccatc 60

tcctgcactg ggagcagctc caacatccat gatgttgggt atgtacactg gtaccagcag 120

cttccaggaa cagcccccaa actcctcatc tatggggcta gcgggcggcc ctcaggggtc 180

cctgaccgat tctctggctc caagtctggc acctcagcct ccctggccat cactgggctc 240

caggctgagg atgaggctga ttattactgt cagtcctatg atacgagcgc cgtcttccct 300

actcctgtgt tcggcggagg gaccaagctg accgtcctag gtcagcccaa ggctgccccc 360

tcggtcactc tgttcccgcc ctcctctgag gagcttcaag ccaacaaggc cacactggtg 420

tgtctcataa gtgacttcta cccgggagcc gtgacagtgg cttggaaagc agatagcagc 480

cccgtcaagg cgggagtgga gaccaccaca ccctccaaac aaagcaacaa caagtacgcg 540

gccagcagct atctgagcct gacgcctgag cagtggaagt cccacagaag ctacagctgc 600

caggtcacgc atgaagggag caccgtggag aagacagtgg cccctacaga atgttcataa 660

<210> 138
<211> 660
<212> DNA
<213> Artificial Sequence

<220>
<223> AC56 VLCL

<400> 138
cagtctgtgc tgacgcagcc gccctcagtg tctggggccc cagggcagag ggtcaccatc 60

tcctgcactg ggagcagctc caacatcatt aataacagtt atgtacactg gtaccagcag 120

cttccaggaa cagcccccaa actcctcatc tatgctccta gcaatcggcc ctcaggggtc 180

cctgaccgat tctctggctc caagtctggc acctcagcct ccctggccat cactgggctc 240

caggctgagg atgaggctga ttattactgt cagtcctatg ataccacggg ggtgatgcct 300

actcctgtgt tcggcggagg gaccaagctg accgtcctag gtcagcccaa ggctgccccc 360

tcggtcactc tgttcccgcc ctcctctgag gagcttcaag ccaacaaggc cacactggtg 420

tgtctcataa gtgacttcta cccgggagcc gtgacagtgg cttggaaagc agatagcagc 480

cccgtcaagg cgggagtgga gaccaccaca ccctccaaac aaagcaacaa caagtacgcg 540

gccagcagct atctgagcct gacgcctgag cagtggaagt cccacagaag ctacagctgc 600

caggtcacgc atgaagggag caccgtggag aagacagtgg cccctacaga atgttcataa 660

<210> 139
<211> 660
<212> DNA

<213> Artificial Sequence

<220>
<223> AC57 VLCL

<400> 139
cagtctgtgc tgacgcagcc gccctcagtg tctggggccc cagggcagag ggtcaccatc 60

tcctgcactg ggagcagctc caacatcatt aataacagtt atgtacactg gtaccagcag 120

cttccaggaa cagcccccaa actcctcatc tatgctccta gcaatcggcc ctcaggggtc 180

cctgaccgat tctctggctc caagtctggc acctcagcct ccctggccat cactgggctc 240

caggctgagg atgaggctga ttattactgt cagtcctatg atactcggtt cgtggtgccg 300

actcctgtgt tcggcggagg gaccaagctg accgtcctag gtcagcccaa ggctgccccc 360

tcggtcactc tgttcccgcc ctcctctgag gagcttcaag ccaacaaggc cacactggtg 420

tgtctcataa gtgacttcta cccgggagcc gtgacagtgg cttggaaagc agatagcagc 480

cccgtcaagg cgggagtgga gaccaccaca ccctccaaac aaagcaacaa caagtacgcg 540

gccagcagct atctgagcct gacgcctgag cagtggaagt cccacagaag ctacagctgc 600

caggtcacgc atgaagggag caccgtggag aagacagtgg cccctacaga atgttcataa 660

<210> 140
<211> 660
<212> DNA
<213> Artificial Sequence

<220>
<223> AC58 VLCL

<400> 140
cagtctgtgc tgacgcagcc gccctcagtg tctggggccc cagggcagag ggtcaccatc 60

tcctgcactg ggagcagctc caacatcggt gttgcgcatg atgtacactg gtaccagcag 120

cttccaggaa cagcccccaa actcctcatc tatgattata gcgagcggcc ctcaggggtc 180

cctgaccgat tctctggctc caagtctggc acctcagcct ccctggccat cactgggctc 240

caggctgagg atgaggctga ttattactgt cagtcctggg ataatgggcc caacggtctt 300

gtgtgggtgt tcggcggagg gaccaagctg accgtcctag gtcagcccaa ggctgccccc 360

tcggtcactc tgttcccgcc ctcctctgag gagcttcaag ccaacaaggc cacactggtg 420

tgtctcataa gtgacttcta cccgggagcc gtgacagtgg cttggaaagc agatagcagc 480

cccgtcaagg cgggagtgga gaccaccaca ccctccaaac aaagcaacaa caagtacgcg 540

gccagcagct atctgagcct gacgcctgag cagtggaagt cccacagaag ctacagctgc 600

caggtcacgc atgaagggag caccgtggag aagacagtgg cccctacaga atgttcataa 660

<210> 141
<211> 660
<212> DNA
<213> Artificial Sequence

<220>
<223> AC59 VLCL

<400> 141
cagtctgtgc tgacgcagcc gccctcagtg tctggggccc cagggcagag ggtcaccatc 60

tcctgcactg ggagcagctc caacatcggt gttgcgcatg atgtacactg gtaccagcag 120

cttccaggaa cagcccccaa actcctcatc tatgattata gcgagcggcc ctcaggggtc 180

cctgaccgat tctctggctc caagtctggc acctcagcct ccctggccat cactgggctc 240

caggctgagg atgaggctga ttattactgt cagtcctggg ataactccga gaacgggctt 300

atttatgtgt tcggcggagg gaccaagctg accgtcctag gtcagcccaa ggctgccccc 360

tcggtcactc tgttcccgcc ctcctctgag gagcttcaag ccaacaaggc cacactggtg 420

tgtctcataa gtgacttcta cccgggagcc gtgacagtgg cttggaaagc agatagcagc 480

cccgtcaagg cgggagtgga gaccaccaca ccctccaaac aaagcaacaa caagtacgcg 540

gccagcagct atctgagcct gacgcctgag cagtggaagt cccacagaag ctacagctgc 600

caggtcacgc atgaagggag caccgtggag aagacagtgg cccctacaga atgttcataa 660

<210> 142
<211> 11
<212> PRT
<213> Artificial Sequence

<220>
<223> AC61 CDRL1

<400> 142
Gln Ala Ser Gln Asn Ile Pro Arg Thr Ile Asn
1 5 10

<210> 143
<211> 7
<212> PRT
<213> Artificial Sequence

<220>
<223> AC61 CDRL2

<400> 143
Asp Ile Ser Asn Leu Glu Thr
1 5

<210> 144
<211> 11
<212> PRT
<213> Artificial Sequence

<220>
<223> AC61 CDRL3

<400> 144
Gln Gln Val Thr Leu Asp Pro Glu Glu Met Thr
1 5 10

<210> 145
<211> 11
<212> PRT
<213> Artificial Sequence

<220>
<223> AC62 CDRL1

<400> 145
Gln Ala Ser Gln Asn Ile Pro Arg Thr Ile Asn
1 5 10

<210> 146
<211> 7
<212> PRT
<213> Artificial Sequence

<220>
<223> AC62 CDRL2

<400> 146
Asp Ile Ser Asn Leu Glu Thr
1 5

<210> 147
<211> 11
<212> PRT
<213> Artificial Sequence

<220>
<223> AC62 CDRL3

<400> 147
Gln Gln Val Thr Leu Glu Pro Glu Leu Thr Thr
1 5 10

<210> 148
<211> 11
<212> PRT
<213> Artificial Sequence

<220>
<223> AC63 CDRL1

<400> 148
Gln Ala Ser Gln Asn Ile Pro Arg Thr Ile Asn
1 5 10

<210> 149
<211> 7

<212> PRT
<213> Artificial Sequence

<220>
<223> AC63 CDRL2

<400> 149
Asp Ile Ser Asn Leu Glu Thr
1               5

<210> 150
<211> 11
<212> PRT
<213> Artificial Sequence

<220>
<223> AC63 CDRL3

<400> 150
Gln Ser Val Thr Leu Trp Pro Glu Leu Thr Thr
1               5                   10

<210> 151
<211> 11
<212> PRT
<213> Artificial Sequence

<220>
<223> AC64 CDRL1

<400> 151
Gln Ala Ser Gln Asn Ile Pro Arg Thr Ile Asn
1               5                   10

<210> 152
<211> 7
<212> PRT
<213> Artificial Sequence

<220>
<223> AC64 CDRL2

<400> 152
Asp Ile Ser Asn Leu Glu Thr
1               5

<210> 153
<211> 11
<212> PRT
<213> Artificial Sequence

<220>
<223> AC64 CDRL3

<400> 153
Gln Gln Val Thr Leu Glu Pro Glu Glu Leu Thr
1               5                   10

<210> 154
<211> 11
<212> PRT
<213> Artificial Sequence

<220>
<223> AC65 CDRL1

<400> 154
Gln Ala Ser Gln Asn Ile Pro Arg Thr Ile Asn
1 5 10

<210> 155
<211> 7
<212> PRT
<213> Artificial Sequence

<220>
<223> AC65 CDRL2

<400> 155
Asp Ile Ser Asn Leu Glu Thr
1 5

<210> 156
<211> 11
<212> PRT
<213> Artificial Sequence

<220>
<223> AC65 CDRL3

<400> 156
Gly Ser Val Thr Leu Asn Pro Glu Leu Thr Thr
1 5 10

<210> 157
<211> 11
<212> PRT
<213> Artificial Sequence

<220>
<223> AC66 CDRL1

<400> 157
Gln Ala Ser Gln Asn Ile Pro Arg Thr Ile Asn
1 5 10

<210> 158
<211> 7
<212> PRT
<213> Artificial Sequence

<220>
<223> AC66 CDRL2

<400> 158
Asp Ile Ser Asn Leu Glu Thr

1 5

<210> 159
<211> 11
<212> PRT
<213> Artificial Sequence

<220>
<223> AC66 CDRL3

<400> 159
Gln Gln Val Thr Leu Asp Pro Glu Glu Asn Thr
1 5 10

<210> 160
<211> 651
<212> DNA
<213> Artificial Sequence

<220>
<223> AC60 VKCK

<400> 160
gacatccaga tgacccagtc tccatcctcc ctgtctgcat ctgtaggaga cagagtcacc 60

atcacttgcc aggcgagtca gaatattcct aggactataa attggtatca gcagaaacca 120

gggaaagccc ctaagctcct gatctacgat atttccaatt tggaaacagg ggtcccatca 180

aggttcagtg gaagtggatc tgggacagat tttactttca ccatcagcag cctgcagcct 240

gaagatattg caacatatta ctgtcagcag gttggtctta attgggacga cagggcgttc 300

ggccaaggga ccaaggtgga aatcaaacgt acggtggctg caccatctgt cttcatcttc 360

ccgccatctg atgagcagtt gaaatctgga actgcctctg ttgtgtgcct gctgaataac 420

ttctatccca gagaggccaa agtacagtgg aaggtggata acgccctcca atcgggtaac 480

tcccaggaga gtgtcacaga gcaggacagc aaggacagca cctacagcct cagcagcacc 540

ctgacgctga gcaaagcaga ctacgagaaa cacaaagtct acgcctgcga agtcacccat 600

cagggcctga gctcgcccgt cacaaagagc ttcaacaggg gagagtgtta a 651

<210> 161
<211> 651
<212> DNA
<213> Artificial Sequence

<220>
<223> AC61 VKCK

<400> 161
gacatccaga tgacccagtc tccatcctcc ctgtctgcat ctgtaggaga cagagtcacc 60

atcacttgcc aggcgagtca gaatattcct aggactataa attggtatca gcagaaacca 120

gggaaagccc ctaagctcct gatctacgat atttccaatt tggaaacagg ggtcccatca 180

aggttcagtg gaagtggatc tgggacagat tttactttca ccatcagcag cctgcagcct 240

gaagatattg caacatatta ctgtcagcag gtcacgcttg accctgagga gatgaccttc 300

ggccaaggga ccaaggtgga aatcaaacgt acggtggctg caccatctgt cttcatcttc 360

ccgccatctg atgagcagtt gaaatctgga actgcctctg ttgtgtgcct gctgaataac 420

ttctatccca gagaggccaa agtacagtgg aaggtggata acgccctcca atcgggtaac 480

tcccaggaga gtgtcacaga gcaggacagc aaggacagca cctacagcct cagcagcacc 540

ctgacgctga gcaaagcaga ctacgagaaa cacaaagtct acgcctgcga agtcacccat 600

cagggcctga gctcgcccgt cacaaagagc ttcaacaggg gagagtgtta a 651

<210> 162
<211> 651
<212> DNA
<213> Artificial Sequence

<220>
<223> AC62 VKCK

<400> 162
gacatccaga tgacccagtc tccatcctcc ctgtctgcat ctgtaggaga cagagtcacc 60

atcacttgcc aggcgagtca gaatattcct aggactataa attggtatca gcagaaacca 120

gggaaagccc ctaagctcct gatctacgat atttccaatt tggaaacagg ggtcccatca 180

aggttcagtg gaagtggatc tgggacagat tttactttca ccatcagcag cctgcagcct 240

gaagatattg caacatatta ctgtcagcag gtcacgcttg agcctgagct gaccaccttc 300

ggccaaggga ccaaggtgga aatcaaacgt acggtggctg caccatctgt cttcatcttc 360

ccgccatctg atgagcagtt gaaatctgga actgcctctg ttgtgtgcct gctgaataac 420

ttctatccca gagaggccaa agtacagtgg aaggtggata acgccctcca atcgggtaac 480

tcccaggaga gtgtcacaga gcaggacagc aaggacagca cctacagcct cagcagcacc 540

ctgacgctga gcaaagcaga ctacgagaaa cacaaagtct acgcctgcga agtcacccat 600

cagggcctga gctcgcccgt cacaaagagc ttcaacaggg gagagtgtta a 651

<210> 163
<211> 651
<212> DNA
<213> Artificial Sequence

<220>
<223> AC63 VKCK

<400> 163
gacatccaga tgacccagtc tccatcctcc ctgtctgcat ctgtaggaga cagagtcacc 60

atcacttgcc aggcgagtca gaatattcct aggactataa attggtatca gcagaaacca 120

gggaaagccc ctaagctcct gatctacgat atttccaatt tggaaacagg ggtcccatca 180

aggttcagtg gaagtggatc tgggacagat tttactttca ccatcagcag cctgcagcct 240

gaagatattg caacatatta ctgtcagtcg gtgacgttgt ggcctgagct tacgaccttc 300

ggccaaggga ccaaggtgga aatcaaacgt acggtggctg caccatctgt cttcatcttc 360

ccgccatctg atgagcagtt gaaatctgga actgcctctg ttgtgtgcct gctgaataac 420

ttctatccca gagaggccaa agtacagtgg aaggtggata acgccctcca atcgggtaac 480

tcccaggaga gtgtcacaga gcaggacagc aaggacagca cctacagcct cagcagcacc 540

ctgacgctga gcaaagcaga ctacgagaaa cacaaagtct acgcctgcga agtcacccat 600

cagggcctga gctcgcccgt cacaaagagc ttcaacaggg gagagtgtta a 651

<210> 164
<211> 651
<212> DNA
<213> Artificial Sequence

<220>
<223> AC64 VKCK

<400> 164
gacatccaga tgacccagtc tccatcctcc ctgtctgcat ctgtaggaga cagagtcacc 60

atcacttgcc aggcgagtca gaatattcct aggactataa attggtatca gcagaaacca 120

gggaaagccc ctaagctcct gatctacgat atttccaatt tggaaacagg ggtcccatca 180

aggttcagtg gaagtggatc tgggacagat tttactttca ccatcagcag cctgcagcct 240

gaagatattg caacatatta ctgtcagcag gtgacccttg agcctgagga gttgaccttc 300

ggccaaggga ccaaggtgga aatcaaacgt acggtggctg caccatctgt cttcatcttc 360

ccgccatctg atgagcagtt gaaatctgga actgcctctg ttgtgtgcct gctgaataac 420

ttctatccca gagaggccaa agtacagtgg aaggtggata acgccctcca atcgggtaac 480

tcccaggaga gtgtcacaga gcaggacagc aaggacagca cctacagcct cagcagcacc 540

ctgacgctga gcaaagcaga ctacgagaaa cacaaagtct acgcctgcga agtcacccat 600

cagggcctga gctcgcccgt cacaaagagc ttcaacaggg gagagtgtta a 651

<210> 165
<211> 651
<212> DNA
<213> Artificial Sequence

<220>
<223> AC65 VKCK

<400> 165
gacatccaga tgacccagtc tccatcctcc ctgtctgcat ctgtaggaga cagagtcacc 60

atcacttgcc aggcgagtca gaatattcct aggactataa attggtatca gcagaaacca 120

gggaaagccc ctaagctcct gatctacgat atttccaatt tggaaacagg ggtcccatca 180

aggttcagtg gaagtggatc tgggacagat tttactttca ccatcagcag cctgcagcct 240

gaagatattg caacatatta ctgtgggagc gtcaccctga atcctgagct tacgaccttc 300

ggccaaggga ccaaggtgga aatcaaacgt acggtggctg caccatctgt cttcatcttc 360

ccgccatctg atgagcagtt gaaatctgga actgcctctg ttgtgtgcct gctgaataac 420

ttctatccca gagaggccaa agtacagtgg aaggtggata acgccctcca atcgggtaac 480

tcccaggaga gtgtcacaga gcaggacagc aaggacagca cctacagcct cagcagcacc 540

ctgacgctga gcaaagcaga ctacgagaaa cacaaagtct acgcctgcga agtcacccat 600

cagggcctga gctcgcccgt cacaaagagc ttcaacaggg gagagtgtta a 651

<210> 166
<211> 651
<212> DNA
<213> Artificial Sequence

<220>
<223> AC66 VKCK

<400> 166
gacatccaga tgacccagtc tccatcctcc ctgtctgcat ctgtaggaga cagagtcacc 60

atcacttgcc aggcgagtca gaatattcct aggactataa attggtatca gcagaaacca 120

gggaaagccc ctaagctcct gatctacgat atttccaatt tggaaacagg ggtcccatca 180

aggttcagtg gaagtggatc tgggacagat tttactttca ccatcagcag cctgcagcct 240

gaagatattg caacatatta ctgtcagcag gtcacgcttg accctgagga gaacaccttc 300

ggccaaggga ccaaggtgga aatcaaacgt acggtggctg caccatctgt cttcatcttc 360

ccgccatctg atgagcagtt gaaatctgga actgcctctg ttgtgtgcct gctgaataac 420

ttctatccca gagaggccaa agtacagtgg aaggtggata acgccctcca atcgggtaac 480

tcccaggaga gtgtcacaga gcaggacagc aaggacagca cctacagcct cagcagcacc 540

ctgacgctga gcaaagcaga ctacgagaaa cacaaagtct acgcctgcga agtcacccat 600

cagggcctga gctcgcccgt cacaaagagc ttcaacaggg gagagtgtta a 651

<210> 167
<211> 216
<212> PRT
<213> Artificial Sequence

<220>
<223> AC60 VKCK

<400> 167
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asn Ile Pro Arg Thr
            20                  25                  30
Ile Asn Trp Tyr Gln Gln Lys Pro Gly Lys Ala Pro Lys Leu Leu Ile
        35                  40                  45
Tyr Asp Ile Ser Asn Leu Glu Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Val Gly Leu Asn Trp Asp
                85                  90                  95
Asp Arg Ala Phe Gly Gln Gly Thr Lys Val Glu Ile Lys Arg Thr Val
            100                 105                 110
Ala Ala Pro Ser Val Phe Ile Phe Pro Pro Ser Asp Glu Gln Leu Lys
        115                 120                 125
Ser Gly Thr Ala Ser Val Val Cys Leu Leu Asn Asn Phe Tyr Pro Arg
    130                 135                 140
Glu Ala Lys Val Gln Trp Lys Val Asp Asn Ala Leu Gln Ser Gly Asn
145                 150                 155                 160
Ser Gln Glu Ser Val Thr Glu Gln Asp Ser Lys Asp Ser Thr Tyr Ser
                165                 170                 175
Leu Ser Ser Thr Leu Thr Leu Ser Lys Ala Asp Tyr Glu Lys His Lys
            180                 185                 190
Val Tyr Ala Cys Glu Val Thr His Gln Gly Leu Ser Ser Pro Val Thr
        195                 200                 205
Lys Ser Phe Asn Arg Gly Glu Cys
    210                 215

<210> 168
<211> 216
<212> PRT
<213> Artificial Sequence

<220>
<223> AC61 VKCK

<400> 168
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asn Ile Pro Arg Thr
            20                  25                  30
Ile Asn Trp Tyr Gln Gln Lys Pro Gly Lys Ala Pro Lys Leu Leu Ile
        35                  40                  45
Tyr Asp Ile Ser Asn Leu Glu Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Val Thr Leu Asp Pro Glu
                85                  90                  95
Glu Met Thr Phe Gly Gln Gly Thr Lys Val Glu Ile Lys Arg Thr Val
            100                 105                 110
Ala Ala Pro Ser Val Phe Ile Phe Pro Pro Ser Asp Glu Gln Leu Lys
        115                 120                 125
Ser Gly Thr Ala Ser Val Val Cys Leu Leu Asn Asn Phe Tyr Pro Arg
    130                 135                 140
Glu Ala Lys Val Gln Trp Lys Val Asp Asn Ala Leu Gln Ser Gly Asn
145                 150                 155                 160

```
Ser Gln Glu Ser Val Thr Glu Gln Asp Ser Lys Asp Ser Thr Tyr Ser
                165                 170                 175
Leu Ser Ser Thr Leu Thr Leu Ser Lys Ala Asp Tyr Glu Lys His Lys
            180                 185                 190
Val Tyr Ala Cys Glu Val Thr His Gln Gly Leu Ser Ser Pro Val Thr
        195                 200                 205
Lys Ser Phe Asn Arg Gly Glu Cys
    210                 215
```

<210> 169
<211> 216
<212> PRT
<213> Artificial Sequence

<220>
<223> AC62 VKCK

<400> 169

```
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asn Ile Pro Arg Thr
            20                  25                  30
Ile Asn Trp Tyr Gln Gln Lys Pro Gly Lys Ala Pro Lys Leu Leu Ile
        35                  40                  45
Tyr Asp Ile Ser Asn Leu Glu Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Val Thr Leu Glu Pro Glu
                85                  90                  95
Leu Thr Thr Phe Gly Gln Gly Thr Lys Val Glu Ile Lys Arg Thr Val
            100                 105                 110
Ala Ala Pro Ser Val Phe Ile Phe Pro Pro Ser Asp Glu Gln Leu Lys
        115                 120                 125
Ser Gly Thr Ala Ser Val Val Cys Leu Leu Asn Asn Phe Tyr Pro Arg
    130                 135                 140
Glu Ala Lys Val Gln Trp Lys Val Asp Asn Ala Leu Gln Ser Gly Asn
145                 150                 155                 160
Ser Gln Glu Ser Val Thr Glu Gln Asp Ser Lys Asp Ser Thr Tyr Ser
                165                 170                 175
Leu Ser Ser Thr Leu Thr Leu Ser Lys Ala Asp Tyr Glu Lys His Lys
            180                 185                 190
Val Tyr Ala Cys Glu Val Thr His Gln Gly Leu Ser Ser Pro Val Thr
        195                 200                 205
Lys Ser Phe Asn Arg Gly Glu Cys
    210                 215
```

<210> 170
<211> 216
<212> PRT
<213> Artificial Sequence

<220>
<223> AC63 VKCK

<400> 170

```
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asn Ile Pro Arg Thr
            20                  25                  30
Ile Asn Trp Tyr Gln Gln Lys Pro Gly Lys Ala Pro Lys Leu Leu Ile
```

```
        35                  40                  45
Tyr Asp Ile Ser Asn Leu Glu Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Ser Val Thr Leu Trp Pro Glu
                85                  90                  95
Leu Thr Thr Phe Gly Gln Gly Thr Lys Val Glu Ile Lys Arg Thr Val
            100                 105                 110
Ala Ala Pro Ser Val Phe Ile Phe Pro Pro Ser Asp Glu Gln Leu Lys
        115                 120                 125
Ser Gly Thr Ala Ser Val Val Cys Leu Leu Asn Asn Phe Tyr Pro Arg
    130                 135                 140
Glu Ala Lys Val Gln Trp Lys Val Asp Asn Ala Leu Gln Ser Gly Asn
145                 150                 155                 160
Ser Gln Glu Ser Val Thr Glu Gln Asp Ser Lys Asp Ser Thr Tyr Ser
                165                 170                 175
Leu Ser Ser Thr Leu Thr Leu Ser Lys Ala Asp Tyr Glu Lys His Lys
            180                 185                 190
Val Tyr Ala Cys Glu Val Thr His Gln Gly Leu Ser Ser Pro Val Thr
        195                 200                 205
Lys Ser Phe Asn Arg Gly Glu Cys
    210                 215
```

<210> 171
<211> 216
<212> PRT
<213> Artificial Sequence

<220>
<223> AC64 VKCK

<400> 171

```
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asn Ile Pro Arg Thr
            20                  25                  30
Ile Asn Trp Tyr Gln Gln Lys Pro Gly Lys Ala Pro Lys Leu Leu Ile
        35                  40                  45
Tyr Asp Ile Ser Asn Leu Glu Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Val Thr Leu Glu Pro Glu
                85                  90                  95
Glu Leu Thr Phe Gly Gln Gly Thr Lys Val Glu Ile Lys Arg Thr Val
            100                 105                 110
Ala Ala Pro Ser Val Phe Ile Phe Pro Pro Ser Asp Glu Gln Leu Lys
        115                 120                 125
Ser Gly Thr Ala Ser Val Val Cys Leu Leu Asn Asn Phe Tyr Pro Arg
    130                 135                 140
Glu Ala Lys Val Gln Trp Lys Val Asp Asn Ala Leu Gln Ser Gly Asn
145                 150                 155                 160
Ser Gln Glu Ser Val Thr Glu Gln Asp Ser Lys Asp Ser Thr Tyr Ser
                165                 170                 175
Leu Ser Ser Thr Leu Thr Leu Ser Lys Ala Asp Tyr Glu Lys His Lys
            180                 185                 190
Val Tyr Ala Cys Glu Val Thr His Gln Gly Leu Ser Ser Pro Val Thr
        195                 200                 205
Lys Ser Phe Asn Arg Gly Glu Cys
    210                 215
```

<210> 172
<211> 216
<212> PRT
<213> Artificial Sequence

<220>
<223> AC65 VKCK

<400> 172
Asp Ile Gln Met Thr Gln Ser Pro Ser Ser Leu Ser Ala Ser Val Gly
1 5 10 15
Asp Arg Val Thr Ile Thr Cys Gln Ala Ser Gln Asn Ile Pro Arg Thr
20 25 30
Ile Asn Trp Tyr Gln Gln Lys Pro Gly Lys Ala Pro Lys Leu Leu Ile
35 40 45
Tyr Asp Ile Ser Asn Leu Glu Thr Gly Val Pro Ser Arg Phe Ser Gly
50 55 60
Ser Gly Ser Gly Thr Asp Phe Thr Phe Thr Ile Ser Ser Leu Gln Pro
65 70 75 80
Glu Asp Ile Ala Thr Tyr Tyr Cys Gly Ser Val Thr Leu Asn Pro Glu
85 90 95
Leu Thr Thr Phe Gly Gln Gly Thr Lys Val Glu Ile Lys Arg Thr Val
100 105 110
Ala Ala Pro Ser Val Phe Ile Phe Pro Pro Ser Asp Glu Gln Leu Lys
115 120 125
Ser Gly Thr Ala Ser Val Val Cys Leu Leu Asn Asn Phe Tyr Pro Arg
130 135 140
Glu Ala Lys Val Gln Trp Lys Val Asp Asn Ala Leu Gln Ser Gly Asn
145 150 155 160
Ser Gln Glu Ser Val Thr Glu Gln Asp Ser Lys Asp Ser Thr Tyr Ser
165 170 175
Leu Ser Ser Thr Leu Thr Leu Ser Lys Ala Asp Tyr Glu Lys His Lys
180 185 190
Val Tyr Ala Cys Glu Val Thr His Gln Gly Leu Ser Ser Pro Val Thr
195 200 205
Lys Ser Phe Asn Arg Gly Glu Cys
210 215

<210> 173
<211> 216
<212> PRT
<213> Artificial Sequence

<220>
<223> AC66 VKCK

<400> 173
Asp Ile Gln Met Thr Gln Ser Pro Ser Ser Leu Ser Ala Ser Val Gly
1 5 10 15
Asp Arg Val Thr Ile Thr Cys Gln Ala Ser Gln Asn Ile Pro Arg Thr
20 25 30
Ile Asn Trp Tyr Gln Gln Lys Pro Gly Lys Ala Pro Lys Leu Leu Ile
35 40 45
Tyr Asp Ile Ser Asn Leu Glu Thr Gly Val Pro Ser Arg Phe Ser Gly
50 55 60
Ser Gly Ser Gly Thr Asp Phe Thr Phe Thr Ile Ser Ser Leu Gln Pro
65 70 75 80
Glu Asp Ile Ala Thr Tyr Tyr Cys Gln Gln Val Thr Leu Asp Pro Glu
85 90 95
Glu Asn Thr Phe Gly Gln Gly Thr Lys Val Glu Ile Lys Arg Thr Val
100 105 110

```
Ala Ala Pro Ser Val Phe Ile Phe Pro Pro Ser Asp Glu Gln Leu Lys
        115                 120                 125
Ser Gly Thr Ala Ser Val Val Cys Leu Leu Asn Asn Phe Tyr Pro Arg
    130                 135                 140
Glu Ala Lys Val Gln Trp Lys Val Asp Asn Ala Leu Gln Ser Gly Asn
145                 150                 155                 160
Ser Gln Glu Ser Val Thr Glu Gln Asp Ser Lys Asp Ser Thr Tyr Ser
                165                 170                 175
Leu Ser Ser Thr Leu Thr Leu Ser Lys Ala Asp Tyr Glu Lys His Lys
            180                 185                 190
Val Tyr Ala Cys Glu Val Thr His Gln Gly Leu Ser Ser Pro Val Thr
        195                 200                 205
Lys Ser Phe Asn Arg Gly Glu Cys
    210                 215
```

<210> 174
<211> 215
<212> PRT
<213> Artificial Sequence

<220>
<223> AC60 VK-H-CL1

<400> 174

```
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asn Ile Pro Arg Thr
            20                  25                  30
Ile Asn Trp Tyr Gln Gln Lys Pro Gly Lys Ala Pro Lys Leu Leu Ile
        35                  40                  45
Tyr Asp Ile Ser Asn Leu Glu Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Val Gly Leu Asn Trp Asp
                85                  90                  95
Asp Arg Ala Phe Gly Gly Gly Thr Lys Leu Thr Val Leu Gly Gln Pro
            100                 105                 110
Lys Ala Ala Pro Ser Val Thr Leu Phe Pro Pro Ser Ser Glu Glu Leu
        115                 120                 125
Gln Ala Asn Lys Ala Thr Leu Val Cys Leu Ile Ser Asp Phe Tyr Pro
    130                 135                 140
Gly Ala Val Thr Val Ala Trp Lys Ala Asp Ser Ser Pro Val Lys Ala
145                 150                 155                 160
Gly Val Glu Thr Thr Thr Pro Ser Lys Gln Ser Asn Asn Lys Tyr Ala
                165                 170                 175
Ala Ser Ser Tyr Leu Ser Leu Thr Pro Glu Gln Trp Lys Ser His Arg
            180                 185                 190
Ser Tyr Ser Cys Gln Val Thr His Glu Gly Ser Thr Val Glu Lys Thr
        195                 200                 205
Val Ala Pro Thr Glu Cys Ser
    210                 215
```

<210> 175
<211> 215
<212> PRT
<213> Artificial Sequence

<220>
<223> AC61 VK-H-CL1

<400> 175
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asn Ile Pro Arg Thr
            20                  25                  30
Ile Asn Trp Tyr Gln Gln Lys Pro Gly Lys Ala Pro Lys Leu Leu Ile
        35                  40                  45
Tyr Asp Ile Ser Asn Leu Glu Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Val Thr Leu Asp Pro Glu
                85                  90                  95
Glu Met Thr Phe Gly Gly Gly Thr Lys Leu Thr Val Leu Gly Gln Pro
            100                 105                 110
Lys Ala Ala Pro Ser Val Thr Leu Phe Pro Pro Ser Ser Glu Glu Leu
        115                 120                 125
Gln Ala Asn Lys Ala Thr Leu Val Cys Leu Ile Ser Asp Phe Tyr Pro
    130                 135                 140
Gly Ala Val Thr Val Ala Trp Lys Ala Asp Ser Ser Pro Val Lys Ala
145                 150                 155                 160
Gly Val Glu Thr Thr Thr Pro Ser Lys Gln Ser Asn Asn Lys Tyr Ala
                165                 170                 175
Ala Ser Ser Tyr Leu Ser Leu Thr Pro Glu Gln Trp Lys Ser His Arg
            180                 185                 190
Ser Tyr Ser Cys Gln Val Thr His Glu Gly Ser Thr Val Glu Lys Thr
        195                 200                 205
Val Ala Pro Thr Glu Cys Ser
    210                 215

<210> 176
<211> 215
<212> PRT
<213> Artificial Sequence

<220>
<223> AC62 VK-H-CL1

<400> 176
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asn Ile Pro Arg Thr
            20                  25                  30
Ile Asn Trp Tyr Gln Gln Lys Pro Gly Lys Ala Pro Lys Leu Leu Ile
        35                  40                  45
Tyr Asp Ile Ser Asn Leu Glu Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Val Thr Leu Glu Pro Glu
                85                  90                  95
Leu Thr Thr Phe Gly Gly Gly Thr Lys Leu Thr Val Leu Gly Gln Pro
            100                 105                 110
Lys Ala Ala Pro Ser Val Thr Leu Phe Pro Pro Ser Ser Glu Glu Leu
        115                 120                 125
Gln Ala Asn Lys Ala Thr Leu Val Cys Leu Ile Ser Asp Phe Tyr Pro
    130                 135                 140
Gly Ala Val Thr Val Ala Trp Lys Ala Asp Ser Ser Pro Val Lys Ala
145                 150                 155                 160
Gly Val Glu Thr Thr Thr Pro Ser Lys Gln Ser Asn Asn Lys Tyr Ala
                165                 170                 175
Ala Ser Ser Tyr Leu Ser Leu Thr Pro Glu Gln Trp Lys Ser His Arg

```
                180                 185                 190
Ser Tyr Ser Cys Gln Val Thr His Glu Gly Ser Thr Val Glu Lys Thr
            195                 200                 205
Val Ala Pro Thr Glu Cys Ser
    210                 215
```

<210> 177
<211> 215
<212> PRT
<213> Artificial Sequence

<220>
<223> AC63 VK-H-CL1

<400> 177

```
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asn Ile Pro Arg Thr
            20                  25                  30
Ile Asn Trp Tyr Gln Gln Lys Pro Gly Lys Ala Pro Lys Leu Leu Ile
        35                  40                  45
Tyr Asp Ile Ser Asn Leu Glu Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Ser Val Thr Leu Trp Pro Glu
                85                  90                  95
Leu Thr Thr Phe Gly Gly Gly Thr Lys Leu Thr Val Leu Gly Gln Pro
            100                 105                 110
Lys Ala Ala Pro Ser Val Thr Leu Phe Pro Pro Ser Ser Glu Glu Leu
        115                 120                 125
Gln Ala Asn Lys Ala Thr Leu Val Cys Leu Ile Ser Asp Phe Tyr Pro
    130                 135                 140
Gly Ala Val Thr Val Ala Trp Lys Ala Asp Ser Ser Pro Val Lys Ala
145                 150                 155                 160
Gly Val Glu Thr Thr Thr Pro Ser Lys Gln Ser Asn Asn Lys Tyr Ala
                165                 170                 175
Ala Ser Ser Tyr Leu Ser Leu Thr Pro Glu Gln Trp Lys Ser His Arg
            180                 185                 190
Ser Tyr Ser Cys Gln Val Thr His Glu Gly Ser Thr Val Glu Lys Thr
        195                 200                 205
Val Ala Pro Thr Glu Cys Ser
    210                 215
```

<210> 178
<211> 215
<212> PRT
<213> Artificial Sequence

<220>
<223> AC64 VK-H-CL1

<400> 178

```
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asn Ile Pro Arg Thr
            20                  25                  30
Ile Asn Trp Tyr Gln Gln Lys Pro Gly Lys Ala Pro Lys Leu Leu Ile
        35                  40                  45
Tyr Asp Ile Ser Asn Leu Glu Thr Gly Val Pro Ser Arg Phe Ser Gly
    50                  55                  60
```

```
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Val Thr Leu Glu Pro Glu
                85                  90                  95
Glu Leu Thr Phe Gly Gly Gly Thr Lys Leu Thr Val Leu Gly Gln Pro
            100                 105                 110
Lys Ala Ala Pro Ser Val Thr Leu Phe Pro Pro Ser Ser Glu Glu Leu
        115                 120                 125
Gln Ala Asn Lys Ala Thr Leu Val Cys Leu Ile Ser Asp Phe Tyr Pro
    130                 135                 140
Gly Ala Val Thr Val Ala Trp Lys Ala Asp Ser Ser Pro Val Lys Ala
145                 150                 155                 160
Gly Val Glu Thr Thr Thr Pro Ser Lys Gln Ser Asn Asn Lys Tyr Ala
                165                 170                 175
Ala Ser Ser Tyr Leu Ser Leu Thr Pro Glu Gln Trp Lys Ser His Arg
            180                 185                 190
Ser Tyr Ser Cys Gln Val Thr His Glu Gly Ser Thr Val Glu Lys Thr
        195                 200                 205
Val Ala Pro Thr Glu Cys Ser
    210                 215
```

<210> 179
<211> 215
<212> PRT
<213> Artificial Sequence

<220>
<223> AK65 VK-H-CL1

<400> 179

```
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asn Ile Pro Arg Thr
            20                  25                  30
Ile Asn Trp Tyr Gln Gln Lys Pro Gly Lys Ala Pro Lys Leu Leu Ile
        35                  40                  45
Tyr Asp Ile Ser Asn Leu Glu Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gly Ser Val Thr Leu Asn Pro Glu
                85                  90                  95
Leu Thr Thr Phe Gly Gly Gly Thr Lys Leu Thr Val Leu Gly Gln Pro
            100                 105                 110
Lys Ala Ala Pro Ser Val Thr Leu Phe Pro Pro Ser Ser Glu Glu Leu
        115                 120                 125
Gln Ala Asn Lys Ala Thr Leu Val Cys Leu Ile Ser Asp Phe Tyr Pro
    130                 135                 140
Gly Ala Val Thr Val Ala Trp Lys Ala Asp Ser Ser Pro Val Lys Ala
145                 150                 155                 160
Gly Val Glu Thr Thr Thr Pro Ser Lys Gln Ser Asn Asn Lys Tyr Ala
                165                 170                 175
Ala Ser Ser Tyr Leu Ser Leu Thr Pro Glu Gln Trp Lys Ser His Arg
            180                 185                 190
Ser Tyr Ser Cys Gln Val Thr His Glu Gly Ser Thr Val Glu Lys Thr
        195                 200                 205
Val Ala Pro Thr Glu Cys Ser
    210                 215
```

<210> 180
<211> 215
<212> PRT

<213> Artificial Sequence

<220>
<223> AK 66 VK-H-CL1

<400> 180
Asp Ile Gln Met Thr Gln Ser Pro Ser Ser Leu Ser Ala Ser Val Gly
1 5 10 15
Asp Arg Val Thr Ile Thr Cys Gln Ala Ser Gln Asn Ile Pro Arg Thr
20 25 30
Ile Asn Trp Tyr Gln Gln Lys Pro Gly Lys Ala Pro Lys Leu Leu Ile
35 40 45
Tyr Asp Ile Ser Asn Leu Glu Thr Gly Val Pro Ser Arg Phe Ser Gly
50 55 60
Ser Gly Ser Gly Thr Asp Phe Thr Phe Thr Ile Ser Ser Leu Gln Pro
65 70 75 80
Glu Asp Ile Ala Thr Tyr Tyr Cys Gln Gln Val Thr Leu Asp Pro Glu
85 90 95
Glu Asn Thr Phe Gly Gly Gly Thr Lys Leu Thr Val Leu Gly Gln Pro
100 105 110
Lys Ala Ala Pro Ser Val Thr Leu Phe Pro Pro Ser Ser Glu Glu Leu
115 120 125
Gln Ala Asn Lys Ala Thr Leu Val Cys Leu Ile Ser Asp Phe Tyr Pro
130 135 140
Gly Ala Val Thr Val Ala Trp Lys Ala Asp Ser Ser Pro Val Lys Ala
145 150 155 160
Gly Val Glu Thr Thr Thr Pro Ser Lys Gln Ser Asn Asn Lys Tyr Ala
165 170 175
Ala Ser Ser Tyr Leu Ser Leu Thr Pro Glu Gln Trp Lys Ser His Arg
180 185 190
Ser Tyr Ser Cys Gln Val Thr His Glu Gly Ser Thr Val Glu Lys Thr
195 200 205
Val Ala Pro Thr Glu Cys Ser
210 215

<210> 181
<211> 214
<212> PRT
<213> Artificial Sequence

<220>
<223> CD47 VK-H-CL1 (VKCL)

<400> 181
Asp Ile Gln Met Thr Gln Ser Pro Ser Ser Leu Ser Ala Ser Val Gly
1 5 10 15
Asp Arg Val Thr Ile Thr Cys Arg Ala Ser Gln Ser Ile Ser Ser Tyr
20 25 30
Leu Asn Trp Tyr Gln Gln Lys Pro Gly Lys Ala Pro Lys Leu Leu Ile
35 40 45
Tyr Ala Ala Ser Ser Leu Gln Ser Gly Val Pro Ser Arg Phe Ser Gly
50 55 60
Ser Gly Ser Gly Thr Asp Phe Thr Leu Thr Ile Ser Ser Leu Gln Pro
65 70 75 80
Glu Asp Phe Ala Thr Tyr Tyr Cys Gln Gln Met His Pro Arg Ala Pro
85 90 95
Lys Thr Phe Gly Gly Gly Thr Lys Leu Thr Val Leu Gly Gln Pro Lys
100 105 110
Ala Ala Pro Ser Val Thr Leu Phe Pro Pro Ser Ser Glu Glu Leu Gln
115 120 125
Ala Asn Lys Ala Thr Leu Val Cys Leu Ile Ser Asp Phe Tyr Pro Gly

130 135 140
Ala Val Thr Val Ala Trp Lys Ala Asp Ser Ser Pro Val Lys Ala Gly
145 150 155 160
Val Glu Thr Thr Thr Pro Ser Lys Gln Ser Asn Asn Lys Tyr Ala Ala
165 170 175
Ser Ser Tyr Leu Ser Leu Thr Pro Glu Gln Trp Lys Ser His Arg Ser
180 185 190
Tyr Ser Cys Gln Val Thr His Glu Gly Ser Thr Val Glu Lys Thr Val
195 200 205
Ala Pro Thr Glu Cys Ser
210

<210> 182
<211> 14
<212> PRT
<213> Artificial Sequence

<220>
<223> AC21 CDRL1

<400> 182
Thr Gly Ser Ser Ser Asn Ile Gly Val Ala His Asp Val His
1 5 10

<210> 183
<211> 7
<212> PRT
<213> Artificial Sequence

<220>
<223> AC21 CDRL2

<400> 183
Asp Tyr Ser Glu Arg Pro Ser
1 5

<210> 184
<211> 13
<212> PRT
<213> Artificial Sequence

<220>
<223> AC21 CDRL3

<400> 184
Gln Ser Trp Asp Asn Ser Asp Ser Gly Leu Ile Tyr Val
1 5 10

<210> 185
<211> 219
<212> PRT
<213> Artificial Sequence

<220>
<223> AC21 VLCL

<400> 185
Gln Ser Val Leu Thr Gln Pro Pro Ser Val Ser Gly Ala Pro Gly Gln
1 5 10 15

```
Arg Val Thr Ile Ser Cys Thr Gly Ser Ser Ser Asn Ile Gly Val Ala
            20                  25                  30
His Asp Val His Trp Tyr Gln Gln Leu Pro Gly Thr Ala Pro Lys Leu
        35                  40                  45
Leu Ile Tyr Asp Tyr Ser Glu Arg Pro Ser Gly Val Pro Asp Arg Phe
    50                  55                  60
Ser Gly Ser Lys Ser Gly Thr Ser Ala Ser Leu Ala Ile Thr Gly Leu
65                  70                  75                  80
Gln Ala Glu Asp Glu Ala Asp Tyr Tyr Cys Gln Ser Trp Asp Asn Ser
                85                  90                  95
Asp Ser Gly Leu Ile Tyr Val Phe Gly Gly Gly Thr Lys Leu Thr Val
            100                 105                 110
Leu Gly Gln Pro Lys Ala Ala Pro Ser Val Thr Leu Phe Pro Pro Ser
        115                 120                 125
Ser Glu Glu Leu Gln Ala Asn Lys Ala Thr Leu Val Cys Leu Ile Ser
    130                 135                 140
Asp Phe Tyr Pro Gly Ala Val Thr Val Ala Trp Lys Ala Asp Ser Ser
145                 150                 155                 160
Pro Val Lys Ala Gly Val Glu Thr Thr Thr Pro Ser Lys Gln Ser Asn
                165                 170                 175
Asn Lys Tyr Ala Ala Ser Ser Tyr Leu Ser Leu Thr Pro Glu Gln Trp
            180                 185                 190
Lys Ser His Arg Ser Tyr Ser Cys Gln Val Thr His Glu Gly Ser Thr
        195                 200                 205
Val Glu Lys Thr Val Ala Pro Thr Glu Cys Ser
    210                 215
```

<210> 186
<211> 660
<212> DNA
<213> Artificial Sequence

<220>
<223> AC21 VLCL

<400> 186
```
cagtctgtgc tgacgcagcc gccctcagtg tctggggccc cagggcagag ggtcaccatc      60

tcctgcactg ggagcagctc caacatcggt gttgcgcatg atgtacactg gtaccagcag     120

cttccaggaa cagcccccaa actcctcatc tatgattata gcgagcggcc ctcaggggtc     180

cctgaccgat tctctggctc caagtctggc acctcagcct ccctggccat cactgggctc     240

caggctgagg atgaggctga ttattactgc cagtcctggg ataattctga ttctggtctt     300

atttatgtgt tcggcggagg gaccaagctg accgtcctag gtcagcccaa ggctgccccc     360

tcggtcactc tgttcccgcc ctcctctgag gagcttcaag ccaacaaggc cacactggtg     420

tgtctcataa gtgacttcta cccgggagcc gtgacagtgg cttggaaagc agatagcagc     480

cccgtcaagg cgggagtgga gaccaccaca ccctccaaac aaagcaacaa caagtacgcg     540

gccagcagct atctgagcct gacgcctgag cagtggaagt cccacagaag ctacagctgc     600

caggtcacgc atgaagggag caccgtggag aagacagtgg cccctacaga atgttcataa     660
```

<210> 187
<211> 24
<212> DNA

<213> Artificial Sequence

<220>
<223> Primer

<400> 187
gtctgaagca ttatgtgttg aagc 24

<210> 188
<211> 23
<212> DNA
<213> Artificial Sequence

<220>
<223> Primer

<400> 188
gtgagtacat tcattgtact gtg 23

<210> 189
<211> 24
<212> DNA
<213> Artificial Sequence

<220>
<223> Primer

<400> 189
ttgtgtgact cttaactctc agag 24

<210> 190
<211> 25
<212> DNA
<213> Artificial Sequence

<220>
<223> Primer

<400> 190
gaggccactt gtgtagcgcc aagtg 25

<210> 191
<211> 21
<212> DNA
<213> Artificial Sequence

<220>
<223> Primer

<400> 191
gtgagtccat ggctgtcact g 21

<210> 192

```
<211> 20
<212> DNA
<213> Artificial Sequence

<220>
<223> Primer

<400> 192
cctgacttgg ctattctcag                                                   20
```